# EUROPEAN PATENT APPLICATION

(11) **EP 2 394 655 A2**
(43) Date of publication of application: **14.12.2011**
(21) Application number: 10183517.1
(22) Date of filing: 04.04.2002
(51) Int. Cl.: A61K 38/02, A61K 38/16, A61K 39/00, A61K 45/00, A61K 47/00, A61K 39/395, C07K 1/00, C07K 16/00, C12N 15/00, C12N 15/70, C07H 21/04, C07K 7/00, A61K 38/08, C12N 9/02, C07K 14/47

(54) **Epitope sequences**

(30) Priority: 06.04.2001 US 282211 P; 07.11.2001 US 337017 P; 07.03.2002 US 363210 P
(62) Divisional of application: 06119117.7
(71) Applicant: Mannkind Corporation, Chatsworth, California 91311 (US)
(72) Inventor: Simard, John, J.L., Vancouver British Columbia V6E 2E9 (CA); Diamond, David, C., West Hills, CA 91304 (US); Liu, Liping, Northridge, CA 91324 (US); Xie, Zhidong, Manassas, VA 20110 (US)
(74) Representative: Lasar, Andrea Gisela

(57) **Abstract**

Disclosed herein are polypeptides, including epitopes, clusters, and antigens. Also disclosed are compositions that include said polypeptides and methods for their use.

SEQUENCE LISTING

<110> CTL IMMUNO THERAPIES CORP.
      SIMARD, John, J.L.
      DIAMOND, David, C.
      LIU, Liping
      XIE, Zhidong

<120> EPITOPE SEQUENCES

<130> CTLIMM.027QPC

<150> US 60/282,211
<151> 2001-04-06

<150> US 60/337,017
<151> 2001-11-07

<150> US 60/363,210
<151> 2002-07-03

<160> 602

<170> FastSEQ for Windows Version 4.0

<210> 1
<211> 10
<212> PRT
<213> Homo sapiens

<400> 1
Phe Leu Pro Trp His Arg Leu Phe Leu Leu
 1               5                   10

<210> 2
<211> 529
<212> PRT
<213> Homo sapiens

<400> 2

<210> 3

<211> 188
<212> PRT
<213> Homo sapiens

<400> 3
Met Asn Gly Asp Asp Ala Phe Ala Arg Arg Pro Thr Val Gly Ala Gln
1               5                   10                  15
Ile Pro Glu Lys Ile Gln Lys Ala Phe Asp Asp Ile Ala Lys Tyr Phe
            20                  25                  30
Ser Lys Glu Glu Trp Glu Lys Met Lys Ala Ser Glu Lys Ile Phe Tyr
        35                  40                  45
Val Tyr Met Lys Arg Lys Tyr Glu Ala Met Thr Lys Leu Gly Phe Lys
    50                  55                  60
Ala Thr Leu Pro Pro Phe Met Cys Asn Lys Arg Ala Glu Asp Phe Gln
65                  70                  75                  80
Gly Asn Asp Leu Asp Asn Asp Pro Asn Arg Gly Asn Gln Val Glu Arg
                85                  90                  95
Pro Gln Met Thr Phe Gly Arg Leu Gln Gly Ile Ser Pro Lys Ile Met
            100                 105                 110
Pro Lys Lys Pro Ala Glu Glu Gly Asn Asp Ser Glu Glu Val Pro Glu
        115                 120                 125
Ala Ser Gly Pro Gln Asn Asp Gly Lys Glu Leu Cys Pro Pro Gly Lys
    130                 135                 140
Pro Thr Thr Ser Glu Lys Ile His Glu Arg Ser Gly Pro Lys Arg Gly
145                 150                 155                 160
Glu His Ala Trp Thr His Arg Leu Arg Glu Arg Lys Gln Leu Val Ile
                165                 170                 175
Tyr Glu Glu Ile Ser Asp Pro Glu Glu Asp Asp Glu
            180                 185


<210> 4
<211> 750
<212> PRT
<213> Homo sapiens

<400> 4
Met Trp Asn Leu Leu His Glu Thr Asp Ser Ala Val Ala Thr Ala Arg
1               5                   10                  15
Arg Pro Arg Trp Leu Cys Ala Gly Ala Leu Val Leu Ala Gly Gly Phe
            20                  25                  30
Phe Leu Leu Gly Phe Leu Phe Gly Trp Phe Ile Lys Ser Ser Asn Glu
        35                  40                  45
Ala Thr Asn Ile Thr Pro Lys His Asn Met Lys Ala Phe Leu Asp Glu
    50                  55                  60
Leu Lys Ala Glu Asn Ile Lys Lys Phe Leu Tyr Asn Phe Thr Gln Ile
65                  70                  75                  80
Pro His Leu Ala Gly Thr Glu Gln Asn Phe Gln Leu Ala Lys Gln Ile
                85                  90                  95
Gln Ser Gln Trp Lys Glu Phe Gly Leu Asp Ser Val Glu Leu Ala His
            100                 105                 110
Tyr Asp Val Leu Leu Ser Tyr Pro Asn Lys Thr His Pro Asn Tyr Ile
        115                 120                 125
Ser Ile Ile Asn Glu Asp Gly Asn Glu Ile Phe Asn Thr Ser Leu Phe
    130                 135                 140
Glu Pro Pro Pro Pro Gly Tyr Glu Asn Val Ser Asp Ile Val Pro Pro
145                 150                 155                 160

Phe Ser Ala Phe Ser Pro Gln Gly Met Pro Glu Gly Asp Leu Val Tyr
                165                 170                 175
Val Asn Tyr Ala Arg Thr Glu Asp Phe Phe Lys Leu Glu Arg Asp Met
            180                 185                 190
Lys Ile Asn Cys Ser Gly Lys Ile Val Ile Ala Arg Tyr Gly Lys Val
        195                 200                 205
Phe Arg Gly Asn Lys Val Lys Asn Ala Gln Leu Ala Gly Ala Lys Gly
    210                 215                 220
Val Ile Leu Tyr Ser Asp Pro Ala Asp Tyr Phe Ala Pro Gly Val Lys
225                 230                 235                 240
Ser Tyr Pro Asp Gly Trp Asn Leu Pro Gly Gly Gly Val Gln Arg Gly
                245                 250                 255
Asn Ile Leu Asn Leu Asn Gly Ala Gly Asp Pro Leu Thr Pro Gly Tyr
            260                 265                 270
Pro Ala Asn Glu Tyr Ala Tyr Arg Arg Gly Ile Ala Glu Ala Val Gly
        275                 280                 285
Leu Pro Ser Ile Pro Val His Pro Ile Gly Tyr Tyr Asp Ala Gln Lys
    290                 295                 300
Leu Leu Glu Lys Met Gly Gly Ser Ala Pro Pro Asp Ser Ser Trp Arg
305                 310                 315                 320
Gly Ser Leu Lys Val Pro Tyr Asn Val Gly Pro Gly Phe Thr Gly Asn
                325                 330                 335
Phe Ser Thr Gln Lys Val Lys Met His Ile His Ser Thr Asn Glu Val
            340                 345                 350
Thr Arg Ile Tyr Asn Val Ile Gly Thr Leu Arg Gly Ala Val Glu Pro
        355                 360                 365
Asp Arg Tyr Val Ile Leu Gly Gly His Arg Asp Ser Trp Val Phe Gly
    370                 375                 380
Gly Ile Asp Pro Gln Ser Gly Ala Ala Val Val His Glu Ile Val Arg
385                 390                 395                 400
Ser Phe Gly Thr Leu Lys Lys Glu Gly Trp Arg Pro Arg Arg Thr Ile
                405                 410                 415
Leu Phe Ala Ser Trp Asp Ala Glu Glu Phe Gly Leu Leu Gly Ser Thr
            420                 425                 430
Glu Trp Ala Glu Glu Asn Ser Arg Leu Leu Gln Glu Arg Gly Val Ala
        435                 440                 445
Tyr Ile Asn Ala Asp Ser Ser Ile Glu Gly Asn Tyr Thr Leu Arg Val
    450                 455                 460
Asp Cys Thr Pro Leu Met Tyr Ser Leu Val His Asn Leu Thr Lys Glu
465                 470                 475                 480
Leu Lys Ser Pro Asp Glu Gly Phe Glu Gly Lys Ser Leu Tyr Glu Ser
                485                 490                 495
Trp Thr Lys Lys Ser Pro Ser Pro Glu Phe Ser Gly Met Pro Arg Ile
            500                 505                 510
Ser Lys Leu Gly Ser Gly Asn Asp Phe Glu Val Phe Phe Gln Arg Leu
        515                 520                 525
Gly Ile Ala Ser Gly Arg Ala Arg Tyr Thr Lys Asn Trp Glu Thr Asn
    530                 535                 540
Lys Phe Ser Gly Tyr Pro Leu Tyr His Ser Val Tyr Glu Thr Tyr Glu
545                 550                 555                 560
Leu Val Glu Lys Phe Tyr Asp Pro Met Phe Lys Tyr His Leu Thr Val
                565                 570                 575
Ala Gln Val Arg Gly Gly Met Val Phe Glu Leu Ala Asn Ser Ile Val
            580                 585                 590
Leu Pro Phe Asp Cys Arg Asp Tyr Ala Val Val Leu Arg Lys Tyr Ala
        595                 600                 605
Asp Lys Ile Tyr Ser Ile Ser Met Lys His Pro Gln Glu Met Lys Thr

610                 615                 620
Tyr Ser Val Ser Phe Asp Ser Leu Phe Ser Ala Val Lys Asn Phe Thr
625                 630                 635                 640
Glu Ile Ala Ser Lys Phe Ser Glu Arg Leu Gln Asp Phe Asp Lys Ser
                645                 650                 655
Asn Pro Ile Val Leu Arg Met Met Asn Asp Gln Leu Met Phe Leu Glu
            660                 665                 670
Arg Ala Phe Ile Asp Pro Leu Gly Leu Pro Asp Arg Pro Phe Tyr Arg
        675                 680                 685
His Val Ile Tyr Ala Pro Ser Ser His Asn Lys Tyr Ala Gly Glu Ser
    690                 695                 700
Phe Pro Gly Ile Tyr Asp Ala Leu Phe Asp Ile Glu Ser Lys Val Asp
705                 710                 715                 720
Pro Ser Lys Ala Trp Gly Glu Val Lys Arg Gln Ile Tyr Val Ala Ala
                725                 730                 735
Phe Thr Val Gln Ala Ala Ala Glu Thr Leu Ser Glu Val Ala
            740                 745                 750

<210> 5
<211> 1964
<212> DNA
<213> Homo sapiens

<400> 5
atcactgtag tagtagctgg aaagagaaat ctgtgactcc aattagccag ttcctgcaga 60
ccttgtgagg actagaggaa gaatgctcct ggctgttttg tactgcctgc tgtggagttt 120
ccagacctcc gctggccatt tccctagagc ctgtgtctcc tctaagaacc tgatggagaa 180
ggaatgctgt ccaccgtgga gcggggacag gagtccctgt ggccagcttt caggcagagg 240
ttcctgtcag aatatccttc tgtccaatgc accacttggg cctcaatttc ccttcacagg 300
ggtggatgac cgggagtcgt ggccttccgt cttttataat aggacctgcc agtgctctgg 360
caacttcatg ggattcaact gtggaaactg caagtttggc ttttggggac caaactgcac 420
agagagacga ctcttggtga gaagaaacat cttcgatttg agtgccccag agaaggacaa 480
attttttgcc tacctcactt tagcaaagca taccatcagc tcagactatg tcatccccat 540
agggacctat ggccaaatga aaaatggatc aacacccatg tttaacgaca tcaatattta 600
tgacctcttt gtctggatgc attattatgt gtcaatggat gcactgcttg ggggatctga 660
aatctggaga gacattgatt ttgcccatga agcaccagct tttctgcctt ggcatagact 720
cttcttgttg cggtgggaac aagaaatcca gaagctgaca ggagatgaaa acttcactat 780
tccatattgg gactggcggg atgcagaaaa gtgtgacatt tgcacagatg agtacatggg 840
aggtcagcac cccacaaatc ctaacttact cagcccagca tcattcttct cctcttggca 900
gattgtctgt agccgattgg aggagtacaa cagccatcag tctttatgca atggaacgcc 960
cgagggacct ttacggcgta atcctggaaa ccatgacaaa tccagaaccc caaggctccc 1020
ctcttcagct gatgtagaat tttgcctgag tttgacccaa tatgaatctg gttccatgga 1080
taaagctgcc aatttcagct ttagaaatac actggaagga tttgctagtc cacttactgg 1140
gatagcggat gcctctcaaa gcagcatgca caatgccttg cacatctata tgaatggaac 1200
aatgtcccag gtacagggat ctgccaacga tcctatcttc cttcttcacc atgcatttgt 1260
tgacagtatt tttgagcagt ggctccgaag gcaccgtcct cttcaagaag tttatccaga 1320
agccaatgca cccattggac ataaccggga atcctacatg gttcctttta taccactgta 1380
cagaaatggt gatttcttta tttcatccaa agatctgggc tatgactata gctatctaca 1440
agattcagac ccagactctt ttcaagacta cattaagtcc tatttggaac aagcgagtcg 1500
gatctggtca tggctccttg gggcggcgat ggtaggggcc gtcctcactg ccctgctggc 1560
agggcttgtg agcttgctgt gtcgtcacaa gagaaagcag cttcctgaag aaaagcagcc 1620
actcctcatg gagaaagagg attaccacag cttgtatcag agccatttat aaaaggctta 1680
ggcaatagag tagggccaaa aagcctgacc tcactctaac tcaaagtaat gtccaggttc 1740
ccagagaata tctgctggta tttttctgta aagaccattt gcaaaattgt aacctaatac 1800
aaagtgtagc cttcttccaa ctcaggtaga acacacctgt ctttgtcttg ctgttttcac 1860
tcagcccttt taacattttc ccctaagccc atatgtctaa ggaaaggatg ctatttggta 1920

atgaggaact gttatttgta tgtgaattaa agtgctctta tttt 1964

<210> 6
<211> 766
<212> DNA
<213> Homo sapiens

<400> 6
ctctctttcg attcttccat actcagagta cgcacggtct gattttctct ttggattctt 60
ccaaaatcag agtcagactg ctcccggtgc catgaacgga gacgacgcct ttgcaaggag 120
acccacggtt ggtgctcaaa taccagagaa gatccaaaag gccttcgatg atattgccaa 180
atacttctct aaggaagagt gggaaaagat gaaagcctcg gagaaaatct tctatgtgta 240
tatgaagaga aagtatgagg ctatgactaa actaggtttc aaggccaccc tcccaccttt 300
catgtgtaat aaacgggccg aagacttcca ggggaatgat ttggataatg accctaaccg 360
tgggaatcag gttgaacgtc ctcagatgac tttcggcagg ctccagggaa tctccccgaa 420
gatcatgccc aagaagccag cagaggaagg aaatgattcg gaggaagtgc cagaagcatc 480
tggcccacaa aatgatggga aagagctgtg cccccccgga aaaccaacta cctctgagaa 540
gattcacgag agatctggac ccaaaagggg ggaacatgcc tggacccaca gactgcgtga 600
gagaaaacag ctggtgattt atgaagagat cagcgaccct gaggaagatg acgagtaact 660
cccctcaggg atacgacaca tgcccatgat gagaagcaga acgtggtgac ctttcacgaa 720
catgggcatg gctgcggacc cctcgtcatc aggtgcatag caagtg 766

<210> 7
<211> 2653
<212> DNA
<213> Homo sapiens

<400> 7
ctcaaaaggg gccggatttc cttctcctgg aggcagatgt tgcctctctc tctcgctcgg 60
attggttcag tgcactctag aaacactgct gtggtggaga aactggaccc caggtctgga 120
gcgaattcca gcctgcaggg ctgataagcg aggcattagt gagattgaga gagactttac 180
cccgccgtgg tggttggagg gcgcgcagta gagcagcagc acaggcgcgg gtcccgggag 240
gccggctctg ctcgcgccga gatgtggaat ctccttcacg aaaccgactc ggctgtggcc 300
accgcgcgcc gcccgcgctg gctgtgcgct ggggcgctgg tgctggcggg tggcttcttt 360
ctcctcggct tcctcttcgg gtggtttata aaatcctcca atgaagctac taacattact 420
ccaaagcata atatgaaagc atttttggat gaattgaaag ctgagaacat caagaagttc 480
ttatataatt ttacacagat accacattta gcaggaacag aacaaaactt tcagcttgca 540
aagcaaattc aatcccagtg gaaagaattt ggcctggatt ctgttgagct agcacattat 600
gatgtcctgt tgtcctaccc aaataagact catcccaact acatctcaat aattaatgaa 660
gatggaaatg agattttcaa cacatcatta tttgaaccac ctcctccagg atatgaaaat 720
gtttcggata ttgtaccacc tttcagtgct ttctctcctc aaggaatgcc agagggcgat 780
ctagtgtatg ttaactatgc acgaactgaa gacttcttta aattggaacg ggacatgaaa 840
atcaattgct ctgggaaaat tgtaattgcc agatatggga aagttttcag aggaaataag 900
gttaaaaatg cccagctggc aggggccaaa ggagtcattc tctactccga ccctgctgac 960
tactttgctc ctggggtgaa gtcctatcca gatggttgga atcttcctgg aggtggtgtc 1020
cagcgtggaa atatcctaaa tctgaatggt gcaggagacc ctctcacacc aggttaccca 1080
gcaaatgaat atgcttatag gcgtggaatt gcagaggctg ttggtcttcc aagtattcct 1140
gttcatccaa ttggatacta tgatgcacag aagctcctag aaaaaatggg tggctcagca 1200
ccaccagata gcagctggag aggaagtctc aaagtgccct acaatgttgg acctggcttt 1260
actggaaact tttctacaca aaaagtcaag atgcacatcc actctaccaa tgaagtgaca 1320
agaatttaca atgtgatagg tactctcaga ggagcagtgg aaccagacag atatgtcatt 1380
ctgggaggtc accgggactc atgggtgttt ggtggtattg accctcagag tggagcagct 1440
gttgttcatg aaattgtgag gagctttgga acactgaaaa aggaagggtg gagacctaga 1500
agaacaattt tgtttgcaag ctgggatgca gaagaatttg gtcttcttgg ttctactgag 1560
tgggcagagg agaattcaag actccttcaa gagcgtggcg tggcttatat taatgctgac 1620
tcatctatag aaggaaacta cactctgaga gttgattgta caccgctgat gtacagcttg 1680
gtacacaacc taacaaaaga gctgaaaagc cctgatgaag gctttgaagg caaatctctt 1740

tatgaaagtt ggactaaaaa aagtccttcc ccagagttca gtggcatgcc caggataagc 1800
aaattgggat ctggaaatga ttttgaggtg ttcttccaac gacttggaat tgcttcaggc 1860
agagcacggt atactaaaaa ttgggaaaca aacaaattca gcggctatcc actgtatcac 1920
agtgtctatg aaacatatga gttggtggaa aagttttatg atccaatgtt taaatatcac 1980
ctcactgtgg cccaggttcg aggagggatg gtgtttgagc tagccaattc catagtgctc 2040
ccttttgatt gtcgagatta tgctgtagtt ttaagaaagt atgctgacaa aatctacagt 2100
atttctatga aacatccaca ggaaatgaag acatacagtg tatcatttga ttcacttttt 2160
tctgcagtaa agaattttac agaaattgct tccaagttca gtgagagact ccaggacttt 2220
gacaaaagca acccaatagt attaagaatg atgaatgatc aactcatgtt tctggaaaga 2280
gcatttattg atccattagg gttaccagac aggccttttt ataggcatgt catctatgct 2340
ccaagcagcc acaacaagta tgcaggggag tcattcccag gaatttatga tgctctgttt 2400
gatattgaaa gcaaagtgga cccttccaag gcctggggag aagtgaagag acagatttat 2460
gttgcagcct tcacagtgca ggcagctgca gagactttga gtgaagtagc ctaagaggat 2520
tctttagaga atccgtattg aatttgtgtg gtatgtcact cagaaagaat cgtaatgggt 2580
atattgataa attttaaaat tggtatattt gaaataaagt tgaatattat atataaaaaa 2640
aaaaaaaaaa aaa 2653

<210> 8
<211> 9
<212> PRT
<213> Homo sapiens

<400> 8
Phe Leu Pro Trp His Arg Leu Phe Leu
1               5


<210> 9
<211> 9
<212> PRT
<213> Homo sapiens

<400> 9
Leu Pro Trp His Arg Leu Phe Leu Leu
1               5


<210> 10
<211> 38
<212> PRT
<213> Homo sapiens

<400> 10
Tyr Phe Ser Lys Glu Glu Trp Glu Lys Met Lys Ala Ser Glu Lys Ile
1               5                   10                  15
Phe Tyr Val Tyr Met Lys Arg Lys Tyr Glu Ala Met Thr Lys Leu Gly
            20                  25                  30
Phe Lys Ala Thr Leu Pro
        35


<210> 11
<211> 9
<212> PRT
<213> Homo sapiens

<400> 11

Phe Ser Lys Glu Glu Trp Glu Lys Met
1               5

<210> 12
<211> 9
<212> PRT
<213> Homo sapiens

<400> 12
Lys Met Lys Ala Ser Glu Lys Ile Phe
1               5

<210> 13
<211> 9
<212> PRT
<213> Homo sapiens

<400> 13
Met Lys Ala Ser Glu Lys Ile Phe Tyr
1               5

<210> 14
<211> 10
<212> PRT
<213> Homo sapiens

<400> 14
Lys Met Lys Ala Ser Glu Lys Ile Phe Tyr
1               5                   10

<210> 15
<211> 9
<212> PRT
<213> Homo sapiens

<400> 15
Lys Ala Ser Glu Lys Ile Phe Tyr Val
1               5

<210> 16
<211> 10
<212> PRT
<213> Homo sapiens

<400> 16
Met Lys Ala Ser Glu Lys Ile Phe Tyr Val
1               5                   10

<210> 17
<211> 10
<212> PRT

<213> Homo sapiens

<400> 17
Lys Ala Ser Glu Lys Ile Phe Tyr Val Tyr
1               5                   10


<210> 18
<211> 9
<212> PRT
<213> Homo sapiens

<400> 18
Ala Ser Glu Lys Ile Phe Tyr Val Tyr
1               5


<210> 19
<211> 9
<212> PRT
<213> Homo sapiens

<400> 19
Arg Lys Tyr Glu Ala Met Thr Lys Leu
1               5


<210> 20
<211> 10
<212> PRT
<213> Homo sapiens

<400> 20
Lys Arg Lys Tyr Glu Ala Met Thr Lys Leu
1               5                   10


<210> 21
<211> 10
<212> PRT
<213> Homo sapiens

<400> 21
Lys Tyr Glu Ala Met Thr Lys Leu Gly Phe
1               5                   10


<210> 22
<211> 9
<212> PRT
<213> Homo sapiens

<400> 22
Tyr Glu Ala Met Thr Lys Leu Gly Phe
1               5

<210> 23
<211> 8
<212> PRT
<213> Homo sapiens

<400> 23
Glu Ala Met Thr Lys Leu Gly Phe
1               5


<210> 24
<211> 10
<212> PRT
<213> Homo sapiens

<400> 24
Phe Leu Pro Ser Asp Tyr Phe Pro Ser Val
1               5                   10


<210> 25
<211> 9
<212> PRT
<213> Homo sapiens

<400> 25
Ala Glu Met Gly Lys Tyr Ser Phe Tyr
1               5


<210> 26
<211> 9
<212> PRT
<213> Homo sapiens

<400> 26
Lys Tyr Ser Glu Lys Ile Ser Tyr Val
1               5


<210> 27
<211> 9
<212> PRT
<213> Homo sapiens

<400> 27
Lys Val Ser Glu Lys Ile Val Tyr Val
1               5


<210> 28
<211> 9
<212> PRT
<213> Homo sapiens

<400> 28
Lys Ser Ser Glu Lys Ile Val Tyr Val

1                   5


<210> 29
<211> 9
<212> PRT
<213> Homo sapiens

<400> 29
Lys Ala Ser Glu Lys Ile Ile Tyr Val
1                   5


<210> 30
<211> 30
<212> PRT
<213> Homo sapiens

<400> 30
Ala Phe Ser Pro Gln Gly Met Pro Glu Gly Asp Leu Val Tyr Val Asn
1                   5                   10                  15
Tyr Ala Arg Thr Glu Asp Phe Phe Lys Leu Glu Arg Asp Met
            20                  25                  30


<210> 31
<211> 23
<212> PRT
<213> Homo sapiens

<400> 31
Gly Met Pro Glu Gly Asp Leu Val Tyr Val Asn Tyr Ala Arg Thr Glu
1                   5                   10                  15
Asp Phe Phe Lys Leu Glu Arg
            20


<210> 32
<211> 9
<212> PRT
<213> Homo sapiens

<400> 32
Met Pro Glu Gly Asp Leu Val Tyr Val
1                   5


<210> 33
<211> 10
<212> PRT
<213> Homo sapiens

<400> 33
Gly Met Pro Glu Gly Asp Leu Val Tyr Val
1                   5                   10

<210> 34
<211> 9
<212> PRT
<213> Homo sapiens

<400> 34
Gly Met Pro Glu Gly Asp Leu Val Tyr
1               5


<210> 35
<211> 10
<212> PRT
<213> Homo sapiens

<400> 35
Gln Gly Met Pro Glu Gly Asp Leu Val Tyr
1               5                   10


<210> 36
<211> 8
<212> PRT
<213> Homo sapiens

<400> 36
Met Pro Glu Gly Asp Leu Val Tyr
1               5


<210> 37
<211> 9
<212> PRT
<213> Homo sapiens

<400> 37
Glu Gly Asp Leu Val Tyr Val Asn Tyr
1               5


<210> 38
<211> 10
<212> PRT
<213> Homo sapiens

<400> 38
Pro Glu Gly Asp Leu Val Tyr Val Asn Tyr
1               5                   10


<210> 39
<211> 10
<212> PRT
<213> Homo sapiens

<400> 39
Leu Val Tyr Val Asn Tyr Ala Arg Thr Glu

1                   5                       10


<210> 40
<211> 9
<212> PRT
<213> Homo sapiens

<400> 40
Val Asn Tyr Ala Arg Thr Glu Asp Phe
1                   5


<210> 41
<211> 10
<212> PRT
<213> Homo sapiens

<400> 41
Tyr Val Asn Tyr Ala Arg Thr Glu Asp Phe
1                   5                       10


<210> 42
<211> 9
<212> PRT
<213> Homo sapiens

<400> 42
Asn Tyr Ala Arg Thr Glu Asp Phe Phe
1                   5


<210> 43
<211> 8
<212> PRT
<213> Homo sapiens

<400> 43
Tyr Ala Arg Thr Glu Asp Phe Phe
1                   5


<210> 44
<211> 9
<212> PRT
<213> Homo sapiens

<400> 44
Arg Thr Glu Asp Phe Phe Lys Leu Glu
1                   5


<210> 45
<211> 30
<212> PRT
<213> Homo sapiens

<400> 45
Arg Gly Ile Ala Glu Ala Val Gly Leu Pro Ser Ile Pro Val His Pro
1               5                   10                  15
Ile Gly Tyr Tyr Asp Ala Gln Lys Leu Leu Glu Lys Met Gly
            20                  25                  30


<210> 46
<211> 25
<212> PRT
<213> Homo sapiens

<400> 46
Ile Ala Glu Ala Val Gly Leu Pro Ser Ile Pro Val His Pro Ile Gly
1               5                   10                  15
Tyr Tyr Asp Ala Gln Lys Leu Leu Glu
            20                  25


<210> 47
<211> 9
<212> PRT
<213> Homo sapiens

<400> 47
Leu Pro Ser Ile Pro Val His Pro Ile
1               5


<210> 48
<211> 10
<212> PRT
<213> Homo sapiens

<400> 48
Gly Leu Pro Ser Ile Pro Val His Pro Ile
1               5                   10


<210> 49
<211> 9
<212> PRT
<213> Homo sapiens

<400> 49
Ile Gly Tyr Tyr Asp Ala Gln Lys Leu
1               5


<210> 50
<211> 10
<212> PRT
<213> Homo sapiens

<400> 50
Pro Ile Gly Tyr Tyr Asp Ala Gln Lys Leu

1                   5                   10


<210> 51
<211> 9
<212> PRT
<213> Homo sapiens

<400> 51
Ser Ile Pro Val His Pro Ile Gly Tyr
1                   5


<210> 52
<211> 10
<212> PRT
<213> Homo sapiens

<400> 52
Pro Ser Ile Pro Val His Pro Ile Gly Tyr
1                   5                   10


<210> 53
<211> 8
<212> PRT
<213> Homo sapiens

<400> 53
Ile Pro Val His Pro Ile Gly Tyr
1                   5


<210> 54
<211> 9
<212> PRT
<213> Homo sapiens

<400> 54
Tyr Tyr Asp Ala Gln Lys Leu Leu Glu
1                   5


<210> 55
<211> 27
<212> PRT
<213> Homo sapiens

<400> 55
Ser Ser Ile Glu Gly Asn Tyr Thr Leu Arg Val Asp Cys Thr Pro Leu
1                   5                   10                  15
Met Tyr Ser Leu Val His Leu Thr Lys Glu Leu
            20                  25


<210> 56
<211> 9

<212> PRT
<213> Homo sapiens

<400> 56
Ile Glu Gly Asn Tyr Thr Leu Arg Val
1               5


<210> 57
<211> 10
<212> PRT
<213> Homo sapiens

<400> 57
Ser Ile Glu Gly Asn Tyr Thr Leu Arg Val
1               5                   10


<210> 58
<211> 8
<212> PRT
<213> Homo sapiens

<400> 58
Glu Gly Asn Tyr Thr Leu Arg Val
1               5


<210> 59
<211> 9
<212> PRT
<213> Homo sapiens

<400> 59
Thr Leu Arg Val Asp Cys Thr Pro Leu
1               5


<210> 60
<211> 10
<212> PRT
<213> Homo sapiens

<400> 60
Tyr Thr Leu Arg Val Asp Cys Thr Pro Leu
1               5                   10


<210> 61
<211> 9
<212> PRT
<213> Homo sapiens

<400> 61
Leu Arg Val Asp Cys Thr Pro Leu Met
1               5

<210> 62
<211> 9
<212> PRT
<213> Homo sapiens

<400> 62
Arg Val Asp Cys Thr Pro Leu Met Tyr
1               5


<210> 63
<211> 10
<212> PRT
<213> Homo sapiens

<400> 63
Leu Arg Val Asp Cys Thr Pro Leu Met Tyr
1               5                   10


<210> 64
<211> 35
<212> PRT
<213> Homo sapiens

<400> 64
Phe Asp Lys Ser Asn Pro Ile Val Leu Arg Met Met Asn Asp Gln Leu
1               5                   10                  15
Met Phe Leu Glu Arg Ala Phe Ile Asp Pro Leu Gly Leu Pro Asp Arg
            20                  25                  30
Pro Phe Tyr
        35


<210> 65
<211> 22
<212> PRT
<213> Homo sapiens

<400> 65
Val Leu Arg Met Met Asn Asp Gln Leu Met Phe Leu Glu Arg Ala Phe
1               5                   10                  15
Ile Asp Pro Leu Gly Leu
            20


<210> 66
<211> 9
<212> PRT
<213> Homo sapiens

<400> 66
Met Met Asn Asp Gln Leu Met Phe Leu
1               5

<210> 67
<211> 10
<212> PRT
<213> Homo sapiens

<400> 67
Arg Met Met Asn Asp Gln Leu Met Phe Leu
1               5                   10


<210> 68
<211> 9
<212> PRT
<213> Homo sapiens

<400> 68
Arg Met Met Asn Asp Gln Leu Met Phe
1               5


<210> 69
<211> 17
<212> PRT
<213> Homo sapiens

<400> 69
Met Leu Leu Ala Val Leu Tyr Cys Leu Leu Trp Ser Phe Gln Thr Ser
1               5                   10                  15
Ala


<210> 70
<211> 661
<212> PRT
<213> Homo sapiens

<400> 70

<210> 71
<211> 309
<212> PRT
<213> Homo sapiens

<400> 71
Met Ser Leu Glu Gln Arg Ser Leu His Cys Lys Pro Glu Glu Ala Leu
 1               5                  10                  15
Glu Ala Gln Gln Glu Ala Leu Gly Leu Val Cys Val Gln Ala Ala Thr
            20                  25                  30
Ser Ser Ser Ser Pro Leu Val Leu Gly Thr Leu Glu Glu Val Pro Thr
        35                  40                  45
Ala Gly Ser Thr Asp Pro Pro Gln Ser Pro Gln Gly Ala Ser Ala Phe
    50                  55                  60
Pro Thr Thr Ile Asn Phe Thr Arg Gln Arg Gln Pro Ser Glu Gly Ser
65                  70                  75                  80
Ser Ser Arg Glu Glu Glu Gly Pro Ser Thr Ser Cys Ile Leu Glu Ser
                85                  90                  95
Leu Phe Arg Ala Val Ile Thr Lys Lys Val Ala Asp Leu Val Gly Phe
            100                 105                 110
Leu Leu Leu Lys Tyr Arg Ala Arg Glu Pro Val Thr Lys Ala Glu Met
        115                 120                 125
Leu Glu Ser Val Ile Lys Asn Tyr Lys His Cys Phe Pro Glu Ile Phe
    130                 135                 140
Gly Lys Ala Ser Glu Ser Leu Gln Leu Val Phe Gly Ile Asp Val Lys
145                 150                 155                 160
Glu Ala Asp Pro Thr Gly His Ser Tyr Val Leu Val Thr Cys Leu Gly
                165                 170                 175
Leu Ser Tyr Asp Gly Leu Leu Gly Asp Asn Gln Ile Met Pro Lys Thr
            180                 185                 190
Gly Phe Leu Ile Ile Val Leu Val Met Ile Ala Met Glu Gly Gly His
        195                 200                 205
Ala Pro Glu Glu Glu Ile Trp Glu Glu Leu Ser Val Met Glu Val Tyr
    210                 215                 220
Asp Gly Arg Glu His Ser Ala Tyr Gly Glu Pro Arg Lys Leu Leu Thr
225                 230                 235                 240
Gln Asp Leu Val Gln Glu Lys Tyr Leu Glu Tyr Arg Gln Val Pro Asp
                245                 250                 255
Ser Asp Pro Ala Arg Tyr Glu Phe Leu Trp Gly Pro Arg Ala Leu Ala
            260                 265                 270
Glu Thr Ser Tyr Val Lys Val Leu Glu Tyr Val Ile Lys Val Ser Ala
        275                 280                 285
Arg Val Arg Phe Phe Phe Pro Ser Leu Arg Glu Ala Ala Leu Arg Glu
    290                 295                 300
Glu Glu Glu Gly Val
305

<210> 72
<211> 314
<212> PRT
<213> Homo sapiens

<400> 72
Met Pro Leu Glu Gln Arg Ser Gln His Cys Lys Pro Glu Glu Gly Leu
1               5                   10                  15
Glu Ala Arg Gly Glu Ala Leu Gly Leu Val Gly Ala Gln Ala Pro Ala
            20                  25                  30
Thr Glu Glu Gln Gln Thr Ala Ser Ser Ser Ser Thr Leu Val Glu Val
        35                  40                  45
Thr Leu Gly Glu Val Pro Ala Ala Asp Ser Pro Ser Pro Pro His Ser
    50                  55                  60
Pro Gln Gly Ala Ser Ser Phe Ser Thr Thr Ile Asn Tyr Thr Leu Trp
65                  70                  75                  80
Arg Gln Ser Asp Glu Gly Ser Ser Asn Gln Glu Glu Glu Gly Pro Arg
                85                  90                  95
Met Phe Pro Asp Leu Glu Ser Glu Phe Gln Ala Ala Ile Ser Arg Lys
            100                 105                 110
Met Val Glu Leu Val His Phe Leu Leu Leu Lys Tyr Arg Ala Arg Glu
        115                 120                 125
Pro Val Thr Lys Ala Glu Met Leu Glu Ser Val Leu Arg Asn Cys Gln
    130                 135                 140
Asp Phe Phe Pro Val Ile Phe Ser Lys Ala Ser Glu Tyr Leu Gln Leu
145                 150                 155                 160
Val Phe Gly Ile Glu Val Val Glu Val Val Pro Ile Ser His Leu Tyr
                165                 170                 175
Ile Leu Val Thr Cys Leu Gly Leu Ser Tyr Asp Gly Leu Leu Gly Asp
            180                 185                 190
Asn Gln Val Met Pro Lys Thr Gly Leu Leu Ile Ile Val Leu Ala Ile
        195                 200                 205
Ile Ala Ile Glu Gly Asp Cys Ala Pro Glu Glu Lys Ile Trp Glu Glu
    210                 215                 220
Leu Ser Met Leu Glu Val Phe Glu Gly Arg Glu Asp Ser Val Phe Ala
225                 230                 235                 240
His Pro Arg Lys Leu Leu Met Gln Asp Leu Val Gln Glu Asn Tyr Leu
                245                 250                 255
Glu Tyr Arg Gln Val Pro Gly Ser Asp Pro Ala Cys Tyr Glu Phe Leu
            260                 265                 270
Trp Gly Pro Arg Ala Leu Ile Glu Thr Ser Tyr Val Lys Val Leu His
        275                 280                 285
His Thr Leu Lys Ile Gly Gly Glu Pro His Ile Ser Tyr Pro Pro Leu
    290                 295                 300
His Glu Arg Ala Leu Arg Glu Gly Glu Glu
305                 310


<210> 73
<211> 314
<212> PRT
<213> Homo sapiens

<400> 73
Met Pro Leu Glu Gln Arg Ser Gln His Cys Lys Pro Glu Glu Gly Leu
1               5                   10                  15

Glu Ala Arg Gly Glu Ala Leu Gly Leu Val Gly Ala Gln Ala Pro Ala
            20                  25                  30
Thr Glu Glu Gln Glu Ala Ala Ser Ser Ser Ser Thr Leu Val Glu Val
        35                  40                  45
Thr Leu Gly Glu Val Pro Ala Ala Glu Ser Pro Asp Pro Pro Gln Ser
    50                  55                  60
Pro Gln Gly Ala Ser Ser Leu Pro Thr Thr Met Asn Tyr Pro Leu Trp
65                  70                  75                  80
Ser Gln Ser Tyr Glu Asp Ser Ser Asn Gln Glu Glu Glu Gly Pro Ser
                85                  90                  95
Thr Phe Pro Asp Leu Glu Ser Glu Phe Gln Ala Ala Leu Ser Arg Lys
            100                 105                 110
Val Ala Glu Leu Val His Phe Leu Leu Leu Lys Tyr Arg Ala Arg Glu
        115                 120                 125
Pro Val Thr Lys Ala Glu Met Leu Gly Ser Val Val Gly Asn Trp Gln
    130                 135                 140
Tyr Phe Phe Pro Val Ile Phe Ser Lys Ala Ser Ser Ser Leu Gln Leu
145                 150                 155                 160
Val Phe Gly Ile Glu Leu Met Glu Val Asp Pro Ile Gly His Leu Tyr
                165                 170                 175
Ile Phe Ala Thr Cys Leu Gly Leu Ser Tyr Asp Gly Leu Leu Gly Asp
            180                 185                 190
Asn Gln Ile Met Pro Lys Ala Gly Leu Leu Ile Ile Val Leu Ala Ile
        195                 200                 205
Ile Ala Arg Glu Gly Asp Cys Ala Pro Glu Glu Lys Ile Trp Glu Glu
    210                 215                 220
Leu Ser Val Leu Glu Val Phe Glu Gly Arg Glu Asp Ser Ile Leu Gly
225                 230                 235                 240
Asp Pro Lys Lys Leu Leu Thr Gln His Phe Val Gln Glu Asn Tyr Leu
                245                 250                 255
Glu Tyr Arg Gln Val Pro Gly Ser Asp Pro Ala Cys Tyr Glu Phe Leu
            260                 265                 270
Trp Gly Pro Arg Ala Leu Val Glu Thr Ser Tyr Val Lys Val Leu His
        275                 280                 285
His Met Val Lys Ile Ser Gly Gly Pro His Ile Ser Tyr Pro Pro Leu
    290                 295                 300
His Glu Trp Val Leu Arg Glu Gly Glu Glu
305                 310

<210> 74
<211> 180
<212> PRT
<213> Homo sapiens

<400> 74
Met Gln Ala Glu Gly Arg Gly Thr Gly Gly Ser Thr Gly Asp Ala Asp
1               5                   10                  15
Gly Pro Gly Gly Pro Gly Ile Pro Asp Gly Pro Gly Gly Asn Ala Gly
            20                  25                  30
Gly Pro Gly Glu Ala Gly Ala Thr Gly Gly Arg Gly Pro Arg Gly Ala
        35                  40                  45
Gly Ala Ala Arg Ala Ser Gly Pro Gly Gly Gly Ala Pro Arg Gly Pro
    50                  55                  60
His Gly Gly Ala Ala Ser Gly Leu Asn Gly Cys Cys Arg Cys Gly Ala
65                  70                  75                  80
Arg Gly Pro Glu Ser Arg Leu Leu Glu Phe Tyr Leu Ala Met Pro Phe

85                  90                  95
Ala Thr Pro Met Glu Ala Glu Leu Ala Arg Arg Ser Leu Ala Gln Asp
            100                 105                 110
Ala Pro Pro Leu Pro Val Pro Gly Val Leu Leu Lys Glu Phe Thr Val
        115                 120                 125
Ser Gly Asn Ile Leu Thr Ile Arg Leu Thr Ala Ala Asp His Arg Gln
    130                 135                 140
Leu Gln Leu Ser Ile Ser Ser Cys Leu Gln Gln Leu Ser Leu Leu Met
145                 150                 155                 160
Trp Ile Thr Gln Cys Phe Leu Pro Val Phe Leu Ala Gln Pro Pro Ser
                165                 170                 175
Gly Gln Arg Arg
            180

<210> 75
<211> 180
<212> PRT
<213> Homo sapiens

<400> 75
Met Gln Ala Glu Gly Arg Gly Thr Gly Gly Ser Thr Gly Asp Ala Asp
1               5                   10                  15
Gly Pro Gly Gly Pro Gly Ile Pro Asp Gly Pro Gly Gly Asn Ala Gly
            20                  25                  30
Gly Pro Gly Glu Ala Gly Ala Thr Gly Gly Arg Gly Pro Arg Gly Ala
        35                  40                  45
Gly Ala Ala Arg Ala Ser Gly Pro Arg Gly Gly Ala Pro Arg Gly Pro
    50                  55                  60
His Gly Gly Ala Ala Ser Ala Gln Asp Gly Arg Cys Pro Cys Gly Ala
65                  70                  75                  80
Arg Arg Pro Asp Ser Arg Leu Leu Glu Leu His Ile Thr Met Pro Phe
                85                  90                  95
Ser Ser Pro Met Glu Ala Glu Leu Val Arg Arg Ile Leu Ser Arg Asp
            100                 105                 110
Ala Ala Pro Leu Pro Arg Pro Gly Ala Val Leu Lys Asp Phe Thr Val
        115                 120                 125
Ser Gly Asn Leu Leu Phe Ile Arg Leu Thr Ala Ala Asp His Arg Gln
    130                 135                 140
Leu Gln Leu Ser Ile Ser Ser Cys Leu Gln Gln Leu Ser Leu Leu Met
145                 150                 155                 160
Trp Ile Thr Gln Cys Phe Leu Pro Val Phe Leu Ala Gln Ala Pro Ser
                165                 170                 175
Gly Gln Arg Arg
            180

<210> 76
<211> 210
<212> PRT
<213> Homo sapiens

<400> 76

<210> 77
<211> 509
<212> PRT
<213> Homo sapiens

<400> 77
Met Glu Arg Arg Arg Leu Trp Gly Ser Ile Gln Ser Arg Tyr Ile Ser
 1               5                  10                  15
Met Ser Val Trp Thr Ser Pro Arg Arg Leu Val Glu Leu Ala Gly Gln
            20                  25                  30
Ser Leu Leu Lys Asp Glu Ala Leu Ala Ile Ala Ala Leu Glu Leu Leu
        35                  40                  45
Pro Arg Glu Leu Phe Pro Pro Leu Phe Met Ala Ala Phe Asp Gly Arg
    50                  55                  60
His Ser Gln Thr Leu Lys Ala Met Val Gln Ala Trp Pro Phe Thr Cys
65                  70                  75                  80
Leu Pro Leu Gly Val Leu Met Lys Gly Gln His Leu His Leu Glu Thr
                85                  90                  95
Phe Lys Ala Val Leu Asp Gly Leu Asp Val Leu Leu Ala Gln Glu Val
            100                 105                 110
Arg Pro Arg Arg Trp Lys Leu Gln Val Leu Asp Leu Arg Lys Asn Ser
        115                 120                 125
His Gln Asp Phe Trp Thr Val Trp Ser Gly Asn Arg Ala Ser Leu Tyr
    130                 135                 140
Ser Phe Pro Glu Pro Glu Ala Ala Gln Pro Met Thr Lys Lys Arg Lys
145                 150                 155                 160
Val Asp Gly Leu Ser Thr Glu Ala Glu Gln Pro Phe Ile Pro Val Glu
                165                 170                 175
Val Leu Val Asp Leu Phe Leu Lys Glu Gly Ala Cys Asp Glu Leu Phe
            180                 185                 190
Ser Tyr Leu Ile Glu Lys Val Lys Arg Lys Lys Asn Val Leu Arg Leu

195                 200                 205
Cys Cys Lys Lys Leu Lys Ile Phe Ala Met Pro Met Gln Asp Ile Lys
    210                 215                 220
Met Ile Leu Lys Met Val Gln Leu Asp Ser Ile Glu Asp Leu Glu Val
225                 230                 235                 240
Thr Cys Thr Trp Lys Leu Pro Thr Leu Ala Lys Phe Ser Pro Tyr Leu
                245                 250                 255
Gly Gln Met Ile Asn Leu Arg Arg Leu Leu Leu Ser His Ile His Ala
            260                 265                 270
Ser Ser Tyr Ile Ser Pro Glu Lys Glu Glu Gln Tyr Ile Ala Gln Phe
        275                 280                 285
Thr Ser Gln Phe Leu Ser Leu Gln Cys Leu Gln Ala Leu Tyr Val Asp
    290                 295                 300
Ser Leu Phe Phe Leu Arg Gly Arg Leu Asp Gln Leu Leu Arg His Val
305                 310                 315                 320
Met Asn Pro Leu Glu Thr Leu Ser Ile Thr Asn Cys Arg Leu Ser Glu
                325                 330                 335
Gly Asp Val Met His Leu Ser Gln Ser Pro Ser Val Ser Gln Leu Ser
            340                 345                 350
Val Leu Ser Leu Ser Gly Val Met Leu Thr Asp Val Ser Pro Glu Pro
        355                 360                 365
Leu Gln Ala Leu Leu Glu Arg Ala Ser Ala Thr Leu Gln Asp Leu Val
    370                 375                 380
Phe Asp Glu Cys Gly Ile Thr Asp Asp Gln Leu Leu Ala Leu Leu Pro
385                 390                 395                 400
Ser Leu Ser His Cys Ser Gln Leu Thr Thr Leu Ser Phe Tyr Gly Asn
                405                 410                 415
Ser Ile Ser Ile Ser Ala Leu Gln Ser Leu Leu Gln His Leu Ile Gly
            420                 425                 430
Leu Ser Asn Leu Thr His Val Leu Tyr Pro Val Pro Leu Glu Ser Tyr
        435                 440                 445
Glu Asp Ile His Gly Thr Leu His Leu Glu Arg Leu Ala Tyr Leu His
    450                 455                 460
Ala Arg Leu Arg Glu Leu Leu Cys Glu Leu Gly Arg Pro Ser Met Val
465                 470                 475                 480
Trp Leu Ser Ala Asn Pro Cys Pro His Cys Gly Asp Arg Thr Phe Tyr
                485                 490                 495
Asp Pro Glu Pro Ile Leu Cys Pro Cys Phe Met Pro Asn
            500                 505

<210> 78
<211> 261
<212> PRT
<213> Homo sapiens

<400> 78
Met Trp Val Pro Val Val Phe Leu Thr Leu Ser Val Thr Trp Ile Gly
1               5                   10                  15
Ala Ala Pro Leu Ile Leu Ser Arg Ile Val Gly Gly Trp Glu Cys Glu
            20                  25                  30
Lys His Ser Gln Pro Trp Gln Val Leu Val Ala Ser Arg Gly Arg Ala
        35                  40                  45
Val Cys Gly Gly Val Leu Val His Pro Gln Trp Val Leu Thr Ala Ala
    50                  55                  60
His Cys Ile Arg Asn Lys Ser Val Ile Leu Leu Gly Arg His Ser Leu
65                  70                  75                  80

Phe His Pro Glu Asp Thr Gly Gln Val Phe Gln Val Ser His Ser Phe
                85                  90                  95
Pro His Pro Leu Tyr Asp Met Ser Leu Leu Lys Asn Arg Phe Leu Arg
            100                 105                 110
Pro Gly Asp Asp Ser Ser His Asp Leu Met Leu Leu Arg Leu Ser Glu
        115                 120                 125
Pro Ala Glu Leu Thr Asp Ala Val Lys Val Met Asp Leu Pro Thr Gln
    130                 135                 140
Glu Pro Ala Leu Gly Thr Thr Cys Tyr Ala Ser Gly Trp Gly Ser Ile
145                 150                 155                 160
Glu Pro Glu Glu Phe Leu Thr Pro Lys Lys Leu Gln Cys Val Asp Leu
                165                 170                 175
His Val Ile Ser Asn Asp Val Cys Ala Gln Val His Pro Gln Lys Val
            180                 185                 190
Thr Lys Phe Met Leu Cys Ala Gly Arg Trp Thr Gly Gly Lys Ser Thr
        195                 200                 205
Cys Ser Gly Asp Ser Gly Gly Pro Leu Val Cys Asn Gly Val Leu Gln
    210                 215                 220
Gly Ile Thr Ser Trp Gly Ser Glu Pro Cys Ala Leu Pro Glu Arg Pro
225                 230                 235                 240
Ser Leu Tyr Thr Lys Val Val His Tyr Arg Lys Trp Ile Lys Asp Thr
                245                 250                 255
Ile Val Ala Asn Pro
            260


<210> 79
<211> 123
<212> PRT
<213> Homo sapiens

<400> 79
Met Lys Ala Val Leu Leu Ala Leu Leu Met Ala Gly Leu Ala Leu Gln
 1               5                  10                  15
Pro Gly Thr Ala Leu Leu Cys Tyr Ser Cys Lys Ala Gln Val Ser Asn
            20                  25                  30
Glu Asp Cys Leu Gln Val Glu Asn Cys Thr Gln Leu Gly Glu Gln Cys
        35                  40                  45
Trp Thr Ala Arg Ile Arg Ala Val Gly Leu Leu Thr Val Ile Ser Lys
    50                  55                  60
Gly Cys Ser Leu Asn Cys Val Asp Asp Ser Gln Asp Tyr Tyr Val Gly
65                  70                  75                  80
Lys Lys Asn Ile Thr Cys Cys Asp Thr Asp Leu Cys Asn Ala Ser Gly
                85                  90                  95
Ala His Ala Leu Gln Pro Ala Ala Ala Ile Leu Ala Leu Leu Pro Ala
            100                 105                 110
Leu Gly Leu Leu Leu Trp Gly Pro Gly Gln Leu
        115                 120


<210> 80
<211> 2817
<212> DNA
<213> Homo sapiens

<400> 80
gtgctaaaaa gatgccttct tcatttggct gtgataggtg ctttgtggct gtgggggcta 60

caaaagtacc cagaaaccag gactggcttg gtgtctcaag gcaactcaga accaaagcct 120
ggaacaggca gctgtatcca gagtggacag aagcccagag acttgactgc tggagaggtg 180
gtcaagtgtc cctcaaggtc agtaatgatg ggcctacact gattggtgca aatgcctcct 240
tctctattgc cttgaacttc cctggaagcc aaaaggtatt gccagatcgg caggttatct 300
gggtcaacaa taccatcatc aatgggagcc aggtgtgggg aggacagcca gtgtatcccc 360
aggaaactga cgatgcctgc atcttccctg atggtggacc ttgcccatct ggctcttggt 420
ctcagaagag aagctttgtt tatgtctgga agacctgggg tgagggactc ccttctcagc 480
ctatcatcca cacttgtgtt tacttctttc tacctgatca cctttctttt ggccgcccct 540
tccaccttaa cttctgtgat tttctctaat cttcattttc ctcttagatc ttttctcttt 600
cttagcacct agcccccttc aagctctatc ataattcttt ctggcaactc ttggcctcaa 660
ttgtagtcct accccatgga atgcctcatt aggacccctt ccctgtcccc ccatatcaca 720
gccttccaaa caccctcaga agtaatcata cttcctgacc tcccatctcc agtgccgttt 780
cgaagcctgt ccctcagtcc cctttgacca gtaatctctt cttccttgct tttcattcca 840
aaaatgcttc aggccaatac tggcaagttc tagggggccc agtgtctcgg ctgagcattg 900
ggacaggcag ggcaatgctg ggcacacaca ccatggaagt gactgtctac catcgccggg 960
gatcccggag ctatgtgcct cttgctcatt ccagctcagc cttcaccatt actggtaagg 1020
gttcaggaag ggcaaggcca gttgtagggc aaagagaagg cagggaggct tggatggact 1080
gcaaaggaga aaggtgaaat gctgtgcaaa cttaaagtag aagggccagg aagacctagg 1140
cagagaaatg tgaggcttag tgccagtgaa gggccagcca gtcagcttgg agttggaggg 1200
tgtggctgtg aaaggagaag ctgtggctca ggcctggttc tcaccttttc tggctccaat 1260
cccagaccag gtgcctttct ccgtgagcgt gtcccagttg cgggccttgg atggagggaa 1320
caagcacttc ctgagaaatc agcctctgac ctttgccctc cagctccatg accccagtgg 1380
ctatctggct gaagctgacc tctcctacac ctgggacttt ggagacagta gtggaaccct 1440
gatctctcgg gcacctgtgg tcactcatac ttacctggag cctggcccag tcactgccca 1500
ggtggtcctg caggctgcca ttcctctcac ctcctgtggc tcctccccag ttccaggcac 1560
cacagatggg cacaggccaa ctgcagaggc ccctaacacc acagctggcc aagtgcctac 1620
tacagaagtt gtgggtacta cacctggtca ggcgccaact gcagagccct ctggaaccac 1680
atctgtgcag gtgccaacca ctgaagtcat aagcactgca cctgtgcaga tgccaactgc 1740
agagagcaca ggtatgacac ctgagaaggt gccagtttca gaggtcatgg gtaccacact 1800
ggcagagatg tcaactccag aggctacagg tatgacacct gcagaggtat caattgtggt 1860
gctttctgga accacagctg cacaggtaac aactacagag tgggtggaga ccacagctag 1920
agagctacct atccctgagc ctgaaggtcc agatgccagc tcaatcatgt ctacggaaag 1980
tattacaggt tccctgggcc ccctgctgga tggtacagcc accttaaggc tggtgaagag 2040
acaagtcccc ctggattgtg ttctgtatcg atatggttcc ttttccgtca ccctggacat 2100
tgtccagggt attgaaagtg ccgagatcct gcaggctgtg ccgtccggtg agggggatgc 2160
atttgagctg actgtgtcct gccaaggcgg gctgcccaag gaagcctgca tggagatctc 2220
atcgccaggg tgccagcccc ctgcccagcg gctgtgccag cctgtgctac ccagcccagc 2280
ctgccagctg gttctgcacc agatactgaa gggtggctcg ggacatactt gcctcaatgt 2340
gtctctggct gataccaaca gcctggcagt ggtcagcacc cagcttatca tgcctggtag 2400
gtccttggac agagactaag tgaggaggga agtggataga ggggacagct ggcaagcagc 2460
agacatgagt gaagcagtgc ctgggattct tctcacaggt caagaagcag gccttgggca 2520
ggttccgctg atcgtgggca tcttgctggt gttgatggct gtggtccttg catctctgat 2580
atataggcgc agacttatga agcaagactt ctccgtaccc cagttgccac atagcagcag 2640
tcactggctg cgtctacccc gcatcttctg ctcttgtccc attggtgaga atagccccct 2700
cctcagtggg cagcaggtct gagtactctc atatgatgct gtgattttcc tggagttgac 2760
agaaacacct atatttcccc cagtcttccc tgggagacta ctattaactg aaataaa 2817

<210> 81
<211> 2420
<212> DNA
<213> Homo sapiens

<400> 81
ggatccaggc cctgccagga aaaatataag ggccctgcgt gagaacagag gggtcatcc 60
actgcatgag agtggggatg tcacagagtc cagcccaccc tcctggtagc actgagaagc 120
cagggctgtg cttgcggtct gcaccctgag ggcccgtgga ttcctcttcc tggagctcca 180
ggaaccaggc agtgaggcct tggtctgaga cagtatcctc aggtcacaga gcagaggatg 240

cacagggtgt gccagcagtg aatgtttgcc ctgaatgcac accaagggcc ccacctgcca 300
caggacacat aggactccac agagtctggc ctcacctccc tactgtcagt cctgtagaat 360
cgacctctgc tggccggctg taccctgagt accctctcac ttcctccttc aggttttcag 420
gggacaggcc aacccagagg acaggattcc ctggaggcca cagaggagca ccaaggagaa 480
gatctgtaag taggcctttg ttagagtctc caaggttcag ttctcagctg aggcctctca 540
cacactccct ctctccccag gcctgtgggt cttcattgcc cagctcctgc ccacactcct 600
gcctgctgcc ctcacgagag tcatcatgtc tcttgagcag aggagtctgc actgcaagcc 660
tgaggaagcc cttgaggccc aacaagaggc cctgggcctg gtgtgtgtgc aggctgccac 720
ctcctcctcc tctcctctgg tcctgggcac cctggaggag gtgcccactg ctgggtcaac 780
agatcctccc cacagtcctc agggagcctc cgcctttccc actaccatca acttcactcg 840
acagaggcaa cccagtgagg gttccagcag ccgtgaagag gaggggccaa gcacctcttg 900
tatcctggag tccttgttcc gagcagtaat cactaagaag gtggctgatt tggttggttt 960
tctgctcctc aaatatcgag ccagggagcc agtcacaaag gcagaaatgc tggagagtgt 1020
catcaaaaat tacaagcact gttttcctga gatcttcggc aaagcctctg agtccttgca 1080
gctggtcttt ggcattgacg tgaaggaagc agaccccacc ggccactcct atgtccttgt 1140
cacctgccta ggtctctcct atgatggcct gctgggtgat aatcagatca tgcccaagac 1200
aggcttcctg ataattgtcc tggtcatgat tgcaatggag ggcggccatg ctcctgagga 1260
ggaaatctgg gaggagctga gtgtgatgga ggtgtatgat gggagggagc acagtgccta 1320
tggggagccc aggaagctgc tcacccaaga tttggtgcag gaaaagtacc tggagtaccg 1380
gcaggtgccg gacagtgatc ccgcacgcta tgagttcctg tggggtccaa gggccctcgc 1440
tgaaaccagc tatgtgaaag tccttgagta tgtgatcaag gtcagtgcaa gagttcgctt 1500
tttcttccca tccctgcgtg aagcagcttt gagagaggag gaagagggag tctgagcatg 1560
agttgcagcc aaggccagtg ggagggggac tgggccagtg caccttccag ggccgcgtcc 1620
agcagcttcc cctgcctcgt gtgacatgag gcccattctt cactctgaag agagcggtca 1680
gtgttctcag tagtaggttt ctgttctatt gggtgacttg gagatttatc tttgttctct 1740
tttggaattg ttcaaatgtt tttttttaag ggatggttga atgaacttca gcatccaagt 1800
ttatgaatga cagcagtcac acagttctgt gtatatagtt taagggtaag agtcttgtgt 1860
tttattcaga ttgggaaatc cattctattt tgtgaattgg gataataaca gcagtggaat 1920
aagtacttag aaatgtgaaa aatgagcagt aaaatagatg agataaagaa ctaaagaaat 1980
taagagatag tcaattcttg ccttatacct cagtctattc tgtaaaattt ttaaagatat 2040
atgcatacct ggatttcctt ggcttctttg agaatgtaag agaaattaaa tctgaataaa 2100
gaattcttcc tgttcactgg ctcttttctt ctccatgcac tgagcatctg ctttttggaa 2160
ggccctgggt tagtagtgga gatgctaagg taagccagac tcatacccac ccatagggtc 2220
gtagagtcta ggagctgcag tcacgtaatc gaggtggcaa gatgtcctct aaagatgtag 2280
ggaaaagtga gagaggggtg agggtgtggg gctccgggtg agagtggtgg agtgtcaatg 2340
ccctgagctg gggcattttg ggctttggga aactgcagtt ccttctgggg gagctgattg 2400
taatgatctt gggtggatcc 2420

<210> 82
<211> 4559
<212> DNA
<213> Homo sapiens

<400> 82
attccttcat caaacagcca ggagtgagga agaggaccct cctgagtgag gactgaggat 60
ccaccctcac cacatagtgg gaccacagaa tccagctcag cccctcttgt cagccctggt 120
acacactggc aatgatctca ccccgagcac acccctcccc ccaatgccac ttcgggccga 180
ctcagagtca gagacttggt ctgaggggag cagacacaat cggcagagga tggcggtcca 240
ggctcagtct ggcatccaag tcaggacctt gagggatgac caaaggcccc tcccaccccc 300
aactcccccg accccaccag gatctacagc ctcaggatcc ccgtcccaat ccctacccct 360
acaccaacac catcttcatg cttaccccca cccccccatc cagatcccca tccgggcaga 420
atccggttcc acccttgccg tgaacccagg gaagtcacgg gcccggatgt gacgccactg 480
acttgcacat tggaggtcag aggacagcga gattctcgcc ctgagcaacg gcctgacgtc 540
ggcggaggga agcaggcgca ggctccgtga ggaggcaagg taagacgccg agggaggact 600
gaggcgggcc tcaccccaga cagagggccc ccaataatcc agcgctgcct ctgctgccgg 660
gcctggacca ccctgcaggg gaagacttct caggctcagt cgccaccacc tcaccccgcc 720
accccccgcc gctttaaccg cagggaactc tggcgtaaga gctttgtgtg accagggcag 780

ggctggttag aagtgctcag ggcccagact cagccaggaa tcaaggtcag gaccccaaga 840
ggggactgag ggcaacccac cccctaccct cactaccaat cccatccccc aacaccaacc 900
ccacccccat ccctcaaaca ccaaccccac ccccaaaccc cattcccatc tcctccccca 960
ccaccatcct ggcagaatcc ggctttgccc ctgcaatcaa cccacggaag ctccgggaat 1020
ggcggccaag cacgcggatc ctgacgttca catgtacggc taagggaggg aaggggttgg 1080
gtctcgtgag tatggccttt gggatgcaga ggaagggccc aggcctcctg gaagacagtg 1140
gagtccttag gggacccagc atgccaggac agggggccca ctgtacccct gtctcaaact 1200
gagccacctt ttcattcagc cgagggaatc ctagggatgc agacccactt cagcaggggg 1260
ttggggccca gcctgcgagg agtcaagggg aggaagaaga gggaggactg aggggacctt 1320
ggagtccaga tcagtggcaa ccttgggctg ggggatcctg ggcacagtgg ccgaatgtgc 1380
cccgtgctca ttgcaccttc agggtgacag agagttgagg gctgtggtct gagggctggg 1440
acttcaggtc agcagaggga ggaatcccag gatctgccgg acccaaggtg tgcccccttc 1500
atgaggactg ggataccccc cggcccagaa agaagggatg ccacagagtc tggaagtccc 1560
ttgttcttag ctctggggga acctgatcag ggatggccct aagtgacaat ctcatttgta 1620
ccacaggcag gaggttgggg aaccctcagg gagataaggt gttggtgtaa agaggagctg 1680
tctgctcatt tcagggggtt gggggttgag aaagggcagt ccctggcagg agtaaagatg 1740
agtaacccac aggaggccat cataacgttc accctagaac caaaggggtc agccctggac 1800
aacgcacgtg ggggtaacag gatgtggccc ctcctcactt gtctttccag atctcaggga 1860
gttgatgacc ttgttttcag aaggtgactc aggtcaacac aggggcccca tctggtcgac 1920
agatgcagtg gttctaggat ctgccaagca tccaggtgga gagcctgagg taggattgag 1980
ggtacccctg ggccagaatg cagcaagggg gccccataga aatctgccct gcccctgcgg 2040
ttacttcaga gaccctgggc agggctgtca gctgaagtcc ctccattatc ctgggatctt 2100
tgatgtcagg gaaggggagg ccttggtctg aaggggctgg agtcaggtca gtagagggag 2160
ggtctcaggc cctgccagga gtggacgtga ggaccaagcg gactcgtcac ccaggacacc 2220
tggactccaa tgaatttgga catctctcgt tgtccttcgc gggaggacct ggtcacgtat 2280
ggccagatgt gggtcccctc atatccttct gtaccatatc agggatgtga gttcttgaca 2340
tgagagattc tcaagccagc aaaagggtgg gattaggccc tacaaggaga aaggtgaggg 2400
ccctgagtga gcacagaggg gaccctccac ccaagtagag tggggacctc acggagtctg 2460
gccaaccctg ctgagacttc tgggaatccg tggctgtgct tgcagtctgc acactgaagg 2520
cccgtgcatt cctctcccag gaatcaggag ctccaggaac caggcagtga ggccttggtc 2580
tgagtcagtg tcctcaggtc acagagcaga ggggacgcag acagtgccaa cactgaaggt 2640
ttgcctggaa tgcacaccaa gggccccacc cgcccagaac aaatgggact ccagagggcc 2700
tggcctcacc ctccctattc tcagtcctgc agcctgagca tgtgctggcc ggctgtaccc 2760
tgaggtgccc tcccacttcc tccttcaggt tctgaggggg acaggctgac aagtaggacc 2820
cgaggcactg gaggagcatt gaaggagaag atctgtaagt aagcctttgt cagagcctcc 2880
aaggttcagt tcagttctca cctaaggcct cacacacgct ccttctctcc ccaggcctgt 2940
gggtcttcat tgcccagctc ctgcccgcac tcctgcctgc tgccctgacc agagtcatca 3000
tgcctcttga gcagaggagt cagcactgca agcctgaaga aggccttgag gcccgaggag 3060
aggccctggg cctggtgggt gcgcaggctc ctgctactga ggagcagcag accgcttctt 3120
cctcttctac tctagtggaa gttaccctgg gggaggtgcc tgctgccgac tcaccgagtc 3180
ctccccacag tcctcaggga gcctccagct tctcgactac catcaactac actctttgga 3240
gacaatccga tgagggctcc agcaaccaag aagaggaggg gccaagaatg tttcccgacc 3300
tggagtccga gttccaagca gcaatcagta ggaagatggt tgagttggtt cattttctgc 3360
tcctcaagta tcgagccagg gagccggtca caaaggcaga aatgctggag agtgtcctca 3420
gaaattgcca ggacttcttt cccgtgatct tcagcaaagc ctccgagtac ttgcagctgg 3480
tctttggcat cgaggtggtg gaagtggtcc ccatcagcca cttgtacatc cttgtcacct 3540
gcctgggcct ctcctacgat ggcctgctgg gcgacaatca ggtcatgccc aagacaggcc 3600
tcctgataat cgtcctggcc ataatcgcaa tagagggcga ctgtgcccct gaggagaaaa 3660
tctgggagga gctgagtatg ttggaggtgt ttgaggggag ggaggacagt gtcttcgcac 3720
atcccaggaa gctgctcatg caagatctgg tgcaggaaaa ctacctggag taccggcagg 3780
tgcccggcag tgatcctgca tgctacgagt tcctgtgggg tccaagggcc ctcattgaaa 3840
ccagctatgt gaaagtcctg caccatacac taaagatcgg tggagaacct cacatttcct 3900
acccacccct gcatgaacgg gctttgagag agggagaaga gtgagtctca gcacatgttg 3960
cagccagggc cagtgggagg gggtctgggc cagtgcacct tccagggccc catccattag 4020
cttccactgc ctcgtgtgat atgaggccca ttcctgcctc tttgaagaga gcagtcagca 4080
ttcttagcag tgagtttctg ttctgttgga tgactttgag atttatcttt ctttcctgtt 4140
ggaattgttc aaatgttcct tttaacaaat ggttggatga acttcagcat ccaagtttat 4200

gaatgacagt agtcacacat agtgctgttt atatagttta ggggtaagag tcctgttttt 4260
tattcagatt gggaaatcca ttccattttg tgagttgtca cataataaca gcagtggaat 4320
atgtatttgc ctatattgtg aacgaattag cagtaaaata catgatacaa ggaactcaaa 4380
agatagttaa ttcttgcctt atacctcagt ctattatgta aaattaaaaa tatgtgtatg 4440
tttttgcttc tttgagaatg caaaagaaat taaatctgaa taaattcttc ctgttcactg 4500
gctcatttct ttaccattca ctcagcatct gctctgtgga aggccctggt agtagtggg 4559

<210> 83
<211> 4204
<212> DNA
<213> Homo sapiens

<400> 83
acgcaggcag tgatgtcacc cagaccacac cccttccccc aatgccactt caggggtac 60
tcagagtcag agacttggtc tgaggggagc agaagcaatc tgcagaggat ggcggtccag 120
gctcagccag gcatcaactt caggaccctg agggatgacc gaaggccccg cccacccacc 180
cccaactccc ccgaccccac caggatctac agcctcagga cccccgtccc aatccttacc 240
ccttgcccca tcaccatctt catgcttacc tccaccccca tccgatcccc atccaggcag 300
aatccagttc cacccctgcc cggaacccag ggtagtaccg ttgccaggat gtgacgccac 360
tgacttgcgc attggaggtc agaagaccgc gagattctcg ccctgagcaa cgagcgacgg 420
cctgacgtcg gcggagggaa gccggcccag gctcggtgag gaggcaaggt aagacgctga 480
gggaggactg aggcgggcct cacctcagac agagggcctc aaataatcca gtgctgcctc 540
tgctgccggg cctgggccac cccgcagggg aagacttcca ggctgggtcg ccactacctc 600
accccgccga cccccgccgc tttagccacg gggaactctg gggacagagc ttaatgtggc 660
cagggcaggg ctggttagaa gaggtcaggg cccacgctgt ggcaggaatc aaggtcagga 720
ccccgagagg gaactgaggg cagcctaacc accaccctca ccaccattcc cgtcccccaa 780
cacccaaccc cacccccatc ccccattccc atccccaccc ccacccctat cctggcagaa 840
tccgggcttt gcccctggta tcaagtcacg gaagctccgg gaatggcggc caggcacgtg 900
agtcctgagg ttcacatcta cggctaaggg agggaagggg ttcggtatcg cgagtatggc 960
cgttgggagg cagcgaaagg gcccaggcct cctggaagac agtggagtcc tgaggggacc 1020
cagcatgcca ggacaggggg cccactgtac ccctgtctca aaccgaggca ccttttcatt 1080
cggctacggg aatcctaggg atgcagaccc acttcagcag ggggttgggg cccagccctg 1140
cgaggagtca tggggaggaa gaagagggag gactgagggg accttggagt ccagatcagt 1200
ggcaaccttg ggctggggga tgctgggcac agtggccaaa tgtgctctgt gctcattgcg 1260
ccttcagggt gaccagagag ttgagggctg tggtctgaag agtgggactt caggtcagca 1320
gagggaggaa tcccaggatc tgcagggccc aaggtgtacc cccaaggggc ccctatgtgg 1380
tggacagatg cagtggtcct aggatctgcc aagcatccag gtgaagagac tgagggagga 1440
ttgagggtac ccctgggaca gaatgcggac tgggggcccc ataaaaatct gccctgctcc 1500
tgctgttacc tcagagagcc tgggcagggc tgtcagctga ggtccctcca ttatcctagg 1560
atcactgatg tcagggaagg ggaagccttg gtctgagggg gctgcactca gggcagtaga 1620
gggaggctct cagaccctac taggagtgga ggtgaggacc aagcagtctc ctcacccagg 1680
gtacatggac ttcaataaat ttggacatct ctcgttgtcc tttccgggag gacctgggaa 1740
tgtatggcca gatgtgggtc ccctcatgtt tttctgtacc atatcaggta tgtgagttct 1800
tgacatgaga gattctcagg ccagcagaag ggagggatta ggccctataa ggagaaaggt 1860
gagggccctg agtgagcaca gaggggatcc tccaccccag tagagtgggg acctcacaga 1920
gtctggccaa ccctcctgac agttctggga atccgtggct gcgtttgctg tctgcacatt 1980
gggggcccgt ggattcctct cccaggaatc aggagctcca ggaacaaggc agtgaggact 2040
tggtctgagg cagtgtcctc aggtcacaga gtagaggggg ctcagatagt gccaacggtg 2100
aaggtttgcc ttggattcaa accaagggcc ccacctgccc cagaacacat ggactccaga 2160
gcgcctggcc tcaccctcaa tactttcagt cctgcagcct cagcatgcgc tggccggatg 2220
taccctgagg tgccctctca cttcctcctt caggttctga ggggacaggc tgacctggag 2280
gaccagaggc ccccggagga gcactgaagg agaagatctg taagtaagcc tttgttagag 2340
cctccaaggt tccattcagt actcagctga ggtctctcac atgctccctc tctccccagg 2400
ccagtgggtc tccattgccc agctcctgcc cacactcccg cctgttgccc tgaccagagt 2460
catcatgcct cttgagcaga ggagtcagca ctgcaagcct gaagaaggcc ttgaggcccg 2520
aggagaggcc ctgggcctgg tgggtgcgca ggctcctgct actgaggagc aggaggctgc 2580
ctcctcctct tctactctag ttgaagtcac cctgggggag gtgcctgctg ccgagtcacc 2640

agatcctccc cagagtcctc agggagcctc cagcctcccc actaccatga actaccctct 2700
ctggagccaa tcctatgagg actccagcaa ccaagaagag gaggggccaa gcaccttccc 2760
tgacctggag tccgagttcc aagcagcact cagtaggaag gtggccgagt tggttcattt 2820
tctgctcctc aagtatcgag ccagggagcc ggtcacaaag gcagaaatgc tggggagtgt 2880
cgtcggaaat tggcagtatt tctttcctgt gatcttcagc aaagcttcca gttccttgca 2940
gctggtcttt ggcatcgagc tgatggaagt ggaccccatc ggccacttgt acatctttgc 3000
cacctgcctg ggcctctcct acgatggcct gctgggtgac aatcagatca tgcccaaggc 3060
aggcctcctg ataatcgtcc tggccataat cgcaagagag ggcgactgtg cccctgagga 3120
gaaaatctgg gaggagctga gtgtgttaga ggtgtttgag gggagggaag acagtatctt 3180
gggggatccc aagaagctgc tcacccaaca tttcgtgcag gaaaactacc tggagtaccg 3240
gcaggtcccc ggcagtgatc ctgcatgtta tgaattcctg tggggtccaa gggccctcgt 3300
tgaaaccagc tatgtgaaag tcctgcacca tatggtaaag atcagtggag gacctcacat 3360
ttcctaccca cccctgcatg agtgggtttt gagagagggg gaagagtgag tctgagcacg 3420
agttgcagcc agggccagtg ggagggggtc tgggccagtg caccttccgg ggccgcatcc 3480
cttagtttcc actgcctcct gtgacgtgag gcccattctt cactctttga agcgagcagt 3540
cagcattctt agtagtgggt ttctgttctg ttggatgact ttgagattat tctttgtttc 3600
ctgttggagt tgttcaaatg ttccttttaa cggatggttg aatgagcgtc agcatccagg 3660
tttatgaatg acagtagtca cacatagtgc tgtttatata gtttaggagt aagagtcttg 3720
ttttttactc aaattgggaa atccattcca ttttgtgaat tgtgacataa taatagcagt 3780
ggtaaaagta tttgcttaaa attgtgagcg aattagcaat aacatacatg agataactca 3840
agaaatcaaa agatagttga ttcttgcctt gtacctcaat ctattctgta aaattaaaca 3900
aatatgcaaa ccaggatttc cttgacttct ttgagaatgc aagcgaaatt aaatctgaat 3960
aaataattct tcctcttcac tggctcgttt cttttccgtt cactcagcat ctgctctgtg 4020
ggaggccctg ggttagtagt ggggatgcta aggtaagcca gactcacgcc tacccatagg 4080
gctgtagagc ctaggacctg cagtcatata attaaggtgg tgagaagtcc tgtaagatgt 4140
agaggaaatg taagagaggg gtgagggtgt ggcgctccgg gtgagagtag tggagtgtca 4200
gtgc 4204

<210> 84
<211> 752
<212> DNA
<213> Homo sapiens

<400> 84
atcctcgtgg gccctgacct tctctctgag agccgggcag aggctccgga gccatgcagg 60
ccgaaggccg gggcacaggg ggttcgacgg gcgatgctga tggcccagga ggccctggca 120
ttcctgatgg cccagggggc aatgctggcg gcccaggaga ggcgggtgcc acgggcggca 180
gaggtccccg gggcgcaggg gcagcaaggg cctcggggcc gggaggaggc gccccgcggg 240
gtccgcatgg cggcgcggct tcagggctga atggatgctg cagatgcggg gccagggggc 300
cggagagccg cctgcttgag ttctacctcg ccatgccttt cgcgacaccc atggaagcag 360
agctggcccg caggagcctg gcccaggatg ccccaccgct tcccgtgcca ggggtgcttc 420
tgaaggagtt cactgtgtcc ggcaacatac tgactatccg actgactgct gcagaccacc 480
gccaactgca gctctccatc agctcctgtc tccagcagct ttccctgttg atgtggatca 540
cgcagtgctt tctgcccgtg tttttggctc agcctccctc agggcagagg cgctaagccc 600
agcctggcgc cccttcctag gtcatgcctc ctcccctagg gaatggtccc agcacgagtg 660
gccagttcat tgtgggggcc tgattgtttg tcgctggagg aggacggctt acatgtttgt 720
ttctgtagaa aataaaactg agctacgaaa aa 752

<210> 85
<211> 2148
<212> DNA
<213> Homo sapiens

<220>
<221> misc_feature
<222> (1)...(2)
<223> n = A,T,C or G

<400> 85
gcttcagggt acagctcccc cgcagccaga agccgggcct gcagcccctc agcaccgctc 60
cgggacaccc cacccgcttc ccaggcgtga cctgtcaaca gcaacttcgc ggtgtggtga 120
actctctgag gaaaaaccat tttgattatt actctcagac gtgcgtggca acaagtgact 180
gagacctaga aatccaagcg ttggaggtcc tgaggccagc ctaagtcgct tcaaaatgga 240
acgaaggcgt ttgtggggtt ccattcagag ccgatacatc agcatgagtg tgtggacaag 300
cccacggaga cttgtggagc tggcagggca gagcctgctg aaggatgagg ccctggccat 360
tgccgccctg gagttgctgc ccagggagct cttcccgcca ctcttcatgg cagcctttga 420
cgggagacac agccagaccc tgaaggcaat ggtgcaggcc tggcccttca cctgcctccc 480
tctgggagtg ctgatgaagg gacaacatct tcacctggag accttcaaag ctgtgcttga 540
tggacttgat gtgctccttg cccaggaggt tcgccccagg aggtggaaac ttcaagtgct 600
ggatttacgg aagaactctc atcaggactt ctggactgta tggtctggaa acagggccag 660
tctgtactca tttccagagc cagaagcagc tcagcccatg acaaagaagc gaaaagtaga 720
tggtttgagc acagaggcag agcagccctt cattccagta gaggtgctcg tagacctgtt 780
cctcaaggaa ggtgcctgtg atgaattgtt ctcctacctc attgagaaag tgaagcgaaa 840
gaaaaatgta ctacgcctgt gctgtaagaa gctgaagatt tttgcaatgc ccatgcagga 900
tatcaagatg atcctgaaaa tggtgcagct ggactctatt gaagatttgg aagtgacttg 960
tacctggaag ctacccacct tggcgaaatt ttctccttac ctgggccaga tgattaatct 1020
gcgtagactc ctcctctccc acatccatgc atcttcctac atttccccgg agaaggaaga 1080
gcagtatatc gcccagttca cctctcagtt cctcagtctg cagtgcctgc aggctctcta 1140
tgtggactct ttatttttcc ttagaggccg cctggatcag ttgctcaggc acgtgatgaa 1200
cccccttgga accctctcaa taactaactg ccggctttcg gaaggggatg tgatgcatct 1260
gtcccagagt cccagcgtca gtcagctaag tgtcctgagt ctaagtgggg tcatgctgac 1320
cgatgtaagt cccgagcccc tccaagctct gctggagaga gcctctgcca ccctccagga 1380
cctggtcttt gatgagtgtg ggatcacgga tgatcagctc cttgccctcc tgccttccct 1440
gagccactgc tcccagctta caaccttaag cttctacggg aattccatct ccatatctgc 1500
cttgcagagt ctcctgcagc acctcatcgg gctgagcaat ctgacccacg tgctgtatcc 1560
tgtccccctg gagagttatg aggacatcca tggtaccctc cacctggaga ggcttgccta 1620
tctgcatgcc aggctcaggg agttgctgtg tgagttgggg cggcccagca tggtctggct 1680
tagtgccaac ccctgtcctc actgtgggga cagaaccttc tatgacccgg agcccatcct 1740
gtgcccctgt ttcatgccta actagctggg tgcacatatc aaatgcttca ttctgcatac 1800
ttggacacta aagccaggat gtgcatgcat cttgaagcaa caaagcagcc acagtttcag 1860
acaaatgttc agtgtgagtg aggaaaacat gttcagtgag gaaaaaacat tcagacaaat 1920
gttcagtgag gaaaaaaagg ggaagttggg gataggcaga tgttgacttg aggagttaat 1980
gtgatctttg gggagataca tcttatagag ttagaaatag aatctgaatt tctaaaggga 2040
gattctggct tgggaagtac atgtaggagt taatccctgt gtagactgtt gtaaagaaac 2100
tgttgaaaat aaagagaagc aatgtgaagc aaaaaaaaaa aaaaaaaa 2148

<210> 86
<211> 1466
<212> DNA
<213> Homo sapiens

<400> 86
agccccaagc ttaccacctg cacccggaga gctgtgtgtc accatgtggg tcccggttgt 60
cttcctcacc ctgtccgtga cgtggattgg tgctgcaccc ctcatcctgt ctcggattgt 120
gggaggctgg gagtgcgaga agcattccca accctggcag gtgcttgtgg cctctcgtgg 180
cagggcagtc tgcggcggtg ttctggtgca cccccagtgg gtcctcacag ctgcccactg 240
catcaggaac aaaagcgtga tcttgctggg tcggcacagc ctgtttcatc ctgaagacac 300
aggccaggta tttcaggtca gccacagctt cccacacccg ctctacgata tgagcctcct 360
gaagaatcga ttcctcaggc caggtgatga ctccagccac gacctcatgc tgctccgcct 420
gtcagagcct gccgagctca cggatgctgt gaaggtcatg gacctgccca cccaggagcc 480
agcactgggg accacctgct acgcctcagg ctggggcagc attgaaccag aggagttctt 540
gaccccaaag aaacttcagt gtgtggacct ccatgttatt tccaatgacg tgtgtgcgca 600
agttcaccct cagaaggtga ccaagttcat gctgtgtgct ggacgctgga cagggggcaa 660
aagcacctgc tcgggtgatt ctgggggccc acttgtctgt aatggtgtgc ttcaaggtat 720

cacgtcatgg ggcagtgaac catgtgccct gcccgaaagg ccttccctgt acaccaaggt 780
ggtgcattac cggaagtgga tcaaggacac catcgtggcc aacccctgag cacccctatc 840
aaccccctat tgtagtaaac ttggaacctt ggaaatgacc aggccaagac tcaagcctcc 900
ccagttctac tgacctttgt ccttaggtgt gaggtccagg gttgctagga aaagaaatca 960
gcagacacag gtgtagacca gagtgtttct taaatggtgt aattttgtcc tctctgtgtc 1020
ctggggaata ctggccatgc ctggagacat atcactcaat ttctctgagg acacagatag 1080
gatggggtgt ctgtgttatt tgtggggtac agagatgaaa gaggggtggg atccacactg 1140
agagagtgga gagtgacatg tgctggacac tgtccatgaa gcactgagca gaagctggag 1200
gcacaacgca ccagacactc acagcaagga tggagctgaa aacataaccc actctgtcct 1260
ggaggcactg ggaagcctag agaaggctgt gagccaagga gggagggtct tcctttggca 1320
tgggatgggg atgaagtaag gagagggact ggaccccctg gaagctgatt cactatgggg 1380
ggaggtgtat tgaagtcctc cagacaaccc tcagatttga tgatttccta gtagaactca 1440
cagaaataaa gagctgttat actgtg 1466

<210> 87
<211> 990
<212> DNA
<213> Homo sapiens

<220>
<221> misc_feature
<222> (1)...(990)
<223> n = A,T,C or G

<400> 87
agggagaggc agtgaccatg aaggctgtgc tgcttgccct gttgatggca ggcttggccc 60
tgcagccagg cactgccctg ctgtgctact cctgcaaagc ccaggtgagc aacgaggact 120
gcctgcaggt ggagaactgc acccagctgg gggagcagtg ctggaccgcg cgcatccgcg 180
cagttggcct cctgaccgtc atcagcaaag gctgcagctt gaactgcgtg gatgactcac 240
aggactacta cgtgggcaag aagaacatca cgtgctgtga caccgacttg tgcaacgcca 300
gcggggccca tgccctgcag ccggctgccg ccatccttgc gctgctccct gcactcggcc 360
tgctgctctg gggacccggc cagctatagg ctctgggggg ccccgctgca gcccacactg 420
ggtgtggtgc cccaggcctt tgtgccactc ctcacagaac ctggcccagt gggagcctgt 480
cctggttcct gaggcacatc ctaacgcaag tttgaccatg tatgtttgca ccccttttcc 540
ccnaaccctg accttcccat gggccttttc caggattccn accnggcaga tcagttttag 600
tganacanat ccgcntgcag atggcccctc caaccntttn tgttgntgtt tccatggccc 660
agcattttcc acccttaacc ctgtgttcag gcacttnttc ccccaggaag ccttccctgc 720
ccaccccatt tatgaattga gccaggtttg gtccgtggtg tcccccgcac ccagcagggg 780
acaggcaatc aggagggccc agtaaaggct gagatgaagt ggactgagta gaactggagg 840
acaagagttg acgtgagttc ctgggagttt ccagagatgg ggcctggagg cctggaggaa 900
ggggccaggc ctcacatttg tggggntccc gaatggcagc ctgagcacag cgtaggccct 960
taataaacac ctgttggata agccaaaaaa 990

<210> 88
<211> 9
<212> PRT
<213> Homo sapiens

<400> 88
Leu Pro His Ser Ser Ser His Trp Leu
1               5

<210> 89
<211> 10
<212> PRT
<213> Homo sapiens

<400> 89
Gln Leu Pro His Ser Ser Ser His Trp Leu
1               5                   10


<210> 90
<211> 9
<212> PRT
<213> Homo sapiens

<400> 90
Leu Ile Tyr Arg Arg Arg Leu Met Lys
1               5


<210> 91
<211> 10
<212> PRT
<213> Homo sapiens

<400> 91
Ser Leu Ile Tyr Arg Arg Arg Leu Met Lys
1               5                   10


<210> 92
<211> 8
<212> PRT
<213> Homo sapiens

<400> 92
Ile Tyr Arg Arg Arg Leu Met Lys
1               5


<210> 93
<211> 9
<212> PRT
<213> Homo sapiens

<400> 93
Leu Pro His Ser Ser Ser His Trp Leu
1               5


<210> 94
<211> 10
<212> PRT
<213> Homo sapiens

<400> 94
Gln Leu Pro His Ser Ser Ser His Trp Leu
1               5                   10


<210> 95

<211> 8
<212> PRT
<213> Homo sapiens

<400> 95
Glu Ser Leu Phe Arg Ala Val Ile
1               5


<210> 96
<211> 10
<212> PRT
<213> Homo sapiens

<400> 96
Ile Leu Glu Ser Leu Phe Arg Ala Val Ile
1               5                   10


<210> 97
<211> 9
<212> PRT
<213> Homo sapiens

<400> 97
Ile Leu Glu Ser Leu Phe Arg Ala Val
1               5


<210> 98
<211> 10
<212> PRT
<213> Homo sapiens

<400> 98
Cys Ile Leu Glu Ser Leu Phe Arg Ala Val
1               5                   10


<210> 99
<211> 9
<212> PRT
<213> Homo sapiens

<400> 99
Cys Ile Leu Glu Ser Leu Phe Arg Ala
1               5


<210> 100
<211> 9
<212> PRT
<213> Homo sapiens

<400> 100
Glu Phe Leu Trp Gly Pro Arg Ala Leu
1               5

<210> 101
<211> 8
<212> PRT
<213> Homo sapiens

<400> 101
Phe Leu Trp Gly Pro Arg Ala Leu
1               5


<210> 102
<211> 10
<212> PRT
<213> Homo sapiens

<400> 102
Phe Leu Trp Gly Pro Arg Ala Leu Ala Glu
1               5                   10


<210> 103
<211> 10
<212> PRT
<213> Homo sapiens

<400> 103
Leu Trp Gly Pro Arg Ala Leu Ala Glu Thr
1               5                   10


<210> 104
<211> 9
<212> PRT
<213> Homo sapiens

<400> 104
Pro Arg Ala Leu Ala Glu Thr Ser Tyr
1               5


<210> 105
<211> 10
<212> PRT
<213> Homo sapiens

<400> 105
Gly Pro Arg Ala Leu Ala Glu Thr Ser Tyr
1               5                   10


<210> 106
<211> 9
<212> PRT
<213> Homo sapiens

<400> 106
Arg Ala Leu Ala Glu Thr Ser Tyr Val
1               5

<210> 107
<211> 9
<212> PRT
<213> Homo sapiens

<400> 107
Leu Ala Glu Thr Ser Tyr Val Lys Val
1               5

<210> 108
<211> 10
<212> PRT
<213> Homo sapiens

<400> 108
Ala Leu Ala Glu Thr Ser Tyr Val Lys Val
1               5                   10

<210> 109
<211> 9
<212> PRT
<213> Homo sapiens

<400> 109
Ala Glu Thr Ser Tyr Val Lys Val Leu
1               5

<210> 110
<211> 10
<212> PRT
<213> Homo sapiens

<400> 110
Leu Ala Glu Thr Ser Tyr Val Lys Val Leu
1               5                   10

<210> 111
<211> 9
<212> PRT
<213> Homo sapiens

<400> 111
Thr Ser Tyr Val Lys Val Leu Glu Tyr
1               5

<210> 112
<211> 10

<212> PRT
<213> Homo sapiens

<400> 112
Glu Thr Ser Tyr Val Lys Val Leu Glu Tyr
1               5                   10


<210> 113
<211> 9
<212> PRT
<213> Homo sapiens

<400> 113
Lys Val Leu Glu Tyr Val Ile Lys Val
1               5


<210> 114
<211> 10
<212> PRT
<213> Homo sapiens

<400> 114
Ser Tyr Val Leu Val Thr Cys Leu Gly Leu
1               5                   10


<210> 115
<211> 9
<212> PRT
<213> Homo sapiens

<400> 115
Tyr Val Leu Val Thr Cys Leu Gly Leu
1               5


<210> 116
<211> 8
<212> PRT
<213> Homo sapiens

<400> 116
Val Leu Val Thr Cys Leu Gly Leu
1               5


<210> 117
<211> 9
<212> PRT
<213> Homo sapiens

<400> 117
Thr Gln Asp Leu Val Gln Glu Lys Tyr
1               5

<210> 118
<211> 10
<212> PRT
<213> Homo sapiens

<400> 118
Leu Thr Gln Asp Leu Val Gln Glu Lys Tyr
1               5                   10


<210> 119
<211> 9
<212> PRT
<213> Homo sapiens

<400> 119
Tyr Gly Glu Pro Arg Lys Leu Leu Thr
1               5


<210> 120
<211> 9
<212> PRT
<213> Homo sapiens

<400> 120
Leu Val Gln Glu Lys Tyr Leu Glu Tyr
1               5


<210> 121
<211> 10
<212> PRT
<213> Homo sapiens

<400> 121
Asp Leu Val Gln Glu Lys Tyr Leu Glu Tyr
1               5                   10


<210> 122
<211> 9
<212> PRT
<213> Homo sapiens

<400> 122
Ser Ala Tyr Gly Glu Pro Arg Lys Leu
1               5


<210> 123
<211> 9
<212> PRT
<213> Homo sapiens

<400> 123

Lys Val Leu Glu Tyr Val Ile Lys Val
1               5

<210> 124
<211> 10
<212> PRT
<213> Homo sapiens

<400> 124
Val Lys Val Leu Glu Tyr Val Ile Lys Val
1               5                   10

<210> 125
<211> 9
<212> PRT
<213> Homo sapiens

<400> 125
Tyr Val Lys Val Leu Glu Tyr Val Ile
1               5

<210> 126
<211> 9
<212> PRT
<213> Homo sapiens

<400> 126
Thr Ser Tyr Val Lys Val Leu Glu Tyr
1               5

<210> 127
<211> 10
<212> PRT
<213> Homo sapiens

<400> 127
Glu Thr Ser Tyr Val Lys Val Leu Glu Tyr
1               5                   10

<210> 128
<211> 9
<212> PRT
<213> Homo sapiens

<400> 128
Val Ile Lys Val Ser Ala Arg Val Arg
1               5

<210> 129
<211> 10
<212> PRT

<213> Homo sapiens

<400> 129
Tyr Val Ile Lys Val Ser Ala Arg Val Arg
1               5                   10


<210> 130
<211> 8
<212> PRT
<213> Homo sapiens

<400> 130
Glu Leu Val His Phe Leu Leu Leu
1               5


<210> 131
<211> 10
<212> PRT
<213> Homo sapiens

<400> 131
Met Val Glu Leu Val His Phe Leu Leu Leu
1               5                   10


<210> 132
<211> 8
<212> PRT
<213> Homo sapiens

<400> 132
Ile Ser Arg Lys Met Val Glu Leu
1               5


<210> 133
<211> 9
<212> PRT
<213> Homo sapiens

<400> 133
Ala Ile Ser Arg Lys Met Val Glu Leu
1               5


<210> 134
<211> 10
<212> PRT
<213> Homo sapiens

<400> 134
Ala Ala Ile Ser Arg Lys Met Val Glu Leu
1               5                   10

<210> 135
<211> 9
<212> PRT
<213> Homo sapiens

<400> 135
Lys Met Val Glu Leu Val His Phe Leu
1               5


<210> 136
<211> 9
<212> PRT
<213> Homo sapiens

<400> 136
Ile Ser Arg Lys Met Val Glu Leu Val
1               5


<210> 137
<211> 10
<212> PRT
<213> Homo sapiens

<400> 137
Ala Ile Ser Arg Lys Met Val Glu Leu Val
1               5                   10


<210> 138
<211> 9
<212> PRT
<213> Homo sapiens

<400> 138
Leu Val His Phe Leu Leu Leu Lys Tyr
1               5


<210> 139
<211> 10
<212> PRT
<213> Homo sapiens

<400> 139
Glu Leu Val His Phe Leu Leu Leu Lys Tyr
1               5                   10


<210> 140
<211> 9
<212> PRT
<213> Homo sapiens

<400> 140
Arg Lys Met Val Glu Leu Val His Phe

1                   5


<210> 141
<211> 9
<212> PRT
<213> Homo sapiens

<400> 141
Leu Gln Leu Val Phe Gly Ile Glu Val
1                   5


<210> 142
<211> 10
<212> PRT
<213> Homo sapiens

<400> 142
Tyr Leu Gln Leu Val Phe Gly Ile Glu Val
1                   5                   10


<210> 143
<211> 9
<212> PRT
<213> Homo sapiens

<400> 143
Gln Leu Val Phe Gly Ile Glu Val Val
1                   5


<210> 144
<211> 10
<212> PRT
<213> Homo sapiens

<400> 144
Leu Gln Leu Val Phe Gly Ile Glu Val Val
1                   5                   10


<210> 145
<211> 9
<212> PRT
<213> Homo sapiens

<400> 145
Ile Glu Val Val Glu Val Val Pro Ile
1                   5


<210> 146
<211> 10
<212> PRT
<213> Homo sapiens

<400> 146
Gly Ile Glu Val Val Glu Val Val Pro Ile
1               5                   10


<210> 147
<211> 9
<212> PRT
<213> Homo sapiens

<400> 147
Phe Gly Ile Glu Val Val Glu Val Val
1               5


<210> 148
<211> 9
<212> PRT
<213> Homo sapiens

<400> 148
Ala Ser Glu Tyr Leu Gln Leu Val Phe
1               5


<210> 149
<211> 10
<212> PRT
<213> Homo sapiens

<400> 149
Lys Ala Ser Glu Tyr Leu Gln Leu Val Phe
1               5                   10


<210> 150
<211> 8
<212> PRT
<213> Homo sapiens

<400> 150
Glu Glu Lys Ile Trp Glu Glu Leu
1               5


<210> 151
<211> 10
<212> PRT
<213> Homo sapiens

<400> 151
Ala Pro Glu Glu Lys Ile Trp Glu Glu Leu
1               5                   10


<210> 152

<211> 8
<212> PRT
<213> Homo sapiens

<400> 152
Ala Pro Glu Glu Lys Ile Trp Glu
1               5


<210> 153
<211> 9
<212> PRT
<213> Homo sapiens

<400> 153
Lys Ile Trp Glu Glu Leu Ser Met Leu
1               5


<210> 154
<211> 10
<212> PRT
<213> Homo sapiens

<400> 154
Glu Lys Ile Trp Glu Glu Leu Ser Met Leu
1               5                   10


<210> 155
<211> 8
<212> PRT
<213> Homo sapiens

<400> 155
Phe Leu Trp Gly Pro Arg Ala Leu
1               5


<210> 156
<211> 9
<212> PRT
<213> Homo sapiens

<400> 156
Phe Leu Trp Gly Pro Arg Ala Leu Ile
1               5


<210> 157
<211> 9
<212> PRT
<213> Homo sapiens

<400> 157
Leu Ile Glu Thr Ser Tyr Val Lys Val
1               5

<210> 158
<211> 10
<212> PRT
<213> Homo sapiens

<400> 158
Ala Leu Ile Glu Thr Ser Tyr Val Lys Val
1               5                   10


<210> 159
<211> 9
<212> PRT
<213> Homo sapiens

<400> 159
Arg Ala Leu Ile Glu Thr Ser Tyr Val
1               5


<210> 160
<211> 9
<212> PRT
<213> Homo sapiens

<400> 160
Ile Glu Thr Ser Tyr Val Lys Val Leu
1               5


<210> 161
<211> 10
<212> PRT
<213> Homo sapiens

<400> 161
Leu Ile Glu Thr Ser Tyr Val Lys Val Leu
1               5                   10


<210> 162
<211> 8
<212> PRT
<213> Homo sapiens

<400> 162
Phe Leu Trp Gly Pro Arg Ala Leu
1               5


<210> 163
<211> 9
<212> PRT
<213> Homo sapiens

<400> 163
Glu Phe Leu Trp Gly Pro Arg Ala Leu
1               5

<210> 164
<211> 9
<212> PRT
<213> Homo sapiens

<400> 164
Phe Leu Trp Gly Pro Arg Ala Leu Val
1               5

<210> 165
<211> 9
<212> PRT
<213> Homo sapiens

<400> 165
Arg Ala Leu Val Glu Thr Ser Tyr Val
1               5

<210> 166
<211> 9
<212> PRT
<213> Homo sapiens

<400> 166
Leu Trp Gly Pro Arg Ala Leu Val Glu
1               5

<210> 167
<211> 10
<212> PRT
<213> Homo sapiens

<400> 167
Phe Leu Trp Gly Pro Arg Ala Leu Val Glu
1               5                   10

<210> 168
<211> 10
<212> PRT
<213> Homo sapiens

<400> 168
Leu Trp Gly Pro Arg Ala Leu Val Glu Thr
1               5                   10

<210> 169
<211> 9

<212> PRT
<213> Homo sapiens

<400> 169
Gly Pro Glu Ser Arg Leu Leu Glu Phe
1               5


<210> 170
<211> 9
<212> PRT
<213> Homo sapiens

<400> 170
Pro Glu Ser Arg Leu Leu Glu Phe Tyr
1               5


<210> 171
<211> 10
<212> PRT
<213> Homo sapiens

<400> 171
Gly Pro Glu Ser Arg Leu Leu Glu Phe Tyr
1               5                   10


<210> 172
<211> 9
<212> PRT
<213> Homo sapiens

<400> 172
Glu Ser Arg Leu Leu Glu Phe Tyr Leu
1               5


<210> 173
<211> 9
<212> PRT
<213> Homo sapiens

<400> 173
Arg Leu Leu Glu Phe Tyr Leu Ala Met
1               5


<210> 174
<211> 9
<212> PRT
<213> Homo sapiens

<400> 174
Leu Glu Phe Tyr Leu Ala Met Pro Phe
1               5

<210> 175
<211> 10
<212> PRT
<213> Homo sapiens

<400> 175
Leu Leu Glu Phe Tyr Leu Ala Met Pro Phe
1               5                   10


<210> 176
<211> 10
<212> PRT
<213> Homo sapiens

<400> 176
Ala Met Pro Phe Ala Thr Pro Met Glu Ala
1               5                   10


<210> 177
<211> 9
<212> PRT
<213> Homo sapiens

<400> 177
Met Pro Phe Ala Thr Pro Met Glu Ala
1               5


<210> 178
<211> 9
<212> PRT
<213> Homo sapiens

<400> 178
Pro Leu Pro Val Pro Gly Val Leu Leu
1               5


<210> 179
<211> 10
<212> PRT
<213> Homo sapiens

<400> 179
Pro Pro Leu Pro Val Pro Gly Val Leu Leu
1               5                   10


<210> 180
<211> 8
<212> PRT
<213> Homo sapiens

<400> 180

Leu Pro Val Pro Gly Val Leu Leu
1               5

<210> 181
<211> 10
<212> PRT
<213> Homo sapiens

<400> 181
Glu Leu Ala Arg Arg Ser Leu Ala Gln Asp
1               5                   10

<210> 182
<211> 9
<212> PRT
<213> Homo sapiens

<400> 182
Val Pro Gly Val Leu Leu Lys Glu Phe
1               5

<210> 183
<211> 10
<212> PRT
<213> Homo sapiens

<400> 183
Pro Val Pro Gly Val Leu Leu Lys Glu Phe
1               5                   10

<210> 184
<211> 8
<212> PRT
<213> Homo sapiens

<400> 184
Leu Pro Val Pro Gly Val Leu Leu
1               5

<210> 185
<211> 9
<212> PRT
<213> Homo sapiens

<400> 185
Thr Val Ser Gly Asn Ile Leu Thr Ile
1               5

<210> 186
<211> 10
<212> PRT

<213> Homo sapiens

<400> 186
Phe Thr Val Ser Gly Asn Ile Leu Thr Ile
1               5                   10


<210> 187
<211> 9
<212> PRT
<213> Homo sapiens

<400> 187
Gly Val Leu Leu Lys Glu Phe Thr Val
1               5


<210> 188
<211> 10
<212> PRT
<213> Homo sapiens

<400> 188
Val Leu Leu Lys Glu Phe Thr Val Ser Gly
1               5                   10


<210> 189
<211> 9
<212> PRT
<213> Homo sapiens

<400> 189
Leu Leu Lys Glu Phe Thr Val Ser Gly
1               5


<210> 190
<211> 9
<212> PRT
<213> Homo sapiens

<400> 190
Val Pro Gly Val Leu Leu Lys Glu Phe
1               5


<210> 191
<211> 10
<212> PRT
<213> Homo sapiens

<400> 191
Pro Val Pro Gly Val Leu Leu Lys Glu Phe
1               5                   10

<210> 192
<211> 9
<212> PRT
<213> Homo sapiens

<400> 192
Ala Ala Asp His Arg Gln Leu Gln Leu
1               5


<210> 193
<211> 9
<212> PRT
<213> Homo sapiens

<400> 193
Ser Ile Ser Ser Cys Leu Gln Gln Leu
1               5


<210> 194
<211> 10
<212> PRT
<213> Homo sapiens

<400> 194
Leu Ser Ile Ser Ser Cys Leu Gln Gln Leu
1               5                   10


<210> 195
<211> 10
<212> PRT
<213> Homo sapiens

<400> 195
Thr Ala Ala Asp His Arg Gln Leu Gln Leu
1               5                   10


<210> 196
<211> 9
<212> PRT
<213> Homo sapiens

<400> 196
Trp Ile Thr Gln Cys Phe Leu Pro Val
1               5


<210> 197
<211> 9
<212> PRT
<213> Homo sapiens

<400> 197
Ser Leu Leu Met Trp Ile Thr Gln Cys

1                   5


<210> 198
<211> 9
<212> PRT
<213> Homo sapiens

<400> 198
Ser Ser Cys Leu Gln Gln Leu Ser Leu
1                   5


<210> 199
<211> 9
<212> PRT
<213> Homo sapiens

<400> 199
Gln Gln Leu Ser Leu Leu Met Trp Ile
1                   5


<210> 200
<211> 9
<212> PRT
<213> Homo sapiens

<400> 200
Ser Cys Leu Gln Gln Leu Ser Leu Leu
1                   5


<210> 201
<211> 10
<212> PRT
<213> Homo sapiens

<400> 201
Ser Ser Cys Leu Gln Gln Leu Ser Leu Leu
1                   5                   10


<210> 202
<211> 9
<212> PRT
<213> Homo sapiens

<400> 202
Thr Gln Cys Phe Leu Pro Val Phe Leu
1                   5


<210> 203
<211> 10
<212> PRT
<213> Homo sapiens

<400> 203
Ile Thr Gln Cys Phe Leu Pro Val Phe Leu
1               5                   10


<210> 204
<211> 9
<212> PRT
<213> Homo sapiens

<400> 204
Pro Met Gln Asp Ile Lys Met Ile Leu
1               5


<210> 205
<211> 10
<212> PRT
<213> Homo sapiens

<400> 205
Met Pro Met Gln Asp Ile Lys Met Ile Leu
1               5                   10


<210> 206
<211> 9
<212> PRT
<213> Homo sapiens

<400> 206
Gln His Leu Ile Gly Leu Ser Asn Leu
1               5


<210> 207
<211> 10
<212> PRT
<213> Homo sapiens

<400> 207
Leu Gln His Leu Ile Gly Leu Ser Asn Leu
1               5                   10


<210> 208
<211> 8
<212> PRT
<213> Homo sapiens

<400> 208
His Leu Ile Gly Leu Ser Asn Leu
1               5


<210> 209

<211> 9
<212> PRT
<213> Homo sapiens

<400> 209
Ile Gly Leu Ser Asn Leu Thr His Val
1               5


<210> 210
<211> 10
<212> PRT
<213> Homo sapiens

<400> 210
Leu Ile Gly Leu Ser Asn Leu Thr His Val
1               5                   10


<210> 211
<211> 9
<212> PRT
<213> Homo sapiens

<400> 211
Val Leu Val His Pro Gln Trp Val Leu
1               5


<210> 212
<211> 10
<212> PRT
<213> Homo sapiens

<400> 212
Gly Val Leu Val His Pro Gln Trp Val Leu
1               5                   10


<210> 213
<211> 9
<212> PRT
<213> Homo sapiens

<400> 213
Gly Val Leu Val His Pro Gln Trp Val
1               5


<210> 214
<211> 9
<212> PRT
<213> Homo sapiens

<400> 214
Trp Val Leu Thr Ala Ala His Cys Ile
1               5

<210> 215
<211> 10
<212> PRT
<213> Homo sapiens

<400> 215
Leu Val His Pro Gln Trp Val Leu Thr Ala
1               5                   10


<210> 216
<211> 10
<212> PRT
<213> Homo sapiens

<400> 216
Val Leu Val His Pro Gln Trp Val Leu Thr
1               5                   10


<210> 217
<211> 9
<212> PRT
<213> Homo sapiens

<400> 217
Leu Val His Pro Gln Trp Val Leu Thr
1               5


<210> 218
<211> 8
<212> PRT
<213> Homo sapiens

<400> 218
Cys Ile Arg Asn Lys Ser Val Ile
1               5


<210> 219
<211> 9
<212> PRT
<213> Homo sapiens

<400> 219
His Cys Ile Arg Asn Lys Ser Val Ile
1               5


<210> 220
<211> 9
<212> PRT
<213> Homo sapiens

<400> 220
His Pro Gln Trp Val Leu Thr Ala Ala
1               5


<210> 221
<211> 10
<212> PRT
<213> Homo sapiens

<400> 221
Ala Ala His Cys Ile Arg Asn Lys Ser Val
1               5                   10


<210> 222
<211> 8
<212> PRT
<213> Homo sapiens

<400> 222
Leu Leu Trp Gly Pro Gly Gln Leu
1               5


<210> 223
<211> 9
<212> PRT
<213> Homo sapiens

<400> 223
Leu Leu Leu Trp Gly Pro Gly Gln Leu
1               5


<210> 224
<211> 10
<212> PRT
<213> Homo sapiens

<400> 224
Gly Leu Leu Leu Trp Gly Pro Gly Gln Leu
1               5                   10


<210> 225
<211> 9
<212> PRT
<213> Homo sapiens

<400> 225
Ala Leu Gln Pro Ala Ala Ala Ile Leu
1               5


<210> 226
<211> 10

<212> PRT
<213> Homo sapiens

<400> 226
His Ala Leu Gln Pro Ala Ala Ala Ile Leu
1               5                   10


<210> 227
<211> 10
<212> PRT
<213> Homo sapiens

<400> 227
Ala Pro Glu Lys Asp Lys Phe Phe Ala Tyr
1               5                   10


<210> 228
<211> 9
<212> PRT
<213> Homo sapiens

<400> 228
Pro Glu Lys Asp Lys Phe Phe Ala Tyr
1               5


<210> 229
<211> 9
<212> PRT
<213> Homo sapiens

<400> 229
Glu Lys Asp Lys Phe Phe Ala Tyr Leu
1               5


<210> 230
<211> 8
<212> PRT
<213> Homo sapiens

<400> 230
Lys Asp Lys Phe Phe Ala Tyr Leu
1               5


<210> 231
<211> 9
<212> PRT
<213> Homo sapiens

<400> 231
Pro Ala Phe Leu Pro Trp His Arg Leu
1               5

<210> 232
<211> 10
<212> PRT
<213> Homo sapiens

<400> 232
Ala Pro Ala Phe Leu Pro Trp His Arg Leu
1               5                   10


<210> 233
<211> 10
<212> PRT
<213> Homo sapiens

<400> 233
Phe Leu Leu Arg Trp Glu Gln Glu Ile Gln
1               5                   10


<210> 234
<211> 9
<212> PRT
<213> Homo sapiens

<400> 234
Arg Leu Phe Leu Leu Arg Trp Glu Gln
1               5


<210> 235
<211> 10
<212> PRT
<213> Homo sapiens

<400> 235
Gly Ser Glu Ile Trp Arg Asp Ile Asp Phe
1               5                   10


<210> 236
<211> 9
<212> PRT
<213> Homo sapiens

<400> 236
Ser Glu Ile Trp Arg Asp Ile Asp Phe
1               5


<210> 237
<211> 9
<212> PRT
<213> Homo sapiens

<400> 237

Arg Ile Trp Ser Trp Leu Leu Gly Ala
1               5

<210> 238
<211> 9
<212> PRT
<213> Homo sapiens

<400> 238
Ser Trp Leu Leu Gly Ala Ala Met Val
1               5

<210> 239
<211> 10
<212> PRT
<213> Homo sapiens

<400> 239
Trp Leu Leu Gly Ala Ala Met Val Gly Ala
1               5                   10

<210> 240
<211> 9
<212> PRT
<213> Homo sapiens

<400> 240
Leu Leu Gly Ala Ala Met Val Gly Ala
1               5

<210> 241
<211> 9
<212> PRT
<213> Homo sapiens

<400> 241
Leu Leu His Glu Thr Asp Ser Ala Val
1               5

<210> 242
<211> 9
<212> PRT
<213> Homo sapiens

<400> 242
Ala Thr Ala Arg Arg Pro Arg Trp Leu
1               5

<210> 243
<211> 9
<212> PRT

<213> Homo sapiens

<400> 243
Thr Pro Lys His Asn Met Lys Ala Phe
1               5

<210> 244
<211> 10
<212> PRT
<213> Homo sapiens

<400> 244
Glu Leu Lys Ala Glu Asn Ile Lys Lys Phe
1               5                   10

<210> 245
<211> 9
<212> PRT
<213> Homo sapiens

<220>
<221> VARIANT
<222> (7)...(7)
<223> Xaa= His or Tyr

<400> 245
Asn Ile Lys Lys Phe Leu Xaa Asn Phe
1               5

<210> 246
<211> 10
<212> PRT
<213> Homo sapiens

<220>
<221> VARIANT
<222> (8)...(8)
<223> Xaa = His or Tyr

<400> 246
Glu Asn Ile Lys Lys Phe Leu Xaa Asn Phe
1               5                   10

<210> 247
<211> 9
<212> PRT
<213> Homo sapiens

<400> 247
Ala Gly Ala Lys Gly Val Ile Leu Tyr
1               5

<210> 248
<211> 10
<212> PRT
<213> Homo sapiens

<400> 248
Pro Leu Met Tyr Ser Leu Val His Asn Leu
1               5                   10


<210> 249
<211> 9
<212> PRT
<213> Homo sapiens

<400> 249
Leu Met Tyr Ser Leu Val His Asn Leu
1               5


<210> 250
<211> 9
<212> PRT
<213> Homo sapiens

<400> 250
Arg Val Asp Cys Thr Pro Leu Met Tyr
1               5


<210> 251
<211> 9
<212> PRT
<213> Homo sapiens

<400> 251
Asp Cys Thr Pro Leu Met Tyr Ser Leu
1               5


<210> 252
<211> 9
<212> PRT
<213> Homo sapiens

<400> 252
Ser Gly Met Pro Arg Ile Ser Lys Leu
1               5


<210> 253
<211> 10
<212> PRT
<213> Homo sapiens

<400> 253
Phe Ser Gly Met Pro Arg Ile Ser Lys Leu

1               5                   10


<210> 254
<211> 28
<212> PRT
<213> Homo sapiens

<400> 254
Arg Leu Thr Ala Ala Asp His Arg Gln Leu Gln Leu Ser Ile Ser Ser
1               5                   10                  15
Cys Leu Gln Gln Leu Ser Leu Leu Met Trp Ile Thr
            20                  25


<210> 255
<211> 28
<212> PRT
<213> Homo sapiens

<400> 255
Ser Ser Cys Leu Gln Gln Leu Ser Leu Leu Met Trp Ile Thr Gln Cys
1               5                   10                  15
Phe Leu Pro Val Phe Leu Ala Gln Pro Pro Ser Gly
            20                  25


<210> 256
<211> 8
<212> PRT
<213> Homo sapiens

<400> 256
Lys Ala Glu Met Leu Glu Ser Val
1               5


<210> 257
<211> 9
<212> PRT
<213> Homo sapiens

<400> 257
Thr Lys Ala Glu Met Leu Glu Ser Val
1               5


<210> 258
<211> 10
<212> PRT
<213> Homo sapiens

<400> 258
Val Thr Lys Ala Glu Met Leu Glu Ser Val
1               5                   10

<210> 259
<211> 9
<212> PRT
<213> Homo sapiens

<400> 259
Met Leu Glu Ser Val Ile Lys Asn Tyr
1               5


<210> 260
<211> 10
<212> PRT
<213> Homo sapiens

<400> 260
Glu Met Leu Glu Ser Val Ile Lys Asn Tyr
1               5                   10


<210> 261
<211> 9
<212> PRT
<213> Homo sapiens

<400> 261
Lys Ala Glu Met Leu Glu Ser Val Ile
1               5


<210> 262
<211> 8
<212> PRT
<213> Homo sapiens

<400> 262
Lys Ala Ser Glu Ser Leu Gln Leu
1               5


<210> 263
<211> 9
<212> PRT
<213> Homo sapiens

<400> 263
Gly Lys Ala Ser Glu Ser Leu Gln Leu
1               5


<210> 264
<211> 9
<212> PRT
<213> Homo sapiens

<400> 264
Ala Ser Glu Ser Leu Gln Leu Val Phe

1               5


<210> 265
<211> 9
<212> PRT
<213> Homo sapiens

<400> 265
Leu Val Phe Gly Ile Asp Val Lys Glu
1               5


<210> 266
<211> 8
<212> PRT
<213> Homo sapiens

<400> 266
Leu Leu Lys Tyr Arg Ala Arg Glu
1               5


<210> 267
<211> 8
<212> PRT
<213> Homo sapiens

<400> 267
Val Ala Asp Leu Val Gly Phe Leu
1               5


<210> 268
<211> 9
<212> PRT
<213> Homo sapiens

<400> 268
Lys Val Ala Asp Leu Val Gly Phe Leu
1               5


<210> 269
<211> 9
<212> PRT
<213> Homo sapiens

<400> 269
Ala Asp Leu Val Gly Phe Leu Leu Leu
1               5


<210> 270
<211> 10
<212> PRT
<213> Homo sapiens

<400> 270
Val Ala Asp Leu Val Gly Phe Leu Leu Leu
1               5                   10


<210> 271
<211> 10
<212> PRT
<213> Homo sapiens

<400> 271
Leu Leu Lys Tyr Arg Ala Arg Glu Pro Val
1               5                   10


<210> 272
<211> 9
<212> PRT
<213> Homo sapiens

<400> 272
Leu Val Glu Thr Ser Tyr Val Lys Val
1               5


<210> 273
<211> 10
<212> PRT
<213> Homo sapiens

<400> 273
Ala Leu Val Glu Thr Ser Tyr Val Lys Val
1               5                   10


<210> 274
<211> 9
<212> PRT
<213> Homo sapiens

<400> 274
Lys Val Leu His His Met Val Lys Ile
1               5


<210> 275
<211> 9
<212> PRT
<213> Homo sapiens

<400> 275
Tyr Val Lys Val Leu His His Met Val
1               5


<210> 276

<211> 9
<212> PRT
<213> Homo sapiens

<400> 276
Pro Arg Ala Leu Val Glu Thr Ser Tyr
1               5


<210> 277
<211> 10
<212> PRT
<213> Homo sapiens

<400> 277
Gly Pro Arg Ala Leu Val Glu Thr Ser Tyr
1               5                   10


<210> 278
<211> 10
<212> PRT
<213> Homo sapiens

<400> 278
Leu Val Glu Thr Ser Tyr Val Lys Val Leu
1               5                   10


<210> 279
<211> 9
<212> PRT
<213> Homo sapiens

<400> 279
Thr Ile Ile Pro Glu Val Pro Gln Leu
1               5


<210> 280
<211> 10
<212> PRT
<213> Homo sapiens

<400> 280
Asp Thr Ile Ile Pro Glu Val Pro Gln Leu
1               5                   10


<210> 281
<211> 10
<212> PRT
<213> Homo sapiens

<400> 281
Glu Val Pro Gln Leu Thr Asp Leu Ser Phe
1               5                   10

<210> 282
<211> 8
<212> PRT
<213> Homo sapiens

<400> 282
Thr Pro Leu Asn Ser Ser Thr Ile
1               5


<210> 283
<211> 8
<212> PRT
<213> Homo sapiens

<400> 283
Ile Gly Leu Arg Trp Thr Pro Leu
1               5


<210> 284
<211> 9
<212> PRT
<213> Homo sapiens

<400> 284
Ser Ile Gly Leu Arg Trp Thr Pro Leu
1               5


<210> 285
<211> 9
<212> PRT
<213> Homo sapiens

<400> 285
Leu Asn Ser Ser Thr Ile Ile Gly Tyr
1               5


<210> 286
<211> 10
<212> PRT
<213> Homo sapiens

<400> 286
Pro Leu Asn Ser Ser Thr Ile Ile Gly Tyr
1               5                   10


<210> 287
<211> 9
<212> PRT
<213> Homo sapiens

<400> 287
Thr Pro Leu Asn Ser Ser Thr Ile Ile
1               5

<210> 288
<211> 8
<212> PRT
<213> Homo sapiens

<400> 288
Ile Gly Tyr Arg Ile Thr Val Val
1               5

<210> 289
<211> 9
<212> PRT
<213> Homo sapiens

<400> 289
Ile Ile Gly Tyr Arg Ile Thr Val Val
1               5

<210> 290
<211> 10
<212> PRT
<213> Homo sapiens

<400> 290
Thr Ile Ile Gly Tyr Arg Ile Thr Val Val
1               5                   10

<210> 291
<211> 9
<212> PRT
<213> Homo sapiens

<400> 291
Ile Gly Tyr Arg Ile Thr Val Val Ala
1               5

<210> 292
<211> 10
<212> PRT
<213> Homo sapiens

<400> 292
Ile Ile Gly Tyr Arg Ile Thr Val Val Ala
1               5                   10

<210> 293
<211> 8

<212> PRT
<213> Homo sapiens

<400> 293
Ser Leu Pro Val Ser Pro Arg Leu
1               5


<210> 294
<211> 9
<212> PRT
<213> Homo sapiens

<400> 294
Gln Ser Leu Pro Val Ser Pro Arg Leu
1               5


<210> 295
<211> 8
<212> PRT
<213> Homo sapiens

<400> 295
Pro Val Ser Pro Arg Leu Gln Leu
1               5


<210> 296
<211> 9
<212> PRT
<213> Homo sapiens

<400> 296
Leu Pro Val Ser Pro Arg Leu Gln Leu
1               5


<210> 297
<211> 10
<212> PRT
<213> Homo sapiens

<400> 297
Ser Leu Pro Val Ser Pro Arg Leu Gln Leu
1               5                   10


<210> 298
<211> 8
<212> PRT
<213> Homo sapiens

<400> 298
Leu Pro Val Ser Pro Arg Leu Gln
1               5

<210> 299
<211> 9
<212> PRT
<213> Homo sapiens

<400> 299
Gln Leu Ser Asn Gly Asn Arg Thr Leu
1               5


<210> 300
<211> 10
<212> PRT
<213> Homo sapiens

<400> 300
Leu Gln Leu Ser Asn Gly Asn Arg Thr Leu
1               5                   10


<210> 301
<211> 9
<212> PRT
<213> Homo sapiens

<400> 301
Trp Val Asn Asn Gln Ser Leu Pro Val
1               5


<210> 302
<211> 9
<212> PRT
<213> Homo sapiens

<400> 302
Pro Val Ser Pro Arg Leu Gln Leu Ser
1               5


<210> 303
<211> 8
<212> PRT
<213> Homo sapiens

<400> 303
Ser Leu Pro Val Ser Pro Arg Leu
1               5


<210> 304
<211> 9
<212> PRT
<213> Homo sapiens

<400> 304

Gln Ser Leu Pro Val Ser Pro Arg Leu
1               5

<210> 305
<211> 8
<212> PRT
<213> Homo sapiens

<400> 305
Pro Val Ser Pro Arg Leu Gln Leu
1               5

<210> 306
<211> 9
<212> PRT
<213> Homo sapiens

<400> 306
Leu Pro Val Ser Pro Arg Leu Gln Leu
1               5

<210> 307
<211> 10
<212> PRT
<213> Homo sapiens

<400> 307
Ser Leu Pro Val Ser Pro Arg Leu Gln Leu
1               5                   10

<210> 308
<211> 8
<212> PRT
<213> Homo sapiens

<400> 308
Leu Pro Val Ser Pro Arg Leu Gln
1               5

<210> 309
<211> 9
<212> PRT
<213> Homo sapiens

<400> 309
Gln Leu Ser Asn Asp Asn Arg Thr Leu
1               5

<210> 310
<211> 10
<212> PRT

<213> Homo sapiens

<400> 310
Leu Gln Leu Ser Asn Asp Asn Arg Thr Leu
1               5                   10


<210> 311
<211> 9
<212> PRT
<213> Homo sapiens

<400> 311
Trp Val Asn Asn Gln Ser Leu Pro Val
1               5


<210> 312
<211> 9
<212> PRT
<213> Homo sapiens

<400> 312
Asn Gln Ser Leu Pro Val Ser Pro Arg
1               5


<210> 313
<211> 8
<212> PRT
<213> Homo sapiens

<400> 313
Ser Leu Pro Val Ser Pro Arg Leu
1               5


<210> 314
<211> 9
<212> PRT
<213> Homo sapiens

<400> 314
Gln Ser Leu Pro Val Ser Pro Arg Leu
1               5


<210> 315
<211> 8
<212> PRT
<213> Homo sapiens

<400> 315
Pro Val Ser Pro Arg Leu Gln Leu
1               5

<210> 316
<211> 9
<212> PRT
<213> Homo sapiens

<400> 316
Leu Pro Val Ser Pro Arg Leu Gln Leu
1               5


<210> 317
<211> 10
<212> PRT
<213> Homo sapiens

<400> 317
Ser Leu Pro Val Ser Pro Arg Leu Gln Leu
1               5                   10


<210> 318
<211> 8
<212> PRT
<213> Homo sapiens

<400> 318
Leu Pro Val Ser Pro Arg Leu Gln
1               5


<210> 319
<211> 9
<212> PRT
<213> Homo sapiens

<400> 319
Gln Leu Ser Asn Gly Asn Arg Thr Leu
1               5


<210> 320
<211> 10
<212> PRT
<213> Homo sapiens

<400> 320
Leu Gln Leu Ser Asn Gly Asn Arg Thr Leu
1               5                   10


<210> 321
<211> 9
<212> PRT
<213> Homo sapiens

<400> 321
Trp Val Asn Gly Gln Ser Leu Pro Val

 1               5


<210> 322
<211> 9
<212> PRT
<213> Homo sapiens

<400> 322
Leu Trp Trp Val Asn Gly Gln Ser Leu
 1               5


<210> 323
<211> 10
<212> PRT
<213> Homo sapiens

<400> 323
Tyr Leu Trp Trp Val Asn Gly Gln Ser Leu
 1               5                   10


<210> 324
<211> 9
<212> PRT
<213> Homo sapiens

<400> 324
Gly Gln Ser Leu Pro Val Ser Pro Arg
 1               5


<210> 325
<211> 8
<212> PRT
<213> Homo sapiens

<400> 325
Asp Met Lys Leu Arg Leu Pro Ala
 1               5


<210> 326
<211> 10
<212> PRT
<213> Homo sapiens

<400> 326
Gly Thr Asp Met Lys Leu Arg Leu Pro Ala
 1               5                   10


<210> 327
<211> 8
<212> PRT
<213> Homo sapiens

<400> 327
His Leu Asp Met Leu Arg His Leu
1               5


<210> 328
<211> 9
<212> PRT
<213> Homo sapiens

<400> 328
Thr His Leu Asp Met Leu Arg His Leu
1               5


<210> 329
<211> 10
<212> PRT
<213> Homo sapiens

<400> 329
Glu Thr His Leu Asp Met Leu Arg His Leu
1               5                   10


<210> 330
<211> 8
<212> PRT
<213> Homo sapiens

<400> 330
Pro Ala Ser Pro Glu Thr His Leu
1               5


<210> 331
<211> 9
<212> PRT
<213> Homo sapiens

<400> 331
Leu Pro Ala Ser Pro Glu Thr His Leu
1               5


<210> 332
<211> 10
<212> PRT
<213> Homo sapiens

<400> 332
Arg Leu Pro Ala Ser Pro Glu Thr His Leu
1               5                   10


<210> 333

<211> 9
<212> PRT
<213> Homo sapiens

<400> 333
Ser Pro Glu Thr His Leu Asp Met Leu
1               5


<210> 334
<211> 10
<212> PRT
<213> Homo sapiens

<400> 334
Ala Ser Pro Glu Thr His Leu Asp Met Leu
1               5                   10


<210> 335
<211> 9
<212> PRT
<213> Homo sapiens

<400> 335
His Leu Asp Met Leu Arg His Leu Tyr
1               5


<210> 336
<211> 10
<212> PRT
<213> Homo sapiens

<400> 336
Thr His Leu Asp Met Leu Arg His Leu Tyr
1               5                   10


<210> 337
<211> 8
<212> PRT
<213> Homo sapiens

<400> 337
Glu Leu Arg Lys Val Lys Val Leu
1               5


<210> 338
<211> 9
<212> PRT
<213> Homo sapiens

<400> 338
Thr Glu Leu Arg Lys Val Lys Val Leu
1               5

<210> 339
<211> 10
<212> PRT
<213> Homo sapiens

<400> 339
Glu Thr Glu Leu Arg Lys Val Lys Val Leu
1               5                   10


<210> 340
<211> 9
<212> PRT
<213> Homo sapiens

<400> 340
Leu Lys Glu Thr Glu Leu Arg Lys Val
1               5


<210> 341
<211> 10
<212> PRT
<213> Homo sapiens

<400> 341
Ile Leu Lys Glu Thr Glu Leu Arg Lys Val
1               5                   10


<210> 342
<211> 9
<212> PRT
<213> Homo sapiens

<400> 342
Met Arg Ile Leu Lys Glu Thr Glu Leu
1               5


<210> 343
<211> 10
<212> PRT
<213> Homo sapiens

<400> 343
Gln Met Arg Ile Leu Lys Glu Thr Glu Leu
1               5                   10


<210> 344
<211> 9
<212> PRT
<213> Homo sapiens

<400> 344
Glu Thr Glu Leu Arg Lys Val Lys Val
1               5

<210> 345
<211> 10
<212> PRT
<213> Homo sapiens

<400> 345
Lys Glu Thr Glu Leu Arg Lys Val Lys Val
1               5                   10

<210> 346
<211> 9
<212> PRT
<213> Homo sapiens

<400> 346
Met Pro Asn Gln Ala Gln Met Arg Ile
1               5

<210> 347
<211> 10
<212> PRT
<213> Homo sapiens

<400> 347
Ala Met Pro Asn Gln Ala Gln Met Arg Ile
1               5                   10

<210> 348
<211> 10
<212> PRT
<213> Homo sapiens

<400> 348
Met Pro Asn Gln Ala Gln Met Arg Ile Leu
1               5                   10

<210> 349
<211> 8
<212> PRT
<213> Homo sapiens

<400> 349
Arg Pro Arg Phe Arg Glu Leu Val
1               5

<210> 350
<211> 9

<212> PRT
<213> Homo sapiens

<400> 350
Cys Arg Pro Arg Phe Arg Glu Leu Val
1               5


<210> 351
<211> 9
<212> PRT
<213> Homo sapiens

<400> 351
Arg Phe Arg Glu Leu Val Ser Glu Phe
1               5


<210> 352
<211> 10
<212> PRT
<213> Homo sapiens

<400> 352
Pro Arg Phe Arg Glu Leu Val Ser Glu Phe
1               5                   10


<210> 353
<211> 9
<212> PRT
<213> Homo sapiens

<400> 353
Glu Cys Arg Pro Arg Phe Arg Glu Leu
1               5


<210> 354
<211> 9
<212> PRT
<213> Homo sapiens

<400> 354
Gly Ala Ala Ser Gly Leu Asn Gly Cys
1               5


<210> 355
<211> 9
<212> PRT
<213> Homo sapiens

<400> 355
Arg Ala Ser Gly Pro Gly Gly Gly Ala
1               5

<210> 356
<211> 9
<212> PRT
<213> Homo sapiens

<400> 356
Pro His Gly Gly Ala Ala Ser Gly Leu
1               5


<210> 357
<211> 10
<212> PRT
<213> Homo sapiens

<400> 357
Gly Pro His Gly Gly Ala Ala Ser Gly Leu
1               5                   10


<210> 358
<211> 10
<212> PRT
<213> Homo sapiens

<400> 358
Ala Pro Arg Gly Pro His Gly Gly Ala Ala
1               5                   10


<210> 359
<211> 8
<212> PRT
<213> Homo sapiens

<400> 359
Val Arg Pro Arg Arg Trp Lys Leu
1               5


<210> 360
<211> 9
<212> PRT
<213> Homo sapiens

<400> 360
Glu Val Arg Pro Arg Arg Trp Lys Leu
1               5


<210> 361
<211> 9
<212> PRT
<213> Homo sapiens

<400> 361

Arg Pro Arg Arg Trp Lys Leu Gln Val
1               5

<210> 362
<211> 9
<212> PRT
<213> Homo sapiens

<400> 362
Pro Arg Arg Trp Lys Leu Gln Val Leu
1               5

<210> 363
<211> 10
<212> PRT
<213> Homo sapiens

<400> 363
Arg Pro Arg Arg Trp Lys Leu Gln Val Leu
1               5                   10

<210> 364
<211> 9
<212> PRT
<213> Homo sapiens

<400> 364
Arg Trp Lys Leu Gln Val Leu Asp Leu
1               5

<210> 365
<211> 10
<212> PRT
<213> Homo sapiens

<400> 365
Arg Arg Trp Lys Leu Gln Val Leu Asp Leu
1               5                   10

<210> 366
<211> 9
<212> PRT
<213> Homo sapiens

<400> 366
Pro Val Glu Val Leu Val Asp Leu Phe
1               5

<210> 367
<211> 8
<212> PRT

<213> Homo sapiens

<400> 367
Val Lys Arg Lys Lys Asn Val Leu
1               5


<210> 368
<211> 9
<212> PRT
<213> Homo sapiens

<400> 368
Lys Val Lys Arg Lys Lys Asn Val Leu
1               5


<210> 369
<211> 10
<212> PRT
<213> Homo sapiens

<400> 369
Glu Lys Val Lys Arg Lys Lys Asn Val Leu
1               5                   10


<210> 370
<211> 8
<212> PRT
<213> Homo sapiens

<400> 370
Lys Val Lys Arg Lys Lys Asn Val
1               5


<210> 371
<211> 8
<212> PRT
<213> Homo sapiens

<400> 371
Arg Lys Lys Asn Val Leu Arg Leu
1               5


<210> 372
<211> 9
<212> PRT
<213> Homo sapiens

<400> 372
Lys Arg Lys Lys Asn Val Leu Arg Leu
1               5

<210> 373
<211> 10
<212> PRT
<213> Homo sapiens

<400> 373
Val Lys Arg Lys Lys Asn Val Leu Arg Leu
1               5                   10


<210> 374
<211> 8
<212> PRT
<213> Homo sapiens

<400> 374
Asp Glu Leu Phe Ser Tyr Leu Ile
1               5


<210> 375
<211> 9
<212> PRT
<213> Homo sapiens

<400> 375
Val Leu Arg Leu Cys Cys Lys Lys Leu
1               5


<210> 376
<211> 10
<212> PRT
<213> Homo sapiens

<400> 376
Asn Val Leu Arg Leu Cys Cys Lys Lys Leu
1               5                   10


<210> 377
<211> 9
<212> PRT
<213> Homo sapiens

<400> 377
Tyr Leu Ile Glu Lys Val Lys Arg Lys
1               5


<210> 378
<211> 8
<212> PRT
<213> Homo sapiens

<400> 378
Gln Ala Trp Pro Phe Thr Cys Leu

1                   5


<210> 379
<211> 9
<212> PRT
<213> Homo sapiens

<400> 379
Val Gln Ala Trp Pro Phe Thr Cys Leu
1                   5


<210> 380
<211> 10
<212> PRT
<213> Homo sapiens

<400> 380
Met Val Gln Ala Trp Pro Phe Thr Cys Leu
1                   5                   10


<210> 381
<211> 8
<212> PRT
<213> Homo sapiens

<400> 381
Leu Pro Leu Gly Val Leu Met Lys
1                   5


<210> 382
<211> 9
<212> PRT
<213> Homo sapiens

<400> 382
Cys Leu Pro Leu Gly Val Leu Met Lys
1                   5


<210> 383
<211> 10
<212> PRT
<213> Homo sapiens

<400> 383
Thr Cys Leu Pro Leu Gly Val Leu Met Lys
1                   5                   10


<210> 384
<211> 9
<212> PRT
<213> Homo sapiens

<400> 384
Gly Val Leu Met Lys Gly Gln His Leu
1               5


<210> 385
<211> 9
<212> PRT
<213> Homo sapiens

<400> 385
Leu Pro Leu Gly Val Leu Met Lys Gly
1               5


<210> 386
<211> 10
<212> PRT
<213> Homo sapiens

<400> 386
Cys Leu Pro Leu Gly Val Leu Met Lys Gly
1               5                   10


<210> 387
<211> 10
<212> PRT
<213> Homo sapiens

<400> 387
Trp Pro Phe Thr Cys Leu Pro Leu Gly Val
1               5                   10


<210> 388
<211> 9
<212> PRT
<213> Homo sapiens

<400> 388
Glu Leu Phe Pro Pro Leu Phe Met Ala
1               5


<210> 389
<211> 9
<212> PRT
<213> Homo sapiens

<400> 389
Pro Arg Glu Leu Phe Pro Pro Leu Phe
1               5


<210> 390

<211> 10
<212> PRT
<213> Homo sapiens

<400> 390
Leu Pro Arg Glu Leu Phe Pro Pro Leu Phe
1               5                   10


<210> 391
<211> 9
<212> PRT
<213> Homo sapiens

<400> 391
Arg Glu Leu Phe Pro Pro Leu Phe Met
1               5


<210> 392
<211> 10
<212> PRT
<213> Homo sapiens

<400> 392
Pro Arg Glu Leu Phe Pro Pro Leu Phe Met
1               5                   10


<210> 393
<211> 8
<212> PRT
<213> Homo sapiens

<400> 393
Arg Pro Ser Leu Tyr Thr Lys Val
1               5


<210> 394
<211> 9
<212> PRT
<213> Homo sapiens

<400> 394
Glu Arg Pro Ser Leu Tyr Thr Lys Val
1               5


<210> 395
<211> 8
<212> PRT
<213> Homo sapiens

<400> 395
Leu Pro Glu Arg Pro Ser Leu Tyr
1               5

<210> 396
<211> 9
<212> PRT
<213> Homo sapiens

<400> 396
Ala Leu Pro Glu Arg Pro Ser Leu Tyr
1               5


<210> 397
<211> 9
<212> PRT
<213> Homo sapiens

<400> 397
Ser Leu Tyr Thr Lys Val Val His Tyr
1               5


<210> 398
<211> 10
<212> PRT
<213> Homo sapiens

<400> 398
Pro Ser Leu Tyr Thr Lys Val Val His Tyr
1               5                   10


<210> 399
<211> 9
<212> PRT
<213> Homo sapiens

<400> 399
Arg Pro Ser Leu Tyr Thr Lys Val Val
1               5


<210> 400
<211> 8
<212> PRT
<213> Homo sapiens

<400> 400
Gly Asn Lys Val Lys Asn Ala Gln
1               5


<210> 401
<211> 8
<212> PRT
<213> Homo sapiens

<400> 401
Ile Ala Arg Tyr Gly Lys Val Phe
1               5


<210> 402
<211> 9
<212> PRT
<213> Homo sapiens

<400> 402
Ala Gln Leu Ala Gly Ala Lys Gly Val
1               5


<210> 403
<211> 9
<212> PRT
<213> Homo sapiens

<400> 403
Lys Val Phe Arg Gly Asn Lys Val Lys
1               5


<210> 404
<211> 9
<212> PRT
<213> Homo sapiens

<400> 404
Gly Asn Lys Val Lys Asn Ala Gln Leu
1               5


<210> 405
<211> 9
<212> PRT
<213> Homo sapiens

<400> 405
Thr Pro Gly Tyr Pro Ala Asn Glu Tyr
1               5


<210> 406
<211> 10
<212> PRT
<213> Homo sapiens

<400> 406
Leu Thr Pro Gly Tyr Pro Ala Asn Glu Tyr
1               5                   10


<210> 407
<211> 9

<212> PRT
<213> Homo sapiens

<400> 407
Gly Tyr Pro Ala Asn Glu Tyr Ala Tyr
1               5


<210> 408
<211> 10
<212> PRT
<213> Homo sapiens

<400> 408
Pro Gly Tyr Pro Ala Asn Glu Tyr Ala Tyr
1               5                   10


<210> 409
<211> 9
<212> PRT
<213> Homo sapiens

<400> 409
Asp Pro Leu Thr Pro Gly Tyr Pro Ala
1               5


<210> 410
<211> 9
<212> PRT
<213> Homo sapiens

<400> 410
Ser Leu Tyr Glu Ser Trp Thr Lys Lys
1               5


<210> 411
<211> 10
<212> PRT
<213> Homo sapiens

<400> 411
Lys Ser Leu Tyr Glu Ser Trp Thr Lys Lys
1               5                   10


<210> 412
<211> 9
<212> PRT
<213> Homo sapiens

<400> 412
Glu Gly Phe Glu Gly Lys Ser Leu Tyr
1               5

<210> 413
<211> 10
<212> PRT
<213> Homo sapiens

<400> 413
Asp Glu Gly Phe Glu Gly Lys Ser Leu Tyr
1               5                   10


<210> 414
<211> 9
<212> PRT
<213> Homo sapiens

<400> 414
Thr Lys Lys Ser Pro Ser Pro Glu Phe
1               5


<210> 415
<211> 10
<212> PRT
<213> Homo sapiens

<400> 415
Trp Thr Lys Lys Ser Pro Ser Pro Glu Phe
1               5                   10


<210> 416
<211> 10
<212> PRT
<213> Homo sapiens

<400> 416
Ser Leu Tyr Glu Ser Trp Thr Lys Lys Ser
1               5                   10


<210> 417
<211> 8
<212> PRT
<213> Homo sapiens

<400> 417
Trp Gly Glu Val Lys Arg Gln Ile
1               5


<210> 418
<211> 9
<212> PRT
<213> Homo sapiens

<400> 418

Ala Trp Gly Glu Val Lys Arg Gln Ile
1               5

<210> 419
<211> 10
<212> PRT
<213> Homo sapiens

<400> 419
Lys Ala Trp Gly Glu Val Lys Arg Gln Ile
1               5                   10

<210> 420
<211> 8
<212> PRT
<213> Homo sapiens

<400> 420
Lys Ala Trp Gly Glu Val Lys Arg
1               5

<210> 421
<211> 9
<212> PRT
<213> Homo sapiens

<400> 421
Ser Lys Ala Trp Gly Glu Val Lys Arg
1               5

<210> 422
<211> 9
<212> PRT
<213> Homo sapiens

<400> 422
Gln Ile Tyr Val Ala Ala Phe Thr Val
1               5

<210> 423
<211> 9
<212> PRT
<213> Homo sapiens

<400> 423
Tyr Val Ala Ala Phe Thr Val Gln Ala
1               5

<210> 424
<211> 9
<212> PRT

<213> Homo sapiens

<400> 424
Trp Gly Glu Val Lys Arg Gln Ile Tyr
1               5


<210> 425
<211> 9
<212> PRT
<213> Homo sapiens

<400> 425
Glu Val Lys Arg Gln Ile Tyr Val Ala
1               5


<210> 426
<211> 9
<212> PRT
<213> Homo sapiens

<400> 426
Thr Val Gln Ala Ala Ala Glu Thr Leu
1               5


<210> 427
<211> 10
<212> PRT
<213> Homo sapiens

<400> 427
Phe Thr Val Gln Ala Ala Ala Glu Thr Leu
1               5                   10


<210> 428
<211> 9
<212> PRT
<213> Homo sapiens

<400> 428
Lys Arg Gln Ile Tyr Val Ala Ala Phe
1               5


<210> 429
<211> 9
<212> PRT
<213> Homo sapiens

<400> 429
Pro Ser Lys Ala Trp Gly Glu Val Lys
1               5

<210> 430
<211> 9
<212> PRT
<213> Homo sapiens

<400> 430
Lys Ala Trp Gly Glu Val Lys Arg Gln
1               5


<210> 431
<211> 9
<212> PRT
<213> Homo sapiens

<400> 431
Trp Lys Glu Phe Gly Leu Asp Ser Val
1               5


<210> 432
<211> 10
<212> PRT
<213> Homo sapiens

<400> 432
Gln Trp Lys Glu Phe Gly Leu Asp Ser Val
1               5                   10


<210> 433
<211> 10
<212> PRT
<213> Homo sapiens

<400> 433
Glu Phe Gly Leu Asp Ser Val Glu Leu Ala
1               5                   10


<210> 434
<211> 9
<212> PRT
<213> Homo sapiens

<400> 434
Glu Leu Arg Gln Lys Glu Ser Lys Leu
1               5


<210> 435
<211> 10
<212> PRT
<213> Homo sapiens

<400> 435
Ala Glu Leu Arg Gln Lys Glu Ser Lys Leu

1                   5                       10


<210> 436
<211> 9
<212> PRT
<213> Homo sapiens

<400> 436
Lys Leu Gln Glu Asn Arg Lys Ile Ile
1                   5


<210> 437
<211> 8
<212> PRT
<213> Homo sapiens

<400> 437
Gln Leu Glu Glu Lys Thr Lys Leu
1                   5


<210> 438
<211> 9
<212> PRT
<213> Homo sapiens

<400> 438
Asn Gln Leu Glu Glu Lys Thr Lys Leu
1                   5


<210> 439
<211> 9
<212> PRT
<213> Homo sapiens

<400> 439
Leu Leu Glu Glu Ser Arg Asp Lys Val
1                   5


<210> 440
<211> 10
<212> PRT
<213> Homo sapiens

<400> 440
Phe Leu Leu Glu Glu Ser Arg Asp Lys Val
1                   5                       10


<210> 441
<211> 9
<212> PRT
<213> Homo sapiens

<400> 441
Glu Ser Arg Asp Lys Val Asn Gln Leu
1               5


<210> 442
<211> 10
<212> PRT
<213> Homo sapiens

<400> 442
Glu Glu Ser Arg Asp Lys Val Asn Gln Leu
1               5                   10


<210> 443
<211> 9
<212> PRT
<213> Homo sapiens

<400> 443
Glu Lys Glu Val His Asp Leu Glu Tyr
1               5


<210> 444
<211> 10
<212> PRT
<213> Homo sapiens

<400> 444
Arg Glu Lys Glu Val His Asp Leu Glu Tyr
1               5                   10


<210> 445
<211> 9
<212> PRT
<213> Homo sapiens

<400> 445
Asp Leu Glu Tyr Ser Tyr Cys His Tyr
1               5


<210> 446
<211> 9
<212> PRT
<213> Homo sapiens

<400> 446
Glu Val His Asp Leu Glu Tyr Ser Tyr
1               5


<210> 447

<211> 10
<212> PRT
<213> Homo sapiens

<400> 447
Glu Val His Asp Leu Glu Tyr Ser Tyr Cys
1               5                   10


<210> 448
<211> 8
<212> PRT
<213> Homo sapiens

<400> 448
Lys Leu Ser Ser Lys Arg Glu Leu
1               5


<210> 449
<211> 8
<212> PRT
<213> Homo sapiens

<400> 449
Glu Leu Lys Asn Thr Glu Tyr Phe
1               5


<210> 450
<211> 9
<212> PRT
<213> Homo sapiens

<400> 450
Arg Glu Leu Lys Asn Thr Glu Tyr Phe
1               5


<210> 451
<211> 8
<212> PRT
<213> Homo sapiens

<400> 451
Lys Arg Gly Gln Arg Pro Lys Leu
1               5


<210> 452
<211> 10
<212> PRT
<213> Homo sapiens

<400> 452
Leu Pro Lys Arg Gly Gln Arg Pro Lys Leu
1               5                   10

<210> 453
<211> 9
<212> PRT
<213> Homo sapiens

<400> 453
Leu Lys Asn Thr Glu Tyr Phe Thr Leu
1               5


<210> 454
<211> 10
<212> PRT
<213> Homo sapiens

<400> 454
Glu Leu Lys Asn Thr Glu Tyr Phe Thr Leu
1               5                   10


<210> 455
<211> 9
<212> PRT
<213> Homo sapiens

<400> 455
Lys Arg Glu Leu Lys Asn Thr Glu Tyr
1               5


<210> 456
<211> 9
<212> PRT
<213> Homo sapiens

<400> 456
Lys Leu Ser Ser Lys Arg Glu Leu Lys
1               5


<210> 457
<211> 9
<212> PRT
<213> Homo sapiens

<400> 457
Gly Gln Arg Pro Lys Leu Ser Ser Lys
1               5


<210> 458
<211> 10
<212> PRT
<213> Homo sapiens

<400> 458
Arg Gly Gln Arg Pro Lys Leu Ser Ser Lys
1               5                   10


<210> 459
<211> 9
<212> PRT
<213> Homo sapiens

<400> 459
Arg Pro Lys Leu Ser Ser Lys Arg Glu
1               5


<210> 460
<211> 8
<212> PRT
<213> Homo sapiens

<400> 460
Leu Glu Tyr Val Arg Glu Glu Leu
1               5


<210> 461
<211> 9
<212> PRT
<213> Homo sapiens

<400> 461
Glu Leu Glu Tyr Val Arg Glu Glu Leu
1               5


<210> 462
<211> 10
<212> PRT
<213> Homo sapiens

<400> 462
Asn Glu Leu Glu Tyr Val Arg Glu Glu Leu
1               5                   10


<210> 463
<211> 10
<212> PRT
<213> Homo sapiens

<400> 463
Glu Leu Lys Gln Lys Arg Glu Asp Glu Val
1               5                   10


<210> 464
<211> 9

<212> PRT
<213> Homo sapiens

<400> 464
Tyr Val Arg Glu Glu Leu Lys Gln Lys
1               5


<210> 465
<211> 9
<212> PRT
<213> Homo sapiens

<400> 465
Gln Leu Asn Val Tyr Glu Ile Lys Val
1               5


<210> 466
<211> 9
<212> PRT
<213> Homo sapiens

<400> 466
Ser Lys Gln Leu Asn Val Tyr Glu Ile
1               5


<210> 467
<211> 9
<212> PRT
<213> Homo sapiens

<400> 467
Ala Glu Ser Lys Gln Leu Asn Val Tyr
1               5


<210> 468
<211> 10
<212> PRT
<213> Homo sapiens

<400> 468
Thr Ala Glu Ser Lys Gln Leu Asn Val Tyr
1               5                   10


<210> 469
<211> 8
<212> PRT
<213> Homo sapiens

<400> 469
Ile Lys Val Asn Lys Leu Glu Leu
1               5

<210> 470
<211> 9
<212> PRT
<213> Homo sapiens

<400> 470
Glu Ile Lys Val Asn Lys Leu Glu Leu
1               5


<210> 471
<211> 10
<212> PRT
<213> Homo sapiens

<400> 471
Tyr Glu Ile Lys Val Asn Lys Leu Glu Leu
1               5                   10


<210> 472
<211> 9
<212> PRT
<213> Homo sapiens

<400> 472
Lys Leu Glu Leu Glu Leu Glu Ser Ala
1               5


<210> 473
<211> 9
<212> PRT
<213> Homo sapiens

<400> 473
Val Tyr Glu Ile Lys Val Asn Lys Leu
1               5


<210> 474
<211> 10
<212> PRT
<213> Homo sapiens

<400> 474
Asn Val Tyr Glu Ile Lys Val Asn Lys Leu
1               5                   10


<210> 475
<211> 9
<212> PRT
<213> Homo sapiens

<400> 475

Glu Leu Glu Ser Ala Lys Gln Lys Phe
1               5

<210> 476
<211> 9
<212> PRT
<213> Homo sapiens

<400> 476
Lys Leu Glu Leu Glu Leu Glu Ser Ala
1               5

<210> 477
<211> 9
<212> PRT
<213> Homo sapiens

<400> 477
Glu Leu Glu Ser Ala Lys Gln Lys Phe
1               5

<210> 478
<211> 8
<212> PRT
<213> Homo sapiens

<400> 478
Lys Glu Lys Leu Lys Arg Glu Ala
1               5

<210> 479
<211> 9
<212> PRT
<213> Homo sapiens

<400> 479
Glu Ala Lys Glu Asn Thr Ala Thr Leu
1               5

<210> 480
<211> 10
<212> PRT
<213> Homo sapiens

<400> 480
Arg Glu Ala Lys Glu Asn Thr Ala Thr Leu
1               5                   10

<210> 481
<211> 10
<212> PRT

<213> Homo sapiens

<400> 481
Lys Leu Lys Arg Glu Ala Lys Glu Asn Thr
1               5                   10


<210> 482
<211> 8
<212> PRT
<213> Homo sapiens

<400> 482
Glu Ala Glu Lys Ile Lys Lys Trp
1               5


<210> 483
<211> 9
<212> PRT
<213> Homo sapiens

<400> 483
Gly Leu Ser Arg Val Tyr Ser Lys Leu
1               5


<210> 484
<211> 10
<212> PRT
<213> Homo sapiens

<400> 484
Glu Gly Leu Ser Arg Val Tyr Ser Lys Leu
1               5                   10


<210> 485
<211> 9
<212> PRT
<213> Homo sapiens

<400> 485
Lys Leu Tyr Lys Glu Ala Glu Lys Ile
1               5


<210> 486
<211> 9
<212> PRT
<213> Homo sapiens

<400> 486
Asn Ser Glu Gly Leu Ser Arg Val Tyr
1               5

<210> 487
<211> 10
<212> PRT
<213> Homo sapiens

<400> 487
Glu Asn Ser Glu Gly Leu Ser Arg Val Tyr
1               5                   10


<210> 488
<211> 9
<212> PRT
<213> Homo sapiens

<400> 488
Leu Ser Arg Val Tyr Ser Lys Leu Tyr
1               5


<210> 489
<211> 10
<212> PRT
<213> Homo sapiens

<400> 489
Gly Leu Ser Arg Val Tyr Ser Lys Leu Tyr
1               5                   10


<210> 490
<211> 10
<212> PRT
<213> Homo sapiens

<400> 490
Leu Glu Asn Ser Glu Gly Leu Ser Arg Val
1               5                   10


<210> 491
<211> 10
<212> PRT
<213> Homo sapiens

<400> 491
Lys Leu Tyr Lys Glu Ala Glu Lys Ile Lys
1               5                   10


<210> 492
<211> 8
<212> PRT
<213> Homo sapiens

<400> 492
Arg Glu Asp Arg Trp Ala Val Ile

1               5


<210> 493
<211> 9
<212> PRT
<213> Homo sapiens

<400> 493
Met Arg Glu Asp Arg Trp Ala Val Ile
1               5


<210> 494
<211> 10
<212> PRT
<213> Homo sapiens

<400> 494
Lys Met Arg Glu Asp Arg Trp Ala Val Ile
1               5                   10


<210> 495
<211> 9
<212> PRT
<213> Homo sapiens

<400> 495
Lys Met Arg Glu Asp Arg Trp Ala Val
1               5


<210> 496
<211> 9
<212> PRT
<213> Homo sapiens

<400> 496
Thr Thr Pro Gly Ser Thr Leu Lys Phe
1               5


<210> 497
<211> 10
<212> PRT
<213> Homo sapiens

<400> 497
Leu Thr Thr Pro Gly Ser Thr Leu Lys Phe
1               5                   10


<210> 498
<211> 8
<212> PRT
<213> Homo sapiens

<400> 498
Gly Ser Thr Leu Lys Gly Ala Ile
1               5


<210> 499
<211> 9
<212> PRT
<213> Homo sapiens

<400> 499
Ile Arg Lys Met Arg Glu Asp Arg Trp
1               5


<210> 500
<211> 8
<212> PRT
<213> Homo sapiens

<400> 500
Arg Leu Glu Met His Phe Lys Leu
1               5


<210> 501
<211> 9
<212> PRT
<213> Homo sapiens

<400> 501
Ser Arg Leu Glu Met His Phe Lys Leu
1               5


<210> 502
<211> 9
<212> PRT
<213> Homo sapiens

<400> 502
Lys Leu Lys Glu Asp Tyr Glu Lys Ile
1               5


<210> 503
<211> 9
<212> PRT
<213> Homo sapiens

<400> 503
Lys Ile Gln His Leu Glu Gln Glu Tyr
1               5


<210> 504

<211> 10
<212> PRT
<213> Homo sapiens

<400> 504
Glu Lys Ile Gln His Leu Glu Gln Glu Tyr
1               5                   10


<210> 505
<211> 9
<212> PRT
<213> Homo sapiens

<400> 505
Glu Asn Ser Arg Leu Glu Met His Phe
1               5


<210> 506
<211> 10
<212> PRT
<213> Homo sapiens

<400> 506
Arg Leu Glu Met His Phe Lys Leu Lys Glu
1               5                   10


<210> 507
<211> 8
<212> PRT
<213> Homo sapiens

<400> 507
Leu Glu Asp Ile Lys Val Ser Leu
1               5


<210> 508
<211> 9
<212> PRT
<213> Homo sapiens

<400> 508
Glu Leu Glu Asp Ile Lys Val Ser Leu
1               5


<210> 509
<211> 10
<212> PRT
<213> Homo sapiens

<400> 509
Lys Glu Leu Glu Asp Ile Lys Val Ser Leu
1               5                   10

<210> 510
<211> 8
<212> PRT
<213> Homo sapiens

<400> 510
Leu Thr Lys Glu Leu Glu Asp Ile
1               5


<210> 511
<211> 9
<212> PRT
<213> Homo sapiens

<400> 511
His Leu Thr Lys Glu Leu Glu Asp Ile
1               5


<210> 512
<211> 9
<212> PRT
<213> Homo sapiens

<400> 512
Ser Leu Gln Arg Ser Val Ser Thr Gln
1               5


<210> 513
<211> 9
<212> PRT
<213> Homo sapiens

<400> 513
Thr Lys Glu Leu Glu Asp Ile Lys Val
1               5


<210> 514
<211> 10
<212> PRT
<213> Homo sapiens

<400> 514
Leu Thr Lys Glu Leu Glu Asp Ile Lys Val
1               5                   10


<210> 515
<211> 10
<212> PRT
<213> Homo sapiens

<400> 515
Asp Ile Lys Val Ser Leu Gln Arg Ser Val
1               5                   10


<210> 516
<211> 8
<212> PRT
<213> Homo sapiens

<400> 516
Lys Met Lys Asp Leu Thr Phe Leu
1               5


<210> 517
<211> 9
<212> PRT
<213> Homo sapiens

<400> 517
Asn Lys Met Lys Asp Leu Thr Phe Leu
1               5


<210> 518
<211> 10
<212> PRT
<213> Homo sapiens

<400> 518
Glu Asn Lys Met Lys Asp Leu Thr Phe Leu
1               5                   10


<210> 519
<211> 9
<212> PRT
<213> Homo sapiens

<400> 519
Leu Leu Glu Glu Ser Arg Asp Lys Val
1               5


<210> 520
<211> 10
<212> PRT
<213> Homo sapiens

<400> 520
Phe Leu Leu Glu Glu Ser Arg Asp Lys Val
1               5                   10


<210> 521
<211> 9

<212> PRT
<213> Homo sapiens

<400> 521
Glu Ser Arg Asp Lys Val Asn Gln Leu
1               5


<210> 522
<211> 10
<212> PRT
<213> Homo sapiens

<400> 522
Glu Glu Ser Arg Asp Lys Val Asn Gln Leu
1               5                   10


<210> 523
<211> 9
<212> PRT
<213> Homo sapiens

<400> 523
Glu Lys Glu Asn Lys Met Lys Asp Leu
1               5


<210> 524
<211> 10
<212> PRT
<213> Homo sapiens

<400> 524
Thr Glu Lys Glu Asn Lys Met Lys Asp Leu
1               5                   10


<210> 525
<211> 9
<212> PRT
<213> Homo sapiens

<400> 525
Glu Asn Lys Met Lys Asp Leu Thr Phe
1               5


<210> 526
<211> 8
<212> PRT
<213> Homo sapiens

<400> 526
Ile Glu Lys Met Ile Thr Ala Phe
1               5

<210> 527
<211> 9
<212> PRT
<213> Homo sapiens

<400> 527
Asn Ile Glu Lys Met Ile Thr Ala Phe
1               5


<210> 528
<211> 10
<212> PRT
<213> Homo sapiens

<400> 528
Ser Asn Ile Glu Lys Met Ile Thr Ala Phe
1               5                   10


<210> 529
<211> 8
<212> PRT
<213> Homo sapiens

<400> 529
Thr Ala Phe Glu Glu Leu Arg Val
1               5


<210> 530
<211> 9
<212> PRT
<213> Homo sapiens

<400> 530
Ile Thr Ala Phe Glu Glu Leu Arg Val
1               5


<210> 531
<211> 10
<212> PRT
<213> Homo sapiens

<400> 531
Met Ile Thr Ala Phe Glu Glu Leu Arg Val
1               5                   10


<210> 532
<211> 9
<212> PRT
<213> Homo sapiens

<400> 532

Lys Met Ile Thr Ala Phe Glu Glu Leu
1               5

<210> 533
<211> 10
<212> PRT
<213> Homo sapiens

<400> 533
Glu Lys Met Ile Thr Ala Phe Glu Glu Leu
1               5                   10

<210> 534
<211> 9
<212> PRT
<213> Homo sapiens

<400> 534
Glu Leu Arg Val Gln Ala Glu Asn Ser
1               5

<210> 535
<211> 10
<212> PRT
<213> Homo sapiens

<400> 535
Asp Leu Asn Ser Asn Ile Glu Lys Met Ile
1               5                   10

<210> 536
<211> 8
<212> PRT
<213> Homo sapiens

<400> 536
Trp Thr Ser Ala Lys Asn Thr Leu
1               5

<210> 537
<211> 9
<212> PRT
<213> Homo sapiens

<400> 537
Thr Pro Leu Pro Lys Ala Tyr Thr Val
1               5

<210> 538
<211> 10
<212> PRT

<213> Homo sapiens

<400> 538
Ser Thr Pro Leu Pro Lys Ala Tyr Thr Val
1               5                   10


<210> 539
<211> 9
<212> PRT
<213> Homo sapiens

<400> 539
Leu Ser Thr Pro Leu Pro Lys Ala Tyr
1               5


<210> 540
<211> 10
<212> PRT
<213> Homo sapiens

<400> 540
Thr Leu Ser Thr Pro Leu Pro Lys Ala Tyr
1               5                   10


<210> 541
<211> 9
<212> PRT
<213> Homo sapiens

<400> 541
Asn Thr Leu Ser Thr Pro Leu Pro Lys
1               5


<210> 542
<211> 10
<212> PRT
<213> Homo sapiens

<400> 542
Lys Asn Thr Leu Ser Thr Pro Leu Pro Lys
1               5                   10


<210> 543
<211> 8
<212> PRT
<213> Homo sapiens

<400> 543
Ile Ser Lys Asp Lys Arg Asp Tyr
1               5

<210> 544
<211> 10
<212> PRT
<213> Homo sapiens

<400> 544
His Gly Ile Ser Lys Asp Lys Arg Asp Tyr
1               5                   10


<210> 545
<211> 9
<212> PRT
<213> Homo sapiens

<400> 545
Lys Arg Asp Tyr Leu Trp Thr Ser Ala
1               5


<210> 546
<211> 10
<212> PRT
<213> Homo sapiens

<400> 546
Ser Lys Asp Lys Arg Asp Tyr Leu Trp Thr
1               5                   10


<210> 547
<211> 8
<212> PRT
<213> Homo sapiens

<400> 547
Glu Asn Lys Met Lys Asp Leu Thr
1               5


<210> 548
<211> 9
<212> PRT
<213> Homo sapiens

<400> 548
Glu Ile Asn Asp Lys Glu Lys Gln Val
1               5


<210> 549
<211> 9
<212> PRT
<213> Homo sapiens

<400> 549
Gln Ile Thr Glu Lys Glu Asn Lys Met

1               5


<210> 550
<211> 9
<212> PRT
<213> Homo sapiens

<400> 550
Ser Leu Leu Leu Ile Gln Ile Thr Glu
1               5


<210> 551
<211> 8
<212> PRT
<213> Homo sapiens

<400> 551
Phe Glu Lys Ile Ala Glu Glu Leu
1               5


<210> 552
<211> 9
<212> PRT
<213> Homo sapiens

<400> 552
Gln Phe Glu Lys Ile Ala Glu Glu Leu
1               5


<210> 553
<211> 10
<212> PRT
<213> Homo sapiens

<400> 553
Lys Gln Phe Glu Lys Ile Ala Glu Glu Leu
1               5                   10


<210> 554
<211> 8
<212> PRT
<213> Homo sapiens

<400> 554
Asp Asn Lys Gln Phe Glu Lys Ile
1               5


<210> 555
<211> 9
<212> PRT
<213> Homo sapiens

<400> 555
Tyr Asp Asn Lys Gln Phe Glu Lys Ile
1               5


<210> 556
<211> 10
<212> PRT
<213> Homo sapiens

<400> 556
Leu Tyr Asp Asn Lys Gln Phe Glu Lys Ile
1               5                   10


<210> 557
<211> 8
<212> PRT
<213> Homo sapiens

<400> 557
Leu Gly Glu Lys Glu Thr Leu Leu
1               5


<210> 558
<211> 9
<212> PRT
<213> Homo sapiens

<400> 558
Val Leu Gly Glu Lys Glu Thr Leu Leu
1               5


<210> 559
<211> 10
<212> PRT
<213> Homo sapiens

<400> 559
Lys Val Leu Gly Glu Lys Glu Thr Leu Leu
1               5                   10


<210> 560
<211> 9
<212> PRT
<213> Homo sapiens

<400> 560
Leu Leu Arg Thr Glu Gln Gln Arg Leu
1               5


<210> 561

<211> 10
<212> PRT
<213> Homo sapiens

<400> 561
Glu Leu Leu Arg Thr Glu Gln Gln Arg Leu
1               5                   10


<210> 562
<211> 9
<212> PRT
<213> Homo sapiens

<400> 562
Thr Glu Gln Gln Arg Leu Glu Asn Tyr
1               5


<210> 563
<211> 10
<212> PRT
<213> Homo sapiens

<400> 563
Arg Thr Glu Gln Gln Arg Leu Glu Asn Tyr
1               5                   10


<210> 564
<211> 9
<212> PRT
<213> Homo sapiens

<400> 564
Glu Asp Gln Leu Ile Ile Leu Thr Met
1               5


<210> 565
<211> 10
<212> PRT
<213> Homo sapiens

<400> 565
Arg Leu Glu Asn Tyr Glu Asp Gln Leu Ile
1               5                   10


<210> 566
<211> 8
<212> PRT
<213> Homo sapiens

<400> 566
Lys Ala Arg Ala Ala His Ser Phe
1               5

<210> 567
<211> 9
<212> PRT
<213> Homo sapiens

<400> 567
Val Val Thr Glu Phe Glu Thr Thr Val
1               5

<210> 568
<211> 10
<212> PRT
<213> Homo sapiens

<400> 568
Phe Val Val Thr Glu Phe Glu Thr Thr Val
1               5                   10

<210> 569
<211> 9
<212> PRT
<213> Homo sapiens

<400> 569
Val Thr Glu Phe Glu Thr Thr Val Cys
1               5

<210> 570
<211> 10
<212> PRT
<213> Homo sapiens

<400> 570
Val Val Thr Glu Phe Glu Thr Thr Val Cys
1               5                   10

<210> 571
<211> 9
<212> PRT
<213> Homo sapiens

<400> 571
Asp Leu Gln Ile Ala Thr Asn Thr Ile
1               5

<210> 572
<211> 9
<212> PRT
<213> Homo sapiens

<400> 572
Ile Ala Thr Asn Thr Ile Cys Gln Leu
1               5

<210> 573
<211> 10
<212> PRT
<213> Homo sapiens

<400> 573
Gln Ile Ala Thr Asn Thr Ile Cys Gln Leu
1               5                   10

<210> 574
<211> 9
<212> PRT
<213> Homo sapiens

<400> 574
Val Met Thr Lys Leu Gly Phe Lys Tyr
1               5

<210> 575
<211> 9
<212> PRT
<213> Homo sapiens

<400> 575
Leu Asn Tyr Glu Val Met Thr Lys Leu
1               5

<210> 576
<211> 10
<212> PRT
<213> Homo sapiens

<400> 576
Lys Leu Asn Tyr Glu Val Met Thr Lys Leu
1               5                   10

<210> 577
<211> 9
<212> PRT
<213> Homo sapiens

<400> 577
Thr Leu Pro Pro Phe Met Arg Ser Lys
1               5

<210> 578
<211> 9

<212> PRT
<213> Homo sapiens

<400> 578
Lys Ile Met Pro Lys Lys Pro Ala Glu
1               5


<210> 579
<211> 10
<212> PRT
<213> Homo sapiens

<400> 579
Ser Leu Gln Arg Ile Phe Pro Lys Ile Met
1               5                   10


<210> 580
<211> 9
<212> PRT
<213> Homo sapiens

<400> 580
Tyr Ile Lys Ser Tyr Leu Glu Gln Ala
1               5


<210> 581
<211> 9
<212> PRT
<213> Homo sapiens

<400> 581
Ser Phe Gln Asp Tyr Ile Lys Ser Tyr
1               5


<210> 582
<211> 10
<212> PRT
<213> Homo sapiens

<400> 582
Asp Ser Phe Gln Asp Tyr Ile Lys Ser Tyr
1               5                   10


<210> 583
<211> 8
<212> PRT
<213> Homo sapiens

<400> 583
Leu Pro Glu Glu Lys Gln Pro Leu
1               5

<210> 584
<211> 9
<212> PRT
<213> Homo sapiens

<400> 584
Gln Leu Pro Glu Glu Lys Gln Pro Leu
1               5


<210> 585
<211> 10
<212> PRT
<213> Homo sapiens

<400> 585
Lys Gln Leu Pro Glu Glu Lys Gln Pro Leu
1               5                   10


<210> 586
<211> 9
<212> PRT
<213> Homo sapiens

<400> 586
Leu Pro Glu Glu Lys Gln Pro Leu Leu
1               5


<210> 587
<211> 10
<212> PRT
<213> Homo sapiens

<400> 587
Gln Leu Pro Glu Glu Lys Gln Pro Leu Leu
1               5                   10


<210> 588
<211> 9
<212> PRT
<213> Homo sapiens

<400> 588
Ser Leu Leu Cys Arg His Lys Arg Lys
1               5


<210> 589
<211> 91
<212> PRT
<213> Homo sapiens

<400> 589

Glu Val Pro Gln Leu Thr Asp Leu Ser Phe Val Asp Ile Thr Asp Ser
1               5                   10                  15
Ser Ile Gly Leu Arg Trp Thr Pro Leu Asn Ser Ser Thr Ile Ile Gly
            20                  25                  30
Tyr Arg Ile Thr Val Val Ala Ala Gly Glu Gly Ile Pro Ile Phe Glu
        35                  40                  45
Asp Phe Val Asp Ser Ser Val Gly Tyr Tyr Thr Val Thr Gly Leu Glu
    50                  55                  60
Pro Gly Ile Asp Tyr Asp Ile Ser Val Ile Thr Leu Ile Asn Gly Gly
65                  70                  75                  80
Glu Ser Ala Pro Thr Thr Leu Thr Gln Gln Thr
                85                  90

<210> 590
<211> 147
<212> PRT
<213> Homo sapiens

<400> 590
Cys Thr Phe Asp Asn Leu Ser Pro Gly Leu Glu Tyr Asn Val Ser Val
1               5                   10                  15
Tyr Thr Val Lys Asp Asp Lys Glu Ser Val Pro Ile Ser Asp Thr Ile
            20                  25                  30
Ile Pro Glu Val Pro Gln Leu Thr Asp Leu Ser Phe Val Asp Ile Thr
        35                  40                  45
Asp Ser Ser Ile Gly Leu Arg Trp Thr Pro Leu Asn Ser Ser Thr Ile
    50                  55                  60
Ile Gly Tyr Arg Ile Thr Val Val Ala Ala Gly Glu Gly Ile Pro Ile
65                  70                  75                  80
Phe Glu Asp Phe Val Asp Ser Ser Val Gly Tyr Tyr Thr Val Thr Gly
                85                  90                  95
Leu Glu Pro Gly Ile Asp Tyr Asp Ile Ser Val Ile Thr Leu Ile Asn
            100                 105                 110
Gly Gly Glu Ser Ala Pro Thr Thr Leu Thr Gln Gln Thr Ala Val Pro
        115                 120                 125
Pro Pro Thr Asp Leu Arg Phe Thr Asn Ile Gly Pro Asp Thr Met Arg
    130                 135                 140
Val Thr Trp
145

<210> 591
<211> 2823
<212> DNA
<213> Homo sapiens

<400> 591
ctgcactttt gataacctga gtcccggcct ggagtacaat gtcagtgttt acactgtcaa 60
ggatgacaag gaaagtgtcc ctatctctga taccatcatc ccaggtaata gaaaataagc 120
tgctatcctg agagtgacat tccaataaga gtggggatta gcatcttaat ccccagatgc 180
ttaagggtgt caactatatt tgggatttaa ttccgatctc ccagctgcac tttccaaaac 240
caagaagtca aagcagcgat ttggacaaaa tgcttgctgt taacactgct ttactgtctg 300
tgcttcactg ggatgctgtg tgttgcagcg agtatgtaat ggagtggcag ccatggcttt 360
aactctgtat tgtctgctca catggaagta tgactaaaac actgtcacgt gtctgtactc 420
agtactgata ggctcaaagt aatatggtaa atgcatccca tcagtacatt tctgcccgat 480
tttacaatcc atatcaattt ccaacagctg cctatttcat cttgcagttt caaatccttc 540

tttttgaaaa ttggatttta aaaaaaagtt aagtaaaagt cacaccttca gggttgttct 600
ttcttgtggc cttgaaagac aacattgcaa aggcctgtcc taaggatagg cttgtttgtc 660
cattgggtta taacataatg aaagcattgg acagatcgtg tccccctttg gactcttcag 720
tagaatgctt ttactaacgc taattacatg ttttgattat gaatgaacct aaaatagtgg 780
caatggcctt aacctaggcc tgtctttcct cagcctgaat gtgcttttga atggcacatt 840
tcacaccata cattcataat gcattagcgt tatggccatg atgttgtcat gagttttgta 900
tgggagaaaa aaaatcaatt tatcacccat ttattatttt ttccggttgt tcatgcaagc 960
ttattttcta ctaaaacagt tttggaatta ttaaaagcat tgctgatact tacttcagat 1020
attatgtcta ggctctaaga atggtttcga catcctaaac agccatatga tttttaggaa 1080
tctgaacagt tcaaattgta ccctttaagg atgttttcaa aatgtaaaaa atatatatat 1140
atatatatat tccctaaaag aatattcctg tttattcttc tagggaagca aactgttcat 1200
gatgcttagg aagtcttttc agagaattta aaacagattg catattacca tcattgcttt 1260
aacattccac caattttact actagtaacc tgatatacac tgctttattt tttcctcttt 1320
ttttccctct attttccttt tgcctccccc tccctttgct ttgtaactca atagaggtgc 1380
cccaactcac tgacctaagc tttgttgata taaccgattc aagcatcggc ctgaggtgga 1440
ccccgctaaa ctcttccacc attattgggt accgcatcac agtagttgcg gcaggagaag 1500
gtatccctat ttttgaagat tttgtggact cctcagtagg atactacaca gtcacagggc 1560
tggagccggg cattgactat gatatcagcg ttatcactct cattaatggc ggcgagagtg 1620
cccctactac actgacacaa caaacgggtg aattttgaaa acttctgcgt ttgagacata 1680
gatggtgttg catgctgcca ccagttactc cggttaaata tggatgtttc atgggggaag 1740
tcagcaattg gccaaagatt cagataggtg gaattggggg gataaggaat caaatgcatc 1800
tgctaaactg attggagaaa aacacatgca atatcttcag tacactctca tttaaaccac 1860
aagtagatat aaagcctaga gaaatacaga tgtctgctct gttaaatata aaatagcaaa 1920
tgttcattca atttgaagac ctagaatttt tcttcttaaa taccaaacac gaataccaaa 1980
ttgcgtaagt accaattgat aagaatatat caccaaaatg taccatcatg ctcttccttc 2040
taccctttga taaactctac catgctcctt ctttgtagct aaaaacccat caaaatttag 2100
ggtagagtgg atgggcattg ttttgaggta ggagaaaagt aaacttggga ccattctagg 2160
ttttgttgct gtcactaggt aaagaaacac ctctttaacc acagtctggg gacaagcatg 2220
caacatttta aaggttctct gctgtgcatg ggaaaagaaa catgctgaga accaatttgc 2280
atgaacatgt tcacttgtaa gtagaattca ctgaatggaa ctgtagctct agatatctca 2340
catgggggga agtttaggac cctcttgtct ttttgtctgt gtgcatgtat ttctttgtaa 2400
agtactgcta tgtttctctt tgctgtgtgg caacttaagc ctcttcggcc tgggataaaa 2460
taatctgcag tggtattaat aatgtacata aagtcaacat atttgaaagt agattaaaat 2520
cttttttaaa tatatcaatg atggcaaaaa ggttaaaggg ggcctaacag tactgtgtgt 2580
agtgttttat ttttaacagt agtacactat aacttaaaat agacttagat tagactgttt 2640
gcatgattat gattctgttt cctttatgca tgaaatattg attttacctt tccagctact 2700
tcgttagctt taattttaaa atacattaac tgagtcttcc ttcttgttcg aaaccagctg 2760
ttcctcctcc cactgacctg cgattcacca acattggtcc agacaccatg cgtgtcacct 2820
ggg 2823

<210> 592
<211> 702
<212> PRT
<213> Homo sapiens

<400> 592
Met Glu Ser Pro Ser Ala Pro Pro His Arg Trp Cys Ile Pro Trp Gln
1               5                   10                  15
Arg Leu Leu Leu Thr Ala Ser Leu Leu Thr Phe Trp Asn Pro Pro Thr
            20                  25                  30
Thr Ala Lys Leu Thr Ile Glu Ser Thr Pro Phe Asn Val Ala Glu Gly
        35                  40                  45
Lys Glu Val Leu Leu Leu Val His Asn Leu Pro Gln His Leu Phe Gly
    50                  55                  60
Tyr Ser Trp Tyr Lys Gly Glu Arg Val Asp Gly Asn Arg Gln Ile Ile
65                  70                  75                  80
Gly Tyr Val Ile Gly Thr Gln Gln Ala Thr Pro Gly Pro Ala Tyr Ser

                85                  90                  95
Gly Arg Glu Ile Ile Tyr Pro Asn Ala Ser Leu Leu Ile Gln Asn Ile
            100                 105                 110
Ile Gln Asn Asp Thr Gly Phe Tyr Thr Leu His Val Ile Lys Ser Asp
        115                 120                 125
Leu Val Asn Glu Glu Ala Thr Gly Gln Phe Arg Val Tyr Pro Glu Leu
    130                 135                 140
Pro Lys Pro Ser Ile Ser Ser Asn Asn Ser Lys Pro Val Glu Asp Lys
145                 150                 155                 160
Asp Ala Val Ala Phe Thr Cys Glu Pro Glu Thr Gln Asp Ala Thr Tyr
                165                 170                 175
Leu Trp Trp Val Asn Asn Gln Ser Leu Pro Val Ser Pro Arg Leu Gln
            180                 185                 190
Leu Ser Asn Gly Asn Arg Thr Leu Thr Leu Phe Asn Val Thr Arg Asn
        195                 200                 205
Asp Thr Ala Ser Tyr Lys Cys Glu Thr Gln Asn Pro Val Ser Ala Arg
    210                 215                 220
Arg Ser Asp Ser Val Ile Leu Asn Val Leu Tyr Gly Pro Asp Ala Pro
225                 230                 235                 240
Thr Ile Ser Pro Leu Asn Thr Ser Tyr Arg Ser Gly Glu Asn Leu Asn
                245                 250                 255
Leu Ser Cys His Ala Ala Ser Asn Pro Pro Ala Gln Tyr Ser Trp Phe
            260                 265                 270
Val Asn Gly Thr Phe Gln Gln Ser Thr Gln Glu Leu Phe Ile Pro Asn
        275                 280                 285
Ile Thr Val Asn Asn Ser Gly Ser Tyr Thr Cys Gln Ala His Asn Ser
    290                 295                 300
Asp Thr Gly Leu Asn Arg Thr Thr Val Thr Thr Ile Thr Val Tyr Ala
305                 310                 315                 320
Glu Pro Pro Lys Pro Phe Ile Thr Ser Asn Asn Ser Asn Pro Val Glu
                325                 330                 335
Asp Glu Asp Ala Val Ala Leu Thr Cys Glu Pro Glu Ile Gln Asn Thr
            340                 345                 350
Thr Tyr Leu Trp Trp Val Asn Asn Gln Ser Leu Pro Val Ser Pro Arg
        355                 360                 365
Leu Gln Leu Ser Asn Asp Asn Arg Thr Leu Thr Leu Leu Ser Val Thr
    370                 375                 380
Arg Asn Asp Val Gly Pro Tyr Glu Cys Gly Ile Gln Asn Glu Leu Ser
385                 390                 395                 400
Val Asp His Ser Asp Pro Val Ile Leu Asn Val Leu Tyr Gly Pro Asp
                405                 410                 415
Asp Pro Thr Ile Ser Pro Ser Tyr Thr Tyr Tyr Arg Pro Gly Val Asn
            420                 425                 430
Leu Ser Leu Ser Cys His Ala Ala Ser Asn Pro Pro Ala Gln Tyr Ser
        435                 440                 445
Trp Leu Ile Asp Gly Asn Ile Gln Gln His Thr Gln Glu Leu Phe Ile
    450                 455                 460
Ser Asn Ile Thr Glu Lys Asn Ser Gly Leu Tyr Thr Cys Gln Ala Asn
465                 470                 475                 480
Asn Ser Ala Ser Gly His Ser Arg Thr Thr Val Lys Thr Ile Thr Val
                485                 490                 495
Ser Ala Glu Leu Pro Lys Pro Ser Ile Ser Ser Asn Asn Ser Lys Pro
            500                 505                 510
Val Glu Asp Lys Asp Ala Val Ala Phe Thr Cys Glu Pro Glu Ala Gln
        515                 520                 525
Asn Thr Thr Tyr Leu Trp Trp Val Asn Gly Gln Ser Leu Pro Val Ser
    530                 535                 540

Pro Arg Leu Gln Leu Ser Asn Gly Asn Arg Thr Leu Thr Leu Phe Asn
545                 550                 555                 560
Val Thr Arg Asn Asp Ala Arg Ala Tyr Val Cys Gly Ile Gln Asn Ser
                565                 570                 575
Val Ser Ala Asn Arg Ser Asp Pro Val Thr Leu Asp Val Leu Tyr Gly
            580                 585                 590
Pro Asp Thr Pro Ile Ile Ser Pro Pro Asp Ser Ser Tyr Leu Ser Gly
        595                 600                 605
Ala Asn Leu Asn Leu Ser Cys His Ser Ala Ser Asn Pro Ser Pro Gln
    610                 615                 620
Tyr Ser Trp Arg Ile Asn Gly Ile Pro Gln Gln His Thr Gln Val Leu
625                 630                 635                 640
Phe Ile Ala Lys Ile Thr Pro Asn Asn Asn Gly Thr Tyr Ala Cys Phe
                645                 650                 655
Val Ser Asn Leu Ala Thr Gly Arg Asn Asn Ser Ile Val Lys Ser Ile
            660                 665                 670
Thr Val Ser Ala Ser Gly Thr Ser Pro Gly Leu Ser Ala Gly Ala Thr
        675                 680                 685
Val Gly Ile Met Ile Gly Val Leu Val Gly Val Ala Leu Ile
    690                 695                 700

<210> 593
<211> 2974
<212> DNA
<213> Homo sapiens

<400> 593
ctcagggcag agggaggaag gacagcagac cagacagtca cagcagcctt gacaaaacgt 60
tcctggaact caagctcttc tccacagagg aggacagagc agacagcaga gaccatggag 120
tctccctcgg cccctcccca cagatggtgc atcccctggc agaggctcct gctcacagcc 180
tcacttctaa ccttctggaa cccgcccacc actgccaagc tcactattga atccacgccg 240
ttcaatgtcg cagaggggaa ggaggtgctt ctacttgtcc acaatctgcc ccagcatctt 300
tttggctaca gctggtacaa aggtgaaaga gtggatggca accgtcaaat tataggatat 360
gtaataggaa ctcaacaagc taccccaggg cccgcataca gtggtcgaga gataatatac 420
cccaatgcat ccctgctgat ccagaacatc atccagaatg acacaggatt ctacacccta 480
cacgtcataa agtcagatct tgtgaatgaa gaagcaactg gccagttccg ggtatacccg 540
gagctgccca agccctccat ctccagcaac aactccaaac ccgtggagga caaggatgct 600
gtggccttca cctgtgaacc tgagactcag gacgcaacct acctgtggtg ggtaaacaat 660
cagagcctcc cggtcagtcc caggctgcag ctgtccaatg gcaacaggac cctcactcta 720
ttcaatgtca caagaaatga cacagcaagc tacaaatgtg aaacccagaa cccagtgagt 780
gccaggcgca gtgattcagt catcctgaat gtcctctatg gcccggatgc ccccaccatt 840
tcccctctaa acacatctta cagatcaggg gaaaatctga acctctcctg ccacgcagcc 900
tctaacccac ctgcacagta ctcttggttt gtcaatggga ctttccagca atccacccaa 960
gagctcttta tccccaacat cactgtgaat aatagtggat cctatacgtg ccaagcccat 1020
aactcagaca ctggcctcaa taggaccaca gtcacgacga tcacagtcta tgcagagcca 1080
cccaaaccct tcatcaccag caacaactcc aaccccgtgg aggatgagga tgctgtagcc 1140
ttaacctgtg aacctgagat tcagaacaca acctacctgt ggtcggtaaa taatcagagc 1200
ctcccggtca gtcccaggct gcagctgtcc aatgacaaca ggaccctcac tctactcagt 1260
gtcacaagga atgatgtagg accctatgag tgtggaatcc agaacgaatt aagtgttgac 1320
cacagcgacc cagtcatcct gaatgtcctc tatggcccag acgaccccac catttccccc 1380
tcatacacct attaccgtcc aggggtgaac ctcagcctct cctgccatgc agcctctaac 1440
ccacctgcac agtattcttg gctgattgat gggaacatcc agcaacacac acaagagctc 1500
tttatctcca acatcactga gaagaacagc ggactctata cctgccaggc caataactca 1560
gccagtggcc acagcaggac tacagtcaag acaatcacag tctctgcgga gctgcccaag 1620
ccctccatct ccagcaacaa ctccaaaccc gtggaggaca aggatgctgt ggccttcacc 1680
tgtgaacctg aggctcagaa cacaacctac ctgtggtggg taaatggtca gagcctccca 1740

gtcagtccca ggctgcagct gtccaatggc aacaggaccc tcactctatt caatgtcaca 1800
agaaatgacg caagagccta tgtatgtgga atccagaact cagtgagtgc aaaccgcagt 1860
gacccagtca ccctggatgt cctctatggg ccggacaccc ccatcatttc ccccccagac 1920
tcgtcttacc tttcgggagc gaacctcaac ctctcctgcc actcggcctc taacccatcc 1980
ccgcagtatt cttggcgtat caatgggata ccgcagcaac acacacaagt tctctttatc 2040
gccaaaatca cgccaaataa taacgggacc tatgcctgtt ttgtctctaa cttggctact 2100
ggccgcaata attccatagt caagagcatc acagtctctg catctggaac ttctcctggt 2160
ctctcagctg gggccactgt cggcatcatg attggagtgc tggttggggt tgctctgata 2220
tagcagccct ggtgtagttt cttcatttca ggaagactga cagttgtttt gcttcttcct 2280
taaagcattt gcaacagcta cagtctaaaa ttgcttcttt accaaggata tttacagaaa 2340
agactctgac cagagatcga gaccatccta gccaacatcg tgaaacccca tctctactaa 2400
aaatacaaaa atgagctggg cttggtggcg cgcacctgta gtcccagtta ctcgggaggc 2460
tgaggcagga gaatcgcttg aacccgggag gtggagattg cagtgagccc agatcgcacc 2520
actgcactcc agtctggcaa cagagcaaga ctccatctca aaaagaaaag aaaagaagac 2580
tctgacctgt actcttgaat acaagtttct gataccactg cactgtctga gaatttccaa 2640
aactttaatg aactaactga cagcttcatg aaactgtcca ccaagatcaa gcagagaaaa 2700
taattaattt catgggacta aatgaactaa tgaggattgc tgattcttta aatgtcttgt 2760
ttcccagatt tcaggaaact ttttttcttt taagctatcc actcttacag caatttgata 2820
aaatatactt ttgtgaacaa aaattgagac atttacattt tctccctatg tggtcgctcc 2880
agacttggga aactattcat gaatatttat attgtatggt aatatagtta ttgcacaagt 2940
tcaataaaaa tctgctcttt gtataacaga aaaa 2974

<210> 594
<211> 1255
<212> PRT
<213> Homo sapiens

<400> 594
Met Glu Leu Ala Ala Leu Cys Arg Trp Gly Leu Leu Leu Ala Leu Leu
1               5                   10                  15
Pro Pro Gly Ala Ala Ser Thr Gln Val Cys Thr Gly Thr Asp Met Lys
            20                  25                  30
Leu Arg Leu Pro Ala Ser Pro Glu Thr His Leu Asp Met Leu Arg His
        35                  40                  45
Leu Tyr Gln Gly Cys Gln Val Val Gln Gly Asn Leu Glu Leu Thr Tyr
    50                  55                  60
Leu Pro Thr Asn Ala Ser Leu Ser Phe Leu Gln Asp Ile Gln Glu Val
65                  70                  75                  80
Gln Gly Tyr Val Leu Ile Ala His Asn Gln Val Arg Gln Val Pro Leu
                85                  90                  95
Gln Arg Leu Arg Ile Val Arg Gly Thr Gln Leu Phe Glu Asp Asn Tyr
            100                 105                 110
Ala Leu Ala Val Leu Asp Asn Gly Asp Pro Leu Asn Asn Thr Thr Pro
        115                 120                 125
Val Thr Gly Ala Ser Pro Gly Gly Leu Arg Glu Leu Gln Leu Arg Ser
    130                 135                 140
Leu Thr Glu Ile Leu Lys Gly Gly Val Leu Ile Gln Arg Asn Pro Gln
145                 150                 155                 160
Leu Cys Tyr Gln Asp Thr Ile Leu Trp Lys Asp Ile Phe His Lys Asn
                165                 170                 175
Asn Gln Leu Ala Leu Thr Leu Ile Asp Thr Asn Arg Ser Arg Ala Cys
            180                 185                 190
His Pro Cys Ser Pro Met Cys Lys Gly Ser Arg Cys Trp Gly Glu Ser
        195                 200                 205
Ser Glu Asp Cys Gln Ser Leu Thr Arg Thr Val Cys Ala Gly Gly Cys
    210                 215                 220
Ala Arg Cys Lys Gly Pro Leu Pro Thr Asp Cys Cys His Glu Gln Cys

225                 230                 235                 240
Ala Ala Gly Cys Thr Gly Pro Lys His Ser Asp Cys Leu Ala Cys Leu
                245                 250                 255
His Phe Asn His Ser Gly Ile Cys Glu Leu His Cys Pro Ala Leu Val
            260                 265                 270
Thr Tyr Asn Thr Asp Thr Phe Glu Ser Met Pro Asn Pro Glu Gly Arg
        275                 280                 285
Tyr Thr Phe Gly Ala Ser Cys Val Thr Ala Cys Pro Tyr Asn Tyr Leu
    290                 295                 300
Ser Thr Asp Val Gly Ser Cys Thr Leu Val Cys Pro Leu His Asn Gln
305                 310                 315                 320
Glu Val Thr Ala Glu Asp Gly Thr Gln Arg Cys Glu Lys Cys Ser Lys
                325                 330                 335
Pro Cys Ala Arg Val Cys Tyr Gly Leu Gly Met Glu His Leu Arg Glu
            340                 345                 350
Val Arg Ala Val Thr Ser Ala Asn Ile Gln Glu Phe Ala Gly Cys Lys
        355                 360                 365
Lys Ile Phe Gly Ser Leu Ala Phe Leu Pro Glu Ser Phe Asp Gly Asp
    370                 375                 380
Pro Ala Ser Asn Thr Ala Pro Leu Gln Pro Glu Gln Leu Gln Val Phe
385                 390                 395                 400
Glu Thr Leu Glu Glu Ile Thr Gly Tyr Leu Tyr Ile Ser Ala Trp Pro
                405                 410                 415
Asp Ser Leu Pro Asp Leu Ser Val Phe Gln Asn Leu Gln Val Ile Arg
            420                 425                 430
Gly Arg Ile Leu His Asn Gly Ala Tyr Ser Leu Thr Leu Gln Gly Leu
        435                 440                 445
Gly Ile Ser Trp Leu Gly Leu Arg Ser Leu Arg Glu Leu Gly Ser Gly
    450                 455                 460
Leu Ala Leu Ile His His Asn Thr His Leu Cys Phe Val His Thr Val
465                 470                 475                 480
Pro Trp Asp Gln Leu Phe Arg Asn Pro His Gln Ala Leu Leu His Thr
                485                 490                 495
Ala Asn Arg Pro Glu Asp Glu Cys Val Gly Glu Gly Leu Ala Cys His
            500                 505                 510
Gln Leu Cys Ala Arg Gly His Cys Trp Gly Pro Gly Pro Thr Gln Cys
        515                 520                 525
Val Asn Cys Ser Gln Phe Leu Arg Gly Gln Glu Cys Val Glu Glu Cys
    530                 535                 540
Arg Val Leu Gln Gly Leu Pro Arg Glu Tyr Val Asn Ala Arg His Cys
545                 550                 555                 560
Leu Pro Cys His Pro Glu Cys Gln Pro Gln Asn Gly Ser Val Thr Cys
                565                 570                 575
Phe Gly Pro Glu Ala Asp Gln Cys Val Ala Cys Ala His Tyr Lys Asp
            580                 585                 590
Pro Pro Phe Cys Val Ala Arg Cys Pro Ser Gly Val Lys Pro Asp Leu
        595                 600                 605
Ser Tyr Met Pro Ile Trp Lys Phe Pro Asp Glu Glu Gly Ala Cys Gln
    610                 615                 620
Pro Cys Pro Ile Asn Cys Thr His Ser Cys Val Asp Leu Asp Asp Lys
625                 630                 635                 640
Gly Cys Pro Ala Glu Gln Arg Ala Ser Pro Leu Thr Ser Ile Val Ser
                645                 650                 655
Ala Val Val Gly Ile Leu Leu Val Val Val Leu Gly Val Val Phe Gly
            660                 665                 670
Ile Leu Ile Lys Arg Arg Gln Gln Lys Ile Arg Lys Tyr Thr Met Arg
        675                 680                 685

Arg Leu Leu Gln Glu Thr Glu Leu Val Glu Pro Leu Thr Pro Ser Gly
    690                 695                 700
Ala Met Pro Asn Gln Ala Gln Met Arg Ile Leu Lys Glu Thr Glu Leu
705                 710                 715                 720
Arg Lys Val Lys Val Leu Gly Ser Gly Ala Phe Gly Thr Val Tyr Lys
                725                 730                 735
Gly Ile Trp Ile Pro Asp Gly Glu Asn Val Lys Ile Pro Val Ala Ile
            740                 745                 750
Lys Val Leu Arg Glu Asn Thr Ser Pro Lys Ala Asn Lys Glu Ile Leu
        755                 760                 765
Asp Glu Ala Tyr Val Met Ala Gly Val Gly Ser Pro Tyr Val Ser Arg
    770                 775                 780
Leu Leu Gly Ile Cys Leu Thr Ser Thr Val Gln Leu Val Thr Gln Leu
785                 790                 795                 800
Met Pro Tyr Gly Cys Leu Leu Asp His Val Arg Glu Asn Arg Gly Arg
                805                 810                 815
Leu Gly Ser Gln Asp Leu Leu Asn Trp Cys Met Gln Ile Ala Lys Gly
            820                 825                 830
Met Ser Tyr Leu Glu Asp Val Arg Leu Val His Arg Asp Leu Ala Ala
        835                 840                 845
Arg Asn Val Leu Val Lys Ser Pro Asn His Val Lys Ile Thr Asp Phe
    850                 855                 860
Gly Leu Ala Arg Leu Leu Asp Ile Asp Glu Thr Glu Tyr His Ala Asp
865                 870                 875                 880
Gly Gly Lys Val Pro Ile Lys Trp Met Ala Leu Glu Ser Ile Leu Arg
                885                 890                 895
Arg Arg Phe Thr His Gln Ser Asp Val Trp Ser Tyr Gly Val Thr Val
            900                 905                 910
Trp Glu Leu Met Thr Phe Gly Ala Lys Pro Tyr Asp Gly Ile Pro Ala
        915                 920                 925
Arg Glu Ile Pro Asp Leu Leu Glu Lys Gly Glu Arg Leu Pro Gln Pro
    930                 935                 940
Pro Ile Cys Thr Ile Asp Val Tyr Met Ile Met Val Lys Cys Trp Met
945                 950                 955                 960
Ile Asp Ser Glu Cys Arg Pro Arg Phe Arg Glu Leu Val Ser Glu Phe
                965                 970                 975
Ser Arg Met Ala Arg Asp Pro Gln Arg Phe Val Val Ile Gln Asn Glu
            980                 985                 990
Asp Leu Gly Pro Ala Ser Pro Leu Asp Ser Thr Phe Tyr Arg Ser Leu
        995                 1000                1005
Leu Glu Asp Asp Asp Met Gly Asp Leu Val Asp Ala Glu Glu Tyr Leu
    1010                1015                1020
Val Pro Gln Gln Gly Phe Phe Cys Pro Asp Pro Ala Pro Gly Ala Gly
1025                1030                1035                1040
Gly Met Val His His Arg His Arg Ser Ser Ser Thr Arg Ser Gly Gly
                1045                1050                1055
Gly Asp Leu Thr Leu Gly Leu Glu Pro Ser Glu Glu Glu Ala Pro Arg
            1060                1065                1070
Ser Pro Leu Ala Pro Ser Glu Gly Ala Gly Ser Asp Val Phe Asp Gly
        1075                1080                1085
Asp Leu Gly Met Gly Ala Ala Lys Gly Leu Gln Ser Leu Pro Thr His
    1090                1095                1100
Asp Pro Ser Pro Leu Gln Arg Tyr Ser Glu Asp Pro Thr Val Pro Leu
1105                1110                1115                1120
Pro Ser Glu Thr Asp Gly Tyr Val Ala Pro Leu Thr Cys Ser Pro Gln
                1125                1130                1135
Pro Glu Tyr Val Asn Gln Pro Asp Val Arg Pro Gln Pro Pro Ser Pro

1140                1145                1150
Arg Glu Gly Pro Leu Pro Ala Ala Arg Pro Ala Gly Ala Thr Leu Glu
        1155                1160                1165
Arg Ala Lys Thr Leu Ser Pro Gly Lys Asn Gly Val Val Lys Asp Val
    1170                1175                1180
Phe Ala Phe Gly Gly Ala Val Glu Asn Pro Glu Tyr Leu Thr Pro Gln
1185                1190                1195                1200
Gly Gly Ala Ala Pro Gln Pro His Pro Pro Pro Ala Phe Ser Pro Ala
                1205                1210                1215
Phe Asp Asn Leu Tyr Tyr Trp Asp Gln Asp Pro Pro Glu Arg Gly Ala
            1220                1225                1230
Pro Pro Ser Thr Phe Lys Gly Thr Pro Thr Ala Glu Asn Pro Glu Tyr
        1235                1240                1245
Leu Gly Leu Asp Val Pro Val
    1250                1255

<210> 595
<211> 4530
<212> DNA
<213> Homo sapiens

<400> 595
   aattctcgag ctcgtcgacc ggtcgacgag ctcgagggtc gacgagctcg agggcgcgcg 60
   cccgcccccc acccctcgca gcaccccgcg ccccgcgccc tcccagccgg gtccagccgg 120
   agccatgggg ccggagccgc agtgagcacc atggagctgg cggccttgtg ccgctggggg 180
   ctcctcctcg ccctcttgcc ccccggagcc gcgagcaccc aagtgtgcac cggcacagac 240
   atgaagctgc ggctccctgc cagtcccgag acccacctgg acatgctccg ccacctctac 300
   cagggctgcc aggtggtgca gggaaacctg gaactcacct acctgcccac caatgccagc 360
   ctgtccttcc tgcaggatat ccaggaggtg cagggctacg tgctcatcgc tcacaaccaa 420
   gtgaggcagg tcccactgca gaggctgcgg attgtgcgag gcacccagct ctttgaggac 480
   aactatgccc tggccgtgct agacaatgga gacccgctga acaataccac ccctgtcaca 540
   ggggcctccc caggaggcct gcgggagctg cagcttcgaa gcctcacaga gatcttgaaa 600
   ggaggggtct tgatccagcg gaacccccag ctctgctacc aggacacgat tttgtggaag 660
   gacatcttcc acaagaacaa ccagctggct ctcacactga tagacaccaa ccgctctcgg 720
   gcctgccacc cctgttctcc gatgtgtaag ggctcccgct gctggggaga gagttctgag 780
   gattgtcaga gcctgacgcg cactgtctgt gccggtggct gtgcccgctg caaggggcca 840
   ctgcccactg actgctgcca tgagcagtgt gctgccggct gcacgggccc caagcactct 900
   gactgcctgg cctgcctcca cttcaaccac agtggcatct gtgagctgca ctgcccagcc 960
   ctggtcacct acaacacaga cacgtttgag tccatgccca atcccgaggg ccggtataca 1020
   ttcggcgcca gctgtgtgac tgcctgtccc tacaactacc tttctacgga cgtgggatcc 1080
   tgcaccctcg tctgccccct gcacaaccaa gaggtgacag cagaggatgg aacacagcgg 1140
   tgtgagaagt gcagcaagcc ctgtgcccga gtgtgctatg gtctgggcat ggagcacttg 1200
   cgagaggtga gggcagttac cagtgccaat atccaggagt ttgctggctg caagaagatc 1260
   tttgggagcc tggcatttct gccggagagc tttgatgggg acccagcctc caacactgcc 1320
   ccgctccagc cagagcagct ccaagtgttt gagactctgg aagagatcac aggttaccta 1380
   tacatctcag catggccgga cagcctgcct gacctcagcg tcttccagaa cctgcaagta 1440
   atccggggac gaattctgca caatggcgcc tactcgctga ccctgcaagg gctgggcatc 1500
   agctggctgg ggctgcgctc actgagggaa ctgggcagtg gactggccct catccaccat 1560
   aacacccacc tctgcttcgt gcacacggtg ccctgggacc agctctttcg gaacccgcac 1620
   caagctctgc tccacactgc caaccggcca gaggacgagt gtgtgggcga gggcctggcc 1680
   tgccaccagc tgtgcgcccg agggcactgc tggggtccag ggcccaccca gtgtgtcaac 1740
   tgcagccagt tccttcgggg ccaggagtgc gtggaggaat gccgagtact gcaggggctc 1800
   cccagggagt atgtgaatgc caggcactgt ttgccgtgcc accctgagtg tcagccccag 1860
   aatggctcag tgacctgttt tggaccggag gctgaccagt gtgtggcctg tgcccactat 1920
   aaggaccctc ccttctgcgt ggcccgctgc cccagcggtg tgaaacctga cctctcctac 1980
   atgcccatct ggaagtttcc agatgaggag ggcgcatgcc agccttgccc catcaactgc 2040
   acccactcct gtgtggacct ggatgacaag ggctgccccg ccgagcagag agccagccct 2100
   ctgacgtcca tcgtctctgc ggtggttggc attctgctgg tcgtggtctt gggggtggtc 2160
   tttgggatcc tcatcaagcg acggcagcag aagatccgga agtacacgat gcggagactg 2220
   ctgcaggaaa cggagctggt ggagccgctg acacctagcg gagcgatgcc caaccaggcg 2280
   cagatgcgga tcctgaaaga gacggagctg aggaaggtga aggtgcttgg atctggcgct 2340
   tttggcacag tctacaaggg catctggatc cctgatgggg agaatgtgaa aattccagtg 2400
   gccatcaaag tgttgaggga aaacacatcc cccaaagcca acaaagaaat cttagacgaa 2460
   gcatacgtga tggctggtgt gggctcccca tatgtctccc gccttctggg catctgcctg 2520
   acatccacgg tgcagctggt gacacagctt atgccctatg gctgcctctt agaccatgtc 2580
   cgggaaaacc gcggacgcct gggctcccag gacctgctga actggtgtat gcagattgcc 2640
   aaggggatga gctacctgga ggatgtgcgg ctcgtacaca gggacttggc cgctcggaac 2700
   gtgctggtca agagtcccaa ccatgtcaaa attacagact tcgggctggc tcggctgctg 2760
   gacattgacg agacagagta ccatgcagat gggggcaagg tgcccatcaa gtggatggcg 2820
   ctggagtcca ttctccgccg gcggttcacc caccagagtg atgtgtggag ttatggtgtg 2880
   actgtgtggg agctgatgac ttttggggcc aaaccttacg atgggatccc agcccgggag 2940
   atccctgacc tgctggaaaa gggggagcgg ctgccccagc cccccatctg caccattgat 3000
   gtctacatga tcatggtcaa atgttggatg attgactctg aatgtcggcc aagattccgg 3060
   gagttggtgt ctgaattctc ccgcatggcc agggaccccc agcgctttgt ggtcatccag 3120
   aatgaggact tgggcccagc cagtcccttg gacagcacct tctaccgctc actgctggag 3180
   gacgatgaca tgggggacct ggtggatgct gaggagtatc tggtacccca gcagggcttc 3240
   ttctgtccag accctgcccc gggcgctggg ggcatggtcc accacaggca ccgcagctca 3300
   tctaccagga gtggcggtgg ggacctgaca ctagggctgg agccctctga agaggaggcc 3360
   cccaggtctc cactggcacc ctccgaaggg gctggctccg atgtatttga tggtgacctg 3420
   ggaatggggg cagccaaggg gctgcaaagc ctccccacac atgaccccag ccctctacag 3480
   cggtacagtg aggaccccac agtacccctg ccctctgaga ctgatggcta cgttgccccc 3540
   ctgacctgca gcccccagcc tgaatatgtg aaccagccag atgttcggcc ccagccccct 3600
   tcgccccgag agggccctct gcctgctgcc cgacctgctg gtgccactct ggaaagggcc 3660
   aagactctct ccccagggaa gaatggggtc gtcaaagacg tttttgcctt tgggggtgcc 3720
   gtggagaacc ccgagtactt gacaccccag ggaggagctg cccctcagcc ccaccctcct 3780
   cctgccttca gcccagcctt cgacaacctc tattactggg accaggaccc accagagcgg 3840
   ggggctccac ccagcacctt caaagggaca cctacggcag agaacccaga gtacctgggt 3900
   ctggacgtgc cagtgtgaac cagaaggcca agtccgcaga agccctgatg tgtcctcagg 3960
   gagcagggaa ggcctgactt ctgctggcat caagaggtgg gagggccctc cgaccacttc 4020
   caggggaacc tgccatgcca ggaacctgtc ctaaggaacc ttccttcctg cttgagttcc 4080
   cagatggctg gaaggggtcc agcctcgttg gaagaggaac agcactgggg agtctttgtg 4140
   gattctgagg ccctgcccaa tgagactcta gggtccagtg gatgccacag cccagcttgg 4200
   ccctttcctt ccagatcctg ggtactgaaa gccttaggga agctggcctg agaggggaag 4260
   cggccctaag ggagtgtcta agaacaaaag cgacccattc agagactgtc cctgaaacct 4320
   agtactgccc cccatgagga aggaacagca atggtgtcag tatccaggct ttgtacagag 4380
   tgcttttctg tttagttttt actttttttg ttttgttttt ttaaagacga aataaagacc 4440
   caggggagaa tgggtgttgt atggggaggc aagtgtgggg ggtccttctc cacacccact 4500
   ttgtccattt gcaaatatat tttggaaaac 4530

<210> 596
<211> 976
<212> PRT
<213> Homo sapiens

<400> 596

<210> 597
<211> 3393
<212> DNA
<213> Homo sapiens

<400> 597
gccctcatag accgtttgtt gtagttcgcg tgggaacagc aacccacggt ttcccgatag 60
ttcttcaaag atatttacaa ccgtaacaga gaaaatggaa aagcaaaagc cctttgcatt 120
gttcgtacca ccgagatcaa gcagcagtca ggtgtctgcg gtgaaacctc agaccctggg 180
aggcgattcc ac[?]ttcttca agagtttcaa caaatgtact gaagatgatt tggagtttcc 240
atttgcaaag actaatctct ccaaaaatgg ggaaaacatt gattcagatc ctgctttaca 300
aaaagttaat ttcttgcccg tgcttgagca ggttggtaat tctgactgtc actatcagga 360
aggactaaaa gactctgatt tggagaattc agagggattg agcagagtgt tttcaaaact 420
gtataaggag gctgaaaaga taaaaaaatg gaaagtaagt acagaagctg aactgagaca 480
gaaagaaagt aagttgcaag aaaacagaaa gataattgaa gcacagcgaa aagccattca 540
ggaactgcaa tttggaaatg aaaaagtaag tttgaaatta gaagaaggaa tacaagaaaa 600
taaagattta ataaaagaga ataatgccac aaggcattta tgtaatctac tcaaagaaac 660
ctgtgctaga tctgcagaaa agacaaagaa atatgaatat gaacgggaag aaaccaggca 720
agtttatatg gatctaaata ataacattga gaaaatgata acagctcatg gggaacttcg 780
tgtgcaagct gagaattcca gactggaaat gcattttaag ttaaaggaag attatgaaaa 840
aatccaacac cttgaacaag aatacaagaa ggaaataaat gacaaggaaa agcaggtatc 900
actactattg atccaaatca ctgagaaaga aaataaaatg aaagatttaa catttctgct 960
agaggaatcc agagataaag ttaatcaatt agaggaaaag acaaaattac agagtgaaaa 1020
cttaaaacaa tcaattgaga aacagcatca tttgactaaa gaactagaag atattaaagt 1080
gtcattacaa agaagtgtga gtactcaaaa ggctttagag gaagatttac agatagcaac 1140
aaaaacaatt tgtcagctaa ctgaagaaaa agaaactcaa atggaagaat ctaataaagc 1200
tagagctgct cattcgtttg tggttactga atttgaaact actgtctgca gcttggaaga 1260
attattgaga acagaacagc aaagattgga aaaaaatgaa gatcaattga aaatacttac 1320
catggagctt caaaagaaat caagtgagct ggaagagatg actaagctta caaataacaa 1380
agaagtagaa cttgaagaat tgaaaaaagt cttgggagaa aaggaaacac ttttatatga 1440
aaataaacaa tttgagaaga ttgctgaaga attaaaagga acagaacaag aactaattgg 1500
tcttctccaa gccagagaga aagaagtaca tgatttggaa atacagttaa ctgccattac 1560
cacaagtgaa cagtattatt caaaagaggt taaagatcta aaaactgagc ttgaaaacga 1620
gaagcttaag aatactgaat taacttcaca ctgcaacaag ctttcactag aaaacaaaga 1680
gctcacacag gaaacaagtg atatgaccct agaactcaag aatcagcaag aagatattaa 1740
taataacaaa aagcaagaag aaaggatgtt gaaacaaata gaaaatcttc aagaaacaga 1800
aacccaatta agaaatgaac tagaatatgt gagagaagag ctaaaacaga aaagagatga 1860
agttaaatgt aaattggaca agagtgaaga aaattgtaac aatttaagga aacaagttga 1920
aaataaaaac aagtatattg aagaacttca gcaggagaat aaggccttga aaaaaaaagg 1980
tacagcagaa agcaagcaac tgaatgttta tgagataaag gtcaataaat tagagttaga 2040
actagaaagt gccaaacaga aatttggaga aatcacagac acctatcaga aagaaattga 2100
ggacaaaaag atatcagaag aaaatctttt ggaagaggtt gagaaagcaa aagtaatagc 2160
tgatgaagca gtaaaattac agaaagaaat tgataagcga tgtcaacata aaatagctga 2220
aatggtagca cttatggaaa aacataagca ccaatatgat aagatcattg aagaaagaga 2280
ctcagaatta ggactttata agagcaaaga acaagaacag tcatcactga gagcatcttt 2340
ggagattgaa ctatccaatc tcaaagctga acttttgtct gttaagaagc aacttgaaat 2400
agaaagagaa gagaaggaaa aactcaaaag agaggcaaaa gaaaacacag ctactcttaa 2460
agaaaaaaaa gacaagaaaa cacaaacatt tttattggaa acacctgaaa tttattggaa 2520
attggattct aaagcagttc cttcacaaac tgtatctcga aatttcacat cagttgatca 2580
tggcatatcc aaagataaaa gagactatct gtggacatct gccaaaaata ctttatctac 2640
accattgcca aaggcatata cagtgaagac accaacaaaa ccaaaactac agcaaagaga 2700
aaacttgaat atacccattg aagaaagtaa aaaaaagaga aaaatggcct ttgaatttga 2760
tattaattca gatagttcag aaactactga tcttttgagc atggtttcag aagaagagac 2820
attgaaaaca ctgtatagga acaataatcc accagcttct catctttgtg tcaaaacacc 2880
aaaaaaggcc ccttcatctc taacaacccc tggacctaca ctgaagtttg gagctataag 2940

aaaaatgcgg gaggaccgtt gggctgtaat tgctaaaatg gatagaaaaa aaaaactaaa 3000
agaagctgaa aagttatttg tttaatttca gagaatcagt gtagttaagg agcctaataa 3060
cgtgaaactt atagttaata ttttgttctt atttgccaga gccacatttt atctggaagt 3120
tgagacttaa aaaatacttg catgaatgat ttgtgtttct ttatattttt agcctaaatg 3180
ttaactacat attgtctgga aacctgtcat tgtattcaga taattagatg attatatatt 3240
gttgttactt tttcttgtat tcatgaaaac tgtttttact aagttttcaa atttgtaaag 3300
ttagcctttg aatgctagga atgcattatt gagggtcatt ctttattctt tactattaaa 3360
atattttgga tgcaaaaaaa aaaaaaaaaa aaa 3393

<210> 598
<211> 188
<212> PRT
<213> Homo sapiens

<400> 598
Met Asn Gly Asp Asp Ala Phe Ala Arg Arg Pro Arg Asp Asp Ala Gln
1               5                   10                  15
Ile Ser Glu Lys Leu Arg Lys Ala Phe Asp Asp Ile Ala Lys Tyr Phe
            20                  25                  30
Ser Lys Lys Glu Trp Glu Lys Met Lys Ser Ser Glu Lys Ile Val Tyr
        35                  40                  45
Val Tyr Met Lys Leu Asn Tyr Glu Val Met Thr Lys Leu Gly Phe Lys
    50                  55                  60
Val Thr Leu Pro Pro Phe Met Arg Ser Lys Arg Ala Ala Asp Phe His
65                  70                  75                  80
Gly Asn Asp Phe Gly Asn Asp Arg Asn His Arg Asn Gln Val Glu Arg
                85                  90                  95
Pro Gln Met Thr Phe Gly Ser Leu Gln Arg Ile Phe Pro Lys Ile Met
            100                 105                 110
Pro Lys Lys Pro Ala Glu Glu Glu Asn Gly Leu Lys Glu Val Pro Glu
        115                 120                 125
Ala Ser Gly Pro Gln Asn Asp Gly Lys Gln Leu Cys Pro Pro Gly Asn
    130                 135                 140
Pro Ser Thr Leu Glu Lys Ile Asn Lys Thr Ser Gly Pro Lys Arg Gly
145                 150                 155                 160
Lys His Ala Trp Thr His Arg Leu Arg Glu Arg Lys Gln Leu Val Val
                165                 170                 175
Tyr Glu Glu Ile Ser Asp Pro Glu Glu Asp Asp Glu
            180                 185

<210> 599
<211> 576
<212> DNA
<213> Homo sapiens

<400> 599
   atgaacggag acgacgcctt tgcaaggaga cccaggcatg atgctcaaat atcagagaag 60
   ttacgaaagg ccttcgatga tattgccaaa tacttctcta agaaagagtg ggaaaagatg 120
   aaatcctcgg agaaaatcgt ctatgtgtat atgaagctaa actatgagct catgactaaa 180
   ctaggtttca aggtcaccct cccacctttc atgcgtagta aacgggctgc agacttccac 240
   gggaatgatt ttggtaacga tcgaaaccac aggaatcagg ttgaacgtcc tcagatgact 300
   ttcggcagcc tccagagaat cttcccgaag atcatgccca agaagccagc agaggaagaa 360
   aatggtttga aggaagtgcc agaggcatct ggcccacaaa atgatgggaa acagctgtgc 420
   ccccccggaa atccaagtac cttggagaag attaacaaga catctggacc caaaaggggg 480
   aaacatgcct ggacccacag actgcgtgag agaaagcagc tggtggttta tgaagagatc 540
   agcgaccctg aggaagatga cgagtaactc ccctcg 576

<210> 600
<211> 262
<212> PRT
<213> Homo sapiens

<400> 600

<210> 601
<211> 269
<212> PRT
<213> Homo sapiens

<400> 601

<210> 602
<211> 269
<212> PRT
<213> Homo sapiens

<400> 602
Met Ile Arg Thr Leu Leu Leu Ser Thr Leu Val Ala Gly Ala Leu Ser
 1               5                  10                  15
Cys Gly Val Ser Thr Tyr Ala Pro Asp Met Ser Arg Met Leu Gly Gly
            20                  25                  30
Glu Glu Ala Arg Pro Asn Ser Trp Pro Trp Gln Val Ser Leu Gln Tyr
        35                  40                  45
Ser Ser Asn Gly Gln Trp Tyr His Thr Cys Gly Gly Ser Leu Ile Ala
    50                  55                  60
Asn Ser Trp Val Leu Thr Ala Ala His Cys Ile Ser Ser Ser Arg Ile
65                  70                  75                  80
Tyr Arg Val Met Leu Gly Gln His Asn Leu Tyr Val Ala Glu Ser Gly
                85                  90                  95
Ser Leu Ala Val Ser Val Ser Lys Ile Val Val His Lys Asp Trp Asn
            100                 105                 110
Ser Asn Gln Val Ser Lys Gly Asn Asp Ile Ala Leu Leu Lys Leu Ala
        115                 120                 125
Asn Pro Val Ser Leu Thr Asp Lys Ile Gln Leu Ala Cys Leu Pro Pro
    130                 135                 140
Ala Gly Thr Ile Leu Pro Asn Asn Tyr Pro Cys Tyr Val Thr Gly Trp
145                 150                 155                 160
Gly Arg Leu Gln Thr Asn Gly Ala Leu Pro Asp Asp Leu Lys Gln Gly
                165                 170                 175
Arg Leu Leu Val Val Asp Tyr Ala Thr Cys Ser Ser Ser Gly Trp Trp

180                 185                 190
Gly Ser Thr Val Lys Thr Asn Met Ile Cys Ala Gly Gly Asp Gly Val
        195                 200                 205
Ile Cys Thr Cys Asn Gly Asp Ser Gly Gly Pro Leu Asn Cys Gln Ala
    210                 215                 220
Ser Asp Gly Arg Trp Glu Val His Gly Ile Gly Ser Leu Thr Ser Val
225                 230                 235                 240
Leu Gly Cys Asn Tyr Tyr Tyr Lys Pro Ser Ile Phe Thr Arg Val Ser
                245                 250                 255
Asn Tyr Asn Asp Trp Ile Asn Ser Val Ile Ala Asn Asn
            260                 265

## Description

### Background of the Invention

### Field_of_theInvention

The present invention generally relates to peptides, and nucleic acids encoding peptides, that are useful epitopes of target-associated antigens. More specifically, the invention relates to epitopes that have a high affinity for MHC class I and that are produced by target-specific proteasomes.

### Description of the Related Art

### Neoplasia and the Immune System

The neoplastic disease state commonly as cancer is thought to result generally from a single cell growing out of control. The uncontrolled growth state typically results from a multistep process in which a series of cellular systems fail, resulting in the genesis of a neoplastic cell. The resulting neoplastic cell rapidly reproduces itself, forms one or more tumors, and eventually may cause the death of the host.

Because the progenitor of the neoplastic cell shares the host's genetic material, neoplastic cells are largely unassailed by the host's immune system. During immune surveillance, the process in which the host's immune system surveys and localizes foreign materials, a neoplastic cell will appear to the host's immune surveillance machinery as a "self cell.

### Viruses and the Immune System

In contrast to cancer cells, virus infection involves the expression of clearly non-self antigens. As a result, many virus infections are successfully dealt with by the immune system with minimal clinical sequela. Moreover, it has been possible to develop effective vaccines for many of those infections that do cause serious disease, A variety of vaccine approaches have been used successfully to combat various diseases. These approaches include subunit vaccines consisting of individual proteins produced through recombinant DNA technology. Notwithstanding these advances, the selection and effective administration of minimal epitopes for use as viral vaccines has remained problematic.

In addition to the difficulties involved in epitope selection stands the problem of viruses that have evolved the capability of evading a host's immune system. Many viruses, especially viruses that establish persistent infections, such as members of the herpes and pox virus families, produce immunomodulatory molecules that permit the virus to evade the host's immune system. The effects of these immunomodulatory molecules on antigen presentation may be overcome by the targeting of select epitopes for administration as immunogenic compositions. To better understand the interaction of neoplastic cells and virally infected cells with the host's immune. system, a discussion of the system's components follows below.

The immune system functions to discriminate molecules endogenous to an organism ("self" molecules) from material exogenous or foreign to the organism ("non-self" molecules). The immune system has two types of adaptive responses to foreign bodies based on the components that mediate the response: a humoral response and a cell-mediated response. The humoral response is mediated by antibodies, while the cell-mediated response involves cells classified as lymphocytes, Recent anticancer and antiviral strategies have focused on mobilizing the host immune system as a means of anticancer or antiviral treatment or therapy.

The immune system functions in three phases to protect the host from foreign bodies: the cognitive phase, the activation phase, and the effector phase. In the cognitive phase, the immune system recognizes and signals the presence of a foreign antigen or invader in the body, The foreign antigen can be, for example, a cell surface marker from a neoplastic cell or a viral protein. Once the system is aware of an invading body, antigen specific cells of the immune system proliferate and differentiate in response to the invader-triggered signals. The last stage is the effector stage in which the effector cells of the immune system respond to and neutralize the detected invader.

An array of effector cells implements an immune response to an invader. One type of effector cell, the B cell, generates antibodies targeted against foreign antigens encountered by the host. In combination with the complement system, antibodies direct the destruction of cells or organisms bearing the targeted antigen. Another type of effector cell is the natural killer cell (NK cell), a type of lymphocyte having the capacity to spontaneously recognize and destroy a variety of virus infected cells as well as malignant cell types. The method used by NK cells to recognize target cells is poorly understood.

Another type of effector cell, the T cell, has members classified into three subcategories, each playing a different role in the immune response. Helper T cells secrete cytokines which stimulate the proliferation of other cells necessary for mounting an effective immune response, while suppressor T cells down-regulate the immune response. A third category of T cell, the cytotoxic T cell (CTL), is capable of directly lysing a targeted cell presenting a foreign antigen on its surface.

### The Major Histocompatibility Complex and T Cell Target Recognition

T cells are antigen-specific immune cells that function in response to specific antigen signals. B lymphocytes and the antibodies they produce are also antigen-specific entities. However, unlike B lymphocytes, T cells do not respond to antigens in a free or soluble form. For a T cell to respond to an antigen, it requires the antigen to be processed to peptides which are then bound to a presenting structure encoded in the major histocompatibility complex (MHC). This requirement is called "MHC restriction" and it is the mechanism by which T cells differentiate "self from "non-self" cells. If an antigen is not displayed by a recognizable MHC molecule, the T cell will not recognize and act on the antigen signal. T cells specific for a peptide bound to a recognizable MHC molecule bind to these MHC-peptids complexes and proceed to the next stages of the immune response.

There are two types of MHC, class I MHC and class II MHC, T Helper cells (CD4⁺) predominately interact with class II MHC proteins while cytolytic T cells (CD8⁺) predominately interact with class I MHC proteins. Both classes of MHC protein are transmembrane proteins with a majority of their structure on the external surface of the cell. Additionally, both classes of MHC proteins have a peptide binding cleft on their external portions, It is in this cleft that small fragments of proteins, endogenous or foreign, are bound and presented to the extracellular environment.

Cells called "professional antigen presenting cells" (pAPCs) display antigens to T cells using the MHC proteins but additionally express various co-stimulatory molecules depending on the particular state of differentiation/activation of the pAPC. When T cells, specific for the peptide bound to a recognizable MHC protein, bind to these MHC-peptide complexes on pAPCs, the specific co-stimulatory molecules that act upon the T cell direct the path of differentiation/activation taken by the T cell. That is, the co-stimulation molecules affect how the T cell will act on antigenic signals in future encounters as it proceeds to the next stages of the immune response.

As discussed above, neoplastic cells are largely ignored by the immune system. A great deal of effort is now being expended in an attempt to harness a host's immune system to aid in combating the presence of neoplastic cells in a host. One such area of research involves the formulation of anticancer vaccines.

### Anticancer Vaccines

Among the various weapons available to an oncologist in the battle against cancer is the immune system of the patient. Work has been done in various attempts to cause the immune system to combat cancer or neoplastic diseases. Unfortunately, the results to date have been largely disappointing. One area of particular interest involves the generation and use of anticancer vaccines.

To generate a vaccine or other immunogenic composition, it is necessary to introduce to a subject an antigen or epitope against which an immune response may be mounted. Although neoplastic cells are derived from and therefore are substantially identical to normal cells on a genetic level, many neoplastic cells are known to present tumor-associated antigens (TuAAs). In theory, these antigens could be used by a subject's immune system to recognize these antigens and attack the neoplastic cells. In reality, however, neoplastic cells generally appear to be ignored by the host's immune system.

A number of different strategies have been developed is an attempt to generate vaccines with activity against neoplastic cells. These strategies include the use of tumor-associated antigens as immunogens. For example, U.S. Patent No. 5,993,828, describes a method for producing an immune response against a particular subunit of the Urinary Tumor Associated Antigen by administering to a subject an effective dose of a composition comprising inactivated tumor cells having the Urinary Tumor Associated Antigen on the cell surface and at least one tumor associated antigen selected from the group consisting of GM-2, GD-2, Fetal Antigen and Melanoma Associated Antigen, Accordingly, this patent describes using whole, inactivated tumor cells as the immunogen in an anticancer vaccine.

Another strategy used with anticancer vaccines involves administering a composition containing isolated tumor antigens. In one approach, MAGE-A1 antigenic peptides were used as an immunogen. (See Chaux, P., et al., "Identification of Five MAGE-A1 Epitopes Recognized by Cytolytic T Lymphocytes Obtained by In Vitro Stimulation with Dendritic Cells Transduced with MAGE-A1," J. Immunol., 163(5):2928-2936 (1999)). There have been several therapeutic trials using MAGE-A1 peptides for vaccination, although the effectiveness of the vaccination regimes was limited. The results of some of these trials are discussed in Vose, J.M., "Tumor Antigens Recognized by T Lymphocytes," 10th European Cancer Conference, Day 2, Sept. 14, 1999.

In another example of tumor associated antigens used as vaccines, Scheinberg, *et al*. treated 12 chronic myelogenous leukemia (CML) patients already receiving interferon (IFN) or hydroxyurea with 5 injections of class I-associated ber-abl peptides with a helper peptide plus the adjuvant QS-21. Scheinberg, D.A., et al., "BCR-ABL Breakpoint Derived Oncogene Fusion Peptide Vaccines Generate Specific Immune Responses in Patients with Chronic Myelogenous Leukemia (CML) [Abstract 1665], American Society of Clinical Oncology 35th Annual Meeting, Atlanta (1999). Proliferative and delayed type hypersensitivity (DTH) T cell responses indicative of T-helper activity were elicited, but no cytolytic killer T cell activity was observed within the fresh blood samples.

Additional examples of attempts to identify TuAAs for use as vaccines are seen in the recent work of Cebon, *et al*, and Scheibenbogen, *et al*. Cebon, *et al*. immunized patients with metastatic melanoma using intradermallly administered MART-1₂₆₋₃₅ peptide with IL-1.2 in increasing doses given either subcutaneously or intravenously. Of the first 15 patients, 1 complete remission, 1 partial remission, and 1 mixed response were noted. Immune assays for T cell generation included DTH, which was seen in patients with or without IL-12. Positive CTL assays were seen in patients with evidence of clinical benefit, but not in patients without tumor regression. Cebon, et al., "Phase I Studies of Immunization with Melan-A and IL-12 in HLA A2+ Positive Patients with Stage III and IV Malignant Melanoma," [Abstract 1671], American Society of Clinical Oncology 35th Annual Meeting, Atlanta (1999).

Scheibenbogen, *et al*. immunized 18 patients with 4 HLA class I restricted tyrosinase peptides, 16 with metastatic melanoma and 2 adjuvant patients. Scheibenbogen, et al., "Vaccination with Tyrosinase peptides and GM-CSF in Metastatic Melanoma: a Phase II Trial," [Abstract 1680], American Society of Clinical Oncology 35th Annual Meeting, Atlanta (1999). Increased CTL activity was observed in 4/15 patients, 2 adjuvant patients, and 2 patients with evidence of tumor regression. As in the trial by Cebon, *et al*, patients with progressive disease did not show boosted immunity. In spite of the various efforts expended to date to generate efficacious anticancer vaccines, no such composition has yet been developed.

### Antiviral Vaccines

Vaccine strategies to protect against viral diseases have had many successes. Perhaps the most notable of these is the progress that has been made against the disease small pox, which has been driven to extinction. The success of the polio vaccine is of a similar magnitude.

Viral vaccines can be grouped into three classifications: live attenuated virus vaccines, such as vaccinia for small pox, the Sabin poliovirus vaccine, and measles mumps and rubella; whole killed or inactivated virus vaccines, such as the Salk poliovirus vaccine, hepatitis A virus vaccine and the typical influenza virus vaccines; and subunit vaccines, such as hepatitis B. Due to their lack of a complete viral genome, subunit vaccines offer a greater degree of safety than those based on whole viruses.

The paradigm of a successful subunit vaccine is the recombinant hepatitis B vaccine based on the viruses envelope protein. Despite much academic interest in pushing the reductionist subunit concept beyond single proteins to individual epitopes, the efforts have yet to bear much fruit. Viral vaccine research has also concentrated on the induction of an antibody response although cellular responses also occur. However, many of the subunit formulations are particularly poor at generating a CTL response.

### Summary of the Invention

Previous methods of priming professional antigen presenting cells (pAPCs) to display target cell epitopes have relied simply on causing the pAPCs to express target-associated antigens (TAAs), or epitopes of those antigens which are thought to have a high affinity for MHC I molecules. However, the proteasomal processing of such antigens results in presentation of epitopes on the pAPC that do not correspond to the epitopes present on the target cells.

Using the knowledge that an effective cellular immune response requires that pAPCs present the same epitope that is presented by the target cells, the present invention provides epitopes that have a high affinity for MHC I, and that correspond to the processing specificity of the housekeeping proteasome, which is active in peripheral cells. These epitopes thus correspond to those presented on target cells. The use of such epitopes in vaccines can activate the cellular immune response to recognize the correctly processed TAA and can result in removal of target cells that present such epitopes. In some embodiments, the housekeeping epitopes provided herein can be used in combination with immune epitopes, generating a cellular immune response that is competent to attack target cells both before and after interferon induction. In other embodiments the epitopes are useful in the diagnosis and monitoring of the target-associated disease and in the generation of immunological reagents for such purposes.

Embodiments of the invention relate to isolated epitopes, and antigens or polypeptides that comprise the epitopes. Preferred embodiments include an epitope or antigen having the sequence as disclosed in Table 1. Other embodiments can include an epitope cluster comprising a polypeptide from Table 1. Further, embodiments include a polypeptide having substantial similarity to the already mentioned epitopes, polypeptides, antigens, or clusters. Other preferred embodiments include a polypeptide having functional similarity to any of the above. Still further embodiments relate to a nucleic acid encoding the polypeptide of any of the epitopes, clusters, antigens, and polypeptides from Table 1 and mentioned herein. For purposes of the following summary, discussions of other embodiments of the invention, when making reference to "the epitope," or "the epitopes" may refer without limitation to all of the foregoing forms of the epitope.

The epitope can be immunologically active. The polypeptide comprising the epitope can be less than about 30 amino acids in length, more preferably, the polypeptide is 8 to 10 amino acids in length, for example. Substantial or functional similarity can include addition of at least one amino acid, for example, and the at least one additional amino acid can be at an N-terminus of the polypeptide. The substantial or functional similarity can include a substitution of at least one amino acid.

The epitope, cluster, or polypeptide comprising the same can have affinity to an HLA-A2 molecule. The affinity can be determined by an assay of binding, by an assay of restriction of epitope recognition, by a prediction algorithm, and the like. The epitope, cluster, or polypeptide comprising the can have affinity to an HLA-B7, HLA-B51 molecule, and the like.

In preferred embodiments the polypeptide can be a housekeeping epitope. The epitope or polypeptide can correspond to an epitope displayed on a tumor cell, to an epitope displayed on a neovasculature cell, and the like. The epitope or polypeptide can be an immune epitope. The epitope, cluster and/or polypeptide can be a nucleic acid.

Other embodiments relate to pharmaceutical compositions comprising the polypeptides, including an epitope from Table 1, a cluster, or a polypeptide comprising the same, and a pharmaceutically acceptable adjuvant, carrier, diluent, excipient, and the like. The adjuvant can be a polynucleotide. The polynucleotide can include a dinucleotide, which can be CpG, for example. The adjuvant can be encoded by a polynucleotide. The adjuvant can be a cytokine and the cytokine can be, for example, GM-CSF.

The pharmaceutical compositions can further include a professional antigen-presenting cell (pAPC). The pAPC can be a dendritic cell, for example. The pharmaceutical composition can further include a second epitope. The second epitope can be a polypeptide, a nucleic acid, a housekeeping epitope, an immune epitope, and the like.

Still further embodiments relate to pharmaceutical compositions that include any of the nucleic acids discussed herein, including those that encode polypeptides that comprise epitopes or antigens from Table 1. Such compositions can include a pharmaceutically acceptable adjuvant, carrier, diluent, excipient, and the like.

Other embodiments relate to recombinant constructs that include such a nucleic acid as described herein, including those that encode polypeptides that comprise epitopes or antigens from Table 1. The constructs can further include a plasmid, a viral vector, an artificial chromosome, and the like, The construct can further include a sequence encoding at least one feature, such as for example, a second epitope, an IRES, an ISS, an NIS, a ubiquitin, and the like.

Further embodiments relate to purified antibodies that specifically bind to at least one of the epitopes in Table 1. Other embodiments relate to purified antibodies that specifically bind to a peptide-MHC protein complex comprising an epitope disclosed in Table 1 or any other suitable epitope. The antibody from any embodiment can be a monoclonal antibody or a polyclonal antibody.

Still other embodiments relate to multimeric MHC-peptide complexes that include an epitope, such as, for example, an epitope disclosed in Table 1. Also, contemplated are antibodies specific for the complexes.

Embodiments relate to isolated T cells expressing a T cell receptor specific for an MHC-peptides complex. The complex can include an epitope, such as, for example, an epitope disclosed in Table 1. The T cell can be produced by an *in vitro* immunization and can be isolated from an immunized animal. Embodiments relate to T cell clones, including cloned T cells, such as those discussed above. Embodiments also relate to polyclonal population of T cells. Such populations can include a T cell, as described above, for example.

Still further embodiments relate to pharmaceutical compositions that include a T cell, such as those described above, for example, and a pharmaceutically acceptable adjuvant, carrier, diluent, excipient, and the like.

Embodiments of the invention relate to isolated protein molecules comprising the binding domain of a T cell receptor specific for an MHC-peptide complex. The complex can include an epitope as disclosed in Table 1. The protein can be multivalent. Other embodiments relate to isolated nucleic acids encoding such proteins. Still further embodiments relate to recombinant constructs that include such nucleic acids,

Other embodiments of the invention relate to host cells expressing a recombinant construct as described herein, including constructs encoding an epitope, cluster or polypeptide comprising the same, disclosed in Table 1, for example. The host cell can be a dendritic cell, macrophage, tumor cell, tumor-derived cell, a bacterium, fungus, protozoan, and the like. Embodiments also relate to pharmaceutical compositions that include a host cell, such as those discussed herein, and a pharmaceutically acceptable adjuvant, carrier, diluent, excipient, and the like.

Still other embodiments relate to vaccines or immunotherapeutic compositions that include at least one component, such as, for example, an epitope disclosed in Table 1 or otherwise described herein; a cluster that includes such an epitope, an antigen or polypeptide that includes such an epitope; a composition as described above and herein; a construct, as described above and herein, a T cell, or a host cell as described above and herein,

Further embodiments relate to methods of treating an animal. The methods can include administering to an animal a pharmaceutical composition, such as, a vaccine or immunotherapeutic composition, including those disclosed above and herein. The administering step can include a mode of delivery, such as, for example, transdermal, intranodal, perinodal, oral, intravenous, intradermal, intramuscular, intraperitoneal, mucosal, aerosol inhalation, and the like. The method can further include a step of assaying to determine a characteristic indicative of a state of a target cell or target cells. The method can include a first assaying step and a second assaying step, wherein the first assaying step precedes the administering step, and wherein the second assaying step follows the administering step. The method can further include a step of comparing the characteristic determined in the first assaying step with the characteristic determined in the second assaying step to obtain a result. The result can be for example, evidence of an immune response, a diminution in number of target cells, a loss of mass or size of a tumor comprising target cells, a decrease in number or concentration of an intracellular parasite infecting target cells, and the like.

Embodiments relate to methods of evaluating immunogenicity of a vaccine or immunotherapeutic composition. The methods can include administering to an animal a vaccine or immunotherapeutic, such as those described above and elsewhere herein, and evaluating immunogenicity based on a characteristic of the animal. The animal can be HLA-transgenic,

Other embodiments relate to methods of evaluating immunogenicity that include *in vitro* stimulation of a T cell with the vaccine or immunotherapeutic composition, such as those described above and elsewhere herein, and evaluating immunogenicity based on a characteristic of the T cell. The stimulation can be a primary stimulation.

Still further embodiments relate to methods of making a passive/adoptive immunotherapeutic. The methods can include combining a T cell or a host cell, such as those described above and elsewhere herein, with a pharmaceutically acceptable adjuvant, carrier, diluent, excipient, and the like.

Other embodiments relate to methods of determining specific T cell frequency, and can include the step of contacting T cells with a MHC-peptide complex comprising an epitope disclosed in Table 1, or a complex comprising a cluster or antigen comprising such an epitope. The contacting step can include at least one feature, such as, for example, immunization, restimulation, detection, enumeration, and the like. The method can further include ELISPOT analysis, limiting dilution analysis, flow cytometry, in situ hybridization, the polymerase chain reaction, any combination thereof, and the like.

Embodiments relate to methods of evaluating immunologic response. The methods can include the above-described methods of determining specific T cell frequency carried out prior to and subsequent to an immunization step.

Other embodiments relate to methods of evaluating immunologic response. The methods can include determining frequency, cytokine production, or cytolytic activity of T cells, prior to and subsequent to a step of stimulation with MHC-peptide complexes comprising an epitope, such as, for example an epitope from Table 1, a cluster or a polypeptide comprising such an epitope.

Further embodiments relate to methods of diagnosing a disease. The methods can include contacting a subject, tissue with at least one component, including, for example, a T cell, a host cell, an antibody, a protein, including those described above and elsewhere herein; and diagnosing the disease based on a characteristic of the tissue or of the component. The contacting step can take place *in vivo* or *in vitro,* for example.

Still other embodiments relate to methods of making a vaccine. The methods can include combining at least one component, an epitope, a composition, a construct, a T cell, a host cell; including any of those described above and elsewhere herein, with a pharmaceutically acceptable adjuvant, carrier, diluent, excipient, and the like.

Embodiments relate to computer readable media having recorded thereon the sequence of any one of SEQ ID NOS: 1 -602, in a machine having a hardware or software that calculates the physical, biochemical, immunologic, molecular genetic properties of a molecule embodying said sequence, and the like.

Still other embodiments relate to methods of treating an animal. The methods can include. combining the method of treating an animal that includes administering to the animal a vaccine or immunotherapeutic composition, such as described above and elsewhere herein, combined with at least one mode of treatment, including, for example, radiation therapy, chemotherapy, biochemotherapy, surgery, and the like.

Further embodiments relate to isolated polypeptides that include an epitope cluster. In preferred embodiments the cluster can be from a target-associated antigen having the sequence as disclosed in any one of Tables 25-44, wherein, the amino acid sequence includes not more than about 80% of the amino acid sequence of the antigen.

Other embodiments relate to vaccines or immunotherapeutic products that include an isolated peptide as described above and elsewhere herein. Still other embodiments relate to isolated polynucleotides encoding a polypeptide as described above and elsewhere herein. Other embodiments relate vaccines or immunotherapeutic products that include these polynucleotides. The polynucleotide can be DNA, RNA, and the like.

Still further embodiments relate to kits comprising a delivery device and any of the embodiments mentioned above and elsewhere herein. The delivery device can be a catheter, a syringe, an internal or external pump, a reservoir, an inhaler, microinjector, a patch, and any other like device suitable for any route of delivery. As mentioned, the kit, in addition to the delivery device also includes any of the embodiments disclosed herein. For example, without limitations, the kit can include an isolated epitope, a polypeptide, a cluster, a nucleic acid, an antigen, a pharmaceutical composition that includes any of the foregoing, an antibody, a T cell, a T cell receptor, an epitope-MHC complex, a vaccine, an and the like. The kit can also include items such as detailed instructions for use and any other like item.

### Brief Description of the Drawings

Figure 1 is a sequence alignment of NY-ESO-1 and several similar protein sequences.
Figure 2 graphically represents a plasmid vaccine backbone useful for delivering nucleic acid-encoded epitopes.
Figures 3A and 3B are FACS profiles showing results of HLA-A2 binding assays for tyrosinase₂₀₇₋₂₁₅ and tyrosinase₂₀₈₋₂₁₆.
   Figure 3C shows cytolytic activity against a tyrosinase epitope by human CTL induced by *in vivo* immunization.
Figure 4 is a T=120 min time point mass spectrum of the fragments produced by proteasomal cleavage of SSX-2₃₁₋₆₈.
Figure 5 shows a binding curve for HLA-A2:SSX-2₄₁₋₄₉ with controls.
Figure 6 shows specific lysis of SSX-2₄₁₋₄₉-pulsed targets by CTL from SSX-2₄₁₋₄₉- immunized HLA-A2 transgenic mice.
Figure 7A, B, and C show results of N-terminal pool sequencing of a T=60 min time point aliquot of the PSMA₁₆₃₋₁₉₂ proteasomal digest.
Figure 8 shows binding curves for HLA-A2:PSMA₁₆₈₋₁₇₇ and HLA-A-2:PSMA₂₈₈₋₂₉₇ with controls.
Figure 9 shows results of N-terminal pool sequencing of a T=60 min. time point aliquot of the PSMA₂₉₁₋₃₁₀ proteasomal digest.
Figure 10 shows binding curves for HLA-A2:PSMA₄₆₁₋₄₆₉, HLA-A2:PSMA₄₆₀₋₄₆₉, and HLA-A2:PSMA₆₆₃₋₆₇₁, with controls.
Figure 11 shows the results of a γ-IFN-based ELISPOT assay detecting PSMA₄₆₃₋₄₇₁- reactive HLA-A1⁺ CD8⁺T cells.
Figure 12 shows blocking of reactivity of the T cells used in figure 10 by anti-HLA-A1 mAb, demonstrating HLA-A1-restricted recognition.
Figure 13 shows a binding curve for HLA-A2:PSMA₆₆₃₋₆₇₁, with controls.
Figure 14 shows a binding curve for HLA-A2:PSMA₆₆₂₋₆₇₁, with controls.
Figure 15. Comparison of anti-peptide CTL responses following immunization with various doses of DNA by different routes of injection.
Figure 16. Growth of transplanted gp33 expressing tumor in mice immunized by i.ln. injection of gp33 epitope-expressing, or control, plasmid.
Figure 17. Amount of plasmid DNA detected by real-time PCR in injected or draining lymph nodes at various times after i.ln. of i.m. injection, respectively.

### Detailed Description of the Preferred Embodiment

### Definitions

Unless otherwise clear from the context of the use of a term herein, the following listed terms shall generally have the indicated meanings for purposes of this description.

PROFESSIONAL ANTIGEN-PRESENTING CELL (pAPC) - a cell that possesses T cell costimulatory molecules and is able to induce a T cell response. Well characterised pAPCs include dendritic cells, B cells, and macrophages.

PERIPHERAL CELL - a cell that is not a pAPC.

HOUSEKEEPING PROTEASOME - a proteasome normally active in peripheral cells, and generally not present or not strongly active in pAPCs.

IMMUNE PROTEASOME - a proteasome normally active in pAPCs; the immune proteasome is also active in some peripheral cells in infected tissues.

EPITOPE - a molecule or substance capable of stimulating an immune response. In preferred embodiments, epitopes according to this definition include but are not necessarily limited to a polypeptide and a nucleic acid encoding a polypeptide, wherein the polypeptide is capable of stimulating an immune response. In other preferred embodiments, epitopes according to this definition include but are not necessarily limited to peptides presented on the surface of cells, the peptides being non-covalently bound to the binding cleft of class I MHC, such that they can interact with T cell receptors.

MHC EPITOPE - a polypeptide having a known or predicted binding affinity for a mammalian class I or class II major histocompatibility complex (MHC) molecule.

HOUSEKEEPING EPITOPE - In a preferred embodiment, a housekeeping epitope is defined as a polypeptide fragment that is an MHC epitope, and that is displayed on a cell in which housekeeping proteasomes are predominantly active, In another preferred embodiment, a housekeeping epitope is defined as a polypeptide containing a housekeeping epitope according to the foregoing definition, that is flanked by one to several additional amino acids. In another preferred embodiment, a housekeeping epitope is defined as a nucleic acid that encodes a housekeeping epitope according to the foregoing definitions.

IMMUNE EPITOPE - In a preferred embodiment, an immune epitope is defined as a polypeptide fragment that is an MHC epitope, and that is displayed on a cell in which immune proteasomes are predominantly active. In another preferred embodiment, an immune epitope is defined as a polypeptide containing an immune epitope according to the foregoing definition, that is flanked by one to several additional amino acids. In another preferred embodiment, an immune epitope is defined as a polypeptide including an epitope cluster sequence, having at least two polypeptide sequences having a known or predicted affinity for a class I MHC. In yet another preferred embodiment, an immune epitope is defined as a nucleic acid that encodes an immune epitope according to any of the foregoing definitions.

TARGET CELL - a cell to be targeted by the vaccines and methods of the invention. Examples of target cells according to this definition include but are not necessarily limited to: a neoplastic cell and a cell harboring an intracellular parasite, such as, for example, a virus, a bacterium, or a protozoan.

TARGET-ASSOCIATED ANTIGEN (TAA) - a protein or polypeptide present in a target cell.

TUMOR-ASSOCIATED ANTIGENS (TuAA) - a TAA, wherein the target cell is a neoplastic cell.

HLA EPITOPE - a polypeptide having a known or predicted binding affinity for a human class I or class II HLA complex molecule.

ANTIBODY - a natural immunoglobulin (Ig), poly- or monoclonal, or any molecule composed in whole or in part of an Ig binding domain, whether derived biochemically or by use of recombinant DNA. Examples include *inter alia*, F(ab), single chain Fv, and Ig variable region-phage coat protein fusions.

ENCODE - an open-ended term such that a nucleic acid encoding a particular amino acid sequence can consist of codons specifying that (poly)peptide, but can also comprise additional sequences either translatable, or for the control of transcription, translation, or replication, or to facilitate manipulation of some host nucleic acid construct.

SUBSTANTIAL SIMILARITY - this term is used to refer to sequences that differ from a reference sequence in an inconsequential way as judged by examination of the sequence. Nucleic acid sequences encoding the same amino acid sequence are substantially similar despite differences in degenerate positions or modest differences in length or composition of any noncoding regions. Amino acid sequences differing only by conservative substitution or minor length variations are substantially similar. Additionally, amino acid sequences comprising housekeeping epitopes that differ in the number of N-terminal flanking residues, or immune epitopes and epitope clusters that differ in the number of flanking residues at either terminus, are substantially similar. Nucleic acids that encode substantially similar amino acid sequences are themselves also substantially similar.

FUNCTIONAL SIMILARITY - this term is used to refer to sequences that differ from a reference sequence in an inconsequential way as judged by examination of a biological or biochemical property, although the sequences may not be substantially similar. For example, two nucleic acids can be useful as hybridization probes for the same sequence but encode differing amino acid sequences. Two peptides that induce cross-reactive CTL responses are functionally similar even if they differ by non-conservative amino acid substitutions (and thus do not meet the substantial similarity definition). Pairs of antibodies, or TCRs, that recognize the same epitope can be functionally similar to each other despite whatever structural differences exist. In testing for functional similarity of immunogenicity one would generally immunize with the "altered" antigen and test the ability of the elicited response (Ab, CTL, cytokine production, etc.) to recognize the target antigen. Accordingly, two sequences may be designed to differ in certain respects while retaining the same function. Such designed sequence variants are among the embodiments of the present invention.

**Table 1A. SEQ ID NOS.* including epitopes in Examples 1-7,13.**

| **SEQ ID NO** | **IDENTITY** | **SEQUENCE** |
|---|---|---|
| 1 | Tyr 207-216 | FLPWHRLFLL |
| 2 | Tyrosinase protein | Accession number**: P14679 |
| 3 | SSX-2 protein | Accession number: NP_003138 |
| 4 | PSMA protein | Accession number: NP_004467 |
| 5 | Tyrosinase cDNA | Accession number: NM_000372 |
| 6 | SSX-2 cDNA | Accession number: NM_003147 |
| 7 | PSMA cDNA | Accession number: NM_004476 |
| 8 | Tyr 207-215 | FLPWHRLFL |
| 9 | Tyr 208-216 | LPWHRLFLL |
| 10 | SSX-2 31-68 | YFSKEEWEKMKASEKIFYVYMKRKYEAMTKLGFK ATLP |
| 11 | SSX-2 32-40 | FSKEEWEKM |
| 12 | SSX-2 39-47 | KMKASEKIF |
| 13 | SSX-2 40-48 | MKASEKIPY |
| 14 | SSX-2 39-49 | KMKASEKIFY |
| 15 | SSX-2 41-49 | KASEKIFYV |
| 16 | SSX-2 40-49 | MKASEKIFYV |
| 17 | SSX-2 41-50 | KASEKIFYVY |
| 18 | SSX-2 42-49 | ASEKIFYVY |
| 19 | SSX-2 53-61 | RKYEAMTKL |
| 20 | SSX-2 52-61 | KRKYEAMTKL |
| 21 | SSX-2 54-63 | KYEAMTKLGF |
| 22 | SSX-2 55-63 | YEAMTKLGF |
| 23 | SSX-2 56-63 | EAMTKLGF |
| 24 | HBV18-27 | FLPSDYFPSV |
| 25 | HLA-B44 binder | AEMGKYSFY |
| 26 | SSX-1 41-49 | KYSEKISYV |
| 27 | SSX-3 41-49 | KVSEKIVYV |
| 28 | SSX-4 41-49 | KSSEKIVYV |
| 29 | SSX-5 41-49 | KASEKIIYV |
| 30 | PSMA163-192 | AFSPQGMPEGDLVYVNYARTEDFFKLERDM |
| 31 | PSMA 168-190 | GMPEGDLVYVNYARTEDFFKLER |
| 32 | PSMA 169-177 | MPEGDLVYV |
| 33 | PSMA 168-177 | GMPEGDLVYV |
| 34 | PSMA 168-176 | GMPEGDLVY |
| 35 | PSMA 167-176 | QGMPEGDLVY |
| 36 | PSMA 169-176 | MPEGDLVY |
| 37 | PSMA 171-179 | EGDLVYVNY |
| 38 | PSMA 170-179 | PEGDLVYVNY |
| 39 | PSMA 174-183 | LVYVNYARTE |
| 40 | PSMA 177-185 | VNYARTEDF |
| 41 | PSMA 176-185 | YVNYARTEDF |
| 42 | PSMA 178-186 | NYARTEDFF |
| 43 | PSMA 179-186 | YARTEDFF |
| 44 | PSMA 181-189 | RTEDFFKLE |
| 45 | PSMA 281-310 | RGIAEΛVGLPSIPVHPIGYYDAQKLLEKMG |
| 46 | PSMA 283-307 | IAEAVGLPSIPVHPIGYYDAQKLLE |
| 47 | PSMA 289-297 | LPSIPVHPI |
| 48 | PSMA 288-297 | GLPSIPVHPI |
| 49 | PSMA 297-305 | IGYYDAQKL |
| 50 | PSMA 296-305 | PIGYYDAQKL |
| 51 | PSMA 291-299 | SIPVHPIGY |
| 52 | PSMA 290-299 | PSIPVHPIGY |
| 53 | PSMA 292-299 | IPVHPIGY |
| 54 | PSMA 299-307 | YYDAQKLLE |
| 55 | PSMA454-481 | SSIEGNYTLRVDCTPLMYSLVHLTKEL |
| 56 | PSMA 456-464 | IEGNYTLRV |
| 57 | PSMA 455-464 | SIEGNYTLRV |
| 58 | PSMA 457-464 | EGNYTLRV |
| 59 | PSMA 461-469 | TLRVDCTPL |
| 60 | PSMA 460-469 | YTLRVDCTPL |
| 61 | PSMA 462-470 | LRVDCTPLM |
| 62 | PSMA 463-471 | RVDCTPLMY |
| 63 | PSMA 462-471 | LRVDCTPLMY |
| 64 | PSMA653-687 | FDKSNPIVLRMMNDQLMFLERAFIDPLGLPDRPFY |
| 65 | PSMA 660-681 | VLRMMNDQLMFLERAFIDPLGL |
| 66 | PSMA 663-671 | MMNDQLMFL |
| 67 | PSMA 662-671 | RMMNDQLMFL |
| 68 | PSMA 662-670 | RMMNDQLMF |
| 69 | Tyr 1-17 | MLLAVLYCLLWSFQTSA |

**Table 1B. SEQ ID NOS.* including epitopes in Examples 14 and 15.**

| **SEQ ID NO** | **IDENTITY** | **SEQUENCE** |
|---|---|---|
| 70 | GP 100 protein² | **Accession number: P40967 |
| 71 | MAGE-1 protein | Accession number: P4335 |
| 72 | MAGE-2 protein | Accession number: P43356 |
| 73 | MAGE-3 protein | Accession number: P43357 |
| 74 | NY-ESO-1 protein | Accession number: P78358 |
| 75 | LAGE-1a protein | Accession number: CAA11116 |
| 76 | LAGE-1b protein | Accession number: CAA11117 |
| 77 | PRAME protein | Accession number: NP 006106 |
| 78 | PSA protein | Accession number: P07288 |
| 79 | PSCA protein | Accession number: 043653 |
| 80 | GP100 cds | Accession number: U20093 |
| 81 | MAGE-1 cds | Accession number: M77481 |
| 82 | MAGE-2 cds | Accession number: L18920 |
| 83 | MAGE-3 cds | Accession number: U03735 |
| 84 | NY-ESO-1 cDNA | Accession number: U87459 |
| 85 | PRAME cDNA | Accession number: NM 006115 |
| 86 | PSA cDNA | Accession number: NM_001648 |
| 87 | PSCA cDNA | Accession number: AF043498 |
| 88 | GP100 630-638 | LPHSSSHWL |
| 89 | GP100 629-638 | QLPHSSSHWL |
| 90 | GP100 614-622 | LIYRRRLMK |
| 91 | GP100 613-622 | SLIYRRRLMK |
| 92 | GP100 615-622 | IYRRRLMK |
| 93 | GP100 630-638 | LPHSSSHWL |
| 94 | GP100 629-638 | QLPHSSSHWL |
| 95 | MAGE-1 95-102 | ESLFRAVI |
| 96 | MAGE-1 93-102 | ILESLFRAVI |
| 97 | MAGE-1 93-101 | ILESLFRAV |
| 98 | MAGE-1 92-101 | CILESLFRAV |
| 99 | MAGE-1 92-100 | CILESLFRA |
| 100 | MAGE-1 263-271 | EFLWGPRAL |
| 101 | MAGE-1 264-271 | FLWGPRAL |
| 102 | MAGE-1 264-273 | FLWGPRALAE |
| 103 | MAGE-1 265-274 | LWGPRALAET |
| 104 | MAGE-1 268-276 | PRALAETSY |
| 105 | MAGE-1 267-276 | GPRALAETSY |
| 106 | MAGE-1 269-277 | RALAETSYV |
| 107 | MAGE-1 271-279 | LAETSYVKV |
| 108 | MAGE-1 270-279 | ALAETSYVKV |
| 109 | MAGE-1 272-280 | AETSYVKVL |
| 110 | MAGE-1 271-280 | LAETSYVKVL |
| 111 | MAGE-1 274-282 | TSYVKVLEY |
| 112 | MAGE-1 273-282 | ETSYVKVLEY |
| 113 | MAGE-1 278-286 | KVLEYVIKV |
| 114 | MAGE-1 168-177 | SYVLVTCLGL |
| 115 | MAGE-1 169-177 | YVLVTCLGL |
| 116 | MAGE-1 170-177 | VLVTCLGL |
| 117 | MAGE-1 240-248 | TQDLVQEKY |
| 118 | MAGE-1 239-248 | LTQDLVQEKY |
| 119 | MAGE-1 232-240 | YGEPRKLLT |
| 120 | MAGE-1 243-251 | LVQEKYLEY |
| 121 | MAGE-1 242-251 | DLVQEKYLEY |
| 122 | MAGE-1 230-238 | SAYGEPRKL |
| 123 | MAGE-1 278-286 | KVLEYVIKV |
| 124 | MAGE-1 277-286 | VKVLEYVIKV |
| 125 | MAGE-1 276-284 | YVKVLEYVI |
| 126 | MAGE-1 274-282 | TSYVKVLEY |
| 127 | MAGE-1 273-282 | ETSYVKVLEY |
| 128 | MAGE-1 283-291 | VIKVSARVR |
| 129 | MAGE-1 282-291 | YVIKVSARVR |
| 130 | MAGE-2 115-122 | ELVHFLLL |
| 131 | MAGE-2 113-122 | MVELVHFLLL |
| 132 | MAGE-2 109-116 | ISRKMVEL |
| 133 | MAGE-2 108-116 | AISRKMVEL |
| 134 | MAGE-2 107-116 | AAISRKMVEL |
| 135 | MAGE-2 112-120 | KMVELVHFL |
| 136 | MAGE-2 109~117 | ISRKMVELV |
| 137 | MAGE-2 108-117 | AISRKMVELV |
| 138 | MAGE-2 116-124 | LVHFLLLKY |
| 139 | MAGE-2 115-124 | ELVHFLLLKY |
| 140 | MAGE-2 111-119 | RKMVELVHF |
| 141 | MAGE-2 158-166 | LQLVFGLEV |
| 142 | MAGE-2 157-166 | YLQLVFGIEV |
| 143 | MAGE-2 159-167 | QLVFGIEVV |
| 144 | MAGE-2 158-167 | LQLVFGIEVV |
| 145 | MAGE-2 164-172 | IEVVEVVPI |
| 146 | MAGE-2 163-172 | GIEVVEVVPI |
| 147 | MAGE-2 162-170 | FGIEVVEVV |
| 148 | MAGE-2 154-162 | ASEYLQLVF |
| 149 | MAGE-2 153-162 | KASEYLQLVF |
| 150 | MAGE-2 218-225 | EEKIWEEL |
| 151 | MAGE-2 216-225 | APEEKIWEEL |
| 152 | MAGE-2 216-223 | APEEKIWE |
| 153 | MAGE-2 220-228 | KIWEELSML |
| 154 | MAGE-2 219-228 | EKIWEELSML |
| 155 | MAGE-2 271-278 | FLWGPRAL |
| 156 | MAGE-2 271-279 | FLWGPRALI |
| 157 | MAGE-2 278-286 | LIETSYVKV |
| 158 | MAGE-2 277-286 | ALIETSYVKV |
| 159 | MAGE-2 276-284 | RALIETSYV |
| 160 | MAGE-2 279-287 | IETSYVKVL |
| 161 | MAGE-2 278-287 | LIETSYVKVL |
| 162 | MAGE-3 271-278 | FLWGPRAL |
| 163 | MAGE-3 270-278 | EFLWGPRAL |
| 164 | MAGE-3 271-279 | FLWGPRALV |
| 165 | MAGE-3 276-284 | RALVETSYV |
| 166 | MAGE-3 272-280 | LWGPRALVE |
| 167 | MAGE-3 271-280 | FLWGPRALVE |
| 168 | MAGE-3 27 2.281 | LWGPRALVET |
| 169 | NY-ESO-1 82-90 | GPESRLLEF |
| 170 | NY-ESO-1 83-91 | PESRLLEFY |
| 171 | NY-ESO-1 82-91 | GPESRLLEFY |
| 172 | NY-ESO-1 84-92 | ESRLLEFYL |
| 173 | NY-ESO-1 86-94 | RLLEFYLAM |
| 174 | NY-ESO-1 88-96 | LEFYLAMPF |
| 175 | NY-ESO-1 87-96 | LLEFYLAMPF |
| 176 | NY-ESO-1 93-102 | AMPFATPMEA |
| 177 | NY-ESO-1 94-102 | MPFATPMEA |
| 178 | NY-ESO~1 115-123 | PLPVPGVLL |
| 179 | NY-ESO-1 114-123 | PPLPVPGVLL |
| 180 | NY-ESO-1 116-123 | LPVPGVLL |
| 181 | NY-ESO-1 103-112 | ELARRSLAQD |
| 182 | NY-ESO-1 118-126 | VPGVLLKEF |
| 183 | NY-ESO-1 117-126 | PVPGVLLKEF |
| 184 | NY-ESO-1 116-123 | LPVPGVLL |
| 185 | NY-ESO-1 127-135 | TVSGNILTI |
| 186 | NY-ESO-1 126-135 | FTVSGNILTI |
| 187 | NY-ESO-1 120-128 | GVLLKEFTV |
| 188 | NY-ESO-1 121-130 | VLLKEFTVSG |
| 189 | NY-ESO-1 122-130 | LLKEFTVSG |
| 190 | NY-ESO-1 118-126 | VPGVLLKEF |
| 191 | NY-ESO-1 117-126 | PVPGVLLKEF |
| 192 | NY-ESO-1 139-147 | AADHRQLQL |
| 193 | NY-ESO-1 148-156 | SISSCLQQL |
| 194 | NY-ESO-1 147-156 | LSISSCLQQL |
| 195 | NY-ESO-1 138-147 | TAADHRQLQL |
| 196 | NY-ESO~1 161-169 | WITQCFLPV |
| 197 | NY-ESO-1 157-165 | SLLMWITQC |
| 198 | NY-ESO-1 150-158 | SSCLQQLSL |
| 199 | NY-ESO-1 154-162 | QQLSLLMWI |
| 200 | NY-ESO-1 151-159 | SCLQQJLSLL |
| 201 | NY-ESO-1 150-159 | SSCLQQLSLL |
| 202 | NY-ESO-1 163-171 | TQCFLPVFL |
| 203 | NY-ESO-1 162-171 | ITQCFLPVFL |
| 204 | PRAME 219-227 | PMQDIKMIL |
| 205 | PRAME 218-227 | MPMQDIKMIL |
| 206 | PRAME 428-436 | QHLIGLSNL |
| 207 | PRAME 427-436 | LQHLIGLSNL |
| 208 | PRAME 429-436 | HLIGLSNL |
| 209 | PRAME431-439 | IGLSNLTHV |
| 210 | PRAME 430-439 | LIGLSNLTHV |
| 211 | PSA 53-61 | VLVHPQWVL |
| 212 | PSA 52-61 | GVLVHPQWVL |
| 213 | PSA 52-60 | GVLVRPQWV |
| 214 | PSA 59-67 | WVLTAAHCI |
| 215 | PSA 54-63 | LVHPQWVLTA |
| 216 | PSA 53-62 | VLVHPQWVLT |
| 217 | PSA 54-62 | LVHPQWVLT |
| 218 | PSA 66~73 | CIRNKSVI |
| 219 | PSA 65-73 | HCIRNKSVI |
| 220 | PSA 56-64 | HPQWVLTAA |
| 221 | PSA 63-72 | AAHCIRNKSV |
| 222 | PSCA 116-123 | LLWGPGQL |
| 223 | PSCA 115-123 | LLLWGPGQL |
| 224 | PSCA 114-123 | GLLLWGPGQL |
| 225 | PSCA 99-107 | ALQPAAAIL |
| 226 | PSCA 98-107 | HALQPAAAIL |
| 227 | Tyr 128-137 | APEKDKFFAY |
| 228 | Tyr 129-137 | PEKDKFFAY |
| 229 | Tyr 130-138 | EKDKFFAYL |
| 230 | Tyr 131-138 | KDKFFAYL |
| 231 | Tyr 205-213 | PAFLPWHRL |
| 232 | Tyr 204-213 | APAFLPWHRL |
| 233 | Tyr 214-223 | FLLR WEQEIQ |
| 234 | Tyr 212-220 | RLFLLRWEQ |
| 235 | Tyr 191-200 | GSEIWRDIDF |
| 236 | Tyr 192-200 | SEIWRDIDF |
| 237 | Tyr 473-481 | RIWSWLLGA |
| 238 | Tyr 476-484 | SWLLGAAMV |
| 239 | Tyr 477-486 | WLLGAAMVGA |
| 240 | Tyr 478-486 | LLGAAMVGA |
| 241 | PSMA 4-12 | LLHETDSAV |
| 242 | PSMA 13-21 | ATARRPRWL |
| 243 | PSMA 53-61 | TPKHNMKAF |
| 244 | PSMA 64-73 | ELKAENIKKF |
| 245 | PSMA 69-77 | NIKKFLH¹NF |
| 246 | PSMA 68-77 | ENIKKFLH^{I}NF |
| 247 | PSMA 220-228 | AGAKGVILY |
| 248 | PSMA 468-477 | PLMYSLVHNL |
| 249 | PSMA 469-477 | LMYSLVHNL |
| 250 | PSMA 463-471 | RVDCTPLMY |
| 251 | PSMA 465-473 | DCTPLMYSL |
| 252 | PSMA 507-515 | SGMPRISKL |
| 253 | PSMA 506-515 | FSGMPRISKL |
| 254 | NY-ESO-1 136-163 | RLTAADHRQLQLSISSCLQQLSLLMWIT |
| 255 | NY-ESO-1 150-177 | SSCLQQLSLLMWITQCFLPVFLAQPPSG |

| | | |
|---|---|---|
| ¹This H was reported as Y in the SWISSPROT database. ²The amino acid at position 274 may be Pro or Leu depending upon the database. The particular analysis presented herein used the Pro. | | |

**Table 1C. SEQ ID NOS.* including epitopes in Example 14.**

| **SEQ ID NO.** | **IDENTITY** | **SEQUENCE** |
|---|---|---|
| 256 | Mage-1 125-132 | KAEMLESV |
| 257 | Mage-1 124-132 | TKAEMLESV |
| 258 | Mage-1 123-132 | VTKAEMLESV |
| 259 | Mage-1 128-136 | MLESVIKNY |
| 260 | Mage-1 127~136 | EMLESVIKNY |
| 261 | Mage-1 125-133 | KAEMLESVI |
| 262 | Mage-1 146-153 | KASESLQL |
| 263 | Mage-1 145-153 | GKASESLQL |
| 264 | Mage-1 147-155 | ASESLQLVF |
| 265 | Mage-1 153-161 | LVFGIDVKE |
| 266 | Mage-1 114-121 | LLKYRARE |
| 267 | Mage-1 106-113 | VADLVGFL |
| 268 | Mage~1 105-113 | KVADLVGFL |
| 269 | Mage-1 107-115 | ADLVGFLLL |
| 270 | Mage-1 106~115 | VADLVGFLLL |
| 271 | Mage-1 114-123 | LLKYRAREPV |
| 272 | Mage-3 278-286 | LVETSYVKV |
| 273 | Mage-3 277-286 | ALVETSYVKV' |
| 274 | Mage~3 285-293 | KVLHHMVKI |
| 275 | Mage-3 283-291 | YVKVLHHMV |
| 276 | Mage-3 275-283 | PRALVETSY |
| 277 | Mage-3 274-283 | GPRALVETSY |
| 278 | Mage-3 278-287 | LVETSYVKVL |
| 279 | ED-B 4'-5 | TIIPEVPQL |
| 280 | ED-B5'-5 | DTIIPEVPQL |
| 281 | ED-B 1-10 | EVPQLTDLSF |
| 282 | ED-B 23-30 | TPLNSSTI |
| 283 | ED-B 18-25 | IGLRWTPL |
| 284 | ED-B 17-25 | SIGLRWTPL |
| 285 | ED-B 25-33 | LNSSTIIGY |
| 286 | ED-B 24-33 | PLNSSTIIGY |
| 287 | ED-B 23-31 | TPLNSSTII |
| 288 | ED-B 31-38 | IGYRITVV |
| 289 | ED-B 30-38 | IIGYRITVV |
| 290 | ED-B 29-38 | TIIGYRITVV |
| 291 | ED-B 31-39 | IGYRITVVA |
| 292 | ED-B 30-39 | IIGYRITVVA |
| 293 | CEA 184-191 | SLPVSPRL |
| 294 | CEA 183-191 | QSLPVSPRL |
| 295 | CEA 186-193 | PVSPRLQL |
| 296 | CEA 185-193 | LPVSPRLQL |
| 297 | CEA 184-193 | SLPVSPRLQL |
| 298 | CEA 185-192 | LPVSPRLQ |
| 299 | CEA 192-200 | QLSNGNRTL |
| 300 | CEA 191-200 | LQLSNGNRTL |
| 301 | CEA 179-187 | WVNNQSLPV |
| 302 | CEA 186-194 | PVSPRLQLS |
| 303 | CEA 362-369 | SLPVSPRL |
| 304 | CEA 361-369 | QSLPVSPRL |
| 305 | CEA 364-371 | PVSPRLQL |
| 306 | CEA 363-371 | LPVSPRLQL |
| 307 | CEA 362-371 | SLPVSPRLQL |
| 308 | CEA 363-370 | LPVSPRLQ |
| 309 | CEA 370-378 | QLSNDNRTL |
| 310 | CEA 369-378 | LQLSNDNRTL |
| 311 | CEA 357-365 | WVNNQSLPV |
| 312 | CEA 360-368 | NQSLPVSPR |
| 313 | CEA 540-547 | SLPVSPRL |
| 314 | CEA 539-547 | QSLPVSPRL |
| 315 | CEA542-549 | PVSPRLQL |
| 316 | CEA 541-549 | LPVSPRLQL |
| 317 | CEA 540-549 | SLPVSPRLQL |
| 318 | CEA 541-548 | LPVSPRLQ |
| 319 | CEA 548-556 | QLSNGNRTL |
| 320 | CEA 547-556 | LQLSNGNRTL |
| 321 | CEA 535-543 | WVNGQSLPV |
| 322 | CEA 533-541 | LWWVNGQSL |
| 323 | CEA 532-541 | YLWWVNGQSL |
| 324 | CEA 538-546 | GQSLPVSPR |
| 325 | Her-2 30-37 | DMKLRLPA |
| 326 | Her-2 28-37 | GTDMKLRLPA |
| 327 | Her-2 42-49 | HLDMLRHL |
| 328 | Her-2 41-49 | THLDMLRHL |
| 329 | Her-2 40-49 | ETHLDMLRHL |
| 330 | Her-2 36-43 | PASPETHL |
| 331 | Her-2 35-43 | LPASPETHL |
| 332 | Her-2 34-43 | RLPASPETHL |
| 333 | Her-2 38-46 | SPETHLDML |
| 334 | Her-2 37-46 | ASPETHLDML |
| 335 | Her-2 42-50 | HLDMLRHLY |
| 336 | Her-241-50 | THLDMLRHLY |
| 337 | Her-2 719-726 | ELRKVKVL |
| 338 | Her-2 718-726 | TELRKVKVL |
| 339 | Her-2 717-726 | ETELRKVKVL |
| 340 | Her-2 715-723 | LKETELRKV |
| 341 | Her-2 714-723 | ILKETELRKV |
| 342 | Her-2 712-720 | MRILKETEL |
| 343 | Her-2 711-720 | QMRILKETEL |
| 344 | Her-2 717-725 | ETELRKVKV |
| 345 | Her-2 716-725 | KETELRKVKV |
| 346 | Her-2 706-714 | MPNQAQMRI |
| 347 | Her-2 705-714 | AMPNQAQMRI |
| 348 | Her-2 706-715 | MPNQAQMRIL |
| 349 | HER-2 966-973 | RPRFRELV |
| 350 | HER-2 965-973 | CRPRFRELV |
| 351 | HER-2 968-976 | RFRELVSEF |
| 352 | HER-2 967-976 | PRFRELVSEF |
| 353 | HER-2 964-972 | ECRPRFREL |
| 354 | NY-ESO-1 67-75 | GAASGLNGC |
| 355 | NY-ESO-1 52-60 | RASGPGGGA |
| 356 | NY-ESO-1 64-72 | PHGGAASGL |
| 357 | NY-ESO-1 63-72 | GPHGGAASGL |
| 358 | NY-ESO-1 60-69 | APRGPHGGAA |
| 359 | PRAME 112-119 | VRPRRWKL |
| 360 | PRAME 111-119 | EVRPRRWKL |
| 361 | PRAME 113-121 | RPRRWKLQV |
| 362 | PRAME 114-122 | PRRWKLQVL |
| 363 | PRAME 113-122 | RPRRWKLQVL |
| 364 | PRAME 116-124 | RWKLQVLDL |
| 365 | PRAME 115-124 | RRWKLQVLDL |
| 366 | FRAME 174-182 | PVEVLVDLF |
| 367 | PRAME 199-206 | VKRKKNVL |
| 368 | PRAME 198-206 | KVKRKKNVL |
| 369 | PRAME 197-206 | EKVKRKKNVL |
| 370 | FRAME 198-205 | KVKRKKNV |
| 371 | PRAME 201-208 | RKKNVLRL |
| 372 | PRAME 200-208 | KRKKNVLRL |
| 373 | PRAME 199-208 | VKRKKNVLRL |
| 374 | PRAME 189-196 | DELFSYLI |
| 375 | PRAME 205-213 | VLRLCCKKL |
| 376 | PRAME 204-213 | NVLRLCCKXL |
| 377 | PRAME 194-202 | YLIEKVKRK |
| 378 | PRAME 74-81 | QAWPFTCL |
| 379 | PRAME 73-81 | VQAWPFTCL |
| 380 | PRAME 72-81 | MVQAWPFTCL |
| 381 | PRAME 81-88 | LPLGYLMK |
| 382 | PRAME 80-88 | CLPLGVLMK |
| 383 | PRAME 79-88 | TCLPLGVLMK |
| 384 | PRAME 84-92 | GVLMKGQHL |
| 385 | PRAME 81-89 | LPLGVLMKG |
| 386 | PRAME 80-89 | CLPLGVLMKG |
| 387 | PRAME 76-85 | WPFTCLPLGV |
| 388 | PRAME 51-59 | ELFPPLFMA |
| 389 | PRAME 49-57 | PRELFPPLF |
| 390 | PRAME 48-57 | LPRELFPPLF |
| 391 | PRAME 50-58 | RELFPPLFM |
| 392 | FRAME 49-58 | PRELFPPLFM |
| 393 | PSA 239-246 | RPSLYTKV |
| 394 | PSA 238-246 | ERPSLYTKV |
| 395 | PSA 236-243 | LPERPSLY |
| 396 | PSA 235-243 | ALPERPSLY |
| 397 | PSA 241-249 | SLYTKVVHY |
| 398 | PSA 240-249 | PSLYTKVVHY |
| 399 | PSA 239-247 | RPSLYTKVV |
| 400 | PSMA 211-218 | GNKVKNAQ |
| 401 | PSMA 202-209 | IARYGKVF |
| 402 | PSMA 217-225 | AQLAGAKGV |
| 403 | PSMA 207-215 | KVFRGNKVK |
| 404 | PSMA 211-219 | GNKVKNAQL |
| 405 | PSMA 269-277 | TPGYPANEY |
| 406 | PSMA 268-277 | LTPGYPANEY |
| 407 | PSMA 271-279 | GYPANEYAY |
| 408 | PSMA 270-279 | PGYPANEYAY |
| 409 | PSMA 266-274 | DPLTPGYPA |
| 410 | PSMA 492-500 | SLYESWTKK |
| 411 | PSMA 491-500 | KSLYESWTKK |
| 412 | PSMA 486-494 | EGFEGKSLY |
| 413 | PSMA 485-494 | DEGFEGKSLY |
| 414 | PSMA 498-506 | TKKSPSPEF |
| 415 | PSMA 497-506 | WTKKSPSPEF |
| 416 | PSMA 492-501 | SLYESWTKKS |
| 417 | PSMA 725-732 | WGEVKRQI |
| 418 | PSMA 724-732 | AWGEVKRQI |
| 419 | PSMA 723~732 | KAWGEVKRQI |
| 420 | PSMA 723-730 | KAWGEVKR |
| 421 | PSMA 722~730 | SKAWGEVKR |
| 422 | PSMA 731-739 | QIYVAAFTV |
| 423 | PSMA 733-741 | YVAAFTVQA |
| 424 | PSMA 725-733 | WGEVKRQIY |
| 425 | PSMA 727-735 | EVKRQIYVA |
| 426 | PSMA 738-746 | TVQAAAETL |
| 427 | PSMA 737-746 | FTVQAAAETL |
| 428 | PSMA 729-737 | KRQIYVAAF |
| 429 | PSMA 721-729 | PSKAWGEVK |
| 430 | PSMA 723-731 | KAWGEVKRQ |
| 431 | PSMA 100-108 | WKEFGLDSV |
| 432 | PSMA 99-108 | QWKEFGLDSV |
| 433 | PSMA 102-111 | EFGLDSVELA |
| 434 | SCP-1 126-134 | ELRQKESKL |
| 435 | SCP-1 125-134 | AELRQKESKL. |
| 436 | SCP-1 133-141 | KLQENRKII |
| 437 | SCP-1 298-305 | QLEEKTKL |
| 438 | SCP-1 297-305 | NQLEEKTKL |
| 439 | SCP-1 288-296 | LLEESRDKV |
| 440 | SCP-1 287-296 | FLLEESRDKV |
| 441 | SCP-1 291-299 | ESRDKVNQL |
| 442 | SCP-1 290-299 | EESRDKVNQL |
| 443 | SCP-1 475-483 | EKEVHDLEY |
| 444 | SCP-1 474-483 | REKEVHDLEY |
| 445 | SCP-1 480-488 | DLEYSYCHY |
| 446 | SCP-1 477-485 | EVHDLEYSY |
| 447 | SCP-1 477-486 | EVHDLEYSYC |
| 448 | SCP-1 502-509 | KLSSKREL |
| 449 | SCP-1 508-515 | ELKNTEYF |
| 450 | SCP-1 507-515 | RELKNTEYF |
| 451 | SCP-1 496-503 | KRGQRPKL |
| 452 | SCP-1 494-503 | LPKRGQRPKL |
| 453 | SCP-1 509-517 | LKNTEYFTL |
| 454 | SCP-1 508-517 | ELKNTEYFTL |
| 455 | SCP-1 506-514 | KRELKNTEY |
| 456 | SCP-1 502-510 | KLSSKRELK |
| 457 | SCP-1 498-506 | GQRPKLSSK |
| 458 | SCP-1 497-506 | RGQRPKLSSK |
| 459 | SCP-1 500-508 | RPKLSSKRE |
| 460 | SCP-1 573-580 | LEYVREEL |
| 461 | SCP-1 572-580 | ELEYVREEL |
| 462 | SCP-1 571-580 | NELEYVREEL |
| 463 | SCP-1 579-587 | ELKQKREDEV |
| 464 | SCP-1575-583 | YVREELKQK |
| 465 | SCP-1 632-640 | QLNVYEIKV |
| 466 | SCP-1 630-638 | SKQLNVYEI |
| 467 | SCP-1 628-636 | AESKQLNVY |
| 468 | SCP-1 627-636 | TAESKQLNVY |
| 469 | SCP-1 638-645 | IKVNKLEL |
| 470 | SCP-1 637-645 | EIKVNKLEL |
| 471 | SCP-1 636-645 | YEIKVNKLEL |
| 472 | SCP-1 642-650 | KLELELESA |
| 473 | SCP-1 635-643 | VYEIKVNKL |
| 474 | SCP-1 634-643 | NVYEIKVNKL |
| 475 | SCP-1 646-654 | ELESAKQKF |
| 476 | SCP-1 642-650 | KLELELESA |
| 477 | SCP-1 646-654 | ELESAKQKF |
| 478 | SCP-1 771-778 | KEKLKREA |
| 479 | SCP-1 777-785 | EAKENTATL |
| 480 | SCP-1 776-785 | REAKENTATL |
| 481 | SCP-1 773-782 | KLKREAKENT |
| 482 | SCP-1 112-119 | EAEKIKKW |
| 483 | SCP-1 101-109 | GLSRVYSKL |
| 484 | SCP-1 100-109 | EGLSRVYSKL |
| 485 | SCP-1 108-116 | KLYKEAEKI |
| 486 | SCP-1 98-106 | NSEGLSRVY |
| 487 | SCP-1 97-106 | ENSEGLSRVY |
| 488 | SCP-1 102-110 | LSRVYSKLY |
| 489 | SCP-1 101-110 | GLSRVYSKLY |
| 490 | SCP-1 96-105 | LENSEGLSRV |
| 491 | SCP-1 108-117 | KLYKEAEKIK |
| 492 | SCP-1 949-956 | REDRWAVI |
| 493 | SCP-1 948-956 | MREDRWAVI |
| 494 | SCP-1 947-956 | KMREDRWAVI |
| 495 | SCP-1 947-955 | KMREDRWAV |
| 496 | SCP-1 934-942 | TTPGSTLKF |
| 497 | SCP-1 933-942 | LTTPGSTLKF |
| 498 | SCP-1 937-945 | GSTLKGAI |
| 499 | SCP-1 945-953 | IRKMREDRW |
| 500 | SCP-1 236-243 | RLEMHFKL |
| 501 | SCP-1 235-243 | SRLEMHFKL |
| 502 | SCP-1 242-250 | KLKEDYEKI |
| 503 | SCP-1 249-257 | KIQHLEQEY |
| 504 | SCP-1 248-257 | EKIQHLEQEY |
| 505 | SCP-1 233-242 | ENSRLEMHF |
| 506 | SCP-1236-245 | RLEMHFKLKE |
| 507 | SCP-1 324-331 | LEDIKVSL |
| 508 | SCP-1 323-331 | ELEDIKVSL |
| 509 | SCP-1 322-331 | KELEDIKVSL |
| 510 | SCP-1 320-327 | LTKELEDI |
| 511 | SCP-1 319-327 | HLTKELEDI |
| 512 | SCP-1 330-338 | SLQRSVSTQ |
| 513 | SCP-1 321-329 | TKELEDIKV |
| 514 | SCP-1 320-329 | LTKELEDIKV |
| 515 | SCP-1 326-335 | DIKVSLQRSV |
| 516 | SCP-1 281-288 | KMKDLTFL |
| 517 | SCP-1 280-288 | NKMKDLTFL |
| 518 | SCP-1 279-288 | ENKMKDLTFL |
| 519 | SCP-1 288-296 | LLEESRDKV |
| 520 | SCP-1 287-296 | FLLEESRDKV |
| 521 | SCP-1 291-299 | ESRDKVNQL |
| 522 | SCP-1 290-299 | EESRDKVNQL |
| 523 | SCP-1 277-285 | EKENKMKDL |
| 524 | SCP-1 276-285 | TEKENKMKDL |
| 525 | SCP-1 279-287 | ENKMKDLTF |
| 526 | SCP-1 218-225 | IEKMITAF |
| 527 | SCP-1 217-225 | NIEKMITAF |
| 528 | SCP-1 216-225 | SNIEKMITAF |
| 529 | SCP-1 223-230 | TAFEELRV |
| 530 | SCP-1 222-230 | ITAFEELRV |
| 531 | SCP-1 221-230 | MITAFEELRV |
| 532 | SCP-1 220-228 | KMITAFEEL |
| 533 | SCP-1 219-228 | EKMITAFEEL |
| 534 | SCP-1 227-235 | ELRVQAENS |
| 535 | SCP-1 213-222 | DLNSNIEKMI |
| 536 | SCP-1 837-844 | WTSAKNTL |
| 537 | SCP-1 846-854 | TPLPKAYTV |
| 538 | SCP-1 845-854 | STPLPKAYTV |
| 539 | SCP-1 844-852 | LSTPLPKAY |
| 540 | SCP-1 843-852 | TLSTPLPKAY |
| 541 | SCP-1 842-850 | NTLSTPLPK |
| 542 | SCP-1 841-850 | KNTLSTPLPK |
| 543 | SCP-1 828-835 | ISKDKRDY |
| 544 | SCP-1 826-835 | HGISKDKRDY |
| 545 | SCP-1 832-840 | KRDYLWTSA |
| 546 | SCP-1 829-838 | SKDKRDYLWT |
| 547 | SCP-1 279-286 | ENKMKDLT |
| 548 | SCP-1 260-268 | EINDKEKQV |
| 549 | SCP-1 274-282 | QITEKENKM |
| 550 | SCP-1 269-277 | SLLLIQITE |
| 551 | SCP-1 453-460 | FEKIAEEL |
| 552 | SCP-1 452-460 | QFEKIAEEL |
| 553 | SCP-1 451-460 | KQFEKIAEEL |
| 554 | SCP-1 449-456 | DNKQFEKI |
| 555 | SCP-1 448-456 | YDNKQFEKI |
| 556 | SCF-1 447-456 | LYDNKQFEKI |
| 557 | SCP-1 440-447 | LGEKETLL |
| 558 | SCP-1 439-447 | VLGEKETLL |
| 559 | SCP-1 438-447 | KVLGEKETLL |
| 560 | SCP-1 390-398 | LLRTEQQRL |
| 561 | SCP-1 389-398 | ELLRTEQQRL |
| 562 | SCP-1 393-401 | TEQQRLENY |
| 563 | SCP-1 392-401 | RTEQQRLENY |
| 564 | SCP-1 402-410 | EDQLIILTM |
| 565 | SCP-1 397-406 | RLENYEDQLI |
| 566 | SCP-1 368-375 | KARAAHSF |
| 567 | SCP-1 376-384 | VVTEFETTV |
| 568 | SCP-1 375-384 | FVVTEFETTV |
| 569 | SCP-1 377-385 | VTEFETTVC |
| 570 | SCP-1 376-385 | VVTEFETTVC |
| 571 | SCP-1 344-352 | DLQIATNTI |
| 572 | SCP-1 347-355 | IATNTICQL |
| 573 | SCP-1 346-355 | QIATNTICQL |
| 574 | SSX4 57-65 | VMTKLGFKY |
| 575 | SSX4 53-61 | LNYEVMTKL |
| 576 | SSX4 52-61 | KLNYEVMTKL |
| 577 | SSX4 66-74 | TLPPFMRSK |
| 578 | SSX4 110-118 | KIMPKKPAE |
| 579 | SSX4 103-112 | SLQRIFPKIM |
| 580 | Tyr 463-471 | YIKSYLEQA |
| 581 | Tyr 459-467 | SFQDYIKSY |
| 582 | Tyr 458-467 | DSFQDYIKSY |
| 583 | Tyr 507-514 | LPEEKQPL |
| 584 | Tyr 506-514 | QLPEEKQPL |
| 585 | Tyr 505-514 | KQLPEEKQPL |
| 586 | Tyr 507-515 | LPEEKQPLL |
| 587 | Tyr 506-515 | QLPEEKQPLL |
| 588 | Tyr 497-505 | SLLCRHKRK |
| 589 | ED-B domain of Fibronectin | EVPQLTDLSFVDITDSSIGLRWTPLNSSTIIGYRI TVVAAGEGIPIFEDFVDSSVGYYTVTGLEPGID YDISVITLINGGESAPTTLTQQT |
| 590 | ED-B domain of Fibronectin with flanking sequence from Fribronectin | CTFDNLSPGLEYNVSVYTVKDDKESVPISDTIIP EVPQLTDLSFVDITDSSIGLRWTPLNSSTIIGYRI TVVAAGEGIPIFEDFVDSSVGYYTVTGLEPGID YDISVITLINGGESAPTTLTQQT AVPPPTDLRFTNIGPDTMRVTW |
| 591 | ED-B domain of Fibronectin cds | Accession number; X07717 |
| 592 | CEA protein | Accession number: P06731 |
| 593 | CEA cDNA | Accession number: NM_004363 |
| 594 | Her2/Neu protein | Accession number: P04626 |
| 595 | Her2/Neu cDNA | Accession number: M11730 |
| 596 | SCP-1 protein | Accession number: Q15431 |
| 597 | SCP-1 cDNA | Accession number: X95654 |
| 598 | SSX-4 protein | Accession number: 060224 |
| 599 | SSX-4 cDNA | Accession number: NM_005636 |

| | | |
|---|---|---|
| *Any of SEQ ID NOS. 1, 8, 9, 11-23, 26-29, 32-44, 47-54, 56-63, 66-68 88-253, and 256-588 can be useful as epitopes in any of the various embodiments of the invention. Any of SEQ ID NOS. 10, 30, 31, 45, 46, 55, 64, 65, 69, 254, and 255 can be useful as sequences containing epitopes or epitope clusters, as described in various embodiments of the invention. **All accession numbers used here and throughout can be accessed through the NCBI databases, for example, through the Entrez seek and retrieval system on the world wide web. | | |

Note that the following discussion sets forth the inventors' understanding of the operation of the invention. However, it is not intended that this discussion limit the patent to any particular theory of operation not set forth in the claims.

In pursuing the development of epitope vaccines others have generated lists of predicted epitopes based on MHC binding motifs. Such peptides can be immunogenic, but may not correspond to any naturally produced antigenic fragment. Therefore, whole antigen will not elicit a similar response or sensitize a target cell to cytolysis by CTL. Therefore such lists do not differentiate between those sequences that can be useful as vaccines and those that cannot. Efforts to determine which of these predicted epitopes are in fact naturally produced have often relied on screening their reactivity with tumor infiltrating lymphocytes (TIL). However, TIL are strongly biased to recognize immune epitopes whereas tumors (and chronically infected cells) will generally present housekeeping epitopes. Thus, unless the epitope is produced by both the housekeeping and immuno- proteasomes, the target cell will generally not be recognized by CTL induced with TIL-identified epitopes. The epitopes of the present invention, in contrast, are generated by the action of a specified proteasome, indicating that they can be naturally produced, and enabling their appropriate use. The importance of the distinction between housekeeping and immune epitopes to vaccine design is more fully set forth in PCT publication WO 01/82963 A2.

The epitopes of the invention include or encode polypeptide fragments of TAAs that are precursors or products of proteasomal cleavage by a housekeeping or immune proteasome, and that contain or consist of a sequence having a known or predicted affinity for at least one allele of MHC I. in some embodiments, the epitopes include or encode a polypeptide of about 6 to 25 amino acids in length, preferably about 7 to 20, amino acids in length, more preferably about 8 to 15 amino acids in length, and still more preferably 9 or 10 amino acids in length. However, it is understood that the polypeptides can be larger as long as N-terminal trimming can produce the MHC epitope or that they do not contain sequences that cause the polypeptides to be directed away from the proteasome or to he destroyed by the proteasome. For immune epitopes, if the larger peptides do not contain such sequences, they can be processed in the pAPC by the immune proteasome. Housekeeping epitopes may also be embedded in longer sequences provided that the sequence is adapted to facilitate liberation of the epitope's by action of the immunoproteasome. The foregoing discussion has assumed that processing of longer epitopes proceeds through action of the immunoproteasome of the pAPC. However, processing can also be accomplished through the contrivance of some other mechanism, such as providing an exogenous protease activity and a sequence adapted so that action of the protease liberates the MHC epitope. The sequences of these epitopes can be subjected to computer analysis in order to calculate physical, biochemical, immunologic, or molecular genetic properties such as mass, isoelectric point, predicted mobility in electrophoresis, predicted binding to other MHC molecules, melting temperature of nucleic acid probes, reverse translations, similarity or homology to other sequences, and the like.

In constructing the polynucleotides encoding the polypeptide epitopes of the invention, the gene sequence of the associated TAA can be used, or the polynucleotide can be assembled from any of the corresponding codons. For a 10 amino acid epitope this can constitute on the order of 10⁶ different sequences, depending on the particular amino acid composition. While large, this is a distinct and readily definable set representing a miniscule fraction of the >10¹⁸ possible polynucleotides of this length, and thus in some embodiments, equivalents of a particular sequence disclosed herein encompass such distinct and readily definable variations on the listed sequence. In choosing a particular one of these sequences to use in a vaccine, considerations such as codon usage, self-complementarity, restriction sites, chemical stability, etc. can be used as will be apparent to one skilled in the art.

The invention contemplates producing peptide epitopes. Specifically these epitopes are derived from the sequence of a TAA, and have known or predicted affinity for at least one allele of MHC I. Such epitopes are typically identical to those produced on target cells or pAPCs.

### Compositions Containing Active Epitopes

Embodiments of the present invention provide polypeptide compositions, including vaccines, therapeutics, diagnostics, pharmacological and pharmaceutical compositions. The various compositions include newly identified epitopes of TAAs, as well as variants of these epitopes. Other embodiments of the invention provide polynucleotides encoding the polypeptide epitopes of the invention. The invention further provides vectors for expression of the polypeptide epitopes for purification. In addition, the invention provides vectors for the expression of the polypeptide epitopes in an APC for use as an anti-tumor vaccine. Any of the epitopes or antigens, or nucleic acids encoding the same, from Table 1 can be used. Other embodiments relate to methods of making and using the various compositions.

A general architecture for a class I MHC-binding epitope can be described, and has been reviewed more extensively in Madden, D.R. Annu. Rev. Immunol. 13:587-622, 1995. Much of the binding energy arises from main chain contacts between conserved residues in the MHC molecule and the N- and C-termini of the peptide. Additional main chain contacts are made but vary among MHC alleles. Sequence specificity is conferred by side chain contacts of so-called anchor residues with pockets that, again, vary among MHC alleles. Anchor residues can be divided into primary and secondary. Primary anchor positions exhibit strong preferences for relatively well-defined sets of amino acid residues. Secondary positions show weaker and/or less well-defined preferences that can often be better described in terms of less favored, rather than more favored, residues. Additionally, residues in some secondary anchor positions are not always positioned to contact the pocket on the MHC molecule at all. Thus, a subset of peptides exists that bind to a particular MHC molecule and have a side chain-pocket contact at the position in question and another subset exists that show binding to the same MHC molecule that does not depend on the conformation the peptide assumes in the peptide-binding groove of the MHC molecule. The C-terminal residue (P ; omega) is preferably a primary anchor residue. For many of the better studied HLA molecules (e.g. A2, A68, B27, B7, B35, and B53) the second position (P2) is also an anchor residue. However, central anchor residues have also been observed including P3 and P5 in HLA-B8, as well as P5 and P (omega)-3 in the murine MHC molecules H-2D^{b} and H-2K^{b}, respectively. Since more stable binding will generally improve immunogenicity, anchor residues are preferably conserved or optimized in the design of variants, regardless of their position.

Because the anchor residues are generally located near the ends of the epitope, the peptide can buckle upward out of the peptide-binding groove allowing some variation in length. Epitopes ranging from 8-11 amino acids have been found for HLA-A68, and up to 13 amino acids for HLA-A2. In addition to length variation between the anchor positions, single residue truncations and extensions have been reported and the N- and C-termini, respectively. Of the non-anchor residues, some point up out of the groove, making no contact with the MHC molecule but being available to contact the TCR, very often P1, P4, and P (omega)-1 for HLA-A2. Others of the non-anchor residues can become interposed between the upper edges of the peptide-binding groove and the TCR, contacting both. The exact positioning of these side chain residues, and thus their effects on binding, MHC fine conformation, and ultimately immunogenicity, are highly sequence dependent. For an epitope to be highly immunogenic it must not only promote stable enough TCR binding for activation to occur, but the TCR must also have a high enough off-rate that multiple TCR molecules can interact sequentially with the same peptide-MHC complex (Kalergis, A.M. et al., Nature Immunol. 2:229-234, 2001. Thus, without further information about the ternary complex, both conservative and non-conservative substitutions at these positions merit consideration when designing variants.

The polypeptide epitope variants can be made, for example, using any of the techniques and guidelines for conservative and non-conservative mutations. Variants can be derived from substitution, deletion or insertion of one or more amino acids as compared with the native sequence. Amino acid substitutions can be the result of replacing one amino acid with another amino acid having similar structural and/or chemical properties, such as the replacement of a threonine with a serine, for example. Such replacements are referred to as conservative amino acid replacements, and all appropriate conservative amino acid replacements are considered to be embodiments of one invention. Insertions or deletions can optionally be in the range of about 1 to 4, preferably 1 to 2, amino acids. It is generally preferable to maintain the "anchor positions" of the peptide which are responsible for binding to the MHC molecule in question. Indeed, immunogenicity of peptides can be improved in many cases by substituting more preferred residues at the anchor positions (Franco, et al., Nature Immunology, 1(2):145-150, 2000. Immunogenicity of a peptide can also often be improved by substituting bulkier amino acids for small amino acids found in non-anchor positions while maintaining sufficient cross-reactivity with the original epitope to constitute a useful vaccine. The variation allowed can be determined by routine insertions, deletions or substitutions of amino acids in the sequence and testing the resulting variants for activity exhibited by the polypeptide epitope. Because the polypeptide epitope is often 9 amino acids, the substitutions preferably are made to the shortest active epitope, for example, an epitope of 9 amino acids.

Variants can also be made by adding any sequence onto the N-terminus of the polypeptide epitope variant. Such N-terminal additions can be from 1 amino acid up to at least 25 amino acids. Because peptide epitopes are often trimmed by N-terminal exopeptidases active in the pAPC, it is understood that variations in the added sequence can have no effect on the activity of the epitope. In preferred embodiments, the amino acid residues between the last upstream proteasomal cleavage site and the N-terminus of the MHC epitope do not include a proline residue. Serwold, T. at al., Nature Immunol. 2:644-651, 2001. Accordingly, effective epitopes can be generated from precursors larger than the preferred 9-mer class I motif.

Generally, peptides are useful to the extent that they correspond to epitopes actually displayed by MHC I on the surface of a target cell or a pACP. A single peptide can have varying affinities for different MHC molecules, binding some well, others adequately, and still others not appreciably (Table 2). MHC alleles have traditionally been grouped according to serologic reactivity which does not reflect the structure of the peptide-binding groove, which can differ among different alleles of the same type. Similarly, binding properties can be shared across types; groups based on shared binding properties have been termed supertypes. There are numerous alleles of MHC I in the human population; epitopes specific to certain alleles can be selected based on the genotype of the patient.

**Table 2. Predicted Binding of Tyrosinase₂₀₇₋₂₁₆ (SEQ ID NO.1) to Various MHC types**

| **MHC** I type | *Half time of dissociation (min) |
|---|---|
| A1 | 0.05 |
| A*0201 | 1311. |
| A*0205 | 50.4 |
| A3 | 2.7 |
| A*1101 (part of the A3 supertype) | 0.012 |
| A24 | 6.0 |
| B7 | 4.0 |
| B8 | 8.0 |
| B14 (part of the B27 supertype) | 60.0 |
| B*2702 | 0.9 |
| B*2705 | 30.0 |
| B*3501 (part of the B7 supertype) | 2.0 |
| B*4403 | 0.1 |
| B*5101 (part of the B7 supertype) | 26.0 |
| B*5102 | 55.0 |
| B*5801 | 0.20 |
| B60 | 0.40 |
| B62 | 2.0 |

| | |
|---|---|
| *HLA Peptide Binding Predictions (world wide web hypertext transfer protocol "access at bimas.dcrt.nih.gov/molbio/hla_bin"). | |

In further embodiments of the invention, the epitope, as peptide or encoding polynucleotide, can be administered as a pharmaceutical composition, such as, for example, a vaccine or an immunogenic composition, alone or in combination with various adjuvants, carriers, or excipients. It should be noted that although the term vaccine may be used throughout the discussion herein, the concepts can be applied and used with any other pharmaceutical composition, including those mentioned herein. Particularly advantageous adjuvants include various cytokines and oligonucleotides containing immonostimulatory sequences (as set forth in greater detail in the co-pending applications referenced herein). Additionally the polynucleoride encoded epitope can be contained in a virus (e.g. vaccinia or adenovirus) or in a microbial host cell (e.g. *Salmonella* or *Listeria monocytogenes)* which is then used as a vector for the polynucleotide (Dietrich, G. et al. Nat. Biotech. 16:181-185, 1998). Alternatively apAPC can be transformed, ex *vivo,* to express the epitope, or pulsed with peptide epitope, to be itself administered as a vaccine. To increase efficiency of these processes, the encoded epitope can be carried by a viral or bacterial vector, or complexed with a ligand of a receptor found on pAPC. Similarly the peptide epitope can be complexed with or conjugated to a pAPC ligand. A vaccine can be composed of more than a single epitope.

Particularly advantageous strategies for incorporating epitopes and/or epitope clusters, into a vaccine or pharmaceutical composition are disclosed in U.S. Patent Application No. 09/560,465 entitled "EPITOPE SYNCHRONIZATION IN ANTIGEN PRESENTING CELLS," filed on April 28, 2000. Epitope clusters for use in connection with this invention are disclosed in U.S. Patent Application No. 09/561,571 entitled "EPITOPE CLUSTERS," filed on April 28, 2000,

Preferred embodiments of the present invention are directed to vaccines and methods for causing a pAPC or population of pAPCs to present housekeeping epitopes that correspond to the epitopes displayed on a particular target cell. Any of the epitopes or antigens in Table 1, can be used for example. In one embodiment, the housekeeping epitope is a TuAA epitope processed by the housekeeping proteasome of a particular tumor type. In another embodiment, the housekeeping epitope is a virus-associated epitope processed by the housekeeping proteasome of a cell infected with a virus. This facilitates a specific T cell response to the target cells. Concurrent expression by the pAPCs of multiple epitopes, corresponding to different induction states (pre- and post-attack), can drive a CTL response effective against target cells as they display either housekeeping epitopes or immune epitopes.

By having both housekeeping and immune epitopes present on the pAPC, this embodiment can optimize the cytotoxic T cell response to a target cell. With dual epitope expression, the pAPCs can continue to sustain a CTL response to the immune-type epitope when the tumor cell switches from the housekeeping proteasome to the immune proteasome with induction by IFN, which, for example, may be produced by tumor-infiltrating CTLs.

In a preferred embodiment, immunization of a patient is with a vaccine that includes a housekeeping epitope. Many preferred TAAs are associated exclusively with a target cell, particularly in the case of infected cells. In another embodiment, many preferred TAAs are the result of deregulated gene expression in transformed cells, but are found also in tissues of the testis, ovaries and fetus. In another embodiment, useful TAAs are expressed at higher levels in the target cell than in other cells. In still other embodiments, TAAs are not differentially expressed in the target cell compare to other cells, but are still useful since they are involved in a particular function of the cell and differentiate the target cell from most other peripheral cells; in such embodiments, healthy cells also displaying the TAA may be collaterally attacked by the induced T cell response, but such collateral damage is considered to be far preferable to the condition caused by the target cell.

The vaccine contains a housekeeping epitope in a concentration effective to cause a pAPC or populations of pAPCs to display housekeeping epitopes. Advantageously, the vaccine can include a plurality of housekeeping epitopes or one or more housekeeping epitopes optionally in combination with one or more immune epitopes. Formulations of the vaccine contain peptides and/or nucleic acids in a concentration sufficient to cause pAPCs to present the epitopes. The formulations preferably contain epitopes in a total concentration of about 1µg-1mg/100µl of vaccine preparation. Conventional dosages and dosing for peptide vaccines and/or nucleic acid vaccines can be used with the present invention, and such dosing regimens are well understood in the art. In one embodiment, a single dosage for an adult human may advantageously be from about 1 to about 5000 µl of such a composition, administered one time or multiple times, e.g., in 2, 3, 4 or more dosages separated by 1 week, 2 weeks, 1 month, or more. insulin pump delivers 1 µl per hour (lowest frequency) ref intranodal method patent.

The compositions and methods of the invention disclosed herein further contemplate incorporating adjuvants into the formulations in order to enhance the performance of the vaccines. Specifically, the addition of adjuvants to the formulations is designed to enhance the delivery or uptake of the epitopes by the pAPCs. The adjuvants contemplated by the present invention are known by those of skill in the art and include, for example, GMCSF, GCSF, IL-2, IL-12, BCG, tetanus toxoid, osteopontin, and ETA-1.

In some embodiments of the invention, the vaccines can include a recombinant organism, such as a virus, bacterium or parasite, genetically engineered to express an epitope in a host. For example, *Listeria monocytogenes,* a gram-positive, facultative intracellular bacterium, is a potent vector for targeting TuAAs to the immune system. In a preferred embodiment, this vector can be engineered to express a housekeeping epitope to induce therapeutic responses. The normal route of infection of this organism is through the gut and can be delivered orally. In another embodiment, an adenovirus (Ad) vector encoding a housekeeping epitope for a TuAA can be used to induce anti-virus or anti-tumor responses. Bone marrow-derived dendritic cells can be transduced with the virus construct and then injected, or the virus can be delivered directly via subcutaneous injection into an animal to induce potent T-cell responses. Another embodiment employs a recombinant vaccinia virus engineered to encode amino acid sequences corresponding to a housekeeping epitope for a TAA. Vaccinia viruses carrying constructs with the appropriate nucleotide substitutions in the form of a minigene construct can direct the expression of a housekeeping epitope, leading to a therapeutic T cell response against the epitope.

The immunization with DNA requires that APCs take up the DNA and express the encoded proteins or peptides. It is possible to encode a discrete class I peptide on the DNA. By immunizing with this construct, APCs can be caused to express a housekeeping epitope, which is then displayed on class I MHC on the surface of cell for stimulating an appropriate CTL response. Constructs generally relying on termination of translation or non-proteasomal proteases for generation of proper termini of housekeeping epitopes have been described in U.S. Patent application No. 09/561,572 entitled EXPRESSION VECTORS ENCODING EPITOPES OF TARGET-ASSOCIATED ANTIGENS, filed on April 28, 2000.

As mentioned, it can be desirable to express housekeeping peptides in the context of a larger protein. Processing can be detected even when a small number of amino acids are present beyond the terminus of an epitope. Small peptide hormones are usually proteolytically processed from longer translation products, often in the size range of approximately 60-120 amino acids. This fact has led some to assume that this is the minimum size that can be efficiently translated. In some embodiments, the housekeeping peptide can be embedded in a translation product of at least about 60 amino acids. In other embodiments the housekeeping peptide can be embedded in a translation product of at least about 50, 30, or 15 amino acids.

Due to differential proteasomal processing, the immune proteasome of the pAPC produces peptides that are different from those produced by the housekeeping proteasome in peripheral body cells. Thus, in expressing a housekeeping peptide in the context of a larger protein, it is preferably expressed in the APC in a context other than its full length native sequence, because, as a housekeeping epitope, it is generally only efficiently processed from the native protein by the housekeeping proteasome, which is not active in the APC. In order to encode the housekeeping epitope in a DNA sequence encoding a larger protein, it is useful to find flanking areas on either side of the sequence encoding the epitope that permit appropriate cleavage by the immune proteasome in order to liberate that housekeeping epitope. Altering flanking amino acid residues at the N-terminus and C-terminus of the desired housekeeping epitope can facilitate appropriate cleavage and generation of the housekeeping epitope in the APC. Sequences embedding housekeeping epitopes can be designed *de novo* and screened to determine which can be successfully processed by immune proteasomes to liberate housekeeping epitopes.

Alternatively, another strategy is very effective for identifying sequences allowing production of housekeeping epitopes in APC. A contiguous sequence of amino acids can be generated from head to tail arrangement of one or more housekeeping epitopes. A construct expressing this sequence is used to immunize an animal, and the resulting T cell response is evaluated to determine its specificity to one or more of the epitopes in the array. By definition, these immune responses indicate housekeeping epitopes that are processed in the pAPC effectively. The necessary flanking areas around this epitope are thereby defined. The use of flanking regions of about 4-6 amino acids on either side of the desired peptide can provide the necessary information to facilitate proteasome processing of the housekeeping epitope by the immune proteasome. Therefore, a sequence ensuring epitope synchronization of approximately 16-22 amino acids can be inserted into, or fused to, any protein sequence effectively to result in that housekeeping epitope being produced in an APC. In alternate embodiments the whole head-to-tail array of epitopes, or just the epitopes immediately adjacent to the correctly processed housekeeping epitope can be similarly transferred from a test construct to a vaccine vector.

In a preferred embodiment, the housekeeping epitopes can be embedded between known immune epitopes, or segments of such, thereby providing an appropriate context for processing. The abutment of housekeeping and immune epitopes can generate the necessary context to enable the immune proteasome to liberate the housekeeping epitope, or a larger fragment, preferably including a correct C-terminus. It can be useful to screen constructs to verify that the desired epitope is produced. The abutment of housekeeping epitopes can generate a site cleavable by the immune proteasome. Some embodiments of the invention employ known epitopes to flank housekeeping epitopes in test substrates; in others, screening as described below are used whether the flanking regions are arbitrary sequences or mutants of the natural flanking sequence, and whether or not knowledge of proteasomal cleavage preferences are used in designing the substrates.

Cleavage at the mature N-terminus of the epitope, while advantageous, is not required, since a variety of N-terminal trimming activities exist in the cell that dan generate the mature N-terminus of the epitope subsequent to proteasomal processing. It is preferred that such N-terminal extension be less than about 25 amino acids in length and it is further preferred that the extension have few or no proline residues. Preferably, in screening, consideration is given not only to cleavage at the ends of the epitope (or at least at its C-terminus), but consideration also can be given to ensure limited cleavage within the epitope.

Shotgun approaches can be used in designing test substrates and can increase the efficiency of screening. In one embodiment multiple epitopes can be assembled one after the other, with individual epitopes possibly appearing more than once. The substrate can be screened to determine which epitopes can be produced. In the case where a particular epitope is of concern a substrate can be designed in which it appears in multiple different contexts. When a single epitope appearing in more than one context is liberated from the substrate additional secondary test substrates, in which individual instances of the epitope are removed, disabled, or are unique, can be used to determine which are being liberated and truly constitute sequences ensuring epitope synchronization.

Several readily practicable screens exist. A preferred *in vitro* screen utilizes proteasomal digestion analysis, using purified immune proteasomes, to determine if the desired housekeeping epitope can be liberated from a synthetic peptide embodying the sequence in question. The position of the cleavages obtained can be determined by techniques such as mass spectrometry, HPLC, and N-terminal pool sequencing; as described in greater detail in U. S. Patent Applications entitled METHOD OF EPITOPE DISCOYERY, EPITOPE SYNCHRONIZATION IN ANTIGEN PRESENTING CELLS, two Provisional U. S. Patent Applications entitled EPITOPE SEQUENCES.

Alternatively, *in vivo* screens such as immunization or target sensitization can be employed. For immunization a nucleic acid construct capable of expressing the sequence in question is used. Harvested CTL can be tested for their ability to recognize target cells presenting the housekeeping epitope in question. Such targets cells are most readily obtained by pulsing cells expressing the appropriate MHC molecule with synthetic peptide embodying the mature housekeeping epitope. Alternatively, cells known to express housekeeping proteasome and the antigen from which the housekeeping epitope is derived, either endogenously or through genetic engineering, can be used. To use target sensitization as a screen, CTL, or preferably a CTL clone, that recognizes the housekeeping epitope can be used. In this case it is the target cell that expresses the embedded housekeeping epitope (instead of the pAPC during immunization) and it must express immune proteasome. Generally, the target cell can be transformed with an appropriate nucleic acid construct to confer expression of the embedded housekeeping epitope. Loading with a synthetic peptide embodying the embedded epitope using peptide loaded liposomes or a protein transfer reagent such as BIOPORTER™ (Gene Therapy Systems, San Diego, CA) represents an alternative.

Additional guidance on nucleic acid constructs useful as vaccines in accordance with the present invention are disclosed in U.S. Patent Application No. 09/561,572 entitled "EXPRESSION VECTORS ENCODING EPITOPES OF TARGET-ASSOCIATED ANTIGENS," filed on April 28, 2000. Further, expression vectors and methods for their design, which are useful in accordance with the present invention are disclosed in U.S. Patent Application No. 60/336,968 (attorney docket number CTLIMM.022PR) entitled "EXPRESSION VECTORS ENCODING EPITOPES OF TARGET-ASSOCIATED ANTIGENS AND METHODS FOR THEIR DESIGN," filed on 11/7/2001.

A preferred embodiment of the present invention includes a method of administering a vaccine including an epitope (or epitopes) to induce a therapeutic immune response. The vaccine is administered to a patient in a manner consistent with the standard vaccine delivery protocols that are known in the art. Methods of administering epitopes of TAAs including, without limitation, transdermal, intranodal, perinodal, oral, intravenous, intradermal, intramuscular, intraperitoneal, and mucosal administration, including delivery by injection, instillation or inhalation. A particularly useful method of vaccine delivery to elicit a CTL response is disclosed in Australian Patent No. 739189 issued January 17, 2002; U.S, Patent Application No. 09/380,534, filed on September 1, 1999; and a Continuation-in-Part thereof U.S. Patent Application No. 09/776,232 both entitled "A METHOD OF INDUCING A CTL RESPONSE," filed on February 2, 2001.

### Reagents Recognizing Epitopes

In another aspect of the invention, proteins with binding specificity for the epitope and/or the epitope-MHC molecule complex are contemplated, as well as the isolated cells by which they can be expressed. In one set of embodiments these reagents take the form of immunoglobulins: polyclonal sera or monoclonal antibodies (mAb), methods for the generation of which are well know in the art. Generation of mAb with specificity for peptide-MHC molecule complexes is known in the art. See, for example, Aharoni et al. Nature 351:147-150, 1991; Andersen et al. Proc. Natl. Acad. Sci. USA 93:1820-1824, 1996; Dadaglio et al. Immunity 6:727-738, 1997; Duc et al, Int. Immunol. 5:427-431, 1993; Eastman et al. Eur. J. Immunol. 26:385-393, 1996; Engberg et al. Immunotechnology 4:273-278, 1999; Porgdor et al. Immunity 6:715-726, 1997; Puri et al. J. Immunol. 158:2471-2476, 1997; and Polakova, K., et al. J. Immunol. 165 342-348, 2000.

In other embodiments the compositions can be used to induce and generate, *in vivo* and *in vitro*, T-cells specific for the any of the epitopes and/or epitope-MHC complexes. In preferred embodiments the epitope can be any one or more of those listed in TABLE 1, for example. Thus, embodiments also relate to and include isolated T cells, T cell clones, T cell hybridomas, or a protein containing the T cell receptor (TCR) binding domain derived from the cloned gene, as well as a recombinant cell expressing such a protein. Such TCR derived proteins can be simply the extra-cellular domains of the TCR, or a fusion with portions of another protein to confer a desired property or function. One example of such a fusion is the attachment of TCR binding domains to the constant regions of an antibody molecule so as to create a divalent molecule. The construction and activity, of molecules following this general pattern have been reported, for example, Plaksin, D. et al. J. Immunol. 158:2218-2227, 1997 and Lebowitz, M.S. et al. Cell Immunol. 192:175-184, 1999. The more general construction and use of such molecules is also treated in U.S. patent 5,830,755 entitled T CELL RECEPTORS AND THEIR USE IN THERAPEUTIC AND DIAGNOSTIC METHODS.

The generation of such T cells can be readily accomplished by standard immunization of laboratory animals, and reactivity to human target cells can be obtained by immunizing with human target cells or by immunizing HLA-transgenic animals with the antigen/epitope. For some therapeutic approaches T cells derived from the same species are desirable. While such a cell can be created by cloning, for example, a murine TCR into a human T cell as contemplated above, *in vitro* immunization of human cells offers a potentially faster option. Techniques for *in vitro* immunization, even using naive donors, are know in the field, for example, Stauss et al., Proc. Natl. Acad. Sci. USA 89:7871-7875, 1992; Salgaller et al. Cancer Res. 55:4972-4979, 1995; Tsai et al., J. Immunol. 158:1796-1802, 1997; and Chung et al., J. Immunother. 22:279-287, 1999.

Any of these molecules can be conjugated to enzymes, radiochemicals, fluorescent tags, and toxins, so as to be used in the diagnosis (imaging or other detection), monitoring, and treatment of the pathogenic condition associated with the epitope, Thus a toxin conjugate can be administered to kill tumor cells, can facilitate imaging of epitope positive tumor, an enzyme conjugate can be used in an ELISA-like assay to diagnose cancer and confirm epitope expression in biopsied tissue, In a further embodiment, such T cells as set forth above, following expansion accomplished through stimulation with the epitope and/or cytokines, can be administered to a patient as an adoptive immunotherapy.

### Reagents Comprising Epitopes

A further aspect of the invention provides isolated epitope-MHC complexes. In a particularly advantageous embodiment of this aspect of the invention, the complexes can be soluble, multimeric proteins such as those described in U. S. Patent No. 5,635,363 (tetramers) or U. S. Patent No. 6,015,884 (Ig-dimers). Such reagents are useful in detecting and monitoring specific T cell responses, and in purifying such T cells.

Isolated MHC molecules complexed with epitopic peptides can also be incorporated into planar lipid bilayers or liposomes. Such compositions can be used to stimulate T cells *in vitro* or, in the case of liposomes, *in vivo*. Co-stimulatory molecules (e.g. B7, CD40, LFA-3) can be incorporated into the same compositions or, especially for *in vitro* work, co-stimulation can be provided by anti-co-receptor antibodies (e.g. anti-CD28, anti-CD154, anti-CD2) or cytokines (e.g. IL-2, IL-12). Such stimulation of T cells can constitute vaccination, drive expansion of T cells *in vitro* for subsequent infusion in an immuotherapy, or constitute a step in an assay of T cell function.

The epitope, or more directly its complex with an MHC molecule, can be an important constituent of functional assays of antigen-specific T cells at either an activation or readout step or both. Of the many assays of T cell function current in the art (detailed procedures can be found in standard immunological references such as Current Protocols in Immunology 1999 John Wiley & Sons Inc., N.Y. two broad classes can be defined, those that measure the response of a pool of cells and those that measure the response of individual cells. Whereas the former conveys a global measure of the strength of a response, the latter allows determination of the relative frequency of responding cells. Examples of assays measuring global response are cytotoxicity assays, ELISA, and proliferation assays detecting cytokine secretion. Assays measuring the responses of individual cells (or small clones derived from them) include limiting dilution analysis (LDA), ELISPOT, flow cytometric detection of unsecreted cytokine (described in U.S. Patent No. 5,445,939, entitled "METHOD FOR ASSESSMENT OF THE MONONUCLEAR LEUKOCYTE IMMUNE SYSTEM" and U.S. Patent Nos 5,656,446; and 5,843,689, both entitled "METHOD FOR THE ASSESSMENT OF THE MONONUCLEAR LEUXOCYTE IMMUNE SYSTEM," reagents for which are sold by Becton, Dickinson & Company under the tradename 'FASTIMMUNE' and detection of specific TCR with tetramers or Ig-dimers as stated and referenced above. The comparative virtues of these techniques have been reviewed in Yee, C, et al, Current Opinion in Immunology, 13:141-146, 2001. Additionally detection of a specific TCR rearrangement or expression can be accomplished through a variety of established nucleic acid based techniques, particularly in situ and single-cell PCR techniques, as will be apparent to one of skill in the art.

These functional assays are used to assess endogenous levels of immunity, response to an immunologic stimulus (e,g, a vaccine), and to monitor immune status through the course of a disease and treatment. Except when measuring endogenous levels of immunity, any of these assays presume a preliminary step of immunization, whether *in vivo* or *in vitro* depending on the nature of the issue being addressed. Such immunization can be carried out with the various embodiments of the invention described above or with other forms of immunogen (e.g., pAPC-tumor cell fusions) that can provoke, similar immunity, With the exception of PCR. and tetramer/Ig-dimer type analyses which can detect expression of the cognate TCR, these assays generally benefit from a step of *in vitro* antigenic stimulation which can advantageously use various embodiments of the invention as described above in order to detect the particular functional activity (highly cytolytic responses can sometimes be detected directly). Finally, detection of cytolytic activity requires epitope-displaying target cells, which can be generated using various embodiments of the invention. The particular embodiment chosen for any particular step depends on the question to be addressed, ease of use, cost, and the like, but the advantages of one embodiment over another for any particular set of circumstances will be apparent to one of skill in the art.

The peptide MHC complexes described in this section have traditionally been understood to be non-covalent associations, However it is possible, and can be advantageous, to create a covalent linkages, for example by encoding the epitope and MHC heavy chain or the epitope, β2-microglobulin, and MHC heavy chain as a single protein (Yu, Y.L.Y., et al., J. Immunol. 168:3145-3149, 2002; Mottez, E., et at., J. Exp. Med. 181:493,1995; Dela Cruz, C. S., et al., Int. Immunol. 12:1293, 2000; Mage, M, G., et al., Proc. Natl. Acad. Sci. USA 89:10658,1992; Toshitani, K., et al., Proc. Natl. Acad, Sci. USA 93:236,1996; Lee, L., et al., Eur. J. Immunol. 24:2633,1994; Chung, D. H., et al., J. Immunol. 163:3699,1999; Uger, R. A. and B. H. Barber, J. Immunol. 160:1598, 1998; Uger, R. A., et al., J. Immunol. 161:60241,1999; and White, J., et al., J. immunol. 162:2671, 1999. Such constructs can have superior stability and overcome roadblocks in the processing- presentation pathway, They can be used in the already described vaccines, reagents, and assays in similar fashion.

### Tumor Associated Antigens

Epitopes of the present invention are derived from the TuAAs tyrosinase (SEQ ID NO. 2), SSX-2, (SEQ ID NO. 3), PSMA (prostate-specific membrane antigen) (SEQ ID NO. 4), GP100, (SEQ ID NO. 70), MAGE-1, (SEQ ID NO. 71), Marge-2, (SEQ ID NO. 72), MAGE-3, (SEQ ID NO. 73), NY-ESO-1, (SEQ ID NO. 74), PRAME, (SEQ ID NO, 77), PSA, (SEQ ID NO. 78), PSCA, (SEQ ID NO, 79), the ED-B domain of fibronectin (SEQ ID NOS 589 and 590), CEA (carcinoembryonic antigen) (SEQ ID NO, 592), Her2/Neu (SEQ ID NO. 594), SCP- (SEQ ID NO, 596) and SSX-4 (SEQ ID NO. 598). The natural coding sequences for these eleven proteins, or any segments within them, can be determined from their cDNA or complete coding (cds) sequences, SEQ ID NOS. 5-7, 80-87, 591, 593, 595, 597, and 599, respectively.

Tyrosinase is a melanin biosynthetic enzyme that is considered one of the most specific markers of melanocytic differentiation. Tyrosinase is expressed in few cell types, primarily in melanocytes, and high levels are often found in melanomas. The usefulness of tyrosinase as a TuAA is taught in U.S. Patent 5,747,271 entitled "METHOD FOR IDENTIFYING INDIVIDUALS SUFFERING FROM A CELLULAR ABNORMALITY SOME OF WHOSE ABNORMAL CELLS PRESENT COMPLEXES OF HLA-A2/TYROSINASE DERIVED PEPTIDES, AND METHODS FOR TREATING SAID INDIVIDUALS".

GP100, also known as PMe117. also is a melanin biosynthetic protein expressed at high levels in melanomas. GP100 as a TuAA is disclosed in U.S. Patent 5,844,075 entitled "MELANOMA ANTIGENS AND THEIR USE IN DIAGNOSTIC AND THERAPEUTIC METHODS,".

SSX-2, also know as Hom-Mel-40, is a member of a family of highly conserved cancer-testis antigens (Gure, A.O. et al. Int. J. Cancer 72:965.971, 1997. Its identification as a TuAA is taught in U.S. Patent 6,025,191 entitled "ISOLATED NUCLEIC ACID MOLECULES WHICH ENCODE A MELANOMA SPECIFIC ANTIGEN AND USES THEREOF,". Cancer-testis antigens are found in a variety of tumors, but are generally absent from normal adult tissues except testis. Expression of different members of the SSX family have been found variously in tumor cell lines. Due to the high degree of sequence identity among SSX family members, similar epitopes from more than one member of the family will be generated and able to bind to an MHC molecule, so that some vaccines directed against one member of this family can cross-react and be effective against other members of this family (see example 3 below),

MAGE-1, MAGE-2, and MAGE-3 are members of another family of cancer-testis antigens originally discovered in melanoma (MAGE is a contraction of melanoma-associated antigen) but found in a variety of tumors. The identification of MAGE proteins as TuAAs is taught in U.S. Patent 5,342,774 entitled NUCLEOTIDE SEQUENCE ENCODING THE TUMOR REJECTION ANTIGEN PRECURSOR, MAGE-1, and in numerous subsequent patents. Currently there are 17 entries for (human) MAGE in the SWISS Protein database. There is extensive similarity among these proteins so in many cases, an epitope from one can induce a cross-reactive response to other members of the family. A few of these have not been observed in tumors, most notably MAGE-H1 and MAGE-D1, which are expressed in testes and brain, and bone marrow stromal cells, respectively. The possibility of cross-reactivity on normal tissue is ameliorated by the fact that they are among the least similar to the other MAGE proteins.

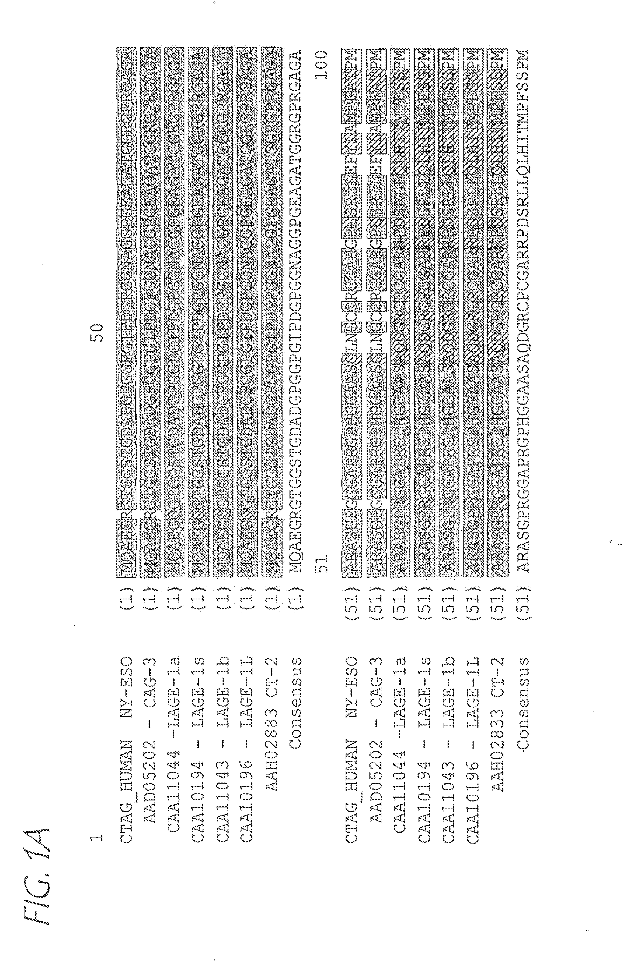

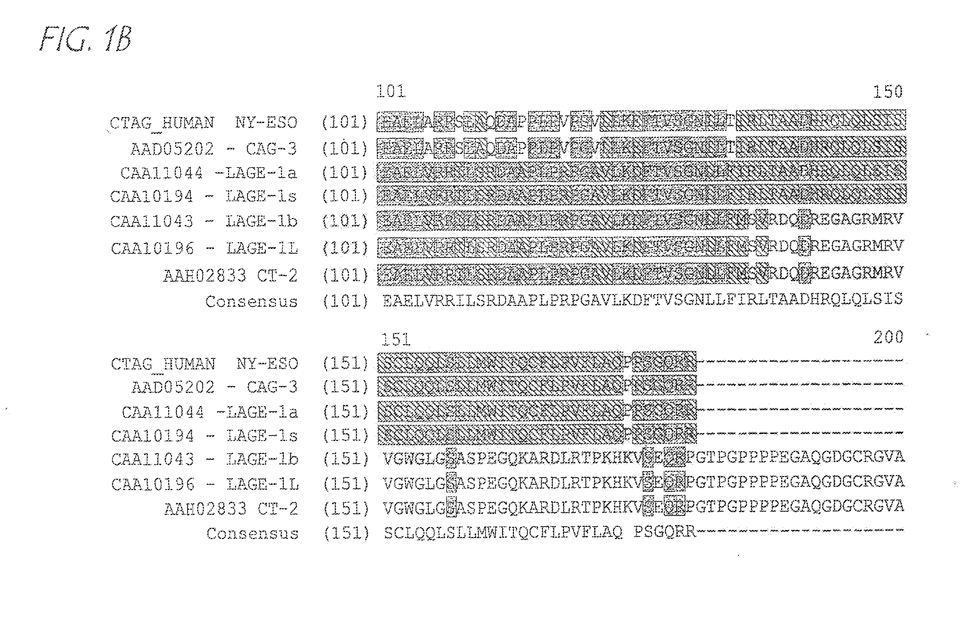

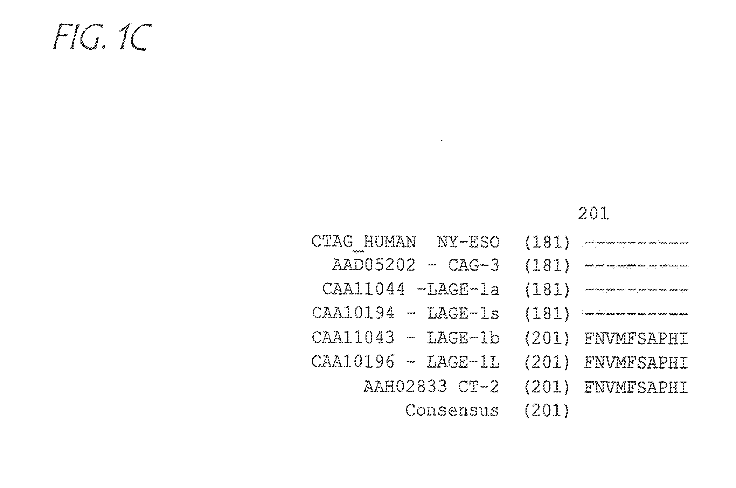

NY-ESO-1, is a cancer-testis antigen found in a wide variety of tumors, also known as CTAG-1 (Cancer-Testis Antigen-1) and CAG-3 (Cancer Antigen-3). NY-ESO-1 as a TuAA is disclosed in U.S. Patent 5,804,381 entitled ISOLATED NUCLEIC ACID MOLECULE ENCODING AN ESOPHAGEAL CANCER ASSOCIATED ANTIGEN, THE ANTIGEN ITSELF, AND USES THEREOF. A paralogous locus encoding antigens with extensive sequence identity, LAGE-1a/s (SEQ ID NO. 75) and LAGE-1b/L (SEQ ID NO. 76), have been disclosed in publicly available assemblies of the human genome, and have been concluded to arise through alternate splicing. Additionally, CT-2 (or CTAG-2, Cancer-Testis Antigen-2) appears to be either an allele, a mutant, or a sequencing discrepancy of LAGE-1b/L. Due to the extensive sequence identity, many epitopes from NY-ESO-1 can also induce immunity to tumors expressing these other antigens. See figure 1. The proteins are virtually identical through amino acid 70. From 71-134 the longest run of identities between NY-ESO-1 and LAGE is 6 residues, but potentially cross-reactive sequences are present. And from 135-180 NY-ESO and LAGE-1a/s are identical except for a single residue, but LAGE-1b/L is unrelated due to the alternate splice. The CAMEL and LAGE-2 antigens appear to derive from the LAGE-1 mRNA, but from alternate reading frames, thus giving rise to unrelated protein sequences. More recently, GenBank Accession AF277315.5, Homo sapiens chromosome X clone RP5-865E18, RP5-1087L19, complete sequence, reports three independent loci in this region which are labeled as LAGE1 (corresponding to CTAG-2 in the genome assemblies), plus LAGE2-A and LAGE2-B (both corresponding to CTAG-1 in the genome assemblies).

PSMA (prostate-specific membranes antigen), a TuAA described in U.S. Patent 5,538,866 entitled "PROSTATE-SPECIFIC MEMBRANES ANTIGEN", is expressed by normal prostate epithelium and, at a higher level, in prostatic cancer. It has also been found in the neovasculature of non-prostatic tumors. PSMA can thus form the basis for vaccines directed to both prostate cancer and to the neovasculature of other tumors. This later concept is more fully described in a provisional U.S. Patent application No. 60/274,063 entitled ANTI-NEOVASCULAR VACCINES FOR CANCER, filed March 7, 2001, and U.S. Application No. 10/094,699, attorney docket number CTLIIMM.015A, filed on March 7, 2002, entitled "ANTI-NEOVASCULAR PREPARATIONS FOR CANCER,". Briefly, as tumors grow they recruit ingrowth of new blood vessels. This is understood to be necessary to sustain growth as the centers of unvascularized tumors are generally necrotic and angiogenesis inhibitors have been reported to cause tumor regression. Such new blood vessels, or neovasculature, express antigens not found in established vessels, and thus can be specifically targeted. By inducing CTL against neovascular antigens the vessels can be disrupted, interrupting the flow of nutrients to (and removal of wastes from) tumors, leading to regression.

Alternate splicing of the PSMA mRNA also leads to a protein with an apparent start at Met₅₈, thereby deleting the putative membrane anchor region of PSMA as described in U.S. Patent 5,935,818 entitled "ISOLATED NUCLEIC ACID MOLECULE ENCODING ALTERNATIVELY SPLICED PROSTATE-SPECIFIC MEMBRANES ANTIGEN AND USES THEREOF". A protein termed PSMA-liks protein, Genbank accession number AF261715, is nearly identical to amino acids 309-750 of PSMA and has a different expression profile. Thus the most preferred epitopes are those with an N-terminus located from amino acid 58 to 308.

FRAME, also know as MAPE, DACE, and OIP4, was originally observed as a melanoma antigen. Subsequently, it has been recognized as a CT antigen, but unlike many CT antigens (e.g., MAGE, GAGE, and BAGE) it is expressed in acute myeloid leukemias. FRAME is a member of the MAPE family which consists largely of hypothetical proteins with which it shares limited sequence similarity. The usefulness of FRAME as a TuAA is taught in U.S. Patent 5,830,753 entitled "ISOLATED NUCLEIC ACID MOLECULES CODING FOR TUMOR REJECTION ANTIGEN PRECURSOR DAGE AND USES THEREOF".

PSA, prostate specific antigen, is a peptidase of the kallikrein family and a differentiation antigen of the prostate. Expression in breast tissue has also been reported. Alternate names include gamma-seminoprotein, kallikrein 3, seminogelase, seminin, and P-30 antigen. PSA has a high degree of sequence identity with the various alternate splicing products prostatic/glandular kallikrein-1 and -2, as well as kalikrein 4, which is also expressed in prostate and breast tissue. Other kallikreins generally share less sequence identity and have different expression profiles. Nonetheless, cross-reactivity that might be provoked by any particular epitope, along with the likelihood that that epitope would be liberated by processing in non-target tissues (most generally by the housekeeping proteasome), should be considered in designing a vaccine.

PSCA, prostate stem cell antigen, and also known as SCAH-2, is a differentiation antigen preferentially expressed in prostate epithelial cells, and overexpresssed in prostate cancers, Lower level expression is seen in some normal tissues including neuroendocrine cells of the digestive tract and collecting ducts of the kidney. PSCA is described in U.S. Patent 5,856,136 entitled "HUMAN STEM CELL ANTIGENS".

Synaptonemal complex protein 1 (SCP-1), also known as HOM-TES-14, is a meiosis- associated protein and also a cancer-testis antigen (Tureci, O., et al. Proc. Natl. Acad. Sci. USA 95;5211-5216, 1998). As a cancer antigen its expression is not cell-cycle regulated and it is found frequently in gliomas, breast, renal cell, and ovarian carcinomas. It has some similarity to myosins, but with few enough identities that cross-reactive epitopes are not an immediate prospect.

The ED-B domain of fibronectin is also a potential target, Fibronectin is subject to developmentally regulated alternative splicing, with the ED-B domain being encoded by a single exon that is used primarily in oncofetal tissues (Matsuura, H. and S. Hakomori Proc. Natl. Acad. Sci, USA 82:6517-6521, 1985; Carnemolla, B. et al. J. Cell Biol. 108: 1139-1148, 1989; Loridon-Rosa, B. et al. Cancer Res.50:1608-1612, 1990; Nicolo, G. et al. Cell Differ, Dev. 32:401-408, 1990; Borsi, L. et al. Exp. Cell Res. 199:98-105, 1992; Oyama, F. et al. Cancer Res. 53:2005-2011, 1993; Mandel, U. et al. APMIS 102:695-702, 1994; Farnoud, M.R. et al. Int. J. Cancer 61:27-34, 1995; Pujuguet, P. et al. Am. J. Pathol. 148:579-592, 1996; Gambler, U. et al. Heart 75:358-362, 1996;Chevalier, X Br. J. Rheumatol. 35:407-415, 1996; Midulla, M. Cancer Res. 60:164-169, 2000).

The ED-B domain is also expressed in fibronectin of the neovasculature (Kaczmarek, J. et al, Int. J. Cancer 59:11-16,1994; Castellani, P. et al. Int. J. Cancer 59:612-.618, 1994; Neri, D. et al. Nat. Biotech. 15:1271-1275, 1997; Karelina, T.V. and A.Z. Eisen Cancer Detect. Prev. 22:438-444, 1998; Tarli, L. et al. Blood 94:192-198; 1999; Castellani, P. et al. Acta Neurochir. (Wien) 142:277-282, 2000). As an oncofetal domain, the ED-B domain is commonly found in the fibronectin expressed by neoplastic cells in addition to being expressed by the neovasculature. Thus, CTL-inducing vaccines targeting the ED-B domain can exhibit two mechanism of action: direct lysis of tumor cells, and disruption of the tumors blood supply through destruction of the tumor-associated As CTL activity can decay rapidly after withdrawal of vaccine, interference with normal angiogenesis can be minimal. The design and testing of vaccines targeted to neovasculature is described in Provisional U.S. Patent Application No. 60/274,063 entitled "ANTl-NEOVASCULATURE VACCINES FOR CANCER" and in U.S. Patent Application No. 10/094,699, attorney docket number CTLIMM.015A, entitled "ANTI-NEOVASCULATURE PREPARATIONS FOR CANCER, filed on date even with Application (March 7, 2002), A tumor cell line is disclosed in Provisional U.S. Application No. 60/363,131, filed on March 7, 2002, attorney docket number entitled "HLA-TRANSEGENIC MURINE TUMOR CELL LINE,".

Carcinoembryonic antigen (CEA) is a paradigmatic oncofetal protein first described in 1965 (Gold and Freedman, J. Exp. Med. 121: 439-462, 1965, Fuller references can be found in the Online Medelian Inheritance in Man; record *114890). It has officially been renamed carcinoembryonic antigen-related cell adhesion molecule 5 (CEACAM5). Its expression is most strongly associated with adenocarcinomas of the epithelial lining of the digestive tract and in fetal colon. CEA is a member of the immunoglobulin supergene family and the defining member of the CEA subfamily.

HER2/NEU is an oncogene related to the epidermal growth factor receptor (van de Vijver, et al., New Eng. J. Med. 319:1239-1245, 1988), and apparently identical to the c-ERBB2 oncogene (Di Fiore, et al., Science 237: 178-192, 1987). The over-expression of ERBB2 has been implicated in the neoplastic transformation of prostate cancer. As HER2 it is amplified and over-expressed in 25-30% of breast cancers among other tumors where expression level is correlated with the aggressiveness of the tumor (Slamon, et al., New Eng. J. Med. 344:783-792, 2001). A more detailed description is available in the Online Medelian Inheritance in Man; record *164870.

Additional disclosure related to embodiments of the present invention is found in U.S. Patent Application No. 10/005,905 (attorney docket number CTLIMM.021CP1) entitled "EPITOPE SYNCHRONIZATION IN ANTIGEN PRESENTING CELLS," filed on November 7, 2001 and a continuation thereof, U.S. Application No. .../..., filed on December 7, 2000, attorney docket number CTLIMM.21CP1C, also entitled "EPITOPE SYNCHRONIZATION IN ANTIGEN PRESENTING CELLS."

Useful epitopes were identified and tested as described in the following examples.
However, these examples are intended for illustration purposes only, and should not be construed as limiting the scope of the invention in any way.

### EXAMPLES

### Sequences of Specific Preferred Epitopes

### Example 1

### Manufacture of epitopes.

### A. Synthetic production of epitopes

Peptides having an amino acid sequence of any of SEQ ID NO: 1, 8, 9, 11-23, 26-29, 32-44, 47-54, 56-63, 66-68 88-253, or 256-588 are synthesized using either FMOC or tBOC solid phase synthesis methodologies. After synthesis, the peptides are cleaved from their supports with either trifluoroacetic acid or hydrogen fluoride, respectively, in the presence of appropriate protective scavengers. After removing the acid by evaporation, the peptides are extracted with ether to remove the scavengers and the crude, precipitated peptide is then lyophilized. Purity of the crude peptides is determined by HPLC, sequence analysis, amino acid analysis, counterion content analysis and other suitable means. If the crude peptides are pure enough (greater than or equal to about 90% pure), they can be used as is. If purification, is required to meet drug substance specifications, the peptides are purified using one or a combination of the following: reprecipitation; reverse-phase, ion exchange, size exclusion or hydrophobic interaction chromatography; or counter-current distribution.

### Drug product formulation

GMP-grade peptides are formulated in a parenterally acceptable aqueous, organic, or aqueous-organic buffer or solvent system in which they remain both physically and chemically stable and biologically potent. Generally, buffers or combinations of buffers or combinations of buffers and organic solvents are appropriate. The pH range is typically between 6 and 9. Organic modifiers or other excipients can be added to help solubilize and stabilize the peptides. These include detergents, lipids, co-solvents, antioxidants, chelators and reducing agents. In the case of a lyophilized product, sucrose or mannitol or other lyophilization aids can be added. Peptide solutions are sterilized by membrane filtration into their final container-closure system and either lyophilized for dissolution in the clinic, or stored until use.

### B. Construction of expression vectors for use as nucleic acid vaccines

The construction of three generic epitope expression vectors is presented below. The particular advantages of these designs are set forth in U.S. Patent Application No. 09/561,572 entitled "EXPRESSION VECTORS ENCODING EPITOPES OF TARGET-ASSOCIATED ANTIGENS,".

A suitable E. coli strain was then transfected with the plasmid and plated out onto a selective medium. Several colonies were grown up in suspension culture and positive clones were identified by restriction mapping. The positive clone was then grown up and aliquotted into storage vials and stored at -70°C.

A mini-prep (QIAprep Spin Mini-prep: Qiagen, Valencia, CA) of the plasmid was then made from a sample of these cells and automated fluorescent dideoxy sequence analysis was used to confirm that the construct had the desired sequence.

### B.1 Construction of pVAX-EP1-IRES-EP2

### Overview:

The starting plasmid for this construct is pVAX1 purchased from Invitrogen (Carlsbad, CA). Epitopes EP1 and EP2 were synthesized by GIBCO BRL (Rockville, MD). The IRES was excised from pIRES purchased from Clontech (Palo Alto, CA).

### Procedure:

1 pIRES was digested with EcoRI and NotI. The digested fragments were separated by agarose gel electrophoresis, and the IRES fragment was purified from the excised band.
2 pVAX1 was digested with EcoRI and NotI, and the pVAX1 fragment was gel-purified.
3 The purified pVAX1 and IRES fragments were then ligated together.
4 Competent E. coli of strain DH5α were transformed with the ligation mixture.
5 Minipreps were made from 4 of the resultant colonies.
6 Restriction enzyme digestion analysis was performed on the miniprep DNA. One recombinant colony having the IRES insert was used for further insertion of EP1 and EP2. This intermediate construct was called pVAX-IRES.
7 Oligonucleotides encoding EP1 and EP2 were synthesized,
8 EP1 was subcloned into pVAX-IRES between AfIII and EcoRI sites, to make pVAX-EP1-IRBS;
9 EP2 was subcloned into pVAX-EP1-IRES between SalI and NotI sites, to make the final construct pVAX-EP1-IRES-EP2.
10 The sequence of the EP1-IRES-EP2 insert was confirmed by DNA sequencing.

### B 2. Construction of pVAX-EP1-IRES-EP2-ISS-NIS

### Overview:

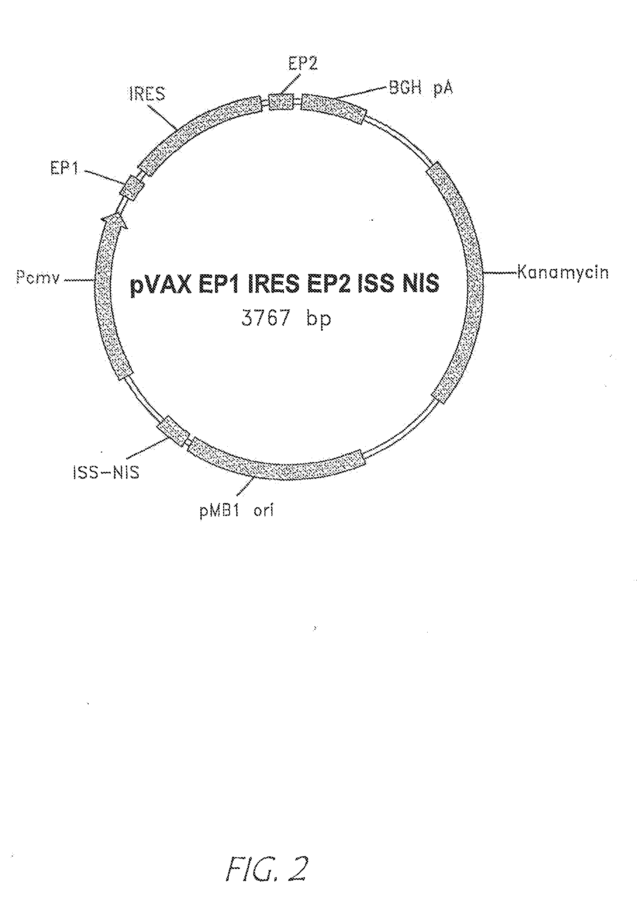

The starting plasmid for this construct was pVAX-EP1-IRES-EP2 (Example 1), The ISS (immunostimulatory sequence) introduced into this construct is AACGTT, and the NIS (standing for nuclear import sequence) used is the SV40 72bp repeat sequence. ISS-NIS was synthesized by GIBCO BRL. See Figure 2.

### Procedure:

1 pVAX-EP1-IRES-EP2 was digested with NruI; the linearized plasmid was gel-purified.
2 ISS-NIS oligonucleotide was synthesized.
3 The purified linearized pVAX-EP1-IRES-EP2 and synthesized ISS-NIS were ligated together.
4 Competent E. coli of strain DH5α were transformed with the ligation product.
5 Minipreps were made from resultant colonies.
6 Restriction enzyme digestions of the minipreps were carried out.
7 The plasmid with the insert was sequenced.

### B3. Construction of pVAX-EP2-UB-EP1

### Overview:

The starting plasmid for this construct was pVAX1 (Invitrogen). EP2 and EP1 were synthesized by GIBCO BRL, Wild type Ubiquitin cDNA encoding the 76 amino acids in the construct was cloned from yeast.

### Procedure:

1 RT-PCR was performed using yeast mRNA. Primers were designed to amplify the complete coding sequence of yeast Ubiquitin.
2 The RT-PCR products were analyzed using agarose gel electrophoresis. A band with the predicted size was gel-purified.
3 The purified DNA band was subcloned into pZERO1 at EcoRV site. The resulting clone was named pZERO-UB.
4 Several clones of pZERO-UB were sequenced to confirm the Ubiquitin sequence before further manipulations.
5 EP1 and EP2 were synthesized.
6 EP2, Ubiquitin and EP1 were ligated and the insert cloned into pVAX1 between BamHI and EcoRI, putting it under control of the CMV promoter.
7 The sequence of the insert EP2-UB-EP1 was confirmed by DNA sequencing.

### Example 2

### Identification of useful epitope variants.

The 10-mer FLPWHRLFLL (SEQ ID NO. 1) is identified as a useful epitope. Based on this sequence, numerous variants are made. Variants exhibiting activity in HLA binding assays (see Example 3, section 6) are identified as useful, and are subsequently incorporated into vaccines.

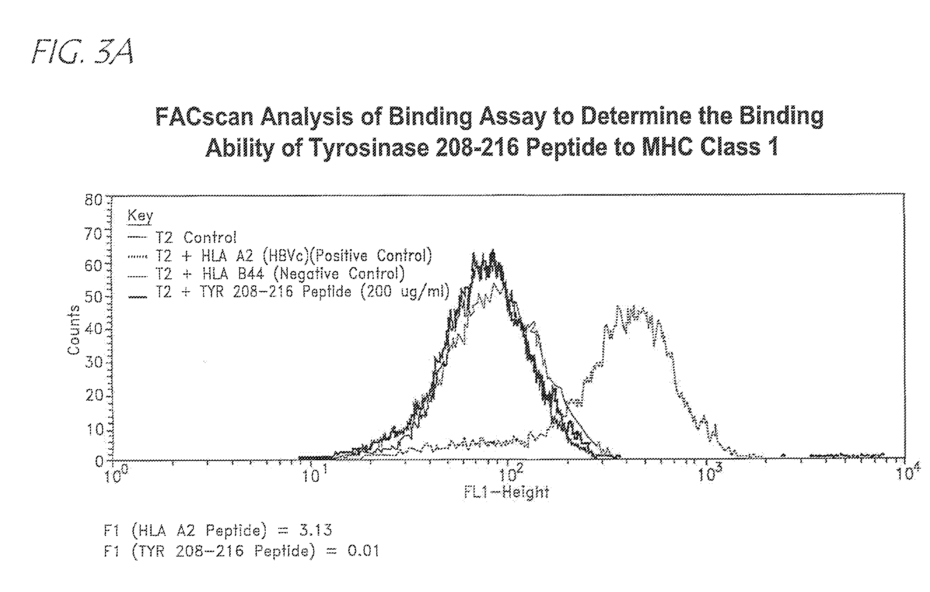

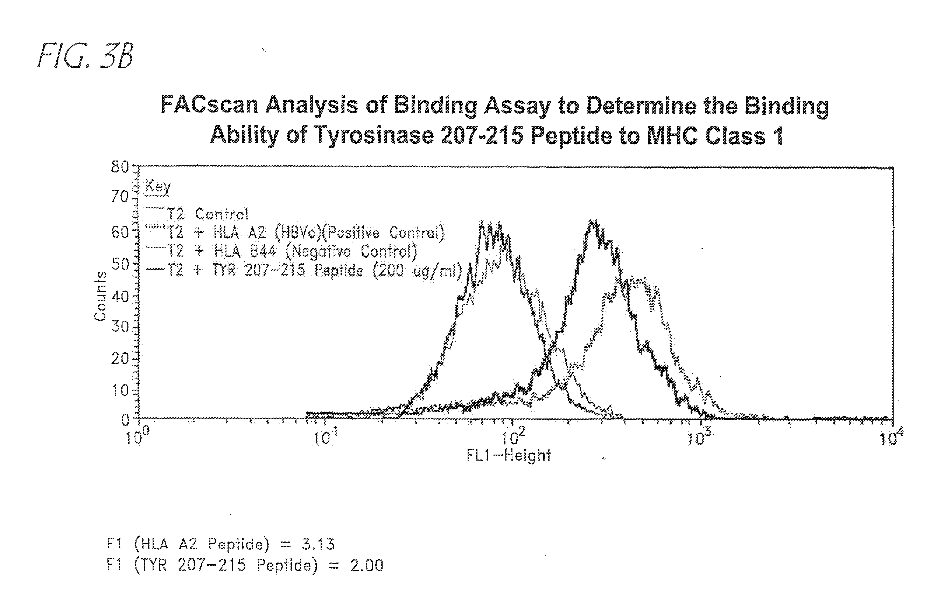

The HLA-A2 binding of length variants of FLPWHRLFLL have been evaluated. Proteasomal digestion analysis indicates that the C-terminus of the 9-mer FLPWHRLFL (SEQ ID NO. 8) is also produced, Additionally the 9-mer LPWHRLFLL (SEQ ID NO, 9) can result from N-terminal trimming of the 10-mer. Both are predicted to bind to the HLA-A*0201 molecule, however of these two 9-mers, FLPWHRLFL displayed more significant binding and is preferred (see Figs. 3A and B).

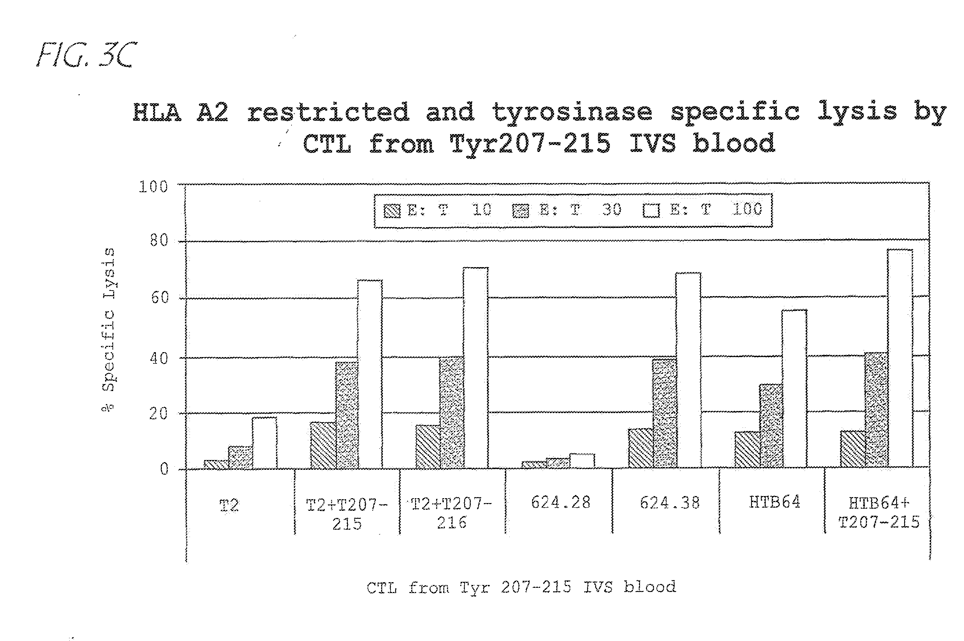

In vitro proteasome digestion and N-terminal pool sequencing indicates that tyrosinase₂₀₇₋₂₁₆ (SEQ ID NO. 1) is produced more commonly than tyrosinase₂₀₇₋₂₁₅ (SEQ ID NO. 8), however the latter peptide displays superior immunogenicity, a potential concern in arriving at an optimal vaccine design. FLPWHRLFL, tyrosinase₂₀₇₋₂₁₅ (SEQ ID NO. 8) was used in an in vitro immunization of HLA-A2⁺ blood to generate CTL (see CTL Induction Cultures below). Using peptide pulsed T2 cells as targets in a standard chromium release assay it was found that the CTL induced by tyrosinase₂₀₇₋₂₁₅ (SEQ ID NO. 8) recognize tyrosinase₂₀₇₋₂₁₆ (SEQ ID NO. 1) targets equally well (see fig. 3C). These CTL also recognize the HLA-A2²⁺, tyrosinase⁺ tumor cell lines 624.38 and HTB64, but not 624.28 an HLA-A2⁻ derivative of 624.38 (fig. 3C). Thus the relative amounts of these two epitopes produced in vivo, does not become a concern in vaccine design.

### CTL induction cultures

PBMCs from normal donors were purified by centrifugation in Ficoll-Hypaque from buffy coats. All cultures were carried out using the autologous plasma (AP) to avoid exposure to potential xenogeneic pathogens and recognition of FBS peptides. To favor the in vitro generation of peptide-specific CTL, we employed autologous dendritic cells (DC) as APCs. DC were generated and CTL were induced with DC and peptide from PBMCs as described (Keogh et al., 2001), Briefly, monocyte-enriched cell fractions were cultured for 5 days with GM-CSF and IL-4 and were cultured for 2 additional days in culture media with 2 µg/ml CD40 ligand to induce maturation. 2 x10⁶ CD8+-enriched T lymphocytes/well and 2 x10⁵ peptide-pulsed DC/well were co-cultured in 24-well plates in 2 ml RPMI supplemented with 10% AP, 10 ng/ml IL-7 and 20 IU/ml IL-2. Cultures were restimulated on days 7 and 14 with autologous irradiated peptide-pulsed DC.

Sequence variants of FLPWHRLFL are constructed as follow. Consistent with the binding coefficient table (see Table 3) from the NIH/BIMAS MHC binding prediction program (see reference in example 3 below), binding can be improved by changing the L at position 9, an anchor position, to V. Binding can also be altered, though generally to a lesser extent, by changes at non-anchor positions. Referring generally to Table 3, binding can be increased by employing residues with relatively larger coefficients. Changes in sequence can also alter immunogenicity independently of their effect on binding to MHC. Thus binding and/or immunogenicity can be improved as follows:
By substituting F,L,M,W, or Y for P at position 3; these are all bulkier residues that can also improve immunogenicity independent of the effect on binding. The amine and hydroxyl-bearing residues, Q and N; and S and T; respectively, can also provoke a stronger, cross-reactive response.
By substituting D or E for W at position 4 to improve binding; this addition of a negative charge can also make the epitope more immunogenic, while in some cases reducing cross-reactivity with the natural epitope. Alternatively the conservative substitutions of F or Y can provoke a cross-reactive response.
By substituting F for H at position 5 to improve binding. H can be viewed as partially charged, thus in some cases the loss of charge can hinder cross-reactivity. Substitution of the fully charged residues R or K at this position can enhance immunogenicity without disrupting charge-dependent cross-reactivity.
By substituting I, L, M, V, F, W, or Y for R at position 6. The same caveats and alternatives apply here as at position 5.
By substituting W or F for L at position 7 to improve binding. Substitution of V, I, S, T, Q, or N at this position are not generally predicted to reduce binding affinity by this model (the NIH algorithm), yet can be advantageous as discussed above.

Y and W, which are equally preferred as the Fs at positions 1 and 8, can provoke a useful cross-reactivity. Finally, while substitutions in the direction of bulkiness are generally favored to improve immunogenicity, the substitution of smaller residues such as A, S, and C, at positions 3-7 can be useful according to the theory that contrast in size, rather than bulkiness per se, is an important factor in immunogenicity. The reactivity of the thiol group in C can introduce other properties as discussed in Chen, J.-L., et al. J. Immunol. 165:948-955, 2000.

**Table 3. 9-mer Coefficient Table for HLA-A*0201***

| HLA Coefficient table for file "A_0201_standard" | | | | | | | | | |
|---|---|---|---|---|---|---|---|---|---|
| Amino Acid Type | 1^{st} | 2^{nd} | 3rd | 4th | 5th | 6th | 7th | 8th | 9th |
| A | 1.000 | 1.000 | 1.000 | 1.000. | 1.000 | 1.000 | 1.000 | 1.000 | 1.000 |
| C | 1.000 | 0.470 | 1.000 | 1.000 | 1.000 | 1.000 | 1.000 | 1.000 | 1.000 |
| D | 0.075 | 1.000 | 0.400 | 4.100 | 1.000 | 1.000 | 0.490 | 1.000 | 0.003 |
| E | 0.075 | 1.400 | 0.064 | 4.100 | 1.000 | 1.000 | 0.490 | 1.000 | 0.003 |
| F | 4.600 | 0.050 | 3.700 | 1.000 | 3.800 | 1.900 | 5.800 | 5.500 | 0.015 |
| G | 1.000 | 0.470 | 1.000 | 1.000 | 1.000 | 1.000 | 0.130 | 1.000 | 0.015 |
| H | 0,034 | 0.050 | 1.000 | 1.000 | 1.000 | 1.000 | 1.000 | 1.000 | 0.015 |
| I | 1.700 | 9.900 | 1.000 | 1.000 | 1.000 | 2.300 | 1.000 | 0.410 | 2.100 |
| K | 3.500 | 0.100 | 0.035 | 1.000 | 1.000 | 1.000 | 1.000 | 1.000 | 0.003 |
| L | 1.700 | 72.000 | 3.700 | 1,000 | 1.000 | 2.300 | 1.000 | 1.000 | 4.300 |
| M | 1.700 | 52.000 | 3.700 | 1.000 | 1.000 | 2.300 | 1.000 | 1.000 | 1.000 |
| N | 1.000 | 0.470 | 1,000 | 1.000 | 1.000 | 1.000 | 1.000 | 1.000 | 0.015 |
| P | 0.022 | 0.470 | 1.000 | 1.000 | 1.000 | 1.000 | 1.000 | 1.000 | 0.003 |
| Q | 1.000 | 7.300 | 1.000 | 1.000 | 1.000 | 1.000 | 1.000 | 1.000 | 0.003 |
| R | 1.000 | 0.010 | 0,076 | 1.000 | 1.000 | 1.000 | 0.200 | 1.000 | 0.003 |
| S | 1.000 | 0.470 | 1.000 | 1.000 | 1.000 | 1.000 | 1.000 | 1.000 | 0.015 |
| T | 1.000 | 1.000 | 1.000 | 1.000 | 1.000 | 1.000 | 1.000 | 1.000 | 1.500 |
| V | 1.700 | 6.300 | 1.000 | 1.000 | 1.000 | 2.300 | 1.000 | 0.410 | 14.000 |
| W | 4.600 | 0.010 | 8.300 | 1.000 | 1.000 | 1.700 | 7.500 | 5.500 | 0.015 |
| Y | 4.600. | 0.010 | 3.200 | 1.000 | 1.000 | 1.500 | 1.000 | 5.500 | 0.015 |

| | | | | | | | | | |
|---|---|---|---|---|---|---|---|---|---|
| *This table and other comparable data thar are publicly available are useful in designing epitope variants and in determining whether a particular variant is substantially similar, or is functionally similar. | | | | | | | | | |

### Example 3

### Cluster Analysis (SSX-2_{31 68}).

### 1. Epitope cluster region prediction:

The computer algorithms: SYFPEITHI (internet http:// access at syfpeithi.bmi-beidelberg.com/Scripts/MHCServer.dll/EpPredict.htm), based on the book "MHC Ligands and Peptide Motifs" by H.G.Rammense, J.Bachmann and S.Stevanovic; and HLA Peptide Binding Predictions (NIH) (internet http:// access at bimas.dert.nih.gov/molbio/hla_bin), described in Parker, K. C., et al., J. Immunol. 152:163, 1994; were used to analyze the protein sequence of SSX-2 (GI:10337583). Epitope clusters (regions with higher than average density of peptide fragments with high predicted MHC affinity) were defined as described fully in U.S. Patent Application No. 09/561,571 entitled "EPITOPE CLUSTERS." filed on April 28, 2000. Using a epitope density ratio cutoff of 2. five and two clusters were defined using the SYFPETIHI and NIH algorithms, respectively, and peptides score cutoffs of 16 (SYFPETHI) and 5 (NIH). The highest scoring peptide with the NIH. algorithm, SSX-2₄₁₋₄₉, with an estimated halftime of dissociation of >1000 min., does not overlap any other predicted epitope but does cluster with SSX-2₅₇₋₆₅ in the NIH analysis.

### 2. Peptide_synthesis and_characterization:

SSX-2₃₁₋₆₈, YFSKEEWEKMKASEKIFYVYMKRKYEAMTKLGFKATLP (SEQ ID NO. 10) was synthesized by MPS (Multiple Peptide Systems, San Diego, CA 92121) using standard solid phase chemistry. According to the provided 'Certificate of Analysis', the purity of this peptide was 95%.

### 3. Proteasome digestion:

Proteasome was isolated from human red blood cells using the proteasome isolation protocol described in U,S. Patent Application No. 09/561,074 entitled "METHOD OF EPITOPH DISCOVERY," filed on April 28, 2000, SDS-PAGE, western-blotting, and ELISA were used as quality control assays. The final concentration of proteasome was 4 mg/ml, which was determined by non-interfering protein assay (Geno Technologies Inc.). Proteasomes were stored at -70°C in 25 µl aliquots.

SSX-2₃₁₋₆₈ was dissolved in Milli-Q water, and a 2 mM stock solution prepared and 20µL aliquots stored at -20°C.

1 tube of proteasome (25 µL) was removed from storage at-70°C and thawed on ice. It was then mixed thoroughly with 12.5µL of 2mM peptide by repipetting (samples were kept on ice). A 5µL sample was immediately removed after mixing and transferred to a tube containing 1.25µL 10%TFA (final concentration of TFA was 2%); the T=0 min sample. The proteasome digestion reaction was then started and carried out at 37°C in a programmable thermal controller. Additional 5µL samples were taken out at 15, 30, 60, 120, 180 and 240 min respectively, the reaction was stopped by adding the sample to 1.25µL 10% TFA as before. Samples were kept on ice or frozen until being analyzed by MALDI-MS. All samples were saved and stored at -20°C for HPLC analysis and N-terminal sequencing. Peptide alone (without proteasome) was used as a blank control: 2 µL peptide + 4µL Tris buffer (20 mM, pH 7.6) + 1.5µL TFA.

### 4. MALDI-TOF MS measurements:

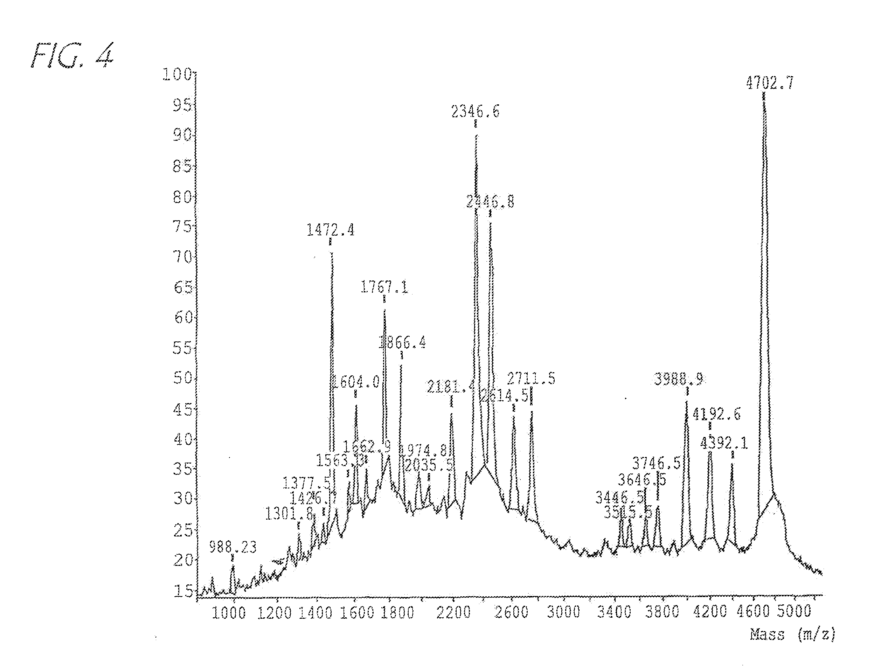

For each time point 0.3 µL of matrix solution (10mg/ml α-cyano-4-hydroxycinnamic acid in AcCN/H₂O (70:30)) was first applied on a sample slide, and then an equal volume of digested sample was mixed gently with matrix solution on the slide, The slide was allowed to dry at ambient air for 3-5 min. before acquiring the mass spectra. MS was performed on a Lasermat 2000 MALDI-TOF mass spectrometer that was calibrated with peptide/protein standards, To improve the accuracy of measurement, the molecular ion weight (MH⁺) of the peptide substrate was used as an internal calibration standard, The mass spectrum of the T=120 min, digested sample is shown in figure 4,

### 5. MS data analysis and epitope identification:

To assign the measured mass peaks, the computer program MS-Product, a tool from the UCSF Mass Spectrometry Facility (http:// accessible at prospector.ucsf.edu/ucsfhtml3.4/msprod.htm), was used so generate all possible fragments (N- and C-terminal ions, and internal fragments) and their corresponding molecular weights. Due to the sensitivity of the mass spectrometer, average molecular weight was used. The mass peaks observed over the course of the digestion were identified as summarized in Table 4.

Fragments co-C-terminal with 8-10 amino acid long sequences predicted to bind HLA by the SYFPEITHI or NIH algorithms were chosen for further study. The digestion and prediction steps of the procedure can be usefully practiced in any order. Although the substrate peptide used in proteasomal digest described here was specifically designed to include predicted HLA-A2.1 binding sequences, the actual products of digestion can be checked after the fact for actual or predicted binding to other MHC molecules. Selected results are shown in Table 5.

**Table 4. SSX-2₃₁₋₆₈ Mass Peak Identification.**

| MS PEAK (measured) | PEPTIDE | SEQUENCE | CALCULATED MASS (MH) |
|---|---|---|---|
| 988.23 | 31-37 | YFSKEEW | 989,08 |
| 1377.68±2.3 | | | |
| 8 | 31-40 | YFSKEEWEKM | 1377.68 |
| 1662.45±1.3 | | | |
| 0 | 31-43 | YFSKEEWEKMKAS | 1663.90 |
| 2181.72±0.8 | | | |
| 5 | 31-47 | YFSKEEWEKMKASEKIF | 2181.52 |
| 2346.6 | 31-48 | YFSKEEWEKMKASEKIFY | 2344.71 |
| 1472.16±1.5 | | | |
| 4 | 38-49 | EKMKASEKIFYV | 1473,77 |
| 2445.78±1.1 | | | |
| 8 | 31-49* | YFSKEEMEKMKASEKIFYV | 2443.84 |
| 2607. | 31-50 | YFSKEEWEKMKASEKIFYVY | 2607.02 |
| 1563.3 | 50-61 | YMKRKYEAMTKL | 1562.93 |
| 3989.9 | 31-61 | YFSKEEWEKMKASEKIFYVYMKRKYEAMTKL | 3987.77 |
| 1603.74±1.5 | 51.63 | | 1603.98 |
| 3 | | MKRKYEAMTKLGF | |
| 1766.45±1.5 | 50-63 | YMKRKYEAMTKLGF | 1767.16 |
| 1866.32±1.2 | | | |
| 2 | 49-63 | VYMKRKYEAMTKLGF | 1866.29 |
| | | YFSKEEWEKMKASEKIFYVYMKRKYEAMTKLG | |
| 4192.6 | 31-63 | F | 4192.00 |
| | | YFSKEEWEKMKASEKIFYVYMKRKYEAMTKLG | |
| 4392.1 | 31-65** | FKA | 4391.25 |

| | | | |
|---|---|---|---|
| **Boldface** sequence correspond to peptides predicted to bind to MHC. * On the basis of mass alone this peak could also have been assigned to the peptide 32-50, however proteasomal removal of just the N-terminal amino acid is unlikely. N-terminal sequencing (below) verifies the assignment to 31-49, ** On the basis of mass this fragment might also represent 33-68. N-terminal sequencing below is consistent with the assignment to 31-65. | | | |

**Table 5. Predicted HLA binding by proteasomally generated fragments**

| **SEQ ID NO,** | **PEPTIDE** | HLA | **SYFPEITHI** | **NIH** |
|---|---|---|---|---|
| 11 | FSKEEWEKM | B*3501 | NP† | 90 |
| 12 | KMKASEKIF | B*08 | 17 | <5 |
| 13 & (14) | (K) MKASEKIFY | A1 | 19 (19) | <5 |
| 15 &(16) | (M) KASEKIFYV | A*0201 | 22(16) | 1017 |
| | | B*08 | 17 | <5 |
| | | B*5101 | 22 (13) | 60 |
| | | B*5102 | NP | 133 |
| | | B*5103 | NP | 121 |
| 17 & (18) | (K) ASEKIFYVY | A1 | 34(19) | 14 |
| 19 & (20) | (K) RKYEAMTKL | A*0201 | 15 | <5 |
| | | A26 | 15 | NP |
| | | B14 | NP | 45 (60) |
| | | B*2705 | 21 | 15 |
| | | B*2709 | 16 | NP |
| | | B*5101 | 15 | <5 |
| 21 | KYEAMTKLGF | A1 | 16 | <5 |
| | | A24 | NP | 300 |
| 22 | | B*4403 | NP | 80 |
| 23 | | B*08 | 22 | <5 |

| | | | | |
|---|---|---|---|---|
| †No prediction | | | | |

As seen in Table 5, N-terminal addition of authentic sequence to epitopes can generate epitopes for the same or different MHC restriction elements. Note in particular the pairing of (K)RKYEAMTKL (SEQ ID NOS 19 and (20)) with HLA-B14, where the 10-mer has a longer predicted halftime of dissociation than the co-C-terminal 9-mer. Also note the case of the 10-mer KYEAMTKLGF (SEQ ID NO. 21) which can be used as a vaccine useful with several MHC types by relying on N-terminal trimming to create the epitopes for HLA-B*4403 and -B*08.

### 6. HLA-A0201 binding assay:

Binding of the candidate epitope KASEKIFYV, SSX-2₄₁₋₄₉, (SEQ ID NO. 15) to HLA-A2.1 was assayed using a modification of the method of Stauss et al., (Proc Natl Acad Sci USA 39(17):7871-5 (1992)). Specifically, T2 cells, which express empty or unstable MHC molecules on their surface, were washed twice, with Iscove's modified Dulbecco's medium (IMDM) and cultured overnight in serum-free AIM-V medium (Life Technologies, Inc., Rockville, MD) supplemented with human β2-microglobulin at 3µg/ml (Sigma, St. Louis, MO) and added peptide, at 800,400, 200, 100, 50, 25, 12.5, and 6.25 µg/ml.in a 96-well flat-bottom plate at 3x10⁵ cells/200 µl/well. Peptide was mixed with the cells by repipeting before distributing to the plate (alternatively peptide can be added to individual wells), and the plate was rocked gently for 2 minutes. Incubation was in a 5% CO₂ incubator at 37°C. The next day the unbound peptide was removed by washing twice with serum free RPMI medium and a saturating amount of anti-class I HLA monoclonal antibody, fluorescein isothiocyanate (FITC)-conjugated anti-HLA A2, A28 (One Lambda, Canoga Park, CA) was added. After incubation for 30 minutes at 4°C, cells were washed 3 times with PBS supplemented with 0.5% BSA, 0.05%(w/y) sodium azide, pH 7.4-7.6 (staining buffer), (Alternatively W6/32 (Sigma) can be used as the anti-class I HLA monoclonal antibody the cells washed with staining buffer and then incubated with fluorescein isothiocyanate (FITC)-conjugated goat F(ab') antimouse-IgG (Sigma) for 30 min at 4°C and washed 3 times as before.) The cells were resuspended in 0.5 ml staining buffer. The analysis of surface HLA-A2.1 molecules stabilized by peptide binding was performed by flow cytometry using a FACScan (Becton Dickinson, San Jose, CA). If flow cytometry is not to be performed immediately the cells can be fixed by adding a quarter volume of 2% paraformaldehyde and storing in the dark at 4 C.

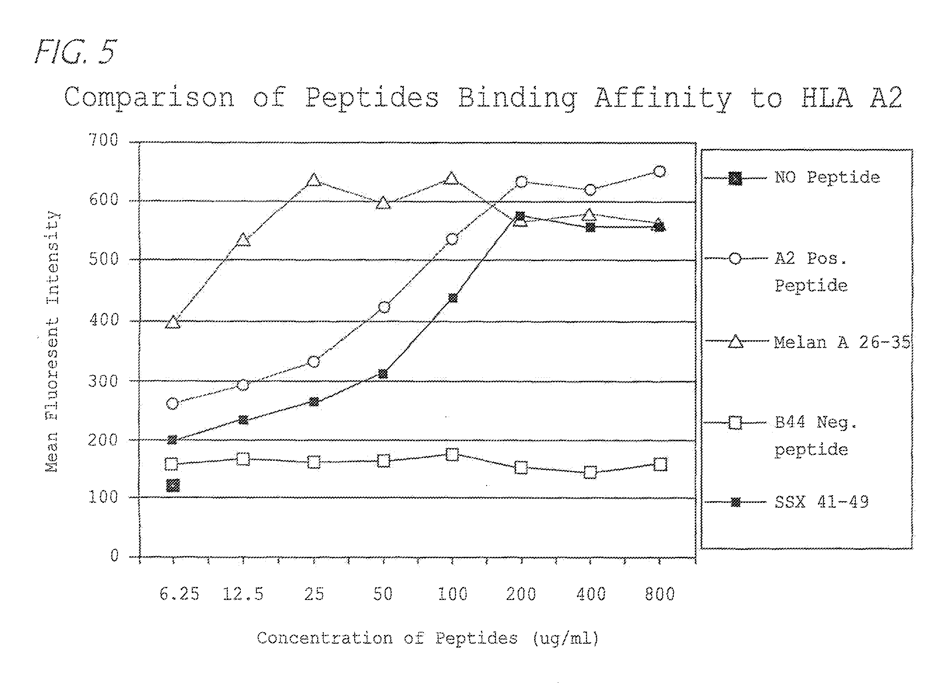

The results of the experiment are shown in Figure 5. SSX-2₄₁₋₄₉ (SEQ ID NO. 15) was found to bind HLA-A2.1 to a similar extent as the known A2.1 binder FLPSDYFPSV (HBV₁₈₋₂₇; SEQ ID NO: 24) used as a positive control. An HLA-B44 binding peptide, AEMGKYSFY (SEQ ID NO: 25), was used as a negative control. The fluoresence obtained from the negative control was similar to the signal obtained when no peptide was used in the assay. Positive, and negative control peptides were chosen from Table 18.3.1 in Current Protocols in Immunology p. 18.3.2, John Wiley and Sons, New York, 1998. 7. Immunogenicity;

### A. In vivo immunization of mice.

HHD1 transgenic A*0201 mice (Pascolo, S., et al, J. Exp. Med. 185:2043-2051, 1997) were anesthetized and injected subcutaneously at the base of the tail, avoiding lateral tail veins, using 100 µl containing 100 mmol of SSX-2₄₁₋₄₉ (SEQ ID NO. 15) and 20 µg of HTL epitope peptide in PBS emulsified with 50 µl ofIFA (incomplete Freund's adjuvant).

### B. Preparation of stimulating cells (LPS blasts).

Using spleens from 2 naive mice for each group of immunized mice, un-immunized mice were sacrificed and the carcasses were placed in alcohol. Using sterile instruments, the top dermal layer of skin on the mouse's left side (lower mid-section) was cut through, exposing the peritoneum. The peritoneum was saturated with alcohol, and the spleen was aseptically extracted. The spleen was placed in a petri dish with serum-free media. Splenocytes were isolated by using sterile plungers from 3 ml syringes to mash the spleens. Cells were collected in a 50 ml conical tubes in serum-free media, rinsing dish well. Cells were centrifuged (12000 rpm, 7 min) and washed one time with RPMI. Fresh spleen cells were resuspended to a concentration of 1x10⁶ cells per ml in RPMI-10%FCS (fetal calf serum), 25g/ml lipopolysaccharide and 7 µg/ml Dextran Sulfate were added. Cell were incubated for 3 days in T-75 flasks at 37°C, with 5% CO₂. Splenic blasts were collected in 50 ml tubes pelleted (12000 rpm, 7 min) and resuspended to 3X10⁷/ml in RPMI. The blasts were pulsed with the priming peptide at 50 µg/ml, RT 4hr, mitomycin C-treated at 25µg/ml, 37°C, 20 min and washed three times with DMEM.

### C. In vitro stimulation.

3 days after LPS stimulation of the blast cells and the same day as peptide loading, the primed mice were sacrificed (at 14 days post immunization) to remove spleens as above. 3x10⁶ splenocytes were co-cultured with 1x10⁶ LPS blasts/well in 24-well plates at 37°C, with 5% CO₂ in DMEM media supplemented with 10% FCS, 5x10⁻⁵ M β-mercaptoethanol, 100%g/ml streptomycin and 100 IU/ml penicillin. Cultures were fed 5% (vol/vol) ConA supernatant on day 3 and assayed for cytolytic activity on day 7 in a ⁵¹Cr-release assay.

### D. Chromium-release assay measuring CTL activity.

To assess peptide specific lysis, 2x10⁶ T2 cells were incubated with 100 µCi sodium chromate together with 50 µg/ml peptide at 37 C for 1 hour. During incubation they were gently shaken every 15 minutes. After labeling and loading, cells were washed three times with 10 ml of DMEM-10% FCS, wiping each tube with a fresh Kimwipe after pouring off the supernatant. Target cells were resuspended in DMEM-10% FBS 1x10⁵/ml. Effector cells were adjusted to 1x10⁷/ml in DMEM-10% FCS and 100 µl serial 3-fold dilutions of effectors were prepared in U-bottom 96-well plates. 100 µl of target cells were added per well. In order to determine spontaneous release and maximum release, six additional wells containing 100 µl of target cells were prepared for each target. Spontaneous release was revealed by incubating the target cells with 100 µl medium; maximum release was revealed by incubating the target cells with 100µl of 2% SDS. Plates were then centrifuged for 5 min at 600 rpm and incubated for 4 hours at 37°C in 5% CO₂ and 80% humidity. After the incubation, plates were then centrifuged for 5 min at 1200 rpm, Supernatants were harvested and counted using a gamma counter. Specific lysis was determined as follows: % specific release = [(experimental release - spontaneous release)/(maximum release - spontaneous release)] x 100.

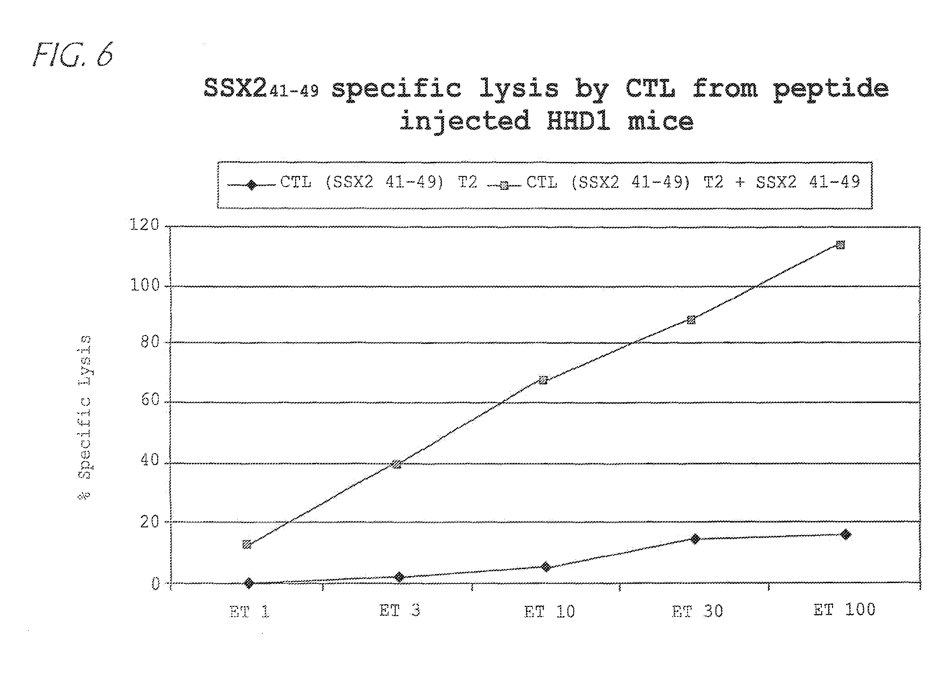

Results of the chromium release assay demonstrating specific lysis of peptide pulsed target cells are shown in figure 6.

### 8. Cross-reactivity with other SSX proteins:

SSX-2₄₁₋₄₉ (SEQ ID NO. 15) shares a high degree of sequence identity with the same region of the other SSX proteins. The surrounding regions have also been generally well conserved. Thus the housekeeping proteasome can cleave following V₄₉ in all five sequences. Moreover, SSX₄₁₋₄₉ is predicted to bind HLA-A*0201 (see Table 6). CTL generated by immunization with SSX-2₄₁₋₄₉ cross-react with tumor cells expressing other SSX proteins.

**Table 6. SSX₄₁₋₄₉-A*0201Predicted Binding**

| **SEQ ID NO**. | **Family Member** | **Sequence** | **SYFPEITHI Score** | **NIH Score** |
|---|---|---|---|---|
| 15 | SSX-2 | KASEKIFYV | 22 | 1017 |
| 26 | SSX-1 | KYSEKISYV | 18 | 1.7 |
| 27 | SSX-3 | KVSEKIVYV | 24 | 1105 |
| 28 | SSX-4 | KSSEKIVBYV | 20 | 82 |
| 29 | SSX-5 | KASEKIIYV | 22 | 175 |

### Example 4

### Cluster Analysis (PSMA₁₆₃₋₁₉₂).

A peptide, AFSPQGMPEGDLVYVNYARTEDFFKLERDM, PSMA₁₆₃₋₁₉₂, (SEQ ID NO. 30), containing an A1 epitope cluster from prostate specific membrane antigen, PSMA₁₆₈₋₁₉₀, (SEQ ID NO. 31) was synthesized using standard solid-phase F-moc chemistry on a 433A ABI Peptide synthesizer. After side chain deprotection and cleavage from the resin, peptide first dissolved in formic acid and then diluted into 30% Acetic acid, was run on a reverse-phase preparative HPLC C4 column at following conditions: linear AB gradient (5% B/min) at a flow rate of 4 ml/mm, where eluent A is 0.1% aqueous TFA and eluent B is 0,1% TFA in acetonitrile. A fraction at time 16.642 min containing the expected peptide, as judged by mass spectrometry, was pooled and lyophilized, The peptide was then subjected to proteasome digestion and mass spectrum analysis essentially as described above. Prominent peaks from the mass spectra are summarized in Table 7.

**Table 7. PSMA₁₆₃₋₁₉₂ Mass Peak Identification.**

| **PEPTIDE** | **SEQUENCE** | **CALCULATE D MASS (MH⁺)** |
|---|---|---|
| 163-177 | AFSPQGMPEGDLVYV | 1610.0 |
| 178-189 | NYARTEDFFKLE | 1533.68 |
| 170-189 | PEGDLVYVNYARTEDFFKLE | 2406.66 |
| 178-191 | NYARTEDFFKLERD | 1804.95 |
| 170.191 | PEGDLVYVNYARTEDFFKLERD | 2677.93 |
| 178-192 | NYARTEDFFKLERDM | 1936.17 |
| 163-176 | AFSPQGMPEGDLVY | 1511.70 |
| 177-192 | VNYARTEDFFKLERDM | 2035.30 |
| 163-179 | AFSPQGMPEGDLVYVNY | 1888.12 |
| 180-192 | ARTEDFFKLERDM | 1658.89 |
| 163-183 | AFSPQGMPEGDLVYVNYARTE | 2345.61 |
| 184-192 | DFFKLERDM | 1201.40 |
| 176-192 | YVNYARTEDFFKLERDM | 2198.48 |
| 167-185 | QGMPEGDLVYVNYARTEDF | 2205.41 |
| 178-186 | NYARTEDFF | 1163.22 |

| | | |
|---|---|---|
| **Boldface** sequences correspond to peptides predicted to bind to MRC, see Table 8. | | |

### N-terminal Pool Sequence Analysis

One aliquot at one hour of the proteasomal digestion (see Example 3 part 3 above) was subjected to N-terminal amino acid sequence analysis by an ABI 473A Protein Sequencer (Applied Biosystems, Foster City, CA). Determination of the sites and efficiencies of cleavage was based on consideration of the sequence cycle, the repetitive yield of the protein sequencer, and the relative yields of amino acids unique in the analyzed sequence. That is if the unique (in the analyzed sequence) residue X appears only in the nth cycle a cleavage site exists n-1 residues before it in the N-terminal direction. In addition to helping resolve any ambiguity in the assignment of mass to sequences, these data also provide a more reliable indication of the relative yield of the various fragments than does mass spectrometry.

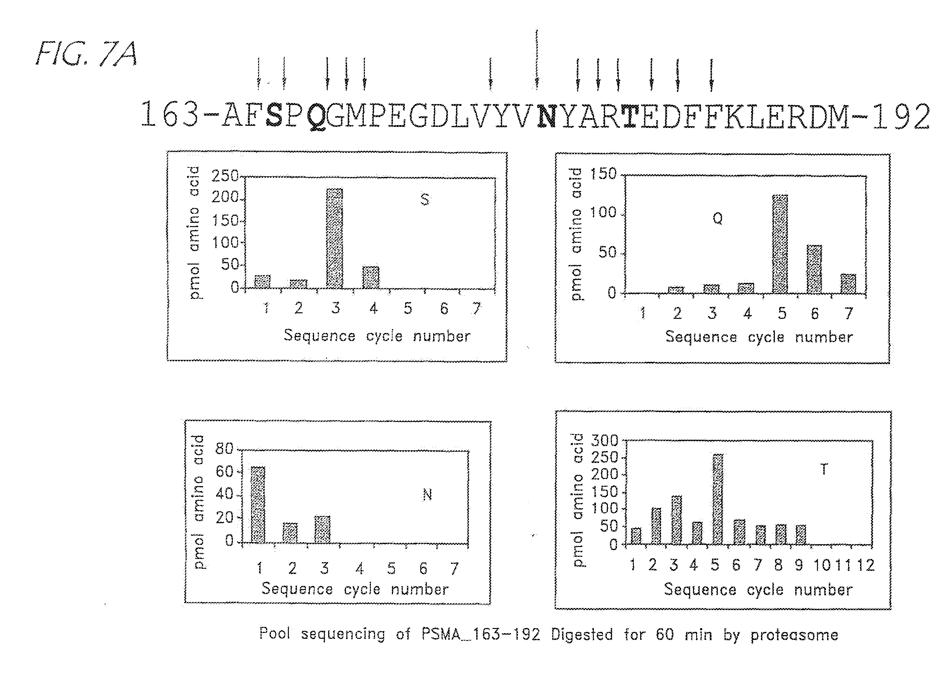

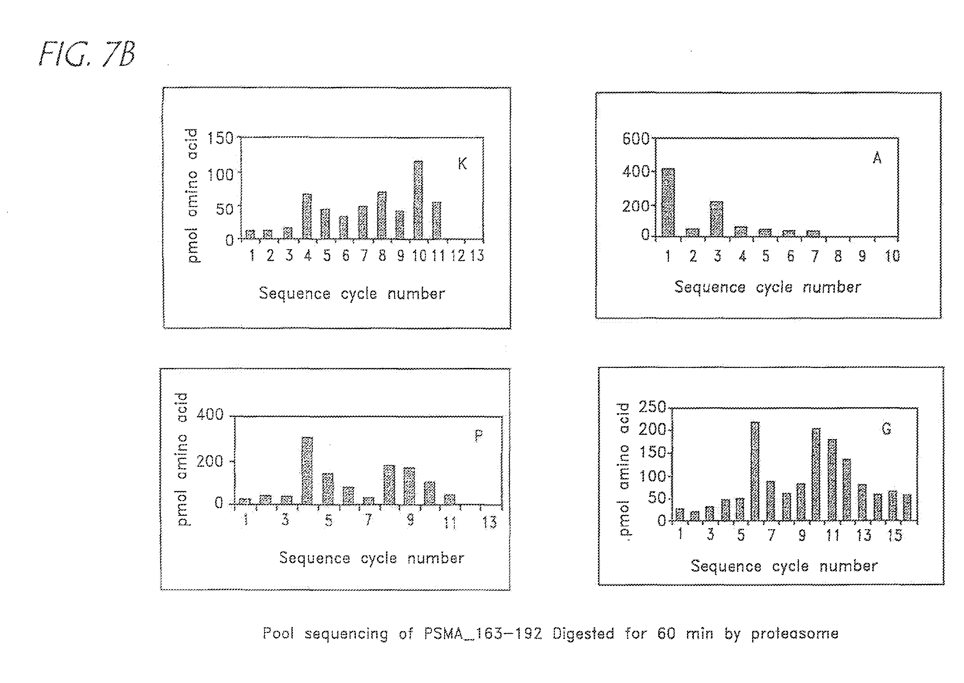

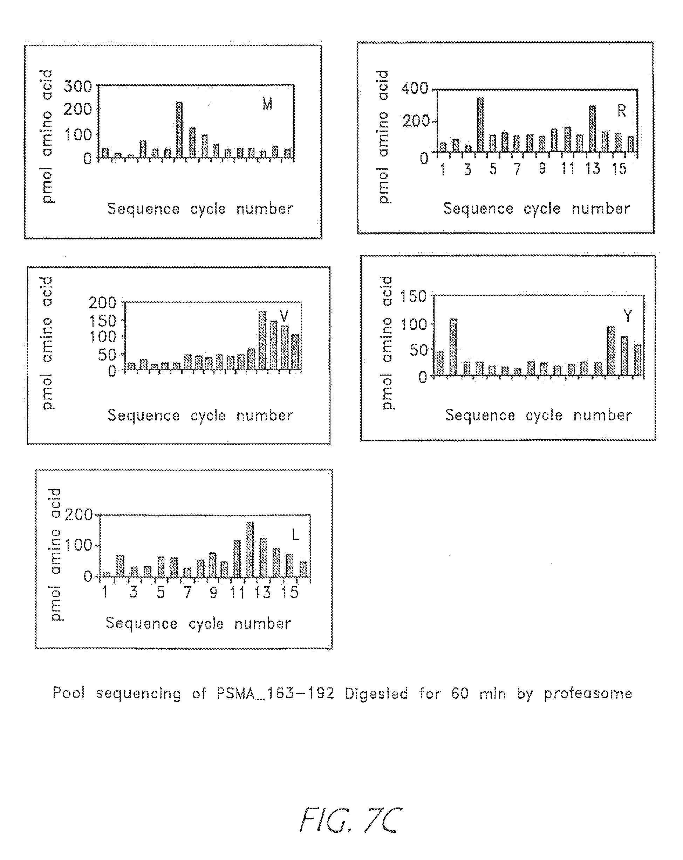

For PSMA₁₆₃₋₁₉₂, (SEQ ID NO. 30) this pool sequencing supports a single major cleavage site after V₁₇₇ and several minor cleavage sites, particularly one after Y₁₇₉. Reviewing the results presented in figures 7A-C reveals the following;
S at the 3^{rd} cycle indicating presence of the N-terminus of the substrate.
Q at the 5^{th} cycle indicating presence of the N-terminus of the substrate.
N at the 1^{st} cycle indicating cleavage after V₁₇₇.
N at the 3^{rd} cycle indicating cleavage after V₁₇₅. Note the fragment 176-192 in Table 7.
T at the 5^{th} cycle indicating cleavage after V₁₇₇.
T at the 1^{st} -3^{rd} cycles, indicating increasingly common cleavages after R₁₈₁, A₁₈₀ and Y₁₇₉.
   Only the last of these correspond to peaks detected by mass spectrometry; 163-179 and 180-192, see Table 7. The absence of the others can indicate that they are on fragments smaller than were examined in the mass spectrum.
K at the 4^{th}, 8^{th}, and 10^{th} cycles indicating cleavages after E₁₈₃, Y₁₇₉ and V₁₇₇, respectively, all of which correspond to fragments observed by mass spectroscopy, See Table 7.
A at the 1^{st} and 3^{rd} cycles indicating presence of the N-terminus of the substrate and cleavage after V₁₇₇, respectively.
P at the 4^{th} and 8^{th} cycles indicating presence of the N-terminus of the substrate.
G at the 6^{th} and 10^{th} cycles indicating presence of the N-terminus of the substrate.
M at the 7^{th} cycle indicating presence of the N-teeminus of the substrate and/or cleavage after F₁₈₅.
M at the 15^{th} cycle indicating cleavage after V₁₇₇.
The 1^{st} cycle can indicate cleavage after D₁₉₁, see Table 7.
R a.t the 4^{th} and 13^{th} cycle indicating cleavage after V₁₇₇₁.
R at the 2^{nd} and 11^{th} cycle indicating cleavage after Y₁₇₉.
V at the 2^{nd}, 6^{th}, and 13^{th} cycle indicating cleavage after V₁₇₅, M₁₆₉ and presence of the N-terminus of the substrate, respectively. Note fragments beginning at 176 and 170 in Table 7.
Y at the 1^{st}, 2^{nd}, and 14^{th} cycles indicating cleavage after V₁₇₅, V₁₇₇, and presence of the N-terminus of the substrate, respectively.
L at the 11^{th} and 12^{th} cycles indicating cleavage after V₁₇₇, and presence of the N-terminus of the substrate, respectively, is the interpretation most consistent with the other data. Comparing to the mass spectrometry results we see that L at the 2^{nd}, 5^{th}, and 9^{th} cycles is consistent with cleavage after F₁₈₆, E₁₈₃ or M₁₆₉, and Y₁₇₉, respectively. See Table 7.

### Epitope Identification

Fragments co-C-terminal with 8-10 amino acid long sequences predicted to bind HLA by the SYFPEITHI or NIH algorithms were chosen for further analysis. The digestion and prediction steps of the procedure can be usefully practiced in any order. Although the substrate peptide used in proteasomal digest described here was specifically designed to include a predicted HLA-A1 binding sequence, the actual products of digestion can be checked after the fact for actual or predicted binding to other MHC molecules. Selected results are shown in Table 8.

**Table 8. Predicted HLA binding by proteasomally generated fragments**

| **SEQ ID NO** | **PEPTIDE** | **HLA** | **SYFPEITHI** | **NIH** |
|---|---|---|---|---|
| 32 & (33) | (G) MPEGDLVY V | A*0201 | 17(27) | (2605) |
| | | B*0702 | 20 | <5 |
| | | B*5101 | 22 | 314 |
| 34 & (35) | (Q) GMPEGDLV Y | A1 | 24(26) | <5 |
| | | A3 | 16 (18) | 36 |
| | | B*2705 | 17 | 25 |
| 36 | MPEGDLVY | B*5101 | 15 | NP† |
| 37 & (38) | (P)EGDLVYVN Y | A1 | 27(15) | 12 |
| | | A26 | 23 (17) | NP |
| 39 | LVYVNYARTE | A3 | 21 | <5 |
| 40 & (41) | (Y) VNYARTED F | A26 | (20) | NP |
| | | B*08 | 15 | <5 |
| | | B*2705 | 12 | 50 |
| 42 | NYARTEDFF | A24 | NP† | 100 |
| | | Cw*0401 | NP | 120 |
| 43 | YARTEDFF | B*08 | 16 | <5 |
| 44 | RTEDFFKLE | A1 | 21 | <5 |
| | | A26 | 15 | NP |

| | | | | |
|---|---|---|---|---|
| †No prediction | | | | |

### HLA-A*0201 binding assay:

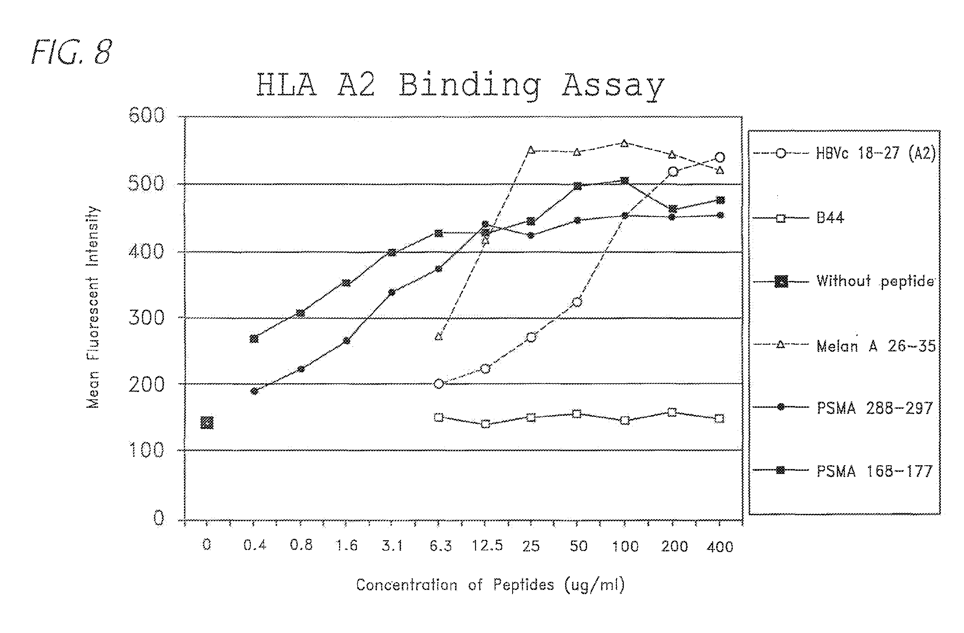

HLA-A*0201 binding studies were preformed with PSMA₁₆₈₋₁₇₇, GMPEGDLVYV, (SEQ ID NO, 33) essentially as described in Example 3 above. As seen in figure 8, this epitope exhibits significant binding at even lower concentrations than the positive control peptides. The Melan-A peptide used as a control in this assay (and throughout this disclosure). ELAGIGILTV, is actually a variant of the natural sequence (EAAGIGILTV) and exhibits a high affinity in this assay.

### Example 5

### Cluster Analysis (PSMA_{281 310}).

Another peptide, RGIAEAVGLPSIPVHPIGYYDAQKLLEKMG, PSMA₂₈₁₋₃₁₀, (SEQ ID NO. 45), containing an A1 epitope cluster from prostate specific membrane antigen, PSMA₂₈₃₋₃₀₇, (SEQ ID NO. 46), was synthesized using standard solid-phase F-moc chemistry on a 433A ABI Peptide synthesizer. After side chain deprotection and cleavage from the resin, peptide in ddH2O was run on a reverse-phase preparative HPLC C18 column at following conditions: linear AB gradient (5% B/min) at a flow rate of 4 ml/min, where eluent A is 0.1% aqueous TFA and eluent B is 0.1% TFA in acetonitrile. A fraction at time 17.061 min containing the expected peptide as judged by mass spectrometry, was pooled and lyophilised. The peptide was then subjected to proteasome digestion and mass spectrum analysis essentially as described above. Prominent peaks from the mass spectra are summarized in Table 9.

**Table 9. PSMA₂₈₁₋₃₁₀ Mass Peak Identification.**

| **PEPTIDE** | **SEQUENCE** | **CALCULATE D MASS (MH⁺)** |
|---|---|---|
| 281-297 | RGIAEVGLPSIPVHPI* | 1727.07 |
| 286-291 | AVGLPSIPVHPI** | 1200.46 |
| 287-297 | VGLPSIPVHPI | 1129.38 |
| 288-297 | GLPSIPVHPI | 1030.25 |
| 298-310 | GYYDAQKLLEKMG‡ | 1516,5 |
| 298-305 | GYYDAQKLS | 958.05 |
| 281-305 | RGIAEAVGLPSIPVHPIGYYDAQKL | 2666.12 |
| 281-307 | RGIAEAVGLPSIPVHPIGYYDAQKLLE | 2908.39 |
| 286-307 | AVGLPSIPVHPIGYYDAQKLLE¶ | 2381.78 |
| 287-307 | VGLPSIPVHPIGYYDAQKLLE | 2310.70 |
| 288-307 | GLFSIPVHPIGYYDAQKLLE# | 2211.57 |
| 281-299 | RGIAEAVGLPSIPVHPIGY | 1947 |
| 286-299 | AVGLPSIPVHPIGY | 1420.69 |
| 287-299 | VGLPSIPVHPIGY | 1349.61 |
| 288-299 | GLPSIPVHPIGY | 1250.48 |
| 287-310 | VGLPSIPVHPIGYYDAQLLEKMG | 2627.14 |
| 288-310 | GLPSIPVHPIGYYDAQKLLEKMG | 2528.01 |

| | | |
|---|---|---|
| **Boldface** sequences correspond to peptides predicted to bind to MHC, see Table 10. *By mass alone this peak could also have been 296-310 or 288-303. **By mass alone this peak could also have been 2.98-307. Combination of HPLC and mass spectrometry show that at some later time points this peak is a mixture of both species. † By mass alone this peak could also have been 289-298. By mass alone this peak could also have been 281-295 or 294-306. § By mass alone this peak could also have been 297-303. ¶ By mass alone this peak could also have been 285-306. # By mass alone this peak could also have been 288-303. None of these alternate assignments are supported N-terminal pool sequence analysis. | | |

### N-terminal Pool Sequence Analysis

One aliquot at one hour of the proteasomal digestion (see Example 3 part 3 above) was subjected to N-terminal amino acid sequence analysis by an ABI 473A Protein Sequencer (Applied Biosystems, Foster City, CA). Determination of the sites and efficiencies of cleavage was based on consideration of the sequence cycle, the repetitive yield of the protein sequencer, and the relative yields of amino acids unique in the analyzed sequence. That is if the unique (in the analysed sequence) residue X appears only in the nth cycle a cleavage site exists n-1 residues before it in the N-terminal direction. In addition to helping resolve any ambiguity in the assignment of mass to sequences, these data also provide a more reliable indication of the relative yield of the various fragments than does mass spectrometry.

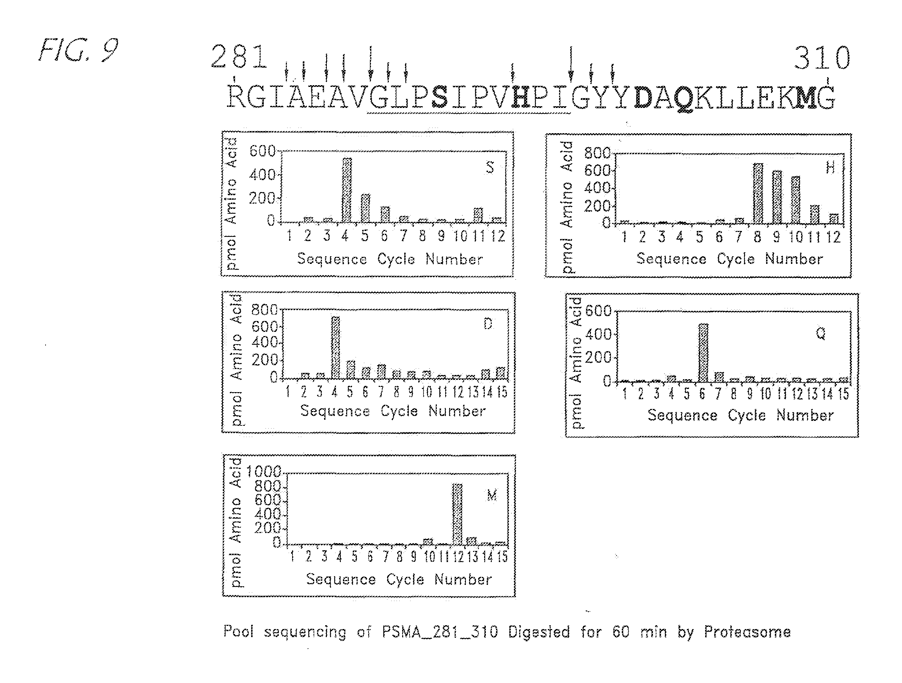

For PSMA₂₈₁₋₃₁₀ (SEQ ID NO. 45) this pool sequencing supports two major cleavage sites after V₂₈₇ and I₂₉₇ among other minor cleavage sites. Reviewing the results presented in Fig. 9 reveals the following:
S at the 4^{th} and 11^{th} cycles indicating cleavage after V₂₈₇ and presence of the N-terminus of the substrate, respectively.
H at the 8^{th} cycle indicating cleavage after V₂₈₇. The lack of decay in peak height at positions 9 and 10 versus the drop in height present going from 10 to 11 can suggest cleavage, after A₂₈₆ and E₂₈₅ as well, rather than the peaks representing latency in the sequencing reaction.
D at the 2^{nd}, 4^{th}, and 7^{th} cycles indicating cleavages alter Y₂₀₉, I297, and V₂₉₄, respectively. This last cleavage is not observed in any of the fragments in Table 10 or in the alternate assignments in the notes below.
Q at the 6^{th} cycle indicating cleavage after I₂₉₇.
M at the 10^{th} and 12^{th} cycle indicating cleavages after Y₂₉₉ and I₂₉₇, respectively.

### Epitope Identification

Fragments co-C-terminal with 8-10 amino acid long sequences predicted to bind HLA by the SYFPEITHI or NIH algorithms were chosen for further study. The digestion and prediction steps of the procedure can be usefully practiced in any order. Although the substrate peptide used in proteasomal digest described here was specifically designed to include a predicted HLA-A1 binding sequence, the actual products of digestion can be checked after the fact for actual or predicted binding to other MHC molecules. Selected results are shown in Table 10.

**Table 10. Predicted HLA binding by proteasomally generated fragment: PSMA 281-310**

| **SEQ ID NO.** | **PEPTIDE** | **IILA** | **SYFPEIHI** | **NIH** |
|---|---|---|---|---|
| 47& (48) | (G) LPSIPVH | A*0201 | 16 (24) | (24) |
| | PI | | | |
| | | B*0702/B7 | 23 | 12 |
| | | B*5101 | 24 | 572 |
| | | Cw*0401 | NP† | 20 |
| 49 & (50) | (P) IGYYDAQ KL | A*0201 | (16) | *<5* |
| | | A26 | (20) | NP |
| | | B*2705 | 16 | 25 |
| | | B*2709 | 15 | NP |
| | | B*5101 | 21 | 57 |
| | | Cw*0301 | NP | 24 |
| 51 & (52) | (P) SIPVHPI GY | A1 | 21 (27) | <5 |
| | | A26 | 22 | NP |
| | | A3 | 16 | <5 |
| 53 | IPVHPIGY | B*5101 | 16 | NP |
| 54 | YYDAQKLLE | A1 | 22 | <5 |

| | | | | |
|---|---|---|---|---|
| †No prediction | | | | |

As seen in Table 10, N-terminal addition of authentic sequence to epitopes can often generate still useful, even better epitopes, for the same or different MHC restriction elements0 Note for example the pairing of (G) LPSIPVHPI with HLA-A*0201, where the 10-mer can be used as a vaccine useful wirh several MHC types by relying on N-terminal trimming to create the epitopes for HLA-B7, -B*5101, and Cw*0401.

### HL-A*0201 binding assay:

HLA-A*0201 binding studies were prefonned with PS-MA₂₈₈₋₂₉₇, GLPSIPVIIPI, (SEQ ID NO. 48) essentially as described in Examples 3 and 4 above. As seen in figure 8, this epitope exhibits significant binding at even lower concentrations than the positive control peptides.

### Example 6

### Cluster Analysis (PSMA₄₅₄₋₄₈₁).

Another peptide, SSIEGNYTLRVDCTPLMYSLVHLTKEL, PSMA₄₅₄₋₄₈₁, (SEQ ID NO. 55) containing an epitope cluster from prostate specific membrane antigen, was synthesized by MPS (purity >95%) and subjected to proteasome digestion and mass spectrum analysis as described above. Prominent peaks from the mass spectra are summarized in Table 11.

**Table 11. PSMA₄₅₄₋₄₈₁ Mass Peak Identification.**

| MS PEAK (measured) | PEPTIDE | SEQUENCE | CALCULATED MASS (MH⁺) |
|---|---|---|---|
| 1238.5 | 454-464 | SSIEGNYTLRV | 1239.78 |
| 1768.38±0.60 | 454-469 | SSIEGNYTLRVDCTPL | 1768.99 |
| 1899.8 | 454-470 | SSIEGNYTLRVDCTPLM | 1900.19 |
| 1097.63±0.91 | 463-471 | RVDCTPLMY | 1098.32 |
| 2062.87±0.68 | 454-471* | SSIEGNYTLRVDCTPLMY | 2063.36 |
| 1153 | 472-481** | SLVHNLTKEL | 1154.36 |
| 1449.93±1.79 | 470-481 | MYSLVHNLTKEL | 1448.73 |

| | | | |
|---|---|---|---|
| **Boldface** sequence correspond to peptides predicted to bind to MHC, see Table 12. * On the basis of mass alone this peak could equally well be assigned to the peptide 455-472 however proteasomal removal of just the N-terminal amino acid is considered unlikely. If the issue were important it could be resolved by N-terminal sequencing. ** On the basis of mass this fragment might also represent 455-464, | | | |

### Epitope Identification

Fragments co-C-terminal with 8-10 amino acid long sequences predicted to bind HLA by the SYPEITHI or NIH algorithms were chosen for further study. The digestion and prediction steps of the procedure can be usefully practiced in any order. Although the substrate peptide used in proteasomal digest described here was specifically designed to include predicted HLA-A2,1 binding sequences, the actual products of digestion can be checked after the fact for actual or predicted binding to other MHC molecules. Selected results are shown in Table 12.

**Table 12. Predicted HLA binding by proteasomally generated fragments**

| **SEQ ID NO** | **PEPTIDE** | **HLA** | **SYFPEITHI** | **NIH** |
|---|---|---|---|---|
| 56 & (57) | (S) IEGNYTLRV | A1 | (19) | <5 |
| | | A*0201 | 16 (22) | <5 |
| 58 | EGNYTLRV | B*5101 | 15 | NP† |
| 59 & (60) | (Y)TLRVDCTPL | A*0201 | 20 (18) | (5) |
| | | A26 | 16 (18) | NP |
| | | B7 | 14 | 40 |
| | | B8 | 23 | <5 |
| | | B*2705 | 12 | 30 |
| | | Cw*0301 | NP | (30) |
| 61 | LRVDCTPLM | B*2705 | 20 | 600 |
| | | B*2709 | 20 | NP |
| 62 & (63) | (L) RVDCTPLMY | A1 | 32 (22) | 125 (13.5) |
| | | A3 | 25 | <5 |
| | | A26 | 22 | NP |
| | | B*2702 | NP | (200) |
| | | B*2705 | 13 (NP) | (1000) |

| | | | | |
|---|---|---|---|---|
| †No prediction | | | | |

As seen in Table 12, N-teminal addition of authentic sequence to epitopes can often generate still useful, even better epitopes, for the same or different MHC restriction elements. Note for example the pairing of (L)RVDCTPLMY (SEQ ID NOS 62 and (63)) with HLA-B*2702/5, where the 10-mer has substantial predicted halftimes of dissociation and the co-C-terminal 9-mer does not. Also note the case of SIEGNYTLRV (SEQ ID NO 57) a predicted HLA-A*0201 epitope which can be used as a vaccine useful with HLA-B*5101 by relying on N-terminal trimming to create the epitope.

### HLA-A*8201 binding assay

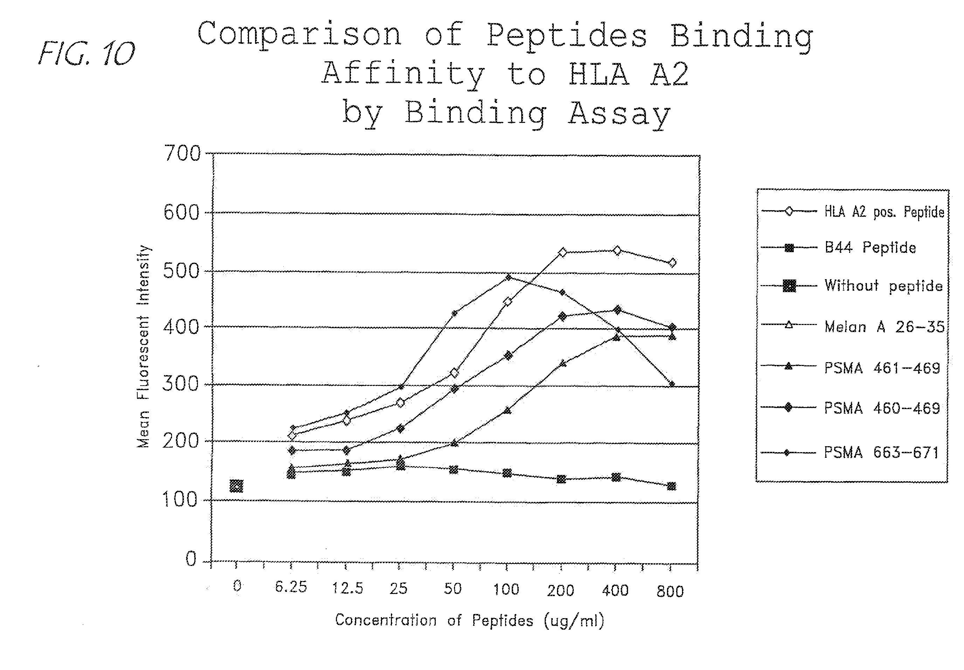

HLA-A*0201 binding studies were preformed, essentially as described in Example 3 above, with PSMA₄₅₀₋₄₆₉, TLRVDCTPL, (SEQ ID NO. 60), As seen in figure 10, this epitope was found to bind HLA-A2.1 to a similar extent as the known A2.1 binder FLPSDYFPSV (HBV₁₈₋₂₇; SEQ ID NO: 24) used as a positive control. Additionally, PSMA₄₆₁₋₄₆₉, (SEQ ID NO. 59) binds nearly as well.

### ELISPOT analysis: SEQ ID NO. 62)

The wells of a nitrocellulose-backed microtiter plate were coated with capture antibody by incubating overnight at 4°C using 50 µL/well of 4µg/murine anti-human γ-IFN monoclonal antibody in coating buffer (35 mM sodium bicarbonate, 15 mM sodium carbonate, pH 9.5). Unbound antibody was removed by washing 4 times 5 min. with PBS. Unbound sites on the membrane then were blocked by adding 200µl/well of RPMI medium with 10% serum and incubating 1 hr. at room temperature. Antigen stimulated CD8⁺ T cells, in 1:3 serial dilutions, were seeded into the wells of the microtiter plate using 100µl/well,starting at 2x10⁵ cells/well. (Prior antigen stimulation was essentially as described in Scheibenbogen, C. et al. Int. J. Cancer 71:932-936, 1997. PSMA₄₆₂₋₄₇₁ (SEQ ID) NO. 62) was added to a final concentration of 10µg/ml and IL-2 to 100 U/ml and the cells cultured at 37°C in a 5% CO₂, water-saturated atmosphere for 40 hrs. Following this incubation the plates were washed with 6 times 200 µl/well of PBS containing 0.05% Tween-20 (PBS-Tween). Detection antibody, 50µl/well of 2g/ml biotinylated murine anti-human γ-IFN monoclonal antibody in PBS+10% fetal calf serum, was added and the plate incubated at room temperature for 2 hrs. Unbound detection antibody was removed by washing with 4 times 200 µl of PBS-Tween. 100µl of avidin-conjugated horseradish peroxidase (Pharmingen, San Diego, CA) was added to each well and incubated at room temperature for 1 hr.
Unbound enzyme was removed by washing with 6 times 200 µl of PBS-Tween. Substrate was prepared by dissolving a 20 mg tablet of 3-amino 9-ethylcoarbasole in 2.5 ml of N, N-dimethylformamide and adding that solution to 47,5 ml of 0.05 M phosphate-citrate buffer (pH 5.0). 25 µl of 30% H₂O₂ was added to the substrate solution immediately before distributing substrate at100 µl/well and incubating the plate at room temperature. After color development (generally 15-30 min.), the reaction was stopped by washing the plate with water. The plate was air dried and the spots counted using a stereomicroscope.

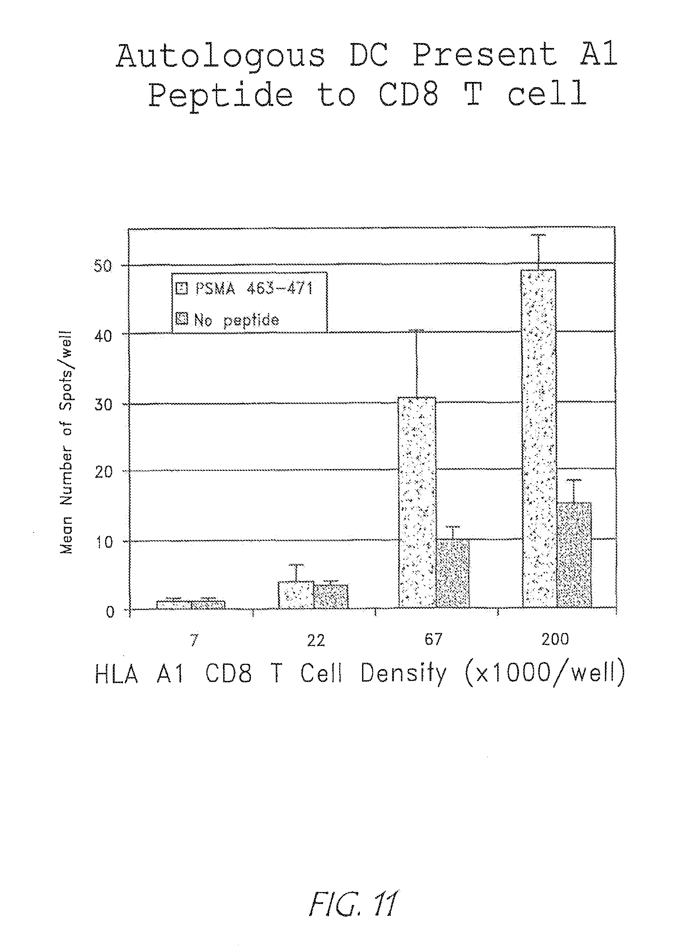

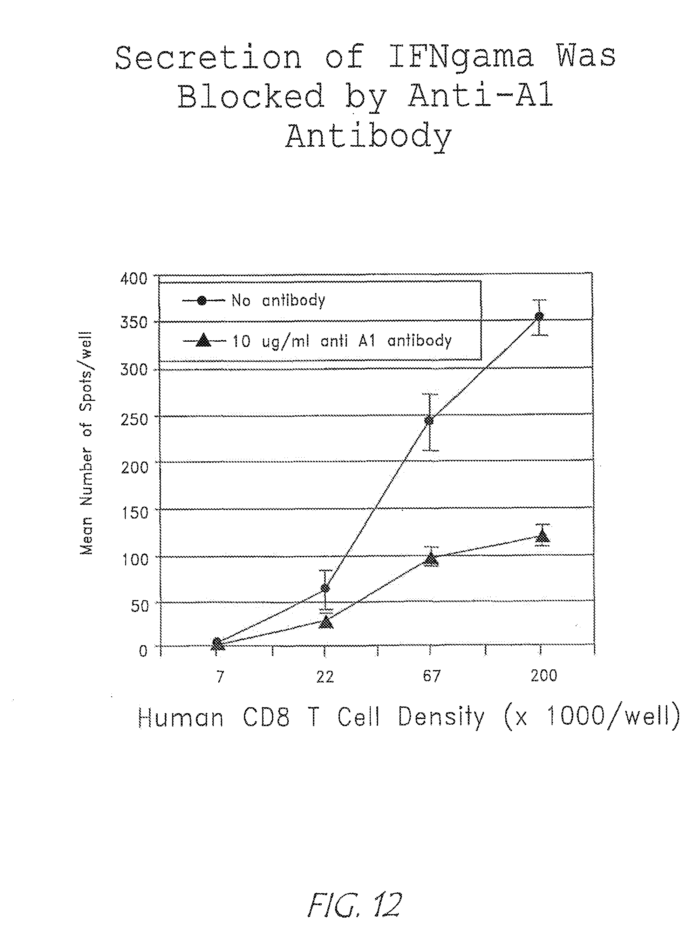

Figure 11 shows the detection of PSMA₄₆₃₋₄₇₁ (SEQ ID NO. 62)-reactive HLA-A1⁺ CD8⁺ T cells previously generated in cultures of HLA-A1⁺ CD8⁺ T cells with autologous dendritic cells plus the peptide. No reactivity is detected from cultures without peptide (data not shown). In this case it can be seen that the peptide reactive T cells are present in the culture at a frequency between 1 in 2.2x10⁴ and 1 in 6.7x10⁴. That this is truly an HLA-Al-restricted response is demonstrated by the ability of anti-HLA-Al monoclonal antibody to block γ-IFN production; see figure 12.

### Example 7

### Cluster Analysis (PSMA₆₅₃₋₆₈₇).

Another peptide, FDKSNPIVLICMMNDQLMFLERKFIDPLGLPDRPFY PSMA₆₅₃₋₆₈₇, (SEQ ID NO. 64) containing an A2 epitope cluster from prostate specific membrane antigen, PSMA₆₆₀₋₆₈₁ (SEQ ID NO 65), was synthesized by MPS (purity >95%) and subjected to proteasome digestion and mass spectrum analysis as described above. Prominent peaks from the mass spectra are summarized in Table 13.

**Table 13. PSMA,₆₅₃₋₆₈₇MASS Peak Identification.**

| MS (measured) | PEAK PEPTIDE | SEQUENCE | CALCULATED MASS (MH⁺) |
|---|---|---|---|
| 906.17±0.65 | 681-687** | LPDRPFY | 908.05 |
| 1287.73±0.76 | 677-687** | DPLGLPDRPFY | 1290.47 |
| 1400.3±1.79 | 676-687 | IDPLGLPDRPFY | 1403.63 |
| 1548.0±1.37 | 675-687 | FIDPLGLPDRPFY | 1550.80 |
| 1619.5±1.51 | 674.687** | AFIDPLGLPDRPFY | 1621.88 |
| 1775.48±1.32 | 673-687* | RAFIDPLGLPDRPFY | 1778.07 |
| 2440.2±1.3 | 653-672 | FDKSNPIVLRMMNDQLMFLE | 2442.93 |
| 1904.63±1.56 | 672-687* | ERAFIDPLGLPDRPFY | 1907.19 |
| 2310.6±2.5 | 653-671 | FDKSNPIVLR**MMNDQLMFL** | 2313.82 |
| 2017.4±1.94 | 671-687 | LERAFIDPLGLPDRPFY | 2020.35 |
| 2197.43±1.78 | 653-670 | FDKSNPIVL**RMMNDQLMF** | 2200.66 |

| | | | |
|---|---|---|---|
| **Boldface** sequence correspond to peptides predicted to bind to MHC, see Table 13. * On the basis of mass alone this peak could equally well be assigned to a peptide beginning at 654, however proteasomal removal of just the N-terminal amino acid is considered unlikely. If the issue were important it could be resolved by N-terminal sequencing. ** On the basis of mass alone these peaks could have been assigned to internal fragments, but given the overall pattern of digestion it was considered unlikely. | | | |

### Epitope Identification

Fragments co-C-terminal with 8-10 amino acid long sequences predicted to bind HLA by the SYFPEITHI or NIH algorithms were chosen for further study. The digestion, and prediction steps of the procedure can be usefully practiced in any order. Although the substrate peptide used in proteasomal digest described here was specifically designed to include predicted HLA-A2.1 binding sequences, the actual products of digestion can be checked after the fact for actual or predicted binding to other MHC molecules. Selected results are shown in Table 14.

**Table 14. Predicted HLA binding by proteasomally generated fragments**

| **SEQ ID NO** | | **PEPTIDE HLA** | **SYFPEITHI** | **NIH** |
|---|---|---|---|---|
| 66 & (67) | (R)MMNDQLMF L | A*0201 | 24 (23) | 1360(722) |
| | | A*0205 | NP† | 71(42) |
| | | A26 | 15 | NP |
| | | B*2705 | 12 | 50 |
| 68 | RMMNDQLMF | B*2705 | 17 | 75 |

| | | | | |
|---|---|---|---|---|
| †No prediction | | | | |

As seen in Table 14, N-terminal addition of authentic sequence to epitopes can generate still useful, even better epitopes, for the same or different MHC restriction elements. Note for example the pairing of (R)MMNDQLMFL (SEQ ID NOS. 66 and (67)) with HLA-A*02, where the 10-mar retains substantial predicted binding potential.

### HLA-A*0201 binding assay

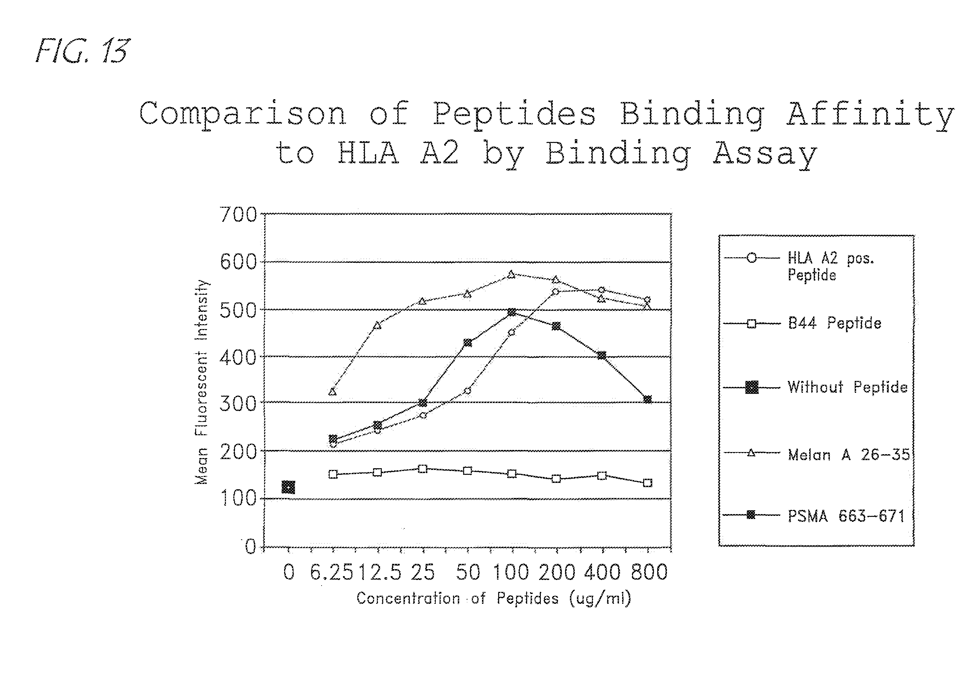

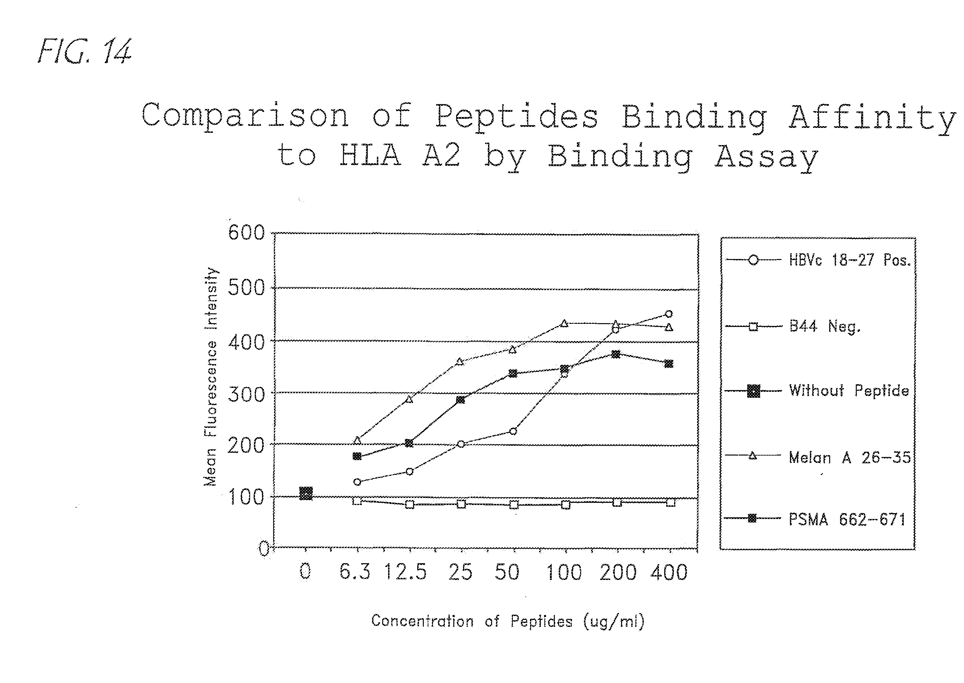

HLA-A*0201 binding studies were preformed, essentially as described in Example 3 above, with PSMA₆₆₃₋₆₇₁, (SEQ ID NO. 66) and PSMA₆₆₂₋₆₇₁, RMMNDQLMFL (SEQ NO. 67). As seen in figures 10, 13 and 14, this epitope exhibits significant binding at even lower concentrations than the positive control peptide (FLPSDYFPSV (HBV₁₈₋₂₇); SEQ ID NO: 24). Though not run in parallel, comparison to the controls suggests that PSMA₆₆₂₋₆₇₁ (which approaches the Melan A peptide in affinity) has the superior binding activity of these two PSMA peptides.

### Example 8

### Vaccinating with epitope vaccines.

### 1 . Vaccination with peptide vaccines:

### A. Intranodal delivery

A formulation containing peptide in aqueous buffer with an antimicrobial agent, an antioxidant, and an immunomodulating cytokine, was injected continuously over several days into the inguinal lymph node using a miniature pumping system developed for insulin delivery (MiniMed; Northridge, CA). This infusion cycle was selected in order to mimic the kinetics of antigen presentation during a natural infection.

### B. Controlled release

A peptide formulation is delivered using controlled PLGA microspheres as is known in the art, which alter the pharmacokinetics of the peptide and improve immunogenicity. This formulation is injected or taken orally.

### C. Gene gun delivery

A peptide formulation is prepared wherein the peptide is adhered to gold microparticles as is known in the art. The particles are delivered in a gene gun, being accelerated at high speed so as to penetrate the skin, carrying the particles into dermal tissues that contain pAPCs.

### D. Aerosol delivery

A peptide formulation is inhaled as an aerosol as is known in the art, for uptake into appropriate vascular or lymphatic tissue in the lungs.

### 2. Vaccination with acid vaccines:

A nucleic acid vaccine is injected into a lymph node using a miniature pumping system, such as the MiniMed insulin pump. A nucleic acid construct formulated in an aqueous buffered solution containing an antimicrobial agent, an antioxidant, and an immunomodulating cytokine, is delivered over a several day infusion cycle in order to mimic the kinetics of antigen presentation during a natural infection.

Optionally, the nucleic acid construct is delivered using controlled release substances, such as PLGA microspheres or other biodegradable substances. These substances are injected or taken orally. Nucleic acid vaccines are given using oral delivery, priming the immune response through uptake into GALT tissues. Alternatively, the nucleic acid vaccines are delivered using a gene gun, wherein the nucleic acid vaccine is adhered to minute gold particles. Nucleic acid constructs can also be inhaled as an aerosol, for uptake into appropriate vascular or lymphatic tissue in the lungs,

### Example 9

### Assays for the effectiveness of epitope vaccines.

### 1. Tetramer analysis:

Class I tetramer analysis is used to determine T cell frequency in an animal before and after administration of a housekeeping epitope. Clonal expansion of T cells in response to an epitope indicates that the epitope is presented to T cells by pAPCs. The specific T cell frequency is measured against the housekeeping epitope before and after administration of the epitope to an animal, to determine if the epitope is present on pAPCs. An increase in frequency of T cells specific to the epitope after administration indicates that the epitope was presented on pAPC.

### 2. Proliferation assay:

Approximately 24 hours after vaccination of an animal with housekeeping epitope, pAPCs are harvested from PBMCs, splenocytes, or lymph node cells, using monoclonal antibodies against specific markers present on pAPCs, fixed to magnetic beads for affinity purification. Crude blood or splenoctye preparation is enriched for pAPCs using this technique. The enriched pAPCs are then used in a proliferation assay against a T cell clone that has been generated and is specific for the housekeeping epitope of interest. The pAPCs are coincubated with the T cell clone and the T cells are monitored for proliferation activity by measuring the incorporation of radiolabeled thymidine by T cells. Proliferation indicates that T cells specific for the housekeeping epitope are being stimulated by that epitope on the pAPCs.

### 3. Chromium release assay:

A human patient, or non-human animal genetically engineered to express human class I MHC, is immunised using a housekeeping epitope. T cells from the immunized subject are used in a standard chromium release assay using human tumor targets or targets engineered to express the same class I MHC. T cell killing of the targets indicates that stimulation of T cells in a patient would be effective at killing a tumor expressing a similar TuAA.

### Example 10

### Induction of CTL response with naked DNA is efficient by Intra-lymph node immunization.

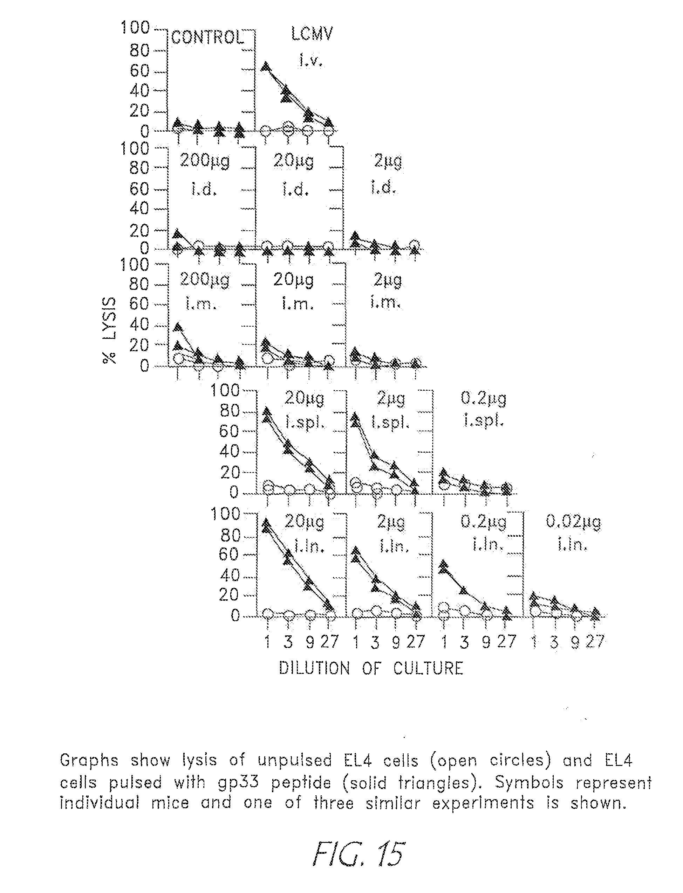

In order to quantitatively compare the CD8⁺ CTL responses induced by different routes of immunization a plasmid DNA vaccine (pEGFPL33A) containing a well-characterized immunodominant CTL epitope from the LCMV-glycoprotein (G) (gp33; amino acids 33-41) (Oehen, S., et al.. Immunology 99, 163-169 2000) was used, as this system allows a comprehensive assessment of antiviral CTL responses. Groups of 2 C57BL/6 mice were immunized once with titrated doses (200-0.02µg) of pEGFPL33A DNA or of control plasmid pEGFP-N3, administered i.m. (intramuscular), i.d. (intradermal), i.spl. (intrasplenic), or i.ln. (intra-lymph node). Positive control mice received 500 pfu LCMV i.v. (intravenous). Ten days after immunization spleen cells were isolated and gp33-specific CTL activity was determined after secondary *in vitro* restimulation. As shown in Fig. 15, i.m. or i.d. immunization induced weakly detectable CTL responses when high doses of pEFGPL33A DNA (200µg) were administered. In contrast, potent gp33-specific CTL responses were elicited by immunization with only 2µg pEFGPL33A DNA i.spl. and with as little as 0.2µg pEFGPL33A DNA given i.ln. (figure 15; symbols represent individual mice and one of three similar experiments is shown). Immunization with the control pEGFP-N3 DNA did not elicit any detectable gp33-specific CTL responses (data not shown).

### Example 11

### Intra-lymph node DNA immunization elicits anti-tumor immunity.

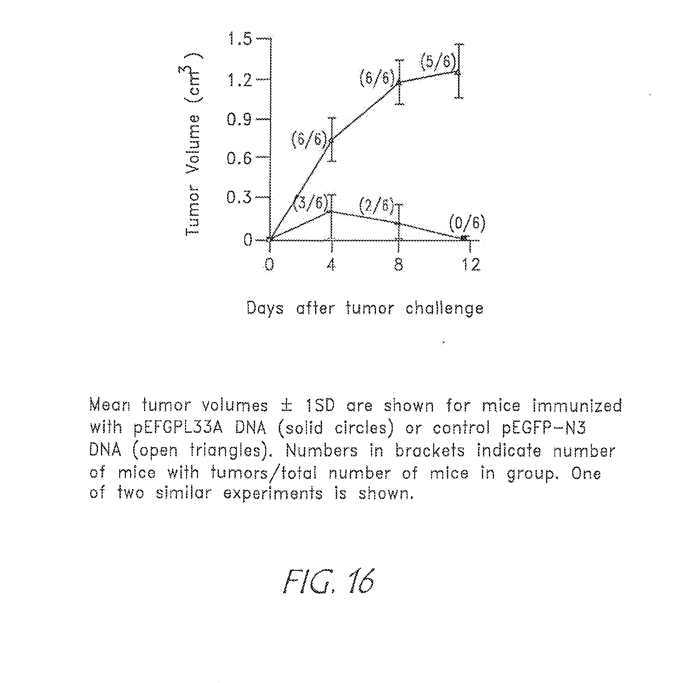

To examine whether the potent CTL responses elicited following i.ln. immunization were able to confer protection against peripheral tumors, groups of 6 C57BL/6mice were immunized three times at 6-day intervals with 10µg of pEFGPL33A DNA or control pEGFP-N3 DNA. Five days after the last immunization small pieces of solid tumors expressing the gp33 epitope (EL4-33) were transplanted s.c. into both flanks and tumor growth was measured every 3-4d. Although the EL4-33 tumors grew well in mice that had been repetitively immunized with control pEGFP-N3 DNA (figure 16), mice which were immunized with pEFGPL33A DNA i.ln. rapidly eradicated the peripheral EL4-33 tumors (figure 16).

### Example 12

### Differences in lymph node DNA content mirrors differences in CTL response following intra-lymph node and intramuscular injection.

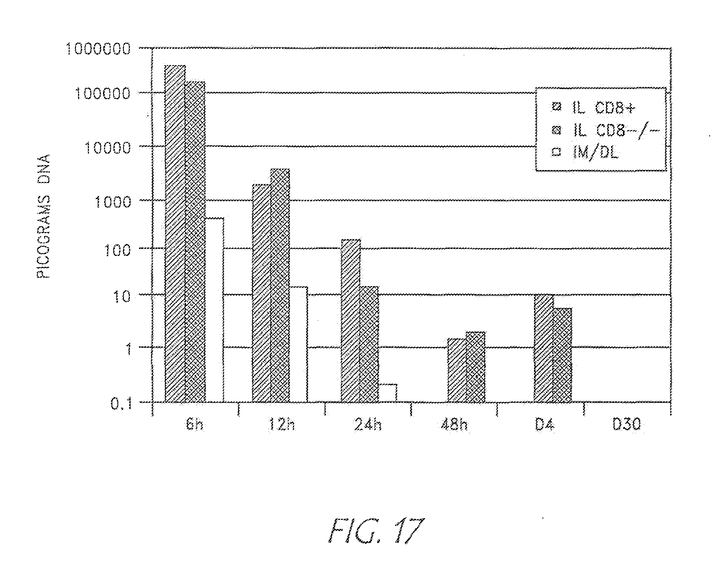

pEFGPL33A DNA was injected i.ln. or i.m. and plasmid content of the injected or draining lymph node was assessed by real time PCR after 6, 12, 24, 48 hours, and 4 and 30 days. At 6, 12, and 24 hours the plasmid DNA content of the injected lymph nodes was approximately three orders of magnitude greater than that of the draining lymph nodes following i.m. injection. No plasmid DNA was detectable in the draining lymph node at subsequent time points (Fig. 17). This is consonant with the three orders of magnitude greater dose needed using i.m. as compared to i.ln. injections to achieve a similar levels of CTL activity. CD8^{-/-} knockout mice, which do not develop a CTL response to this epitope, were also injected i.ln. showing clearance of DNA from the lymph node is not due to CD8⁺ CTL killing of cells in the lymph node. This observation also supports the conclusion that i.ln. administration will not provoke immunopathological damage to the lymph node.

### Example 13

### Administration of a DNA plasmid formulation of a therapeutic vaccine for melanoma to humans.

SYNCHROTOPE TA2M, a melanoma vaccine, encoding the HLA-A2-restricted tyrosinase epitope SEQ ID NO. 1 and epitope cluster SEQ ID NO. 69, was formulated in 1% Benzyl alcohol, 1% ethyl alcohol, 0.5mM EDTA, citrate-phosphate, pH 7.6. Aliquots of 80, 160, and 320 µg DNA/ml were prepared for loading into MINIMED 407C infusion pumps. The catheter of a SILHOUETTE infusion set was placed into an inguinal lymph node visualized by ultrasound imaging. The assembly of pump and infusion set was originally designed for the delivery of insulin to diabetics and the usual 17mm catheter was substituted with a 31mm catheter for this application. The infusion set was kept patent for 4 days (approximately 96 hours) with an infusion rate of about 25 µl/hour resulting in a total infused volume of approximately 2.4 ml. Thus the total administered dose per infusion was approximately 200, and 400 µg; and can be 800 µg, respectively, for the three concentrations described above. Following an subjects were given a 10 day rest period before starting a subsequent infusion. Given the continued residency of plasmid DNA in the lymph node after administration (as in example 12) and the usual kinetics of CTL response following disappearance of antigen, this schedule will be sufficient to maintain the immunologic CTL response.

### Example 14

### Additional Epitopes.

The methodologies described above, and in particular in examples 3-7, have been applied to additional synthetic peptide substrates, leading to the identification of further epitopes as set for the in tables 15-36 below. The substrates used here were designed to identify products of housekeeping proteasomal processing that give rise to HLA-A*0201 binding epitopes, but additional MHC-binding reactivities can be predicted, as discussed above. Many such reactivities are disclosed, however, these listings are meant to be exemplary, not exhaustive or limiting. As also discussed above, individual components of the analyses can be used in varying combinations and orders. The digests of the NY-ESO-1 substrates 136-163 and 150-177 (SEQ ID NOS. 254 and 255, respectively) yielded fragments that did not fly well in MALDI-TOF mass spectrometry. However, they were quite amenable to N-terminal peptide pool sequencing, thereby allowing identification of cleavage sites. Not all of the substrates necessarily meet the formal definition of an epitope cluster as referenced in example 3. Some clusters are so large, e.g. NY-ESO-1₈₆₋₁₇₁, that it was more convenient to use substrates spanning only a portion of this cluster. In other cases, substrates were extended beyond clusters meeting the formal definition to include neighboring predicted epitopes. In some instances, actual binding activity may have dictated what substrate was made, as with for example the MAGE epitopes reported here, where HLA binding activity was determined for a selection of peptides with predicted affinity, before synthetic substrates were designed.

**Table 15 GP100: Preferred Epitopes Revealed by Housekeeping Proteasome Digestion †Scores are given from the two binding prediction programs referenced above (see example 3).**

| | | | **SEQ ID NO** | **HLA Binding Predictions (SYFPEITHI)†** | | | | | |
|---|---|---|---|---|---|---|---|---|---|
| **Substrate** | **Epitope** | **Sequence** | | **A*0201** | **A1** | **A3** | **B7** | **B8** | **Comments** |
| 609-644 | 630-638* | LPHSSSHWL | 88 | | | | 20/80 | 16/<5 | *The digestion of 609-644 have generated the same epitopes. |
| | 629-638*. | QLPHSSSHWL | 89 | 21/117 | | | | | |
| | 614-622 | LIYRRRLMK | 90 | | | 32/20 | | | |
| | 613-622 | SLIYRRRLMK | 91 | 14/<5 | | 29/60 | | | |
| | 615-622 | IYRRRLMK | 92 | | | | | 15/<5 | |
| 622-650 | 630-638* | LPHSSSHWL | 93 | | | | 20/80 | 16/<5 | |
| | 629-638* | QLPHSSSHWL | 94 | 21/117 | | | | | |

**Table 16A MAGE-1: Preferred Epitopes Revealed by Housekeeping Proteasome Digestion**

| | | | **SEQ ID NO** | **HLA Binding Predictions (SYFPEITHI/NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| 86-109 | 95-102 | ESLFRAVI | 95 | | | | | 16/<5 | |
| | 93-102 | ILESLFRAVI | 96 | 21/<5 | | 20/<5 | | | |
| | 93-101 | ILESLFRAV | 97 | 23/<5 | | | | | |
| | 92-101 | CILESLFRAV | 98 | 23/55 | | | | | |
| | 92-100 | CILESLFRA | 99 | 20/138 | | | | | |
| 263-292 | 263-271 | EFLWGPRAL | 100 | | | | | | A26 (R21), A24 (NIH 30) |
| | 264-271 | FLWGPRAL | 101 | | | | | 17/<5 | |
| | 264-273 | PLWGPRALAE | 102 | 16/<5 | | 19/<5 | | | |
| | 265-274 | LWGPRALAET | 103 | 16/<5 | | | | | |
| | 268-276 | PRALAETSY | 104 | 15/<5 | | | | | |
| | 267-276 | GPRALAETSY | 105 | 15/<5 | | | <15/<5 | | B4403 (NIH 7); B3501 (NIH 120) |
| | 269-277 | RALAETSYV | 106 | 18/20 | | | | | |
| | 271-279 | LAETSYVKV | 107 | 19/<5 | | | | | |
| | 270-279 | ALAETSYVKV | 108 | 30/427 | | 19/<5<5 | | | |
| | 272-280 | AETSYVKVL | 109 | 15/<5 | | | | | (N-IH 36) B4403 (NIH 36) |
| | 271-280 | LAETSYVKLVL | 110 | 18/<5 | | | <15/<5 | | |
| | 274-282 | TSYVKVLEY | 111 | | 26/<5 | | | | B4403 (NIH 14) |
| | 273-282 | ETSYVKVLEY | 112 | | 28/6 | | | | A26 (R 31), B4403 (NIH 14) |
| | 278-286 | KVLEYVIKV | 113 | 26/743 | | 16/<5 | | | |

**Table 16B MAGE-1: Preferred Epitopes Revealed by Housekeeping Proteasome Digestion**

| | | | **SEQ ID NO** | **HLA Binding Predictions (SY FPEITHI/NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| 168-193 | 168-177 | SYVLVTCLGL | 114 | | | | | | A24 (NIH 300) |
| | 169-177 | YVLVTCLGL | 115 | 20/32 | | 15/<5 | <15/20 | | |
| | 170-177 | VLVTCLGL | 116 | | | | | 17/<5 | |
| 229-258 | 240-248 | TQDLVQEKY | 117 | | 29/<5 | | | | |
| | 239-248 | LTQDLVQEKY | 118 | | 23/<5 | | | | A26 (R22) |
| | 232-240 | YGEPRKLLT | 119 | | 24/11 | | | | |
| | 243-251 | LVQEKYLEY | 120 | | 21/<5 | 21/<5 | | | A26 (R 28) |
| | 242-251 | DLVQEKYLEY | 121 | | 22/<5 | 19/<5 | | | A26 (R 30) |
| | 230-238 | SAYGEPRKL | 122 | 21/<5 | | | | | B5101 (25/121) |
| 272-297 | 278-286 | KVLEYVIKV | 123 | 26/743 | | 16/<5 | | | |
| | 277-286 | VKVLEYVIKV | 124 | 17/<5 | | | | | |
| | 276-284 | YVKVLEYVI | 125 | 15/<5 | | 15/<5 | | 17/<5 | |
| | 274-282 | TSYVKVLEY | 126 | | 26/<5 | | | | |
| | 273-282 | ETSYVKVLEY | 127 | | 28/6 | | | | |
| | 283-291 | VIKVSARVR | 128 | | | 20/<5 | | | |
| | 282-291 | YVIKVSARVR | 129 | | | 24/<5 | | | |

| | | | | | | | | | |
|---|---|---|---|---|---|---|---|---|---|
| †Scores are given from the two binding prediction programs referenced above (ses example 3). R indicates a SYFPETTHI score. | | | | | | | | | |

**Table 17A MAGE-2: Preferred Epitopes Revealed by Housekeeping Proteasome Digestion**

| | | | **SEQ ID NO** | **HLA Binding Predictions (SYFPEITHI/NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| 107-126 | 115-122 | ELVKFLLL | 130 | | | | | 18/<5 | |
| | 113-122 | MVELVHFLLL | 131 | | 21/<5 | | | | A26 (R 22) |
| | | ISRKMVEL | 132 | | | | | 17/<5 | |
| | 108-116 | AISRKMVEL | 133 | 25/7 | | 19/<5 | 16/12 | 26/<5 | |
| | 107-116 | AAISRKMVEL | 134 | 22/<5 | | | 14/36 | n.p./16 | |
| | 112-120 | KMVELVHFL | 135 | 27/2800 | | | | | |
| | 109-117 | ISRKMVELV | 136 | 16/<5 | | | | | |
| | 108-117 | AISRKMVELV | 137 | 24/11 | | | | | |
| | 116-124 | LVHFLLLKY | 138 | 23/<5 | 23/<5 | 19/<5 | | | A26 (R 26) |
| | 115-124 | ELVRFLLLKY | 139 | | 24/<5 | 19/5 | | | A26 (R 29) |
| | 111-119 | RKMVELVHF | 140 | | | | | | |
| 145-175 | 158-166 | LQLVFGIEV | 141 | 17/168 | | | | | |
| | 157-166 | YLQLVFGIEV | 142 | 24/1215 | | | | | |
| | 159-167 | QLVFGIEVV | 143 | 25/32 | | 18/<5 | | | |
| | 158-167 | LQLVFGIEVV | 144 | 18/20 | | | | | |
| | 164-172 | IEVVEVVPI | 145 | 16/<5 | | | | | |
| | 163-172 | GIEVVEVVPI | 146 | 22/<5 | | | | | |
| | 162-170 | FGIEVVEVV | 147 | 19/<5 | | | | | B5101(24/69.212) |
| | 154-162 | ASEYLQLVF | 148 | | 22/68 | | | | |
| | 153-162 | KASEYLQLVF | 149 | | | 15/<5 | | | |

| | | | | | | | | | |
|---|---|---|---|---|---|---|---|---|---|
| †Scores are given from the two binding prediction programs referenced above (see example 3). R indicates a SYFPEITHI score. | | | | | | | | | |

**Table 17B MAGE-2: Preferred Epitopes Revealed by Housekeeping Proteasome Digestion**

| | | | | **HLA Binding Predictions (SYFPEITHI/NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| 213-233 | 218-225 | EEKTWEEL | 150 | | | | | 22/<5 | |
| | 216-225 | APEEKIWEEL | 151 | 15/<5 | | | 22/72 | | |
| | 216-223 | APEEKIWE | 152 | | | | | 18/<5 | |
| | 220-228 | KIWEELSML | 153 | 26/804 | | 16/<5 | | 16/<5 | A26 (R 26) |
| | 219-228 | EKIWEELSML | 154 | | | | | | A26 (R 22) |
| 271-291 | 271-278 | FLWGPRAL | 155 | | | | | 17/<5 | |
| | 271-279 | FLWGPRALI | 156 | 25/398 | | 16/7 | | | |
| | 278-286 | LIETSYVKV | 157 | 23/<5 | | | | | |
| | 277-286 | ALIETSYVKV | 158 | 30/427 | | 21/<5 | | | B5101 (20/55) |
| | 276-284 | RALIFTSYV | 159 | 18/19 | | | | | |
| | 279-287 | IETSYVKVL | 160 | 15/<5 | | | | | A26 (R 22) |
| | 278-287 | LIETSYVKVL | 161 | 22/<5 | | | | | |

| | | | | | | | | | |
|---|---|---|---|---|---|---|---|---|---|
| †Scores are given from the two binding prediction programs referenced above (see example 3). R indicates a SYFPEITHI score. | | | | | | | | | |

**Table 18 MAGE-3: Preferred Epitopes Revealed by Housekeeping Proteasome Digestion**

| | | | **SEQ ID NO** | **HLA Binding Predictions (SYFPEITHI/NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| 267-286 | | 271-278 FLWGPRAL | 162 | | | | | 17/<5 | |
| | 270-278 | EFLWGPRAL | 163 | | | | | | A26 (R 21); A24 (NIH 30) |
| | 271-279 | FLWGPRALV | 164 | 27/2655 | | 16/<5 | | | |
| | 276-284 | RALVETSYV | 165 | 18/19 | | | | | B5101 (20/55) |
| | 272-280 | LWGPRALVE | 166 | | | 15/<5 | | | |
| | 271-280 | FLWGPRALVE | 167 | 15/<5 | | 22/<5 | | | |
| | 272-281 | LWGPRALVET | 168 | 16/<5 | | | | | |

| | | | | | | | | | |
|---|---|---|---|---|---|---|---|---|---|
| †Scores are given from the two binding prediction programs referenced above (see example 3). R. indicates a SYPPETTHI score. | | | | | | | | | |

**Table 19A NY-ESO-1: Preferred Epitopes Revealed by Housekeeping Proteasome Digestion**

| | | | **SEQ ID NO** | **HLA Binding Predictions (SYFPETTHI/NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| 81-113 | 82-90 | GPESRLLEF | 169 | | 16/11 | | 18/<5 | 22/<5 | |
| | 83-91 | PESRLLEFY | 170 | | 15/<5 | | | | B4403 (NIH 18) |
| | 82-91 | GPESRLLEFY | 171 | | 25/11 | | | | |
| | 84-92 | ESRLLEFYL | 172 | | | | | 19/8 | |
| | 86-94 | RLLEFYLAM | 173 | 21/430 | | 21/<5 | | | |
| | 88-96 | LEFYLAMPF | 174 | | | | | | B4403 (NIH 60) |
| | 87-96 | LLEFYLAMPF | 175 | | <15/45 | 18/<5 | | | |
| | 93-102 | AMPFATPMEA | 176 | 15/<5 | | | | | |
| | 94-102 | MPFATPMEA | 177 | | | | 17/<5 | | |
| 101-133 | 115-123 | PLPVPGVLL | 178 | 20/<5 | | 17/<5 | 16/<5 | 18/<5 | |
| | 114-123 | PPLPVPGVLL | 179 | | | | 23/12 | | |
| | 116-123* | LPVPGVLL | 180 | | | | | 16/<5 | **Comment *** Evidence of the same epitope obtained from two digests. |
| | 103-112 | ELARRSLAQD | 181 | 15/<5 | | 20/<5 | | | |
| | 118-126* | VPGVLLKEF | 182 | | | | 17/<5 | 16/<5 | |
| | 117-126* | PVPGVLLKEF | 183 | | | 16/<5 | | | |
| 116-145 | 116-123* | LPVPGVLL | 184 | | | | | 16/<5 | |
| | 127-135 | TVSGNILTI | 185 | 21/<5 | | 19/<5 | | | |
| | 126-135 | FTVSGNILTI | 186 | 20/<5 | | | | | |
| | 120-128 | GVLLKEFTV | 187 | 20/130 | | 18/<5 | | | |
| | 121-130 | VLLKEFTVSG | 188 | 17/<5 | | 18/<5 | | | |
| | 122-130 | LLKEFTVSG | 189 | 20/<5 | | 18/<5 | | | |
| | 118-126* | VPGVLLKEF | 190 | | | | 17/<5 | 16/<5 | |
| | 117-126* | PVPGVLLKEF | 191 | | | 16/<5 | | | |

| | | | | | | | | | |
|---|---|---|---|---|---|---|---|---|---|
| †Scores are given from the two binding prediction programs referenced above (see example 3). | | | | | | | | | |

**Table 19B NY-ESO-1: Preferred Epitopes Revealed by Housekeeping Proteasome Digestion**

| | | | **SEQ ID NO** | **HLA Binding Predictions (SYFPEITHI/NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| 136-163 | 139-147 | AADHRQLQL | 192 | | 17/<5 | | | 22/<5 | |
| | 148-156 | SISSCLQQL | 193 | 24/7 | | | | | A26 (R 25) |
| | 147-156 | LSISSCLQQL | 194 | 8/<5 | | | | | |
| | 138-147 | TAADHRQLQL | 195 | 18/<5 | | | | | |
| 156-177 | 161-169 | WITQCFLPV | 196 | 18/84 | | | | | |
| | 157-165 | SLLMWTTQC | 197 | 18/42 | | 17/<5 | | | |
| | 150-158 | SSCLQQLSL | 198 | 15/<5 | | | | | |
| | 154-162 | QQLSLLMWI | 199 | 15/50 | | | | | |
| | 151-159 | SCLQQLSLL | 200 | 18/<5 | | | | | |
| | 150-159 | SSCLQQLSLL | 201 | 16/<5 | | | | | |
| | 163-171 | TQCFLPVFL | 202 | <15/12 | | | | | |
| | 162-171 | IIQCFLPVFL | 203 | 18/<5 | | | | | A26 (R 19) |

| | | | | | | | | | |
|---|---|---|---|---|---|---|---|---|---|
| †Scores are given from the two binding prediction programs referenced above (see example 3). R indicates a SYFPEITHI score | | | | | | | | | |

**Table 20 PRAME: Preferred Epitopes Revealed by Housekeeping Proteasome Digestion**

| | | | **SEQ ID NO** | **HLA binding predictions (SYFPEITHI/NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| 211-245 | 219-227 | PMQDIKMIL | 204 | 16/<5 | | | | 16/n.d. | A26 (R 20) |
| | 218-227 | MPMQDIKMIL | 205 | | | | <15/240 | | |
| 411-446 | 428-436 | QHLIGLSNL | 206 | 18/<5 | | | | | |
| | 427-436 | LQHLIGLSNL | 207 | 16/8 | | | | | |
| | 429-436 | HLIGLSNL | 208 | | | | | 17/<5 | B15 (R 21) |
| | 431-439 | IGLSNLTHV | 209 | 18/7 | | | | | B*5101 (R 22) |
| | 430-439 | LIGLSNLTHV | 210 | 24/37 | 24/37 | | | | |

| | | | | | | | | | |
|---|---|---|---|---|---|---|---|---|---|
| †Scores are given from the two binding prediction programs referenced above (see example 3). R indicates a SYFPEITHI score. | | | | | | | | | |

**Table 21 PSA: Preferred Epitopes Revealed by Housekeeping Proteasome Digestion**

| | | | **SEQ ID NO** | **HLA Binding Predictions (SYFPEITHI /NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| **42-77** | 53-61 | VLVHPQWVL | 211 | 22/112 | | | <15/6 | 17/<5 | |
| | 52-61 | GVLVHPQWVL | 212 | 17/21 | | 16/<5 | <15/30 | | A26 (R 18) |
| | 52-60 | GVLVHPQWV | 213 | 17/124 | | | | | |
| | 59-67 | WVLTAAHCI | 214 | 15/16 | | | | | |
| | 54-63 | LVHPQWVLTA | 215 | 19/<5 | | 20/<5 | | | A26 (R 16) |
| | 53-62 | VLVHPQWVLT | 216 | 17/22 | | | | | |
| | 54-62 | LVHPQWVLT | 217 | | | 17/n.d. | | | |
| 55-95 | 66-73 | CIRNKSVI | 218 | | | | | 26/20 | |
| | 65-73 | HCIRNKSVI | 219 | | | | | <15/16 | |
| | 56-64 | HPQWVLTAA | 220 | | | | 18/<5 | | |
| | 63-72 | AAHCIRNKSV | 221 | 17/<5 | | | | | |

| | | | | | | | | | |
|---|---|---|---|---|---|---|---|---|---|
| †Scores are given from the two binding prediction programs referenced above (see example 3). R indicates a SYFPEITHI score. | | | | | | | | | |

**Table 22 PSCA: Preferred Epitopes Revealed by Housekeeping Proteasome Digestion**

| | | | **SEQ ID NO** | **HLA Binding Predictions (SYFPEITHI /NIH)†** | | | | | |
|---|---|---|---|---|---|---|---|---|---|
| **Substrate** | **Epitope** | **Sequence** | | **A*201** | **A1** | **A3** | **B7** | **B8** | **Other** |
| **93-123*** | 116-123 | LLWGPGQL | 222 | | | | 16/<5 | | |
| | 115-123 | LLLWGPGQL | 223 | <15/18 | | | | | |
| | 114-123 | GLLLWGPGQL | 224 | <15/10 | | | | | |
| | 99-107 | ALQPAAAIL | 225 | 26/9 | | 22/<5 | <15/12 | 16/<5 | A26 (R 19) |
| | 98-107 | HALQPAAAIL | 226 | 18/<5 | | | <15/12 | | |

| | | | | | | | | | |
|---|---|---|---|---|---|---|---|---|---|
| *L123 is the C-terminus of the natural protein. †Scores are given from the two binding prediction programs referenced above (see example 3). | | | | | | | | | |

**Table 23 Tyrosinase: Preferred Epitopes Revealed by Housekeeping Proteasome Digestion**

| | | | **SEQ ID NO** | **HLA Binding Predictions (SYFPEITHI /NIH)†** | | | | | |
|---|---|---|---|---|---|---|---|---|---|
| **Substrate** | **Epitope** | **Sequence** | | **A*0201** | **A1** | **A3** | **B7** | **B8** | **Other** |
| **128-157** | 128-137 | APEKDKFFAY | 227 | | 29/6 | | 15/<5 | | B4403 (NIH 14) |
| | 129-137 | PEKDKFFAY | 228 | | 18/<5 | | | 21/<5 | |
| | 130-138 | EKDKFFAYL | 229 | | | | 15/<5 | | |
| | 131-138 | KDKFFAYL | 230 | | | | | 20/<5 | |
| **197-228** | 205-213 | PAFLPWHRL | 231 | | | | | 15/<5 | |
| | 204-213 | APAFLPWHRL | 232 | | | | 23/360 | | |
| | 207-216 | FLPWHRLFLL | 1 | 25/1310 | | | | <15/8 | |
| | 208-216 | LPWHRLFLL | 9 | 17/26 | | | 20/80 | 24/16 | |
| | 214-223 | FLLRWEQEIQ | 233 | | | 15/<5 | | | |
| | 212-220 | RLFLLRWEQ | 234 | | | 16/<5 | | | |
| **191-211** | 191-200 | GSEIWRDIDF | 235 | | 18/68 | | | | |
| | 192-200 | SEIWRDIDF | 236 | | | | | 16/<5 | B4403 (NH 400) |
| **207-230** | 207-215 | FLWHRLFL | 8 | 22/540 | | | <15/6 | 17/<5 | |
| **466-484** | 473-481 | RIWSWLLCA | 237 | 19/13 | | 15/<5 | | | |
| **476-497** | 476-484 | SWLLGAAMV | 238 | 18/<5 | | | | | |
| | 477-486 | WLLGAAMVGA | 239 | 21/194 | | 18/<5 | | | |
| | 478-486 | LLGAAMVGA | 240 | 19/19 | | 16/<5 | | | |

| | | | | | | | | | |
|---|---|---|---|---|---|---|---|---|---|
| †Scores are given from the two binding prediction programs referenced above (see example 3). | | | | | | | | | |

**Table 24 PSMA: Preferred Epitopes Revealed by Housekeeping Proteasome Digestion**

| | | | **SEQ ID NO** | **HLA Binding Predictions (SYFPEITHI /NIH)†** | | | | | |
|---|---|---|---|---|---|---|---|---|---|
| **Substrate** | **Epitope** | **Sequence** | | **A*0201** | **A1** | **A3** | **B7** | **B7** | **Other** |
| **1-30** | 4-12 | LLHETDSAV | 241 | 25/485 | | 15/<5 | | | A26 (R 19) |
| | 13-21 | ATARRPRWL | 242 | 18/<5 | | | | 18/<5 | A26 (R 19) |
| **53-80** | 53-61 | TPKHNMKAF | 243 | | | | | 24/<5 | |
| | 64-73 | ELKAENIKKF | 244 | | | 17/<5 | | | A26 (R 30) |
| | 69-77 | NIKKFLHNF | 245 | | | | | | A26 (R 27) |
| | 68-77 | ENIKKFLH¹NF | 246 | | | | | | A26 (R 24) |
| **215**-**244** | 220-228 | AGAKGVILY | 247 | | 25/<5 | | | | |
| **457-489** | 468-477 | PLMYSLVHNL | 248 | 22/<5 | | | | | |
| | 469-477 | LMYSLVHNL | 249 | 27/193 | | <15/9 | | | |
| | 463-471 | RVDCTPLMY | 250 | | 32/125 | 25/<5 | | | A26(R 22) |
| | 465-473 | DCTPLMYSL | 251 | | | | | | A26 (R 22) |
| **503-533** | 507-515 | SGMPRISKL | 252 | 21/<5 | | | | 21<5 | |
| | 506-515 | FSGMPRISKL | 253 | 17/<5 | | | | | |

| | | | | | | | | | |
|---|---|---|---|---|---|---|---|---|---|
| ¹This H was reported as Y in the SWISSPROT database. †Scores are given from the two binding prediction programs referenced above (see example 3). | | | | | | | | | |

**Table 25A MAGE-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPETTHI** | **NIH** |
| | 125-132 | KAEMLESV | 256 | B5101 | 19 | n.a. |
| | 124-132 | TKAEMLESV | 257 | A0201 | 20 | <5 |
| | 123-132 | VTKAEMLESV | 258 | A0201 | 20 | <5 |
| | 128-136 | MLESVLKNY | 259 | A1 | 28 | 45 |
| | | | | A26 | 24 | n.a. |
| Mage-1 119-146 | | | | A3 | 17 | 5 |
| | 127-136 | EMLESVIKNY | 260 | A1 | 15 | <1.0 |
| | | | | A26 | 23 | <1.0 |
| | 125-133 | KAEMLESVI | 261 | B5101 | 23 | 100 |
| | | | | A24 | N.A. | 4 |
| | 146-153 | KASESLQL | 262 | B08 | 16 | <1.0 |
| | | | | B5101 | 17 | N.A. |
| | 145-153 | GKASESLQL | 263 | B2705 | 17 | 1 |
| Mage-1 143-170 | | | | B2709 | 16 | N.A. |
| | 147-155 | ASESLQLVF | 264 | A1 | 22 | 68 |
| | 153-161 | LVFGIDVKE | 265 | A26 | 16 | N.A. |
| | | LVFGIDVKE | | A3 | 16 | <1.0 |

**Table 25B MAGE-1: Preferred Epitopes Reveled by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 114-121 | LLKYRARE | 266 | B8 | 25 | <1.0 |
| | 106-113 | VADLVGFL | 267 | B8 | 16 | <1.0 |
| | | | | B5101 | 21 | N.A. |
| | 105-113 | KVADLVGFL | 268 | A0201 | 23 | 44 |
| | | | | A26 | 25 | N.A. |
| | | | | A3 | 16 | <5 |
| | | | | B0702 | 14 | 20 |
| Mage-1 99-125 | | | | B2705 | 14 | 30 |
| | 107-115 | ADLVGFLLL | 269 | A0201 | 17 | <5 |
| | | | | B0702 | 15 | <5 |
| | | | | B2705 | 16 | 1 |
| | 106-115 | VADLVGFLLL | 270 | A0201 | 16 | <5 |
| | | | | A0201 | 22 | 3 |
| | 114-123 | LLKYRAREPV | 271 | A0201 | 20 | 2 |

**Table 26 MAGE-3: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type HLA type** | **SYFPEITHI** | **NIH** |
| | 271-278 | FLWGPRAL | 162 | B08 | 17 | <5 |
| | 270-278 | EFLWGPRAL | 163 | A26 | 21 | N.A. |
| | | | | A24 | N.A. | 30 |
| | | | | B1510 | 16 | N.A. |
| | 271-279 | FLWGPRALV | 164 | A0201 | 27 | 2655 |
| | | | | A3 | 16 | 2 |
| | 278-286 | LVETSYVKV | 272 | A0201 | 19 | <1.0 |
| | | | | A26 | 17 | N.A. |
| | 277-286 | ALVETSYVKV | 273 | A0201 | 28 | 428 |
| Mage-3 267-295 | | | | A26 | 16 | <5 |
| | | | | A3 | 18 | <5 |
| | 285-293 | KVLHHMVKI | 274 | A0201 | 19 | 27 |
| | | | | A3 | 19 | <5 |
| | 276-284 | RALVETSYV | 165 | A0201 | 18 | 20 |
| | 283-291 | YVKVLHHMV | 275 | A0201 | 17 | <1.0 |
| | 275-283 | PRALVETSY | 276 | A1 | 17 | <1.0 |
| | 274-283 | GPRALVETSY | 277 | A1 | 15 | <1.0 |
| | 278-287 | LVETSYVKVL | 278 | A0201 | 18 | <1.0 |
| | 272-281 | LWGPRALVET | 168 | A0201 | 16 | <1.0 |
| | 271-280 | FLWGPRALVE | 167 | A3 | 22 | <5 |

**Table 27A Fibronectin ED-B: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 4'-5** | *TIIP*EVPQL^{†} | 279 | A0201 | 27 | 7 |
| | | | | A26 | 28 | N.A. |
| | | | | A3 | 17 | <5 |
| | | | | B8 | 15 | <5 |
| | | | | B1510 | 15 | N.A. |
| ED-B 14'-21* | | | | B2705 | 17 | 10 |
| | | | | B2709 | 15 | N.A. |
| | 5'-5** | DTIIPEVPQL† | 280 | A0201 | 20 | <5 |
| | | | | A26 | 32 | N.A. |
| | 1-10 | EVPQLTDLSF | 281 | A26 | 29 | N.A. |

| | | | | | | |
|---|---|---|---|---|---|---|
| *This substrate contains the 14 amino acids from fibronectin flanking ED-B to the N-terminal side. **These peptides span the junction between the N-terminus of the ED-B domain and the rest of fibronectin. ^{†}The *italicized* lettering indicates sequence outside the ED-B domain. | | | | | | |

**Table 27B Fibronectin ED-B: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFFEITHI** | **NIH** |
| | 23-30 | TPLNSSTI | 282 | B5101 | 22 | N.A. |
| | 18-25 | IGLRWTPL | 283 | B5101 | 18 | N.A. |
| | 17-25 | SIGLRWTPL | 284 | A0201 | 20 | 5 |
| | | | | A26 | 18 | N.A. |
| | | | | B08 | 25 | <5 |
| | 25-33 | LNSSTIIGY | 285 | A1 | 19 | <5 |
| ED-B 8-35 | | | | A26 | 16 | <5 |
| | 24-33 | PLNSSTIIGY | 286 | A1 | 20 | <5 |
| | | | | A26 | 24 | N.A. |
| | | | | A3 | 16 | <5 |
| | 23-31 | TPLNSSTII | 287 | B0702 | 17 | 8 |
| | | | | B5101 | 25 | 440 |

**Table 27C Fibronectin ED-B: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEIHI** | **NIH** |
| | 31-38 | IGYRITVV | 288 | B5101 | 25 | N.A. |
| | 30-38 | IIGYRITVV | 289 | A0201 | 23 | 15 |
| | | | | A3 | 17 | <1.0 |
| | | | | B08 | 15 | <1.0 |
| | | | | B5101 | 15 | 3 |
| | 29-38 | TIIGYRITVV | 290 | A0201 | 26 | 9 |
| | | | | A26 | 18 | N.A. |
| | | | | A3 | 18 | <5 |
| | 23-30 | TPLNSSTI | 282 | B5101 | 22 | N.A. |
| ED-B 20-49 | 25-33 | LNSSTIIGY | 285 | A1 | 19 | <5 |
| | | | | A26 | 16 | N.A. |
| | 24-33 | PLNSSTIIGY | 286 | A26 | 24 | N.A. |
| | | | | A3 | 16 | <5 |
| | 31-39 | IGYRITVVA | 291 | A3 | 17 | <5 |
| | 30-39 | IIGYRITVVA | 292 | A0201 | 15 | <5 |
| | | | | A3 | 18 | <5 |
| | 23-31 | TPLNSSTII | 287 | B0702 | 17 | 8 |
| | | | | B5101 | 25 | 440 |

**Table 28A CEA: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 184-191 | SLPVSPRL | 293 | B08 | 19 | <5 |
| | 183-191 | QSLPVSPRL | 294 | A0201 | 15 | <5 |
| | | | | B1510 | 15 | |
| | | | | B2705 | 18 | 10 |
| | | | | B2709 | 15 | |
| | 186-193 | PVSPRLQL | 295 | B08 | 18 | <5 |
| | 185-193 | LPVSPRLQL | 296 | B0702 | 26 | 180 |
| | | | | B08 | 16 | <5 |
| | | | | B5101 | 19 | 130 |
| | 184-193 | SLPVSPRLQL | 297 | A0201 | 23 | 21 |
| CEA 176-202 | | | | A26 | 18 | N.A. |
| | | | | A3 | 18 | <5 |
| | 185-192 | LPVSPRLQ | 298 | B5101 | 17 | N.A. |
| | 192-200 | QLSNGNRTL | 299 | A0201 | 21 | 4 |
| | | | | A26 | 16 | N.A. |
| | | | | A3 | 19 | <5 |
| | | | | B08 | 17 | <5 |
| | | | | B1510 | 15 | |
| | 191-200 | LQLSNGNRTL | 300 | A0201 | 16 | 3 |
| | 179-187 | WVNNQSLPV | 301 | A0201 | 16 | 28 |
| | 186-194 | PVSPRLOLS PVSPRLQLS | 302 | A26 | 17 | N.A. |
| | | | | A3 | 15 | <5 |

**Table 28B CEA: Preferred Epitopes Revealed by Housekeeping Proteasome Digestion**

| **Substrate** | **Epitope** | **Sequence** | **Seq. ID No.** | **Bi nding Prediction** | | |
|---|---|---|---|---|---|---|
| | | | | **IILA type** | **SYFPEITHI** | **NIH** |
| | 362-369 | SLPVSPRL | 303 | B08 | 19 | <1.0 |
| | 361-369 | QSLPVSPRL | 304 | A0201 | 15 | <1.0 |
| | | | | B2705 | 18 | 10 |
| | | | | B2709 | 15 | |
| | 364-371 | PVSPRLQL | 305 | B08 | 18 | <1.0 |
| | 363-371 | LPVSPRLQL | 306 | B0702 | 26 | 180 |
| | | | | B08 | 16 | <1.0 |
| | | | | B5101 | 19 | 130 |
| | 362-371 | SLPVSPRLQL | 307 | A0201 | 23 | 21 |
| | | | | A26 | 18 | N.A. |
| CEA 354-380 | | | | A24 | N.A. | 6 |
| | | | | A3 | 18 | <5 |
| | 363-370 | LPVSPRLQ | 308 | B5101 | 17 | N.A. |
| | 370-378 | QLSNDNRTL | 309 | A0201 | 22 | 4 |
| | | | | A26 | 16 | N.A. |
| | | | | A3 | 17 | <1.0 |
| | | | | B08 | 17 | <1.0 |
| | 369-378 | LQLSMDNRTL | 310 | A0201 | 16 | 3 |
| | 357-365 | WVNNQSLPV | 311 | A0201 | 16 | 28 |
| | 360-368 | NQSLPVSPR | 312 | B2705 | 14 | 100 |

**Table 28C CEA: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **RLA type** | **SYFPEITHI** | **NIH** |
| | 540-547 | SLPVSPRL | 313 | B08 | 19 | <5 |
| | 539-547 | QSLPVSPRL | 314 | A0201 | 15 | <5 |
| | | | | B1510 | 15 | <5 |
| | | | | B2705 | 18 | 10 |
| | | | | B2709 | 15 | |
| | 542-549 | PVSPKLQL | 315 | B08 | 18 | <5 |
| | 541-549 | LPVSPRLQL | 316 | B0702 | 26 | 180 |
| | | | | 316 B08 | 16 | <1.0 |
| | | | | B5101 | 19 | 130 |
| | 540-549 | SLPVSPRLQL | 317 | A0201 | 23 | 21 |
| | | | | A26 | 18 | N.A. |
| CEA 532-558 | | | | A3 | 18 | <5 |
| | 541-548 | LPVSPRLQ | 318 | B5101 | 17 | N.A. |
| | 548-556 | QLSNGNRTL | 319 | A0201 | 24 | 4 |
| | | | | A26 | 16 | N.A. |
| | | | | A3 | 19 | <1.0 |
| | | | | B08 | 17 | <1.0 |
| | | | | B1510 | 15 | |
| | 547-556 | LQLSNGNRTL | 320 | A0201 | 16 | 3 |
| | 535-543 | WVNGQSLPV | 321 | A0201 | 18 | 28 |
| | | | | A3 | 15 | <1.0 |
| | 533-541 | LWWYNGQSL | 322 | A0201 | 15 | <5 |

**Table 28D CEA: Preferred Epitopes Revealed by Housekeeping Proteasome Digestion**

| **Substrate** | **Epitope** | **Seq. Sequence** | **ID No.** | **Binding Prediction** | | |
|---|---|---|---|---|---|---|
| | | | | **HLA type** | **SYFPEITHI** | **NIH** |
| CEA 532-558 (continued) | 532-541 | YLWWVNGQSL | 323 | A0201 | 25 | 816 |
| | | | | A26 | 18 | N.A. |
| | 538-546 | GQSLPVSPR | 324 | B2705 | 17 | 100 |

**Table 29A HER2/NEU: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 30-37 | DMKLRLPA | 325 | B08 | 19 | 8 |
| | 28-37 | GTDMKLRLPA | 326 | A1 | 23 | 6 |
| | 42-49 | HLDMLRHL | 327 | B08 | 17 | <5 |
| | 41-49 | THLDMLRHL | 328 | A0201 | 17 | <5 |
| | | | | B1510 | 24 | N.A. |
| | 40-49 | ETHLDMLRHL | 329 | A26 | 29 | N.A. |
| | 36-43 | PASPETHL | 330 | B5101 | 17 | N.A. |
| | 35-43 | LPASPETHL | 331 | A0201 | 15 | <5 |
| | | | | B5101 | 20 | 130 |
| | | | | B5102 | N.A. | 100 |
| Her-2 25-52 | 34-43 | RLPASPETHL | 332 | A0201 | 20 | 21 |
| | 38-46 | SPETHLDML | 333 | A0201 | 15 | <5 |
| | | | | B0702 | 20 | 24 |
| | | | | B08 | 18 | <5 |
| | | | | B5101 | 18 | 110 |
| | 37-46 | ASPETHLDML | 334 | A0201 | 18 | <5 |
| | 42-50 | HLDMLRHLY | 335 | A1 | 29 | 25 |
| | | | | A26 | 20 | N.A. |
| | | | | A3 | 17 | 4 |
| | 41-50 | THLDMLRHLY | 336 | A1 | 18 | <1.0 |

**Table 29B HER2/NEU: Preferred Epitopes Revealed by Housekeeping Proleasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 719-726 | ELRKVKVL | 337 | B08 | 24 | 16 |
| | 718-726 | TELRKVKVL | 338 | A0201 | 16 | 1 |
| | | | | B08 | 22 | <5 |
| | | | | B5101 | 16 | <5 |
| | 717-726 | ETELRKVKVL | 339 | A1 | 18 | 2 |
| | | | | A26 | 28 | 6 |
| | 715-723 | LKETELRKV | 340 | A0201 | 17 | <5 |
| | | | | B5101 | 15 | <5 |
| | 714-723 | ILKETFLRKV | 341 | A0201 | 29 | 8 |
| | 712-720 | MRILKETEL | 342 | A0201 | 15 | <5 |
| | | | | B08 | 22 | <5 |
| Her-2 705-732 | | | | B2705 | 27 | 2000 |
| | | | | B2709 | 21 | N.A. |
| | 711-720 | QMRILKETEL | 343 | A0201 | 20 | 2 |
| | | | | B0702 | 13 | 40 |
| | 717-725 | ETELRKVKV | 344 | A1 | 18 | 5 |
| | | | | A26 | 18 | N.A. |
| | 716-725 | KETELRKVKV | 345 | A0201 | 16 | 19 |
| | 706-714 | MPNQAQMRI | 346 | B0702 | 16 | 8 |
| | | | | B5101 | 22 | 629 |
| | 705-714 | AMPNQAQMRI | 347 | A0201 | 18 | 8 |
| | 706-715 | MPNQAQMRIL | 348 | B0702 | 20 | 80 |

**Table 29C HER2/NEU: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 966-973 | RPRFRELV | 349 | B08 | 20 | 24 |
| | | | | B5101 | 18 | N.A. |
| | 965-973 | CRPRFRELV | 350 | B2709 | 18 | |
| | 968-976 | RFRELVSEF | 351 | A26 | 25 | N.A. |
| | | | | A24 | N.A. | 32 |
| | | | | A3 | 15 | <5 |
| | | | | B08 | 16 | <5 |
| Her-2 954-982 | | | | B2705 | 19 | |
| | 967-976 | PRFRELVSEF | 352 | A26 | 18 | N.A. |
| | 964-972 | ECRPRFREL | 353 | A26 | 21 | N.A. |
| | | | | A24 | N.A. | 6 |
| | | | | B0702 | 15 | 40 |
| | | | | B8 | 27 | 640 |
| | | | | B1510 | 16 | <5 |

**Table 30 NY-ESO-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFFEITHI** | **NIH** |
| | 67-75 | GAASGLNGC | 354 | A0201 | 15 | <5 |
| | 52-60 | RASGPGGGA | 355 | B0702 | 15 | <5 |
| NY-ESO-1 51-77 | 64-72 | PHGGAASGL | 356 | B1510 | 21 | N.A. |
| | 63-72 | GPHGGAASGL | 357 | B0702 | 22 | 80 |
| | 60-69 | APRGPHGGAA | 358 | B0702 | 23 | 60 |

**Table 31A PRAME: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 112-119 | VRPRRWKL | 359 | B08 | 19 | |
| | 111-119 | EVRPRRWKL | 360 | A26 | 27 | N.A. |
| | | | | A24 | N.A. | 5 |
| | | | | A3 | 19 | N.A. |
| | | | | B0702 | 15 | (B7) 300.00 |
| | | | | BOS | 26 | 160 |
| | 113-121 | RPRRWKLQV | | | | |
| | | | | B5101 | 19 | 110 |
| FRAME 103-135 | 114-122 | PRRWKLQVL | 362 | B08 | 26 | <5 |
| | | | | B2705 | 23 | 200 |
| | 113-122 | RPRRWKLQVL | 363 | B0702 | 24 | (B7) 800.00 |
| | | | | B8 | N.A. | 160 |
| | | | | B5101 | N.A. | 61 |
| | | | | B5102 | N.A. | 61 |
| | | | | A24 | N.A. | 10 |
| | 116-124 | RWKLQVLDL | 364 | B08 | 22 | <5 |
| | | | | B2705 | 17 | 3 |
| | 115-124 | RRWKLQVLDL | 365 | A0201 | 16 | <5 |
| PRAME 161-187 | 174-182 | PVEVLVDLF | 366 | A26 | 25 | N.A. |

**Table 31B PRAME: Preferred Epitopes Revealed by Housekeeping Proteasome Digestion**

| **Substrate** | **Epitope** | **Sequence** | **Seq.ID No.** | **Binding Prediction** | | |
|---|---|---|---|---|---|---|
| | | | | **HLA type** | **SYFPERITHI** | **NIH** |
| | 199-206 | VKRKKNVL | 367 | B08 | 27 | 8 |
| | 198-206 | KVKRKKNVL | 368 | A0201 | 16 | <1.0 |
| | | | | A26 | 20 | N.A. |
| | | | | A3 | 22 | <1.0 |
| | | | | B08 | 30 | 40 |
| | | | | B2705 | 16 | |
| | 197-206 | EKVKRKKNVL | 369 | A26 | 15 | N.A, |
| | 198-205 | KVKRKKNV | 370 | B08 | 20 | 6 |
| | 201-208 | RKKNVLRL | 371 | B08 | 20 | <5 |
| | 200-208 | KRKKNLRLRL | 372 | A0201 | 15 | <1.0 |
| | | | | A26 | 15 | N.A. |
| PRAME 185-215 | | | | B0702 | 15 | <1.0 |
| | | | | B08 | 21 | <1.0 |
| | | | | B2705 | 28 | |
| | | | | B2709 | 25 | |
| | 199-208 | VKRKKNVLRL | 373 | A0201 | 16 | <1.0 |
| | | | | B0702 | 16 | 4 |
| | 189-196 | DELFSYLI | 374 | B5101 | 15 | N.A. |
| | 205-213 | VLRLCCKKL | 375 | A0201 | 22 | 3 |
| | | | | A26 | 17 | N.A. |
| | | | | B08 | 25 | 8 |
| | 204-213 | NVLRL CCKKL NVLRLCCKKL | 376 | A0201 | 17 | 7 |
| | | | | A26 | 19 | N.A. |

**Table 31C PRAME: Preferred Epitopes revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 194-202 | YLLEKVKRK | 377 | A0201 | 20 | <1.0 |
| | | | | A26 | 18 | N.A. |
| PRAME 185-215 | | | | A3 | 25 | 68 |
| (continued) | | | | B08 | 20 | <1.0 |
| | | | | B2705 | 17 | |
| | 74-81 | QAWPFTCL | 378 | B5101 | 17 | n.a. |
| | 73-81 | VQACWPFTCL | 379 | A0201 | 14 | 7 |
| | | | | A24 | n.a. | 5 |
| | | | | B0702 | 16 | 6 |
| | 72-81 | MVQAWPFTCL | 380 | A26 | 22 | n.a. |
| | | | | A24 | n.a. | 7 |
| | | | | B0702 | 13 | 30 |
| | S1-88 | LPLGVLMK | 381 | B5101 | 18 | n.a. |
| | 80-88 | CLPLGVLMK | 382 | A0201 | 17 | <1.0 |
| PRAME 71-98 | | | | A3 | 27 | 20 |
| | 79-88 | TCLPLGVLMK | 383 | A1 | 12 | 10 |
| | | | | A3 | 19 | 3 |
| | 84-92 | GVLMKGQHL | 384 | A0201 | 18 | 7 |
| | | | | A26 | 21 | n.a. |
| | | | | B08 | 21 | 4 |
| | 81-89 | LPLGVLMKG | 385 | B5101 | 20 | 2 |
| | 80-89 | CLPLGVLMKG | 386 | A0201 | 16 | <1.0 |
| | 76-85 | WPFTCLPLGV | 387 | B0702 | 18 | 4 |

**Table 31D PRAME: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 51-59 | ELFPPLFMA | 388 | A0201 | 19 | 18 |
| | | | | A26 | 23 | N.A. |
| | 49-57 | PRELFPPLF | 389 | B2705 | 22 | |
| | | | | B2709 | 19 | |
| PRAME 39-65 | 48-57 | LPRELFPPLF | 390 | B0702 | 19 | 4 |
| | 50-58 | RELFPPLFM | 391 | B2705 | 16 | |
| | | | | B2705 | 15 | |
| | 49-58 | PRELFPPLFM | 392 | A1 | 16 | <1.0 |

**Table 32 PSA: Preferred Epitopes Revealed by Housekeeping Proteasome Digestion**

| **Substrate** | **Epitope** | **Sequence** | **Seq. ID No** | **Binding Prediction** | | |
|---|---|---|---|---|---|---|
| | | | | **HLA type** | **SYFPEITHI** | **NIH** |
| | 239-246 | RPSLYTKV | 393 | B5101 | 21 | N.A. |
| | 238-246 | ERPSLYTKV | 394 | B2705 | 15 | 60 |
| | 236-243 | LPERPSLY | 395 | B5101 | 18 | N.A. |
| | 235-243 | ALPERPSLY | 396 | A1 | 19 | <1.0 |
| | | | | A26 | 22 | N.A. |
| | | | | A3 | 26 | 6 |
| | | | | B08 | 16 | <1.0 |
| | | | | B2705 | 11 | 15 |
| | | | | B2709 | 19 | N.A. |
| PSA 232-258 | 241-249 | SLYTKVVHY | 397 | A0201 | 20 | <1.0 |
| | | | | A1 | 19 | <1.0 |
| | | | | A26 | 25 | N.A. |
| | | | | A3 | 26 | 60 |
| | | | | B08 | 20 | <1.0 |
| | | | | B2705 | 13 | 75 |
| | 240-249 | PSLYTKVVHY | 398 | A1 | 20 | <1.0 |
| | | | | A26 | 16 | N.A. |
| | 239-247 | RPSLYTKVV | 399 | B0702 | 21 | 4 |
| | | | | B5101 | 23 | 110 |

**Table 33A PSMA: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 211-218 | GNKVKNAQ | 400 | B08 | 22 | <5 |
| | 202-209 | IARYGKVF | 401 | B08 | 18 | <5 |
| PSMA 202-228 | 217-225 | AQLAGAKGV | 402 | A0201 | 16 | 26 |
| | 207-215 | KVFRGNKVK | 403 | A3 | 32 | 15 |
| | 211-219 | GNKVKNAQL | 404 | B8 | 33 | 80 |
| | | | | B2705 | 17 | 20 |
| | 269-277 | TPGYPANEY | 405 | A1 | 16 | <5 |
| | 268-277 | LTPGYPANEY | 406 | A1 | 21 | 1 |
| PSMA 255-282 | 271-279 | GYPANEYAY | 407 | A1 | 15 | <5 |
| | 270-279 | PGYPANEYAY | 408 | A1 | 19 | <5 |
| | 266-274 | DPLTPGYPA | 409 | B0702 | 21 | 3 |
| | | | | B5101 | 17 | 20 |
| | 492-500 | SLYESWKK | 410 | A0201 | 17 | <5 |
| | | | | A3 | 27 | 150 |
| | | | | B2705 | 18 | 150 |
| | 491-500 | KSLYESWTKK | 411 | A3 | 16 | <5 |
| PSMA | 486-494 | EGFEGKSLY | 412 | A1 | 19 | <5 |
| 483-509 | | | | A26 | 21 | N.A. |
| | | | | B2705 | 16 | <5 |
| | 485-494 | DEGFEGKSLY | 413 | A1 | 17 | <5 |
| | | | | A26 | 17 | N.A. |
| | 498-506 | TKKSPSPEF | 414 | B08 | 17 | <5 |

**Table 33B PSMA: Preferred Epitopes Revealed by Housekeeping Broteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 497-506 | WTKKSPSPEF | 415 | A26 | 24 | N.A. |
| PSMA 483-509 | 492-501 | SLYESWTKKS | 416 | A0201 | 16 | <5 |
| (continued) | | | | A3 | 16 | <5 |
| | 725-732 | WGEVKRQI | 417 | B08 B5101 | 17 | <5 N.A. |
| | | | | B5101 | 17 | N.A. |
| | 724-732 | AWGEVKRQI | 418 | B5101 | 15 | 6 |
| | 723-732 | KAWGEVKRQI | 419 | A0201 | 16 | <1.0 |
| | 723-730 | KAWGEVKR | 420 | B5101 | 15 | N.A. |
| | 722-730 | SKAWGVKR | 421 | B2705 | 15 | <5 |
| | 731-739 | QIYVAAFTV | 422 | A0201 | 21 | 177 |
| | | | | A3 | 21 | <1.0 |
| | | | | B5101 | 15 | 5 |
| PSMA 721-749 | 733-741 | YVAAFTVQA | 423 | A0201 | 17 | 6 |
| | | | | A3 | 20 | <1.0 |
| | 725-733 | WGEVKRQIY | 424 | A1 | 26 | 11 |
| | 727- 735 | EVKRQIYVA | 425 | A26 | 22 | N.A. |
| | | | | 425 | 18 | <1.0 |
| | 1738-746 | TVQAAAETL | 426 | A26 | 18 | N.A. |
| | | | | A3 | 19 | <1.0 |
| | 737-746 | FTVQAAAETL | 427 | A0201 | 17 | <1.0 |
| | | | | A26 | 19 | N.A. |

**Table 33C PSMA: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 729-737 | KRQIYVAAF | 428 | A26 | 16 | N.A. |
| | | | | B2705 | 24 | 3000 |
| PSMA 721-749 (continued) | | | | B2709 | 21 | N.A. |
| | 721-729 | PSKAWGEVK | 429 | A3 | 20 | <1.0 |
| | 723-731 | KAWGEVKRQ | 430 | B5101 | 16 | <1.0 |
| | 100-108 | WKEFGLDSV | 431 | A0201 | 16 | <5 |
| PSMA 95-122 | 99-108 | QWKEFGLDSV | 432 | A0201 | 17 | <5 |
| | 102-111 | EFGLDSVELA | 433 | A26 | 16 | N.A. |

**Table 34A SCP-I: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 126-134 | ELRQKESKL | 434 | A0201 | 20 | <5 |
| | | | | A26 | 26 | N.A. |
| | | | | A3 | 17 | <5 |
| SCP-1 117-143 | | | | B0702 | 13 | (B7) 40.00 |
| | | | | B8 | 34 | 320 |
| | 125-134 | AELRQKESKL | 435 | A0201 | 16 | <5 |
| | 133-141 | KLQENRKII | 436 | A0201 | 20 | 61 |
| | 298-305 | QLEEKTKL | 437 | B08 | 28 | 2 |
| | 297-305 | NQLEETKL NQLEEKTKL | 438 | A0201 | 16 | 33 |
| | | | | B2705 | 19 | 200 |
| | 288-296 | LLEESRDKV | 439 | A0201 | 25 | 15 |
| SCP-1 281-308 | | | | B5101 | 15 | 3 |
| | 287-296 | FLLEESRDKV | 440 | A0201 | 27 | 2378 |
| | 291-299 | ESRDKVNQL | 441 | 426 | 21 | N.A. |
| | | | | B08 | 29 | 240 |
| | 290-299 | EESRDKVNQL | 442 | A26 | 19 | N.A. |
| | 475 483 | EKEVHDLEY | 443 | A1 | 31 | 11 |
| | | | | A26 | 17 | N.A. |
| | 474-483 | REKEVKDLEY | 444 | A1 | 21 | <1.0 |
| SCP-1471-498 | 480-488 | DLEYSYCHY | 445 | A1 | 26 | 45 |
| | | | | A26 | 30 | N.A. |
| | | | | A3 | 16 | <5 |
| | 477-485 | EVHDLEYSY | 446 | A1 | 15 | 1 |

**Table 34B SCP-1 : Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| SCP-1 471-498 (continued) | 477-485 | EVHDLEYSY | | A26 | 29 | N.A. |
| | | | | A3 | 19 | <1.0 |
| | 477-486 | EVHDLEYSYC | 4147 | A26 | 22 | N.A. |
| SCP-1 493-520 | 02-509 | KLSSKREL | 448 | B08 | 26 | 4 |
| | 508-515 | ELKNTEYF | 449 | B08 | 24 | <1.0 |
| | 50-515 | RELKNTEYF | 450 | B2705 | 18 | 45 |
| | | | | B4403 | N.A. | 120 |
| | 496-503 | KRGQRPKL | 451 | B08 | 18 | <1.0 |
| | 494.503 | LPKRGQRPKL | 452 | B0702 | 22 | 120 |
| | | | | B8 | N.A. | 30 |
| | | | | B5101 | N.A | 130 |
| | | | | B3501 | N.A. | 60 |
| | 509-517 | LKNIEYFTL | 453 | A0201 | 15 | <5 |
| | 508-517 | ELKNTEYFTL | 454 | A0201 | 18 | <1.0 |
| | | | | A26 | 27 | N.A. |
| | | | | A3 | 16 | <1.0 |
| | 506-514 | KLELKNTEY | 455 | A1 | 26 | 2 |
| | | | | B2705 | 26 | 3000 |
| | 502-510 | KLSSKRELK | 456 | A3 | 25 | 60 |
| | 498-506 | GQRKLSSF | 457 | A3 | 22 | 4 |
| | | | | B2705 | 18 | 200 |
| | 497-506 | RGQRPKLSSK | 458 | A3 | 22 | <1.0 |
| | 500-508 | RPKLSSKRE | 459 | B08 | 18 | <1.0 |

**Table 34C SCP-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFEITHI** | **NIH** |
| | 573-580 | LEYVREEL | 460 | B08 | 19 | <5 |
| | 572-580 | ELEYVREEL | 461 | A0201 | 17 | <1.0 |
| | | | | A26 | 23 | N.A. |
| | | | | A24 | N.A. | 9 |
| | | | | B08 | 20 | N.A. |
| SCP-1 570-596 | 571-580 | N ELEYVREEL | 462 | A0201 | 16 | 4 |
| | 579-587 | ELKQKRDEV | 463 | A0201 | 19 | <1.0 |
| | | | | A26 | 18 | N.A. |
| | | | | B08 | 29 | 48 |
| | 575-583 | YVREELKQK | 464 | A26 | 17 | N.A. |
| | | | | A3 | 27 | 2 |
| | 632-640 | QLNVYEIKV | 465 | A0201 | 24 | 70 |
| | 630-638 | SKQLNVYEI | 466 | A0201 | 17 | <55 |
| SCP-1 618-645 | 628-636 | AESKQLNVY | 467 | A1 | 19 | <5 |
| | | | | A26 | 16 | <N.A. |
| | 627-636 | TAESKQLNVY | 468 | A1 | 26 | 45 |
| | | | | A26 | 15 | N.A. |

**Table 34D SCP-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 638-645 | IKVNKLEL | 469 | B08 | 21 | <1.0 |
| | 637-645 | EIKVNKLEL | 470 | A0201 | 17 | <1.0 |
| | | | | A26 | 26 | N.A |
| | | | | B08 | 28 | 8 |
| | | | | B1510 | 15 | N.A. |
| | 636-645 | YEIKVNKLEL | 471 | A0201 | 17 | 2 |
| | 642-650 | KLELELESA | 472 | A0201 | 20 | 1 |
| | | | | A3 | 16 | <1.0 |
| SCP-1 633-660 | 635-643 | VYEIKVNKL | 473 | A0201 | 18 | <1.0 |
| | | | | A24 | N.A. | 396 |
| | | | | B08 | 22 | <1.0 |
| | 634-643 | NVYEIKVNKL | 474 | A0201 | 24 | 56 |
| | | | | A26 | 25 | N.A. |
| | | | | A24 | N.A. | 6 |
| | | | | A3 | 15 | <5 |
| | | | | B0702 | 11 | (B7) 20 |
| | | | | B08 | N.A. | 6 |
| | 646-654 | ELESAKQKF | 475 | A26 | 27 | N.A. |
| | 642-.650 | KLELELESA | 476 | A0201 | 20 | 1 |
| | | | | A3 | 16 | <1.0 |
| scP-1 640-668 | 646-654 | ELESAKLQKF | 477 | A26 | 27 | N.A. |

**Table 34E SCP-1: Preferred Epitopes Revealed by Housekeeping Proteasome Digestion.**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 771-178 | KEKLKREA | 478 | B08 | 21 | <5 |
| | 775-785 | EAKENTATL | 479 | A0201 | 18 | <5 |
| | | | | A26 | 18 | N.A |
| | | | | A24 | N.A. | 5 |
| SCP-1 768-796 | | | | B0702 | 13 | 12 |
| | | | | B08 | 28 | 48 |
| | | | | B5101 | 20 | 121 |
| | 776-785 | REAKENTATL | 480 | AQ201 | 16 | <5 |
| | 713-782 | KLKREAKENT | 481 | A3 | 17 | <5 |
| | 112-119 | EAEKIKKW | 482 | B5101 | 17 | N.A. |
| | 101-109 | GLSRVYTSKL | 483 | A0201 | 23 | 32 |
| | | | | A26 | 22 | N.A |
| | | | | A24 | N.A. | 6 |
| | | | | A3 | 17 | 3 |
| | | | | B08 | 17 | <1.0 |
| | 100-109 | EGLSRVYSKL | 484 | A26 | 21 | N.A |
| SCP-1 92-125 | | | | A24 | N.A. | 9 |
| | 108-116 | KLYKEAEKI | 485 | A0201 | 22 | 57 |
| | | | | A3 | 20 | 9 |
| | | | | B5101 | 18 | 5 |
| | 98-106 | NSEGLSRVY | 486 | A1 | 31 | 68 |
| | 97-106 | ENSEGLSRVY | 487 | A26 | 18 | N.A. |
| | 102-110 | LSRVYSKLY | 488 | A1 | 22 | <1.0 |

**Table 34F SCP-1: Preferred Epitopes Reveled by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFEITHI** | **NIH** |
| | 101-110 | GLSRVYSKLY | 489 | A1 | 18 | <1.0 |
| | | | | A26 | 18 | N.A. |
| SCP-125 | | | | A3 | 19 | 18 |
| 1 92 (continued) | 96-105 | LENSEGLSRV | 490 | A0201 | 17 | 5 |
| | 108-117 | KLYKEAEKIK | 491 | A3 | 27 | 150 |
| | 949-956 | REDRWAVI | 492 | B5101 | 15 | N.A. |
| | 948-956 | MREDRWAVI | 493 | B2705 | 18 | 600 |
| | | | | B2709 | 18 | N.A. |
| | | | | B5101 | 15 | 1 |
| | 947-956 | KMREDRWAVI | 494 | A0201 | 21 | 6 |
| SCP-1 931-958 | | | | B08 | N.A. | 15 |
| | 947-955 | KMREDRWAV | 495 | A0201 | 22 | 411 |
| | 934-942 | TTPGSTLKF | 496 | A26 | 25 | N.A. |
| | 933-942 | LTTPGSTLKF | 497 | A26 | 23 | N.A. |
| | 937-945 | GSTLELFGAI | 498 | B08 | 19 | 1 |
| | 945-953 | IRKMKEDRW | 499 | B08 | 19 | <5 |
| | 236-243 | RLEMHFKL | 500 | B08 | 16 | <5 |
| | 235-243 | SRLEMHFKL | 501 | A0201 | 18 | <5 |
| SCP-1 232-259 | | | | B2705 | 25 | 2000 |
| | | | | B2709 | 22 | |
| | 242-250 | KLKEDYEKI | 502 | A0201 | 22 | 4 |

**Tale 34G SCP-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFEITHI** | **NIH** |
| | | | | A26 | 16 | N.A. |
| | | | | A3 | 15 | 3 |
| | | | | B08 | 24 | <5 |
| | | | | B5101 | 14 | 2 |
| | 249-257 | KIQHLEQEY | 503 | A1 | 15 | <5 |
| SGP-1 232-259 | | | | A26 | 23 | N.A. |
| (continued) | | | | A3 | 17 | <5 |
| | 248 257 | EKIQHLEQEY | 504 | A1 | 15 | <5 |
| | | | | A26 | 21 | N.A. |
| | 233-242 | ENSRLEMHF | 505 | A26 | 19 | N.A. |
| | 236-245 | RLEMHFKLKE | 506 | A1 | 19 | <5 |
| | | | | 506 | 17 | <5 |
| | 324-331 | LEDIKVSL, | 507 | B08 | 20 | <1.0 |
| | 323-331 | ELEDIKVSL | 508 | A0201 | 21 | <1.0 |
| | | | | A26 | 25 | N.A. |
| | | | | A24 | N.A. | 10 |
| | | | | A3 | 17 | <1.0 |
| SCP-1 310-340 | | | | B08 | 19 | <1.0 |
| | | | | B1510 | 16 | N.A. |
| | 322-331 | KELEDIKVSL | 509 | A0201 | 19 | 22 |
| | 320-327 | LTKELEDI | 500 | B08 | 18 | <5 |
| | 319-327 | HLTKELEDE | 511 | A0201 | 21 | <1.0 |
| | 330-338 | SLQRSVSTQ | 512 | A0201 | 18 | <1.0 |

**Table 34H SCP-L - Preferred Epitope Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| SCP-1 310-340 (continued) | 321-329 | TKELEDIKV | 513 | A1 | 16 | <1.0 |
| | 320-329 | TKELEDIKV | 514 | A020 | 19 | <1.0 |
| | 326-335 | DIKVSLQRSV | 5i5 5 | A26 | 18 | N.A. |
| | 281-288 | KMKDLTFL | 516 | B08 | 20 | 3 |
| | 280--288 | NKMKDLTFL | 517 | A0201 | 15 | 1 |
| | 279-288 | ENKMKDLTFL | 518 | A26 | 19 | N.A. |
| | 288-296 | LLEESRDKV | 519 | A0201 | 25 | 15 |
| | | | | B5101 | 15 | 3 |
| | 287-296 | FLLEESRDKV | 520 | A0201 | 27 | 2378 |
| SPCP-1 272-305 | 291-299 | ESRDKVNQL | 521 | A26 | 21 | N.A. |
| | | | | B08 | 29 | 240 |
| | 290-299 | EESRDKVNQL | 522 | A26 | 19 | N.A. |
| | 277-285 | EKENKMKDL | 523 | A26 | 19 | N.A. |
| | | | | B08 | 23 | <1.0 |
| | 276-285 | TEKENKMKDL | 524 | A26 | 15 | N.A. |
| | 279-287 | ENKMXDLTF | 525 | A26 | 18 | N.A. |
| | | | | B08 | 28 | 4 |
| | 218-225 | IEKMITAF | 526 | B08 | 17 | <5 |
| | 217-225 | NTFKMTTAF | 527 | A26 | 26 | N.A. |
| | 216-225 | SNIEKMITAF | 528 | A26 | 19 | N.A. |
| SCP-1211-239 | 223-230 | TAFEFLRV | 529 | B5101 | 23 | N.A. |
| | 222-230 | ITAFEELRV | 530 | A0201 | 18 | 2 |
| | 221-230 | MITAFEELRV | 531 | A0201 | 18 | 16 |

**Table 34I. SCP-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| | 220-228 | KMITAFEEL | 532 | A0201 | 23 | 50 |
| | | | | A26 | 15 | N.A. |
| | | | | A24 | N.A. | 16 |
| SCP-1 2111-239 | 219-228 | EKMTTAFEEL | 533 | A26 | 19 | N.A. |
| (contimued) | 227-235 | ELRVQAENS | 534 | A3 | 16 | <1.0 |
| | | | | B08 | 15 | <1.0 |
| | 213-222 | DLNSNIEKMI | 535 | A0201 | 17 | <1.0 |
| | | | | A26 | 16 | N.A. |
| | 837-844 | WTSAKNTL | 536 | B08 | 20 | 4 |
| | 846-854 | TPLPKAYTV | 537 | A0201 | 18 | 2 |
| | | | | B0702 | 17 | 4 |
| | | | | B08 | 16 | 2 |
| | | | | B5101 | 25 | 220 |
| SCP-1 836-863 | 845-354 | STPLPKAYTV | 538 | A0201 | 19 | <5 |
| | 844-852 | LSTPLPKAY | 539 | A1 | 23 | 8 |
| | 843-852 | TLSTPLPKAY | 540 | A1 | 16 | <1.0 |
| | | | | A26 | 19 | N.A. |
| | | | | A3 | 18 | 2 |
| | 842-850 | NTLSTPLPK | 541 | A3 | 16 | 3 |
| | 841-850 | KNTLSTPLPK | 542 | A3 | 18 | <1.0 |

**Table 34J SCP-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| SCP-1 819-845 | 828-835 | ISKDKRDY | 543 | B08 | 21 | 3 |
| | | | | A26 | 21 | N.A. |
| | 826-835 | HGISKDKRDY | 544 | A1 | 15 | <5 |
| | 832-840 | KRDYLWTSA | 545 | B2705 | 16 | 600 |
| | 829-838 | SKDKRDYLWT | 546 | A1 | 18 | <5 |
| SCP-1 260-288 | 279-286 | ENKMKDLT | 547 | B08 | 22 | 8 |
| | 260-268 | EINDIKEKQV | 548 | A0201 | 17 | 3 |
| | | | | A26 | 19 | N.A. |
| | | | | B08 | 17 | <5 |
| | 274-282 | QITEKENKM | 549 | A0201 | 17 | 3 |
| | | | | A26 | 22 | N.A. |
| | | | | B08 | 16 | <5 |
| | 269-277 | SLLLIQJTE | 550 | A0201 | 16 | <1.0 |
| | | | | A3 | 18 | <1.0 |
| SCP-1 437-464 | 453-460 | FEKIAEEL | 551 | B08 | 21 | <1.0 |
| | 452-460 | QFEKIABEEL | 552 | B2705 | 15 | |
| | 451-460 | KOFEKIAEEL | 553 | A0201 | 16 | 56 |
| | 449-456 | DNKQFEIKI | 554 | B08 | 16 | 2 |
| | | | | B5101 | 16 | N.A. |
| | 448-456 | YDNKQFEKI | 555 | B5101 | 16 | 1 |
| | 447-456 | LYDNKQFEKI | 556 | A1 | 15 | <1.0 |

**Table 34K SCP-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| SCP-1 437-464 (continued) | 440-447 | LGEKETLL | 557 | B5101 | 16 | N.A. |
| | 439-447 | VLGEKETLL | 558 | A0201 | 24 | 149 |
| | | | | A26 | 19 | N.A. |
| | | | | B08 | 29 | 12 |
| | 438-447 | KVLGEKETLL | 559 | A0201 | 19 | 24 |
| | | | | A26 | 20 | N.A. |
| | | | | A24 | N.A. | 12 |
| | | | | A3 | 18 | <1.0 |
| | | | | B0702 | 14 | 20 |
| SCP-1 383-412 | 390-398 | LLRTEQQRL | 560 | A0201 | 22 | 3 |
| | | | | A26 | 18 | N.A. |
| | | | | B08 | 22 | 1.6 |
| | | | | B2705 | 15 | 30 |
| | 389-398 | ELLRTEQQRL | 561 | A0201 | 1.9 | 6 |
| | | | | A26 | 24 | N.A. |
| | | | | A.3 | 15 | <1.0 |
| | 393-401 | TEQQRLENY | 562 | A1 | 15 | <5 |
| | | | | A26 | 16 | N.A. |
| | 392-401 | RTEQQRLENY | 563 | A1 | 31 | 113 |
| | | | | A26 | 26 | N.A. |
| | 402-410 | EDQLIILTM | 564 | A26 | 18 | N.A. |
| | 397-406 | RLENYEDQLI | 565 | A0201 | 17 | <1.0 |
| | | | | A3 | 15 | <1.0 |

**Table 34L SCP-1: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| SCP-1 366-394 | 368-375 | KARAAHSF | 566 | B08 | 16 | <1.0 |
| | 376-384 | VVTEFETTV | 567 | A0201 | 19 | 161 |
| | | | | A3 | 16 | <1.0 |
| | 375-384 | FVVTEFETTV | 568 | A0201 | 17 | 106 |
| | 377-385 | VTEFETTVC | 569 | A1 | 18 | 2 |
| | 376-385 | VVTEFETTVC | 570 | A3 | 16 | <5 |
| SCP-1 331-357 | 344-352 | DLQIATNTI | 571 | A0201 | 22 | <5 |
| | | | | A3 | 15 | <1.0 |
| | | | | B5101 | 17 | 11 |
| | 347-355 | IATNTICQL | 572 | A0201 | 19 | 1 |
| | | | | B08 | 16 | <1.0 |
| | | | | B5101 | 20 | 79 |
| | 346-355 | QIATNTICQL | 573 | A0201 | 24 | 7 |
| | | | | A26 | 24 | N.A. |

**Table 35 SSX-4: Preferred Epitopes Revealed by Housekeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| SSX445-76 | 57-65 | VMTKLGFKV | 574 | A0201 | 21 | 495 |
| | 53-61 | LNYEVMTKL | 575 | A0201 | 17 | 7 |
| | 52-61 | KLNYEVMTKL | 576 | A0201 | 23 | 172 |
| | | | | A26 | 21 | N.A. |
| | | | | A24 | N.A. | 18 |
| | | | | A3 | 14 | 4 |
| | | | | B7 | N.A. | 4 |
| | 66-74 | TLPPFMRSK | 577 | A26 | 16 | N.A. |
| | | | | A3 | 25 | 14 |
| SSX4 98-124 | 110-118 | KIMPKKPAE | 578 | A0201 | 15 | <5 |
| | | | | A26 | 15 | N.A. |
| | | | | A3 | 16 | <5 8 |
| | 103-112 | SLQRIFPKIM | 579 | A0201 | 15 | |
| | | | | A26 | 16 | N.A. |
| | | | | A3 | 15 | <5 |

**Table 36 Tyrosinase: Preferred Epitopes Revealed by Housekeeping Proteasome Digestion**

| **Substrate** | **Epitope** | **Sequence** | **Seq. ID No.** | **Binding Prediction** | | |
|---|---|---|---|---|---|---|
| | | | | **HLA type** | **SYFPEITHI** | **NIH** |
| Tyr 445-474 | 463-471 | YIKSYLEQA | 580 | A0201 | 18 | <5 |
| | | | | A26 | 17 | N.A. |
| | 459-467 | SFQDYIKSY | 581 | A1 | 18 | <5 |
| | | | | A26 | 22 | N.A. |
| | 458-467 | DSFQDYIKSY | 582 | A1 | 19 | <5 |
| | | | | A26 | 24 | N.A. |
| Tyr 490-518 | 507-514 | LPEEKQPL | 583 | B08 | 28 | 5 |
| | | | | B5101 | 18 | N.A. |
| | 506-514 | QLPEEKQPL | 584 | A0201 | 22 | 88 |
| | | | | A26 | 20 | N.A. |
| | | | | A24 | N.A. | 9 |
| | | | | B08 | 18 | <5 |
| | 505-514 | KQLPEEKQPL | 585 | A020 | 15 | 28 |
| | | | | A24 | N.A. | 17 |
| | 507-515 | LPEEKQPLL | 586 | A0201 | 15 | <5 |
| | | | | B0702 | 21 | 24 |
| | | | | B08 | 28 | 5 |
| | | | | B5101 | 21 | 157 |
| | 506-515 | QLPEEKQPLL | 587 | A0201 | 23 | 88 |
| | | | | A26 | 20 | N.A. |
| | | | | A24 | N.A. | 7 |
| | 497-505 | SLLCRBKRK | 588 | A3 | 25 | 15 |

The following tables (37-60) present 9-mer epitopes predicted for HLA-A2 binding using both the SYPPEITHI and NIH algorithms and the epitope density of regions of overlapping epitopes, and of epitopes in the whole protein, and the ratio of these two densities. (The ratio must exceed one for there to be a cluster by the above definition; requiring higher values of this ratio reflect preferred embodiments). Individual 9-mers are ranked by score and identified by the position of their first amino in the complete protein sequence. Each potential cluster from a protein is numbered. The range of ammo acid positions within the complete sequence that the cluster covers is indicated as are the rankings of the individual predicted epitopes it is made up of.

**Table 37 BIMAS-NIH/Parker algorithm Results for gp100**

| **Rank** | **Start** | **Score** | **Rank** | **Start** | **Score** |
|---|---|---|---|---|---|
| **1** | 619 | 1493 | **21** | 416 | 19 |
| **2** | 602 | 413 | **22** | 25 | 18 |
| **3** | 162 | 226 | **23** | 566 | 17 |
| **4** | 18 | 118 | **24** | 603 | 15 |
| **5** | 178 | 118 | **25** | 384 | 14 |
| **6** | 273 | 117 | **26** | 13 | 14 |
| **7** | 601 | 81 | **27** | 290 | 12 |
| **8** | 243 | 63 | **28** | 637 | 10 |
| **9** | 606 | 60 | **29** | 639 | 9 |
| **10** | 373 | 50 | **30** | 485 | 9 |
| **11** | 544 | 36 | **31** | 453 | 8 |
| **12** | 291 | 29 | **32** | 102 | 8 |
| **13** | 592 | 29 | **33** | 399 | 8 |
| **14** | 268 | 29 | **34** | 456 | 7 |
| **15** | 47 | 27 | **35** | 113 | 7 |
| **16** | 585 | 26 | **36** | 622 | 7 |
| **17** | 576 | 21 | **37** | 69 | 7 |
| **18** | 465 | 21 | **38** | 604 | 6 |
| **19** | 570 | 20 | **39** | 350 | 6 |
| **20** | 9 | 19 | **40** | 583 | 5 |

**Table 38 SYFPEITM (Rammensee algorithm) Results for gp100**

| **Rank** | **Start** | **Score** | **Rank** | **Start** | **Score** | **Rank** | **Start** | **Score** |
|---|---|---|---|---|---|---|---|---|
| **1** | 606 | 30 | **37** | 291 | 20 | **73** | 60 | 18 |
| **2** | 162 | 29 | **38** | 269 | 20 | **74** | 17 | 18 |
| **3** | 456 | 28 | **39** | 2 | 20 | **75** | 613 | 17 |
| **4** | 18 | 28 | **40** | 610 | 19 | **76** | 599 | 17 |
| **5** | 602 | 27 | **41** | 594 | 19 | **77** | 572 | 17 |
| **6** | 598 | 27 | **42** | 591 | 19 | **78** | 557 | 17 |
| **7** | 601 | 26 | **43** | 583 | 19 | **79** | 556 | 17 |
| **8** | 597 | 26 | **44** | 570 | 19 | **80** | 512 | 17 |
| **9** | 13 | 26 | **45** | 488 | 19 | **81** | 406 | 17 |
| **10** | 585 | 25 | **46** | 446 | 19 | **82** | 324 | 17 |
| **11** | 449 | 25 | **47** | 322 | 19 | **83** | 290 | 17 |
| **12** | 4 | 25 | **48** | 267 | 19 | **84** | 101 | 17 |
| **13** | 603 | 24 | **49** | 250 | 19 | **85** | 95 | 17 |
| **14** | 576 | 24 | **50** | 205 | 19 | **86** | 635 | 16 |
| **15** | 453 | 24 | **51** | 180 | 19 | **37** | 588 | 16 |
| **16** | 178 | 24 | **52** | 169 | 19 | **88** | 584 | 16 |
| **17** | 171 | 24 | **53** | 88 | 19 | **89** | 577 | 16 |
| **18** | 11 | 24 | **54** | 47 | 19 | **90** | 559 | 16 |
| **19** | 619 | 23 | **55** | 10 | 19 | **91** | 539 | 16 |
| **20** | 280 | 23 | **56** | 648 | 18 | **92** | 494 | 16 |
| **21** | 268 | 23 | **57** | 605 | 18 | **93** | 482 | 16 |
| **22** | 592 | 22 | **58** | 604 | 18 | **94** | 468 | 16 |
| **23** | 544 | 22 | **59** | 595 | 18 | **95** | 442 | 16 |
| **24** | 465 | 22 | **60** | 571 | 18 | **96** | 413 | 16 |
| **25** | 399 | 22 | **61** | 569 | 18 | **97** | 408 | 16 |
| **26** | 373 | 22 | **62** | 450 | 18 | **98** | 402 | 16 |
| **27** | 273 | 22 | **63** | 409 | 18 | **99** | 286 | 16 |
| **28** | 243 | 22 | **64** | 400 | 18 | **100** | 234 | 16 |
| **29** | 566 | 21 | **65** | 371 | 18 | **101** | 217 | 16 |
| **30** | 563 | 21 | **66** | 343 | 18 | **102** | 211 | 16 |
| **31** | 485 | 21 | **67** | 298 | 18 | **103** | 176 | 16 |
| **32** | 384 | 21 | **68** | 209 | 18 | **104** | 107 | 16 |
| **33** | 350 | 21 | **69** | 102 | 18 | **105** | 96 | 16 |
| **34** | 9 | 21 | **70** | 97 | 18 | **106** | 80 | 16 |
| **35** | 463 | 20 | **71** | 76 | 18 | **107** | 16 | 16 |
| **36** | 397 | 20 | **72** | 69 | 18 | **108** | 14 | 16 |
| | | | | | | **109** | 7 | 16 |

**Table 39**

| **Prediction of clusters for gp100** | | | | | | |
|---|---|---|---|---|---|---|
| Total AAs: 661 | | | | | | |
| Total 9-mers: 653 | | | | | | |
| SYFPEITHI 16: 109 9-mers | | | | | | |
| NIH 5: 40 9-mers | | | | | | |
| | | | | **Epitopes/AA** | | |
| | **Cluster #** | **AAs** | **Epitopes (by Rank)** | **Cluster** | **Whole Pr** | **Ratio** |
| **SYFPEITHI** | 1 | 2 to 26 | 39, 12, 109, 34, 55, 11, 9, 108, 107, 74, 4 | 0.440 | 0.165 | 2.668 |
| | 2 | 89-115 | 72, 71, 106, 53, 85, 105, 70,84,69,104 | 0.213 | 0.165 | 1.290 |
| | 3 | 95-115 | 85, 105, 70, 84, 69 | 0.238 | 0.165 | 1.444 |
| | 4 | 162-188 | 2, 52, 17, 103, 16, 51 | 0.222 | 0,165 | 1.348 |
| | 5 | 205-225 | 50, 68, 105, 101 | 0.190 | 0.165 | 1.155 |
| | 6 | 243-258 | 28, 49 | 0.125 | 0.165 | 0.758 |
| | 7 | 267-306 | 48, 21, 38, 27, 20, 99, 83, 37, 67 | 0.225 | 0.165 | 1.364 |
| | 8 | 322-332 | 47,82 | 0.182 | 0.165 | 1.103 |
| | 9 | 343-358 | 66,33 | 0.125 | 0.165 | 0.758 |
| | 10 | 371-381 | 65,26 | 0.182 | 0.165 | 1.103 |
| | 11 | 397-421 | 36, 25, 64, 98, 81, 97, 63, 96 | 0.320 | 0.165 | 1.941 |
| | 12 | 442-476 | 95, 46, 11, 62, 15, 3, 35, 24, 94 | 0.257 | 0.165 | 1.559 |
| | 13 | 482-502 | 93, 31, 45, 93 | 0.190 | 0.165 | 1.165 |
| | 14 | 539-552 | 91,23 | 0.143 | 0.165 | 0.866 |
| | 15 | 556-627 | 79, 78, 90, 30, 29, 61, 44, 60, 77, 14, 89, 43, 88, 10, 87, 42, 22, 41, 59, 8, 6, 76, 7, 5, 13, 58, 57, 1, 40, 75, 19 | 0.431 | 0.165 | 2.611 |
| **NIH** | 1 | 9 to 33 | 20, 26, 4, 22 | 0.160 | 0.061 | 2.644 |
| | 2 | 268-281 | 14, 6 | 0.143 | 0.061 | 2.361 |
| | 3 | 290-299 | 27, 12 | 0.200 | 0.061 | 3.305 |
| | 4* | 102-121 | 32,35 | 0.100 | 0.061 | 1.653 |
| | 5* | 373-392 | 10,25 | 0.100 | 0.061 | 1.653 |
| | 6 | 453-473 | 31, 34, 18 | 0.143 | 0.061 | 2.361 |
| | 7 | 566-600 | 23, 19, 17, 40, 16, 13 | 0.171 | 0.061 | 2.833 |
| | 8 | 601-614 | 7, 2, 24, 38, 9 | 0.357 | 0.061 | 5.902 |
| | 9 | 619-630 | 1, 36 | 0.17 | 0.061 | 2.754 |
| | 10 | 637-647 | 28, 29 | 0.18 | 0.061 | 3.005 |

| | | | | | | |
|---|---|---|---|---|---|---|
| *Nearby but not overlapping epitopes | | | | | | |

**Table 40 BIMAS-NIH/Parker algorithm Results for PSMA**

| **Rank** | **Start** | **Score** |
|---|---|---|
| **1** | 663 | 1360 |
| **2** | 711 | 1055 |
| **3** | 4 | 485 |
| **4** | 27 | 400 |
| **5** | 26 | 375 |
| **6** | 668 | 261 |
| **7** | 707 | 251 |
| **8** | 469 | 193 |
| **9** | 731 | 177 |
| **10** | 35 | 67 |
| **11** | 33 | 64 |
| **12** | 554 | 59 |
| **13** | 427 | 50 |
| **14** | 115 | 47 |
| **15** | 20 | 40 |
| **16** | 217 | 26 |
| **17** | 583 | 24 |
| **18** | 415 | 19 |
| **19** | 193 | 14 |
| **20** | 240 | 12 |
| **21** | 627 | 11 |
| **22** | 260 | 10 |
| **23** | 130 | 10 |
| **24** | 741 | 9 |
| **25** | 3 | 9 |
| **26** | 733 | 8 |
| **27** | 726 | 7 |
| **28** | 286 | 6 |
| **29** | 174 | 5 |
| **30** | 700 | 5 |

**Table 41 SYFPEITHI (Rammensee algorithm) Results for PSMA**

| **Rank** | **Start** | **Score** | **Rank** | **Start** | **Score** | **Rank** | **Start** | **Score** |
|---|---|---|---|---|---|---|---|---|
| **1** | 469 | 27 | **31** | 26 | 20 | **61** | 305 | 17 |
| **2** | 27 | 27 | **32** | 3 | 20 | **62** | 304 | 17 |
| **3** | 741 | 26 | **33** | 583 | 19 | **63** | 286 | 17 |
| **4** | 711 | 26 | **34** | 579 | 19 | **64** | 282 | 17 |
| **5** | 354 | 25 | **35** | 554 | 19 | **65** | 169 | 17 |
| **6** | 4 | 25 | **36** | 550 | 19 | **66** | 142 | 17 |
| **7** | 663 | 24 | **37** | 547 | 19 | **67** | 122 | 17 |
| **8** | 130 | 24 | **38** | 390 | 19 | **68** | 738 | 16 |
| **9** | 57 | 24 | **39** | 219 | 19 | **69** | 634 | 16 |
| **10** | 707 | 23 | **40** | 193 | 19 | **70** | 631 | 16 |
| **11** | 260 | 23 | **41** | 700 | 18 | **71** | 515 | 16 |
| **12** | 20 | 23 | **42** | 472 | 18 | **72** | 456 | 16 |
| **13** | 603 | 22 | **43** | 364 | 18 | **73** | 440 | 16 |
| **14** | 218 | 22 | **44** | 317 | 18 | **74** | 385 | 16 |
| **15** | 109 | 22 | **45** | 253 | 18 | **75** | 373 | 16 |
| **16** | 731 | 21 | **46** | 91 | 18 | **76** | 365 | 16 |
| **17** | 668 | 21 | **47** | 61 | 18 | **77** | 361 | 16 |
| **18** | 660 | 21 | **48** | 13 | 18 | **78** | 289 | 16 |
| **19** | 507 | 21 | **49** | 733 | 17 | **79** | 278 | 16 |
| **20** | 454 | 21 | **50** | 673 | 17 | **80** | 258 | 16 |
| **21** | 427 | 21 | **51** | 671 | 17 | **81** | 247 | 16 |
| **22** | 358 | 21 | **52** | 642 | 17 | **82** | 217 | 16 |
| **23** | 284 | 21 | **53** | 571 | 17 | **83** | 107 | 16 |
| **24** | 115 | 21 | **54** | 492 | 17 | **84** | 100 | 16 |
| **25** | 33 | 21 | **55** | 442 | 17 | **85** | 75 | 16 |
| **26** | 606 | 20 | **56** | 441 | 17 | **86** | 37 | 16 |
| **27** | 568 | 20 | **57** | 397 | 17 | **87** | 30 | 16 |
| **28** | 473 | 20 | **58** | 391 | 17 | **88** | 21 | 16 |
| **29** | 461 | 20 | **59** | 357 | 17 | | | |
| **30** | 200 | 20 | **60** | 344 | 17 | | | |

**Table 42**

| **Prediction of clusters for prostate-specific membrane antigen (PSMA)** | | | | | | |
|---|---|---|---|---|---|---|
| Total AAs: 750 | | | | | | |
| Total 9-mers: 742 | | | | | | |
| SYFPEITHI 16: 88 9-mers | | | | | | |
| NIH 5: 30 9-mers | | | | | | |
| | | | | **Epitopes/AA** | | |
| | **Clutster#** | **Aas** | **Epitopes (by rank)** | **Cluster** | **Whole Pr** | **Ratio** |
| **SYFPEITHI** | 1 | 3 to 12 | 32, 6 | 0.200 | 0.117 | 1.705 |
| | 2 | 13-45 | 13, 12, 88, 31, 2, 87, 25, 86 | 0.242 | 0.117 | 2.066 |
| | 3 | 57-69 | 9, 47 | 0.154 | 0.117 | 1.311 |
| | 4 | 100-138 | 84, 83, 15, 24, 67.8 | 0.154 | 0.117 | 1.311 |
| | 5 | 193-208 | 40, 30 | 0.111 | 0.117 | 0.947 |
| | 6 | 217-227 | 82, 14, 39 | 0.273 | 0.117 | 2.324 |
| | 7 | 247-268 | 81, 45, 80,11 | 0.182 | 0.117 | 1.550 |
| | 8 | 278-297 | 79, 64, 23, 63, 78 | 0.250 | 0.117 | 2.131 |
| | 9 | 354-381 | 5, 59, 22, 77, 43.76,75 | 0.250 | 0.117 | 2.131 |
| | 10 | 385-405 | 74, 38, 58, 57 | 0.190 | 0,117 | 1.623 |
| | 11 | 440-450 | 73, 56, 55 | 0.273 | 0.117 | 2.324 |
| | 12 | 454-481 | 20, 72, 29, 1, 42, 28 | 0.214 | 0.117 | 1.826 |
| | 13 | 507-523 | 17,71 | 0.118 | 0.117 | 1.003 |
| | 14 | 547-562 | 37,36,35 | 0.188 | 0.117 | 1.598 |
| | 15 | 568-591 | 27, 53, 34, 33 | 0.167 | 0.117 | 1.420 |
| | 16 | 603-614 | 13, 26 | 0.167 | 0.117 | 1.420 |
| | 17 | 631-650 | 70, 69, 52 | 0.150 | 0.117 | 1.278 |
| | 18 | 660-681 | 18, 7, 17, 51, 50 | 0.227 | 0.117 | 1.937 |
| | 19 | 700-719 | 41, 10, 4 | 0.150 | 0.117 | 1.278 |
| | 20 | 731-749 | 16, 49, 68, 3 | 0.211 | 0.117 | 1.794 |
| **NIH** | 1 | 3 to12 | 25, 3 | 0.200 | 0.040 | 5.000 |
| | 2 | 20-43 | 15, 5, 4, 11, 10 | 0.208 | 0.040 | 5.208 |
| | 3* | 415-435 | 18, 13 | 0.095 | 0.040 | 2.381 |
| | 4 | 663-676 | 1, 6 | 0.143 | 0.040 | 3.571 |
| | 5 | 700-715 | 30, 7, 3 | 0.183 | 0.040 | 4.688 |
| | 6 | 726-749 | 27, 9, 26, 24 | 0.167 | 0.040 | 4.167 |

| | | | | | | |
|---|---|---|---|---|---|---|
| *Nearby but not overlapping epitopes | | | | | | |

**Table 43 BIMAS-NIH/Parker algorithm Results for PSA**

| **Rank** | **Start** | **Score** |
|---|---|---|
| **1** | 7 | 607 |
| **2** | 170 | 243 |
| **3** | 52 | 124 |
| **4** | 53 | 112 |
| **5** | 195 | 101 |
| **6** | 165 | 23 |
| **7** | 72 | 18 |
| **8** | 245 | 18 |
| **9** | 2 | 16 |
| **10** | 59 | 16 |
| **11** | 122 | 15 |
| **12** | 125 | 15 |
| **13** | 191 | 13 |
| **14** | 9 | 8 |
| **15** | 14 | 6 |
| **16** | 175 | 5 |
| **17** | 130 | 5 |

**Table 44 SYFPEITHI (Rammensee algorithm) Results for PSA**

| **Rank** | **Start** | **Score** |
|---|---|---|
| **1** | 72 | 26 |
| **2** | 170 | 22 |
| **3** | 53 | 22 |
| **4** | 7 | 22 |
| **5** | 234 | 21 |
| **6** | 166 | 21 |
| **7** | 140 | 21 |
| **8** | 66 | 21 |
| **9** | 241 | 20 |
| **10** | 175 | 20 |
| **11** | 12 | 20 |
| **12** | 41 | 19 |
| **13** | 20 | 19 |
| **14** | 14 | 19 |
| **15** | 130 | 18 |
| **16** | 124 | 18 |
| **17** | 121 | 18 |
| **18** | 47 | 18 |
| **19** | 17 | 18 |
| **20** | 218 | 17 |
| **21** | 133 | 17 |
| **22** | 125 | 17 |
| **23** | 122 | 17 |
| **24** | 118 | 17 |
| **25** | 110 | 17 |
| **26** | 67 | 17 |
| **27** | 52 | 17 |
| **28** | 21 | 17 |
| **29** | 16 | 17 |
| **30** | 2 | 17 |
| **31** | 184 | 16 |
| **32** | 179 | 16 |
| **33** | 158 | 16 |
| **34** | 79 | 16 |
| **35** | 73 | 16 |
| **36** | 4 | 16 |

**Table 45**

| **Prediction of clusters for prostate specific antigen (PSA)** | | | | | | |
|---|---|---|---|---|---|---|
| Total AAs: 261 | | | | | | |
| Total 9-mers: 253 | | | | | | |
| SYFPEITHI 16: 36 9-mers | | | | | | |
| NIH 5:17 9-mers | | | | | | |
| | | | | | **Epitopes/AA** | |
| | **Cluster #** | **AAs** | **Epitopes(by rank)** | **Cluster** | **Whole Pr** | **Ratio** |
| **SYFPEITHI** | 1 | 2 to 29 | 30, 36, 4, 11, 14, 29, 19, 13, 28 | 0.321 | 0.138 | 2.330 |
| | 2 | 41-61 | 12, 18, 27,3 | 0.190 | 0.138 | 1.381 |
| | 3 | 66-87 | 8, 26, 1, 35, 34 | 0.227 | 0.138 | 1.648 |
| | 4 | 110-148 | 25, 24, 17, 23, 16, 22, 15, 21, 7 | 0.184 | 0.138 | 1.332 |
| | 5 | 158-192 | 33, 6, 2, 10, 32, 31 | 0.171 | 0.138 | 1.243 |
| | 6 | 234-249 | 5, 9 | 0.125 | 0.138 | 0.906 |
| | 7* | 118-133 | 24, 17, 23, 16, 22 | 0,313 | 0.138 | 2.266 |
| | 8* | 118-138 | 24 ,17, 23, 16, 22, 15 | 0,286 | 0.138 | 2.071 |
| **NIH** | 1 | 2-22 | 9, 1, 14, 15 | 0.190 | 0.065 | 2.924 |
| | 2 | 52-67 | 3, 4, 10 | 0.188 | 8.065 | 2.879 |
| | 3 | 122-138 | 11, 12, 17 | 0.176 | 0.065 | 2.709 |
| | 4 | 165-183 | 6, 2, 16 | 0.158 | 0.065 | 2.424 |
| | 5 | 191-203 | 13,5 | 0.154 | 0.085 | 2.362 |
| | 6** | 52-80 | 3, 4, 10, 7 | 0.138 | 0,065 | 2.118 |

| | | | | | | |
|---|---|---|---|---|---|---|
| *These clusters are internal to the less preferred cluster #4. **Includes a nearby but not overlapping epitope, | | | | | | |

### Example 15

### Evaluating Likelihood of Epitope Cross-reactivity on Non-target Tissues.

As noted above PSA is a member of the kallikrein family of proteases, which is itself a subset of the serine protease family. While the members of this family sharing the greatest degree of sequence identity with PSA also share similar expression profiles, it remains possible that individual epitope sequences might be shared with proteins having distinctly different expression profiles. A first step in evaluating the likelihood of undesirable cross-reactivity is the identification of shared sequences. One way to accomplish this is to conduct a BLAST search of an epitope sequence against the SWISSPROT or Entrez non-redundant peptide sequence databases using the "Search for short nearly exact matches" option; hypertext transfer protocol accessible on the world wide web (http://www) at "ncbi.nlm.nih.gov/blast/index.html". Thus searching SEQ ID NO. 214, WVLTAAHCI, against SWISSPROT (limited to entries for homo sapiens) one finds four exact matches, including PSA. The other three are from kallikrein 1 (tissue kallikrein), and elastase 2A and 2B. While these nine amino acid segments are identical, the flanking sequences are quite distinct, particularly on the C-terminal side, suggesting that processing may proceed differently and that thus the same epitope may not be liberated from these other proteins. (Please note that kallikrein naming is confused. Thus the kallilkrein 1 [accession number P06870] is a different protein than the one [accession number AAD13817] mentioned in the paragraph on PSA above in the section on tumor-associated antigens).

It is possible to test this possibility in several ways, Synthetic peptides containing the epitope sequence embedded in the context of each of these proteins can be subjected to *in vitro* proteasomal digestion and analysis as described above. Alternatively, cells expressing these other proteins, whether by natural or recombinant expression, can be used as targets in a cytotoxicity (or similar) assay using CD8⁺ T cells that recognize the epitope, in order to determine if the epitope is processed and presented.

### Example 16

### Epitope Clusters,

Known and predicted epitopes are generally not evenly distributed across the sequences of protein antigens. As referred to above, we have defined segments of sequence containing a higher than average density of (known or predicted) epitopes as epitope clusters. Among the uses of epitope clusters is the incorporation of their sequence into substrate peptides used in proteasomal digestion analysis as described herein. Epitope clusters can also be useful as vaccine components. A fuller discussion of the definition and uses of epitope clusters is found in U.S. Patent Application No. 09/561,571 entitled EPITOPE CLUSTERS.

**Table 46 BIMAS-NIH/Parker algorithm Results for PSCA**

| **Rank** | **Start** | **Score** |
|---|---|---|
| **1** | 43 | P53 |
| **2** | 5 | 84 |
| **3** | 7 | 79 |
| **4** | 109 | 36 |
| **5** | 105 | 25 |
| **6** | 108 | 24 |
| **7** | 14 | 21 |
| **8** | 20 | 18 |
| **9** | 115 | 17 |
| **10** | 42 | 15 |
| **11** | 36 | 15 |
| **12** | 99 | 9 |
| **13** | 58 | 8 |

**Table 47 SYFPEITHI (Rammensee algorithm) Results for PSCA**

| **Rank** | **Start** | **Score** | **Rank** | **Start** | **Score** |
|---|---|---|---|---|---|
| **1** | 108 | 30 | **17** | 54 | 19 |
| **2** | 14 | 30 | **18** | 12 | 19 |
| **3** | 105 | 29 | **19** | 4 | 19 |
| **4** | 5 | 28 | **20** | 1 | 19 |
| **5** | 115 | 26 | **21** | 112 | 18 |
| **6** | 99 | 26 | **22** | 101 | 18 |
| **7** | 7 | 26 | **23** | 98 | 18 |
| **8** | 109 | 24 | **24** | 51 | 18 |
| **9** | 53 | 23 | **25** | 43 | 18 |
| **10** | 107 | 21 | **26** | 106 | 17 |
| **11** | 20 | 21 | **27** | 104 | 17 |
| **12** | 8 | 21 | **28** | 83 | 17 |
| **13** | 13 | 20 | **29** | 63 | 17 |
| **14** | 102 | 19 | **30** | 50 | 17 |
| **15** | 60 | 19 | **31** | 3 | 17 |
| **16** | 57 | 19 | **32** | 9 | 16 |
| | | | **33** | 92 | 16 |

**Table 48**

| **Prediction of clusters for prostate stern cell antigen (PSCA)** | | | | | | |
|---|---|---|---|---|---|---|
| Total AAs: 123 | | | | | | |
| Total 9-mers: 115 | | | | | | |
| SYFPEITHI 16: 33; | | | | | | |
| SYFPEITHI 20:13 | | | | | | |
| NIH 5: 13 | | | | | **Epitopes/AA** | |
| | | | | | | |
| | **Cluster #** | **AAs** | **Epitopes (by rank)** | **Cluster** | **Whole Pr** | **Ratio** |
| **SYFPEITHI >16** | 1 | 1 to 28 | 20, 31, 19, 4, 7, 12, 33, 18, 13, 2, 11 | 0.393 | 0.268 | 1.464 |
| | 2 | 43-71 | 25, 30, 24, 9, 17, 16, 15, 29 | 0.276 | 0.268 | 1.028 |
| | 3 | 92-123 | 32, 23, 6, 27, 14, 22, 3, 26, 10, | 0.406 | 0.268 | 1.514 |
| | | | 1, 8, 21, 5 | | | |
| **SYFPEITHI >20** | 1 | 5 to 28 | 4, 7, 12, 13, 2, 11 | 0.250 | 0.106 | 2.365 |
| | 2 | 99-123 | 6, 3, 10, 1, 8, 6 | 0.240 | 0.106 | 2.271 |
| **NIH** | 1 | 5 to 28 | 2, 3, 7, 8 | 0.167 | 0.106 | 1.577 |
| | 2 | 36-51 10, 11, 1 | | 0.188 | 0.106 | 1.774 |
| | 3 | 99-123 | 12, 5, 6, 4, 9 | 0.200 | 0.106 | 1.892 |
| | 4* | 105-116 | 5, 6, 4 | 0,250 | 0.106 | 2,365 |

| | | | | | | |
|---|---|---|---|---|---|---|
| This cluster is internal to the less preferred cluster #6 | | | | | | |

In tables 49-60 epitope prediction and cluster analysis data for each algorithm are presented together in a single table.

### Table 49

### Prediction of clusters for MAGE-1 (NIH algorithm)

Total AAs: 309
Total 9-mers: 301
NIH 5:19 9-mers

| **Cluster #** | **AAs** | **Epitope Rank** | **Start Position** | **NIH Score** | **Cluster** | **Epitopes/AA Whole Pr.** | **Ratio** |
|---|---|---|---|---|---|---|---|
| 1 | 18-32 | 16 | 18 | 9 | 0.133 | 0.063 | 2.112 |
| | | 19 | 24 | 7 | | | |
| 2 | 101-113 | 14 | 101 | 11 | 0.154 | 0.063 | 2.442 |
| | | 7 | 105 | 44 | | | |
| 3 | 146-159 | 9 | 146 | 32 | 0.143 | 0.063 | 2.263 |
| | | 3 | 151 | 169 | | | |
| 4 | 169-202 | 10 | 169 | 32 | 0.176 | 0.063 | 2.796 |
| | | 13 | 174 | 16 | | | |
| | | 18 | 181 | 8 | | | |
| | | 17 | 187 | 8 | | | |
| | | 6 | 188 | 74 | | | |
| | | 5 | 194 | 110 | | | |
| 5 | 264-277 | 2 | 264 | 190 | 0.143 | 0.063 | 2.263 |
| | | 12 | 269 | 20 | | | |
| 6 | 278-290 | 1 | 278 | 743 | 0.154 | 0.063 | 2.437 |
| | | | 11 | 282 | 28 | | |

### Table 50

### Prediction of clusters for MAGE-1 (SYFPEITHI algorithm)

Total AAs: 309
Total 9-mers: 301
SYFPEITHI 16: 46 9-mers

| **Cluster#** | **Aas** | **Epitope Rank** | **Start Position** | **SYFPEITHI Score** | **Cluster** | **Epitopes/AA Whole** | **Ratio** |
|---|---|---|---|---|---|---|---|
| 1 | 7-49 | 22 | 7 | 19 | 0.233 | 0R53 | 1.522 |
| | | 9 | 15 | 22 | | | |
| | | 27 | 18 | 18 | | | |
| | | 16 | 20 | 20 | | | |
| | | 28 | 22 | 18 | | | |
| | | 29 | 24 | 18 | | | |
| | | 33 | 31 | 17 | | | |
| | | 30 | 35 | 18 | | | |
| | | 2 | 38 | 26 | | | |
| 17 | | | 41 | 20 | | | |
| 2 | 89-132 | 10 | 89 | 22 | 0.273 | 0.153 | 1.783 |
| | | 18 | 92 | 20 | | | |
| | | 7 | 93 | 23 | | | |
| | | 23 | 96 | 19 | | | |
| | | 43 | 98 | 16 | | | |
| | | 4 | 101 | 25 | | | |
| | | 8 | 105 | 23 | | | |
| | | 34 | 107 | 17 | | | |
| | | 35 | 108 | 17 | | | |
| | | 36 | 113 | 17 | | | |
| | | 37 | 118 | 17 | | | |
| | | 19 | 124 | 20 | | | |
| 3 | 167-203 | 44 | 167 | 16 | 0.270 | 0.153 | 1.766 |
| | | 20 | 169 | 20 | | | |
| | | 12 | 174 | 21 | | | |
| | | 24 | 181 | 19 | | | |
| | | 6 | 187 | 24 | | | |
| | | 31 | 188 | 18 | | | |
| | | 25 | 191 | 19 | | | |
| | | 38 | 192 | 17 | | | |
| | | 1 | 194 | 27 | | | |
| | | 13 | 195 | 21 | | | |
| 4 | 230-246 | 14 | 230 | 21 | 0.118 | 0.153 | 0.769 |
| | | 39 | 238 | 17 | | | |
| 5 | 264-297 | 15 | 264 | 21 | 0.235 | 0.153 | 1.538 |
| | | 32 | 269 | 18 | | | |
| | | 40 | 270 | 17 | | | |
| | | 26 | 271 | 19 | | | |
| | | 46 | 275 | 16 | | | |
| | | 3 | 278 | 26 | | | |
| | | 21 | 282 | 20 | | | |
| | | 41 | 289 | 17 | | | |

**Table 51**

| **Prediction of clusters for MAGE-2 (NIH algorithm)** | | | | | | | |
|---|---|---|---|---|---|---|---|
| Total AAs: 314 | | | | | | | |
| Total 9-mers: 308 | | | | | | | |
| NIH >= 5: 20 9-mers | | | | | | | |
| | | | | | | | |
| | | | | | | **Epitope/AA** | |
| **Cluster #** | **AAs** | **Epitope Rank** | **Start Position** | **NIH Score** | **Cluster** | **Whole Pr.** | **Ratio** |
| 1 | 101-120 | 18 | 101 | 5.373 | 0.150 | 0.065 | 2.310 |
| | | 16 | 108 | 6.756 | | | |
| | | 1 | 112 | 2800.697 | | | |
| 2 | 153-167 | 8 | 153 | 31.883 | 0.200 | 0.065 | 3.080 |
| | | 4 | 158 | 168.552 | | | |
| | | 7 | 159 | 32.138 | | | |
| 3 | 169-211 | 14 | 169 | 8.535 | 0.209 | 0.065 | 3.223 |
| | | 19 | 174 | 5.346 | | | |
| | | 6 | 176 | 49.993 | | | |
| | | 11 | 181 | 15.701 | | | |
| | | 15 | 188 | 7.536 | | | |
| | | 12 | 195 | 12.809 | | | |
| | | 5 | 200 | 88.783 | | | |
| | | 10 | 201 | 16.725 | | | |
| | | 17 | 203 | 5.609 | | | |
| 4 | 271-284 | 3 | 271 | 398.324 | 0.143 | 0.065 | 2.200 |
| | | 9 | 276 | 19.658 | | | |

**Table 52**

| **Prediction of clusters for MAGE-2 (SYFPEITHI algorithm)** | | | | | | | |
|---|---|---|---|---|---|---|---|
| Total AAs: 314 | | | | | | | |
| Total 9-mers: 308 | | | | | | | |
| SYFPEITHI 16: 52 9-mers | | | | | | | |
| | | | | | | | |
| **Cluster #** | **AAS** | **Epitope Rank** | **Start Position** | **SYFPEITHI Score** | **Cluster** | **Epitopes/AA Whole Pr.** | **Ratio** |
| 1 | 15-32 | 13 | 15 | 21 | 0.278 | 0.169 | 1.645 |
| | | 29 | 18 | 18 | | | |
| | | 43 | 20 | 16 | | | |
| | | 30 | 22 | 18 | | | |
| | | 21 | 24 | 19 | | | |
| 2 | 37-56 | 31 | 37 | 18 | 0.250 | 0.169 | 1.481 |
| | | 16 | 40 | 20 | | | |
| | | 44 | 44 | 16 | | | |
| | | 14 | 45 | 21 | | | |
| | | 22 | 48 | 19 | | | |
| 3 | 96-133 | 36 | 96 | 17 | 0.211 | 0.169 | 1.247 |
| | | 46 | 101 | 16 | | | |
| | | 6 | 108 | 25 | | | |
| | | 47 | 109 | 16 | | | |
| | | 2 | 112 | 27 | | | |
| | | 37 | 120 | 17 | | | |
| | | 38 | 125 | 17 | | | |
| | | 17 | 131 | 20 | | | |
| 4 | 153-216 | 12 | 153 | 22 | 0.344 | 0.169 | 2.036 |
| | | 39 | 158 | 17 | | | |
| | | 7 | 159 | 25 | | | |
| | | 23 | 161 | 19 | | | |
| | | 24 | 162 | 19 | | | |
| | | 48 | 164 | 16 | | | |
| | | 49 | 167 | 16 | | | |
| | | 32 | 170 | 18 | | | |
| | | 50 | 171 | 16 | | | |
| | | 4 | 174 | 26 | | | |
| | | 9 | 176 | 24 | | | |
| | | 51 | 177 | 16 | | | |
| | | 15 | 181 | 21 | | | |
| | | 25 | 188 | 19 | | | |
| | | 18 | 194 | 20 | | | |
| | | 33 | 195 | 18 | | | |
| | | 19 | 198 | 20 | | | |
| | | 3 | 200 | 27 | | | |
| | | 1 | 201 | 28 | | | |
| | | 40 | 202 | 17 | | | |
| | | 10 | 203 | 23 | | | |
| | | 52 | 208 | 16 | | | |
| 5 | 237-254 | 26 | 237 | 19 | 0.167 | 0.169 | 0.987 |
| | | 27 | 245 | 19 | | | |
| | | 34 | 246 | 18 | | | |
| 6 | 271-299 | 8 | 271 | 25 | 0.241 | 0.169 | 1430 |
| | | 35 | 276 | 18 | | | |
| | | 41 | 277 | 17 | | | |
| | | 11 | 278 | 23 | | | |
| | | 28 | 283 | 19 | | | |
| | | 20 | 285 | 20 | | | |
| | | 42 | 291 | 17 | | | |

**Table 53**

| **Prediction of clusters for MAGE-3 (NIH algorithm)** | | | | | | | |
|---|---|---|---|---|---|---|---|
| Total AAs: 314 | | | | | | | |
| Total 9-mers: 308 | | | | | | | |
| NIH 5: 22 9-mers | | | | | | | |
| | | | | | | | |
| **Cluster #** | **AAs** | **Epitope Rank** | **Start Position** | **NIH Score** | **Cluster** | **Epitopes/AA Whole Pr.** | **Ratio** |
| 1 | 101-120 | 15 | 101 | 11.002 | 0.200 | 0.071 | 2.800 |
| | | 21 | 105 | 6.488 | | | |
| | | 8 | 108 | 49.134 | | | |
| | | 2 | 112 | 339.313 | | | |
| 2 | 153-167 | 18 | 153 | 7.776 | 0.200 | 0.071 | 2.800 |
| | | 6 | 158 | 51.77 | | | |
| | | 22 | 159 | 5.599 | | | |
| 3 | 174-209 | 17 | 174 | 8.832 | 0.194 | 0.071 | 2.722 |
| | | 7 | 176 | 49.993 | | | |
| | | 13 | 181 | 15.701 | | | |
| | | 19 | 188 | 7.536 | | | |
| | | 14 | 195 | 12.809 | | | |
| | | 5 | 200 | 88.783 | | | |
| | | 12 | 201 | 16.725 | | | |
| 4 | 237-251 | 16 | 237 | 10.868 | 0.200 | 0.071 | 2.800 |
| | | 4 | 238 | 148.896 | | | |
| | | 20 | 243 | 6.88 | | | |
| 5 | 271-284 | 1 | 271 | 2655.495 | 0.143 | 0.071 | 2.000 |
| | | 11 | 276 | 19.658 | | | |

**Table 54**

| **Prediction of clusters for MAGE-3 (SYFPEITHI algorithm)** | | | | | | | |
|---|---|---|---|---|---|---|---|
| Total AAs: 314 | | | | | | | |
| Total 9-mers: 308 | | | | | | | |
| SYFPEITHI 16: 47 9-mers | | | | | | | |
| | | | | | | | |
| **Cluster #** | **AAs** | **Epitope Rank** | **Start Position** | **SYFPEITHI Score** | **Cluster** | **Epitopes/AA Whole Pr.** | **Ratio** |
| 1 | 15-32 | 12 | 15 | 21 | 0.278 | 0.153 | 1.820 |
| | | 26 | 18 | 18 | | | |
| | | 37 | 20 | 16 | | | |
| | | 27 | 22 | 18 | | | |
| | | 18 | 24 | 19 | | | |
| 2 | 38-56 | 38 | 38 | 16 | 0.263 | 0,153 | 1.725 |
| | | 15 | 40 | 20 | | | |
| | | 39 | 44 | 16 | | | |
| | | 13 | 45 | 21 | | | |
| | | 19 | 48 | 19 | | | |
| 3 | 101-142 | 28 | 101 | 18 | 0.190 | 0.153 | 1.248 |
| | | 40 | 105 | 16 | | | |
| | | 1 | 108 | 31 | | | |
| | | 6 | 112 | 25 | | | |
| | | 31 | 120 | 17 | | | |
| | | 32 | 125 | 17 | | | |
| | | 16 | 131 | 20 | | | |
| | | 41 | 134 | 16 | | | |
| 4 | 153-216 | 20 | 153 | 19 | 0.313 | 0.153 | 2.048 |
| | | 29 | 156 | 18 | | | |
| | | 33 | 158 | 17 | | | |
| | | 21 | 159 | 19 | | | |
| | | 34 | 161 | 17 | | | |
| | | 42 | 164 | 16 | | | |
| | | 43 | 167 | 16 | | | |
| | | 10 | 174 | 22 | | | |
| | | 8 | 176 | 23 | | | |
| | | 14 | 181 | 21 | | | |
| | | 22 | 188 | 19 | | | |
| | | 44 | 193 | 16 | | | |
| | | 11 | 194 | 22 | | | |
| | | 23 | 195 | 19 | | | |
| | | 45 | 197 | 16 | | | |
| | | 17 | 198 | 20 | | | |
| | | 3 | 200 | 27 | | | |
| | | 2 | 201 | 28 | | | |
| | | 35 | 202 | 17 | | | |
| | | 46 | 208 | 16 | | | |
| 5 | 220-230 | 5 | 220 | 26 | 0.182 | 0.153 | 1.191 |
| | | 47 | 222 | 16 | | | |
| 6 | 237-246 | 7 | 237 | 25 | 0.200 | 0.153 | 1.311 311 |
| | | 9 | 238 | 23 | | | |
| 7 | 271-293 | 4 | 271 | 27 | 0.217 | 0.153 | 1.425 |
| | | 30 | 276 | 18 | | | |
| | | 24 | 278 | 19 | | | |
| | | 36 | 283 | 17 | | | |
| | | 25 | 285 | 19 | | | |

**Table 55**

| **Prediction of clusters for PRAME (NIH algorithm)** | | | | | | | |
|---|---|---|---|---|---|---|---|
| Total AAs: 509 | | | | | | | |
| Total 9-mers: 501 | | | | | | | |
| NIH 5: 40 9-mers | | | | | | | |
| | | | | | | | |
| **Cluster#** | **AAs** | **Epitope Rank** | **Start Position** | **NIH Score** | **Cluster** | **Epitopes/AA Whole Pr.** | **Ratio** |
| 1 | 33-47 | 20 | 33 | 18 | 0.133 | 0.080 | 1.670 |
| | | 17 | 39 | 21 | | | |
| 2 | 71-81 | 9 | 71 | 50 | 0.2 | 0.07984 | 2.505 |
| | | 32 | 73 | 7 | | | |
| 3 | 99-108 | 23 | 100 | 15 | 0.2 | 0.07984 | 2.505 |
| | | 24 | 99 | 13 | | | |
| 4 | 126-135 | 38 | 126 | 5 | 0.2 | 0.07984 | 2.505 |
| | | 35 | 127 | 6 | | | |
| 5 | 224-246 | 5 | 224 | 124 | 0.130 | 0.080 | 1.634 |
| | | 8 | 230 | 63 | | | |
| | | 39 | 238 | 5 | | | |
| 6 | 290-303 | 18 | 290 | 18 | 0.214 | 0.080 | 2.684 |
| | | 14 | 292 | 23 | | | |
| | | 7 | 295 | 66 | | | |
| 7 | 305-324 | 28 | 305 | 10 | 0.200 | 0.080 | 2.505 |
| | | 30 | 308 | 8 | | | |
| | | 25 | 312 | 13 | | | |
| | | 36 | 316 | 6 | | | |
| 8 | 394-409 | 2 | 394 | 182 | 0.188 | 0.080 | 2.348 |
| | | 12 | 397 | 42 | | | |
| | | 31 | 401 | 7 | | | |
| 9 | 422-443 | 10 | 422 | 49 | 0.227 | 0.080 | 2.847 |
| | | 3 | 425 | 182 | | | |
| | | 34 | 431 | 7 | | | |
| | | 29 | 432 | 9 | | | |
| | | 4 | 435 | 160 | | | |
| 10 | 459-487 | 15 | 459 | 21 | 0.172 | 0.080 | 2.159 |
| | | 11 | 462 | 45 | | | |
| | | 22 | 466 | 15 | | | |
| | | 40 | 472 | 5 | | | |
| | | 37 | 479 | 6 | | | |

**Table 56**

| **Prediction of clusters for PRAME (SYFPEITHI algorithm)** | | | | | | | |
|---|---|---|---|---|---|---|---|
| Total AAs: 509 | | | | | | | |
| Total 9-mers: 501 | | | | | | | |
| SYFPEITFII 17:809-mers | | | | | | | |
| | | | | | | | |
| **Cluster #** | **AAs** | **Epitope Rank** | **Start Position** | **SYFPEITHI Score** | **Cluster** | **Epitopes/AA Whole Pr.** | **Ratio** |
| 1 | 18-59 | 65 | 18 | 17 | 0.238 | 0.160 | 1.491 |
| | | 50 | 21 | 18 | | | |
| | | 66 | 26 | 17 | | | |
| | | 35 | 33 | 20 | | | |
| | | 22 | 34 | 22 | | | |
| | | 51 | 37 | 18 | | | |
| | | 5 | 39 | 27 | | | |
| | | 23 | 40 | 22 | | | |
| | | 13 | 44 | 24 | | | |
| | | 46 | 51 | 19 | | | |
| 2 | 78-115 | 36 | 78 | 20 | 0.263 | 0.160 | 1.648 |
| | | 67 | 80 | 17 | | | |
| | | 52 | 84 | 18 | | | |
| | | 24 | 86 | 22 | | | |
| | | 53 | 91 | 18 | | | |
| | | 25 | 93 | 22 | | | |
| | | 9 | 99 | 25 | | | |
| | | 8 | 100 | 26 | | | |
| | | 54 | 103 | 18 | | | |
| | | 55 | 107 | 18 | | | |
| 3 | 191-202 | 56 | 191 | 18 | 0.167 | 0.160 | 1.044 |
| | | 38 | 194 | 20 | | | |
| 4 | 205-215 | 26 | 205 | 22 | 0.182 | 0.160 | 1.139 |
| | | 27 | 207 | 22 | | | |
| 5 | 222-238 | 47 | 222 | 19 | 0.235 | 0.160 | 1.474 |
| | | 14 | 224 | 24 | | | |
| | | 69 | 227 | 17 | | | |
| | | 57 | 230 | 18 | | | |
| 6 | 241-273 | 70 | 241 | 17 | 0.212 | 0.160 | 1.328 |
| | | 15 | 248 | 24 | | | |
| | | 71 | 255 | 17 | | | |
| | | 30 | 258 | 21 | | | |
| | | 39 | 259 | 20 | | | |
| | | 58 | 261 | 18 | | | |
| | | 40 | 265 | 20 | | | |
| 7 | 290-342 | 72 | 290 | 17 | 0.208 | 0.160 | 1.300 |
| | | 48 | 293 | 19 | | | |
| | | 31 | 298 | 21 | | | |
| | | 73 | 301 | 17 | | | |
| | | 18 | 305 | 23 | | | |
| | | 6 | 308 | 27 | | | |
| | | 10 | 312 | 25 | | | |
| | | 19 | 316 | 23 | | | |
| | | 28 | 319 | 22 | | | |
| | | | | | | | |

| **Prediction of clusters for PRAME (SYFPEITHI algorithm)** | | | | | | | |
|---|---|---|---|---|---|---|---|
| Total AAs: 509 | | | | | | | |
| Total 9-mers: 501 | | | | | | | |
| SYFPEITHI 17:80 9-mers | | | | | | | |
| | | | | | | | |
| **Cluster #** | **AAs** | **Epitope Rank** | **Start Position** | **SYFPEITHI Score** | **Cluster** | **Epitopes/AA Whole Pr.** | **Ratio** |
| | | 41 | 326 | 20 | | | |
| | | 74 | 334 | 17 | | | |
| 8 | 343-363 | 59 | 343 | 18 | 0.238 | 0.160 | 1.491 |
| | | 60 | 348 | 18 | | | |
| | | 75 | 351 | 17 | | | |
| | | 20 | 353 | 23 | | | |
| | | 76 | 355 | 17 | | | |
| 9 | 364-447 | 49 | 364 | 19 | 0.250 | 0.160 | 1.566 |
| | | 32 | 371 | 21 | | | |
| | | 11 | 372 | 25 | | | |
| | | 61 | 375 | 18 | | | |
| | | 77 | 382 | 17 | | | |
| | | 21 | 390 | 23 | | | |
| | | 78 | 391 | 17 | | | |
| | | 1 | 394 | 30 | | | |
| | | 42 | 397 | 20 | | | |
| | | 62 | 403 | 18 | | | |
| | | 33 | 410 | 21 | | | |
| | | 43 | 418 | 20 | | | |
| | | 34 | 419 | 21 | | | |
| | | 7 | 422 | 27 | | | |
| | | 2 | 425 | 29 | | | |
| | | 79 | 426 | 17 | | | |
| | | 63 | 428 | 18 | | | |
| | | 64 | 431 | 18 | | | |
| | | 12 | 432 | 25 | | | |
| | | 16 | 435 | 24 | | | |
| | | 80 | 439 | 17 | | | |
| 10 | 455-474 | 29 | 455 | 22 | 0.200 | 0.160 | 1.253 |
| | | 17 | 459 | 24 | | | |
| | | 4 | 462 | 28 | | | |
| | | 3 | 466 | 29 | | | |

**Table 57**

| **Predication of clusters for CEA (NIH algorithm)** | | | | | | | |
|---|---|---|---|---|---|---|---|
| Total AAs:702 | | | | | | | |
| Total 9-mers: 694 | | | | | | | |
| NIH 5: 30 9-mers | | | | | | | |
| | | | | | | | |
| **Cluster #** | **AA** | **Peptides Rank** | **Start Position** | **Score** | **Cluster** | **Peptides/AAs Whole Pr.** | **Ratio** |
| 1 | 17-32 | 5 | 17 | 79.041 | 0.188 | 0.043 | 4.388 |
| | | 7 | 18 | 46.873 | | | |
| | | 20 | 24 | 12.668 | | | |
| 2 | 113-129 | 2 | 113 | 167.991 | 0.118 | 0.043 | 2.753 |
| | | 15 | 121 | 21.362 | | | |
| 3 | 172-187 | 25 | 172 | 9.165 | 0.125 | 0.043 | 2.925 |
| | | 14 | 179 | 27.995 | | | |
| 4 | 278-291 | 30 | 278 | 5.818 | 0.143 | 0.043 | 3.343 |
| | | 17 | 283 | 19.301 | | | |
| 5 | 0350-365 | 9 | 350 | 43.075 | 0.125 | 0.043 | 2.925 |
| | | 12 | 357 | 27.995 | | | |
| 6 | 528-543 | 8 | 528 | 43.075 | 0.125 | 0.043 | 2.925 |
| | | 13 | 535 | 27.995 | | | |
| 7 | 631-645 | 23 | 631 | 9.563 | 0.200 | 0.043 | 4.680 |
| | | 19 | 634 | 13.381 | | | |
| | | 24 | 637 | 9.245 | | | |
| 8 | 691-702 | 1 | 691 | 196.407 | 0.167 | 0.043 | 3.900 |
| | | 27 | 694 | 7.769 | | | |

**Table 58**

| **Predication of clusters for CEA (SYFPEITHI algorithm)** | | | | | | | |
|---|---|---|---|---|---|---|---|
| Total AAs:702 | | | | | | | |
| Total 9-mers: 694 | | | | | | | |
| SYFPEITHI 16: 81 9-mers | | | | | | | |
| | | | | | | | |
| **Cluster #** | **AA** | **Peptides Rank** | **Start Position** | **Score** | **Cluster** | **Peptides/AAs Whole Pr.** | **Ratio** |
| 1 | 5-36 | 67 | 5 | 16 | 0.250 | 0.117 | 2.140 |
| | | 23 | 12 | 19 | | | |
| | | 24 | 16 | 19 | | | |
| | | 9 | 17 | 22 | | | |
| | | 25 | 18 | 19 | | | |
| | | 32 | 19 | 18 | | | |
| | | 68 | 23 | 16 | | | |
| | | 33 | 28 | 18 | | | |
| 2 | 37-62 | 41 | 37 | 17 | 0.269 | 0.117 | 2.305 |
| | | 20 | 44 | 20 | | | |
| | | 26 | 45 | 19 | | | |
| | | 42 | 46 | 17 | | | |
| | | 27 | 50 | 19 | | | |
| | | 43 | 53 | 17 | | | |
| | | 44 | 54 | 17 | | | |
| 3 | 99-115 | 14 | 99 | 21 | 0.235 | 0.117 | 2.014 |
| | | 5 | 100 | 23 | | | |
| | | 45 | 104 | 17 | | | |
| | | 34 | 107 | 18 | | | |
| 4 | 116-129 | 69 | 116 | 16 | 0.143 | 0.117 | 1.223 |
| | | 21 | 121 | 20 | | | |
| 5 | 172-187 | 46 | 172 | 17 | 0.125 | 0.117 | 1.070 |
| | | 70 | 179 | 16 | | | |
| 6 | 192-202 | 3 | 192 | 24 | 0.182 | 0.117 | 1.557 |
| | | 47 | 194 | 17 | | | |
| 7 | 226-241 | 48 | 226 | 17 | 0.188 | 0.117 | 1.605 |
| | | 49 | 229 | 17 | | | |
| | | 15 | 233 | 21 | | | |
| 8 | 307-318 | 11 | 307 | 22 | 0.250 | 0.117 | 2.140 |
| | | 71 | 308 | 16 | | | |
| | | 51 | 310 | 17 | | | |
| 9 | 319-349 | 52 | 319 | 17 | 0.129 | 0.117 | 1.105 |
| | | 53 | 327 | 17 | | | |
| | | 72 | 335 | 16 | | | |
| | | 35 | 341 | 18 | | | |
| 10 | 370-388 | 12 | 370 | 22 | 0.211 | 0.117 | 1.802 |
| | | 54 | 372 | 17 | | | |
| | | 74 | 375 | 16 | | | |
| | | 6 | 380 | 23 | | | |
| 11 | 403-419 | 56 | 403 | 17 | 0.235 | 0.117 | 2.014 |
| | | 57 | 404 | 17 | | | |
| | | 58 | 407 | 17 | | | |
| | | 28 | 411 | 19 | | | |
| 12 | 427-442 | 59 | 427 | 17 | 0.188 | 0.117 | 1.605 |
| | | 75 | 432 | 16 | | | |
| | | 76 | 434 | 16 | | | |
| 13 | 450-462 | 77 | 450 | 16 | 0.154 | 0.117 | 1.317 |
| | | 13 | 454 | 22 | | | |
| 14 | 488-505 | 36 | 488 | 18 | 0.167 | 0.117 | 1.427 |
| | | 18 | 492 | 21 | | | |
| | | 60 | 497 | 17 | | | |
| 15 | 548-558 | 4 | 548 | 24 | 0.182 | 0.117 | 1.557 |
| | | 61 | 550 | 17 | | | |
| 16 | 565-577 | 62 | 565 | 17 | 0.154 | 0.117 | 1.317 |
| | | 19 | 569 | 21 | | | |
| 17 | 579-597 | 78 | 579 | 16 | 0.143 | 0.117 | 1.223 |
| | | 79 | 582 | 16 | | | |
| | | 7 | 589 | 23 | | | |
| 18 | 605-618 | 2 | 605 | 25 | 0.143 | 0.117 | 1.223 |
| | | 38 | 610 | 18 | | | |
| 19 | 631-669 | 29 | 631 | 19 | 0.154 | 0.117 | 1.317 |
| | | 63 | 637 | 17 | | | |
| | | 80 | 644 | 16 | | | |
| | | 64 | 652 | 17 | | | |
| | | 39 | 660 | 18 | | | |
| | | 81 | 661 | 16 | | | |
| 20 | 675-702 | 22 | 675 | 20 | 0.286 | 0.117 | 2.446 |
| | | 30 | 683 | 19 | | | |
| | | 31 | 687 | 19 | | | |
| | | 40 | 688 | 18 | | | |
| | | 65 | 690 | 17 | | | |
| | | 1 | 691 | 31 | | | |
| | | 66 | 692 | 17 | | | |
| | | 8 | 694 | 23 | | | |

**Table 59**

| **Predication of clusters for SCP-1 (NIH algorithm)** | | | | | | | |
|---|---|---|---|---|---|---|---|
| Total AAs: 976 | | | | | | | |
| Total 9-mers: 968 | | | | | | | |
| NIH 5: 37 9-mers | | | | | | | |
| | | | | | | | |
| **Cluster #** | **AA** | **Peptides Rank** | **Start Position** | **Score** | **Cluster** | **Peptides/AAs Whole Pr.** | **Ratio** |
| 1 | 101-116 | 15 | 101 | 40.589 | 0.125 | 0.038 | 3.270 |
| | | 13 | 108 | 57.255 | | | |
| 2* | 281-305 | 14 | 281 | 44.944 | 0.12 | 0.038 | 3.139 |
| | | 24 | 288 | 15.203 | | | |
| | | 17 | 297 | 32.857 | | | |
| 3 | 431-447 | 8 | 431 | 80.217 | 0.073 | 0.038 | 1.914 |
| | | 26 | 438 | 11.861 | | | |
| | | 4 | 439 | 148.896 | | | |
| 4 | 557-579 | 11 | 557 | 64.335 | 0.174 | 0.038 | 4.550 |
| | | 19 | 560 | 24.937 | | | |
| | | 6 | 564 | 87.586 | | | |
| | | 18 | 571 | 32.765 | | | |
| 5 | 635-650 | 10 | 635 | 69.552 | 0.125 | 0.038 | 3.270 |
| | | 34 | 642 | 6.542 | | | |
| 6 | 755-767 | 36 | 755 | 5.599 | 0.154 | 0.038 | 4.025 |
| | | 35 | 759 | 5.928 | | | |
| 7 | 838-854 | 2 | 838 | 284.517 | 0.118 | 0.038 | 3.078 |
| | | 28 | 846 | 11.426 | | | |

**Table 60**

| **Prediction of clusters for SCP-1** | | | | | | | |
|---|---|---|---|---|---|---|---|
| Total AAs: 976 | | | | | | | |
| Total 9-mers: 968 | | | | | | | |
| Rammensee 16: 118 9-mers | | | | | | | |
| | | | | | | | |
| **Cluster #** | **AA** | **Peptides Rank** | **Start Position** | **Score** | **Cluster** | **Peptides/AAs Whole Pr.** | **Ratio** |
| 1 | 8-28 | 99 | 8 | 16 | 0.143 | 0.121 | 1.182 |
| | | 77 | 15 | 17 | | | |
| | | 100 | 20 | 16 | | | |
| 2 | 63-80 | 78 | 63 | 17 | 0.222 | 0.121 | 1.838 |
| | | 50 | 66 | 19 | | | |
| | | 102 | 69 | 16 | | | |
| | | 60 | 72 | 18 | | | |
| 3 | 94-123 | 79 | 94 | 17 | 0.133 | 0.121 | 1.103 |
| | | 12 | 101 | 23 | | | |
| | | 17 | 108 | 22 | | | |
| | | 103 | 115 | 16 | | | |
| 4 | 126-158 | 35 | 126 | 20 | 0.182 | 0.121 | 1.504 |
| | | 36 | 133 | 20 | | | |
| | | 51 | 139 | 19 | | | |
| | | 80 | 140 | 17 | | | |
| | | 61 | 143 | 18 | | | |
| | | 37 | 150 | 20 | | | |
| 5 | 161-189 | 38 | 161 | 20 | 0,207 | 0.121 | 1.711 |
| | | 52 | 165 | 19 | | | |
| | | 81 | 171 | 17 | | | |
| | | 82 | 177 | 17 | | | |
| | | 62 | 178 | 18 | | | |
| | | 39 | 181 | 20 | | | |
| 6 | 213-230 | 40 | 213 | 20 | 0.167 | 0.121 | 1.379 |
| | | 13 | 220 | 23 | | | |
| | | 28 | 222 | 21 | | | |
| 7 | 235-250 | 63 | 235 | 18 | 0.125 | 0.121 | 1.034 |
| | | 18 | 242 | 22 | | | |
| 8 | 260-296 | 83 | 260 | 17 | 0.243 | 0.121 | 2.012 |
| | | 105 | 262 | 16 | | | |
| | | 84 | 267 | 17 | | | |
| | | 106 | 269 | 16 | | | |
| | | 41 | 270 | 20 | | | |
| | | 64 | 271 | 18 | | | |
| | | 85 | 274 | 17 | | | |
| | | 19 | 281 | 22 | | | |
| | | 3 | 288 | 25 | | | |
| 9 | 312-338 | 108 | 312 | 16 | 0.148 | 0.121 | 1.225 |
| | | 29 | 319 | 21 | | | |
| | | 30 | 323 | 21 | | | |
| | | 65 | 330 | 18 | | | |
| 10 | 339-355 | 66 | 339 | 18 | 0.235 | 0.121 | 1.946 |
| | | 31 | 340 | 21 | | | |
| | | 42 | 344 | 20 | | | |
| | | 53 | 347 | 19 | | | |
| 11 | 376-447 | 54 | 376 | 19 | 0.194 | 0.121 | 1.608 |
| | | 43 | 382 | 20 | | | |
| | | 44 | 386 | 20 | | | |
| | | 20 | 390 | 22 | | | |
| | | 55 | 397 | 19 | | | |
| | | 8 | 404 | 24 | | | |
| | | 86 | 407 | 17 | | | |
| | | 45 | 411 | 20 | | | |
| | | 67 | 417 | 18 | | | |
| | | 21 | 425 | 22 | | | |
| | | 46 | 431 | 20 | | | |
| | | 68 | 432 | 18 | | | |
| | | 32 | 438 | 21 | | | |
| | | 7 | 439 | 24 | | | |
| 12 | 455-488 | 33 | 455 | 21 | 0.235 | 0.121 | 1.946 |
| | | 47 | 459 | 20 | | | |
| | | 56 | 462 | 19 | | | |
| | | 87 | 463 | 17 | | | |
| | | 88 | 466 | 17 | | | |
| | | 14 | 470 | 23 | | | |
| | | 109 | 473 | 16 | | | |
| | | 34 | 480 | 21 | | | |
| 13 | 515-530 | 57 | 515 | 19 | 0.125 | 0.121 | 1.034 |
| | | 22 | 522 | 22 | | | |
| 14 | 557-590 | 8 | 557 | 24 | 0.147 | 0.121 | 1.216 |
| | | 23 | 564 | 22 | | | |
| | | 9 | 571 | 24 | | | |
| | | 90 | 575 | 17 | | | |
| | | 58 | 582 | 19 | | | |
| 15 | 610-625 | 69 | 610 | 18 | 0.125 | 0.121 | 1.034 |
| | | 91 | 617 | 17 | | | |
| 16 | 633-668 | 92 | 633 | 17 | 0.222 | | |
| | | 10 | 635 | 24 | | | |
| | | 70 | 638 | 18 | | | |
| | | 93 | 640 | 17 | | | |
| | | 48 | 642 | 20 | | | |
| | | 49 | 645 | 20 | | | |
| | | 111 | 652 | 16 | | | |
| | | 112 | 660 | 16 | | | |
| 17 | 674-665 | 71 | 674 | 18 | 0.167 | 0.121 | 1.379 |
| | | 11 | 677 | 24 | | | |
| 18 | 687-702 | 1 | 687 | 26 | 0.125 | 0.121 | 1.034 |
| | | 94 | 694 | 17 | | | |
| 19 | 744-767 | 113 | 744 | 16 | 0.250 | 0.121 | 2.068 |
| | | 95 | 745 | 17 | | | |
| | | 4 | 745 | 25 | | | |
| | | 24 | 752 | 22 | | | |
| | | 2 | 755 | 26 | | | |
| | | 72 | 759 | 18 | | | |
| 20 | 812-827 | 97 | 812 | 17 | 0.125 | 0.121 | 1.034 |
| | | 115 | 819 | 16 | | | |
| 21 | 838-857 | 116 | 838 | 16 | 0.150 | 0.121 | 1.241 |
| | | 25 | 846 | 22 | | | |
| | | 74 | 849 | 18 | | | |
| 22 | 896-913 | 117 | 896 | 16 | 0.222 | 0.121 | 1.838 |
| | | 98 | 899 | 17 | | | |
| | | 26 | 902 | 22 | | | |
| | | 76 | 905 | 18 | | | |

The embodiments of the invention are applicable to and contemplate variations in the sequences of the target antigens provided herein, including those disclosed in the various databases that are accessible by the world wide web. Specifically for the specific sequences disclosed herein, variation in sequences can be found by using the provided accession numbers to access information for each antigen.

### TYROSINASE PROTEIN; SEQ ID NO 2

1 

61 

121 

181 

241 

301 

361 

421 

481 

### SSX-2 PROTEIN; SEQ ID NO 3

1 MNGDDAFARR PTVGAQIPEK IQKAFDDIAK YFSKEEWEKM KASEKIFYVY
MKRKYEAMTK
61 LGFKATLPPF MCNKRAEDFQ GNDLDNDPNR GNQVERPQMT FGRLQGISPK
IMPKKPAEEG
121 NDSEEVPEAS GPQNDGKELC PPGKPTTSEK IHERSGPKRG EHAWTHRLRE
RKQLVIYEEI
181 SDPEEDDE

### PSMA PROTEIN; SEQ ID NO 4

1 MWNLLHETDS AVATARRPRW LCAGALVLAG GFFLLGFLFG WFIKSSNEAT
NITPKHNMKA
61 FLDELKAENI KKFLYNFTQI PHLAGTEQNF QLAKQIQSQW KEFGLDSVEL
AHYDVLLSYP
121 NKTHPNYISI INEDGNEIFN TSLFEPPPPG YENVSDIVPP FSAFSPQGMP
EGDLVYVNYA
181 RTEDFFKLER DMKINCSGKI VIARYGKVFR GNKVKNAQLA GAKGVILYSD
PADYFAPGVK
241 SYPDGWNLPG GGVQRGNILN LNGAGDPLTP GYPANEYAYR RGIAEAVGLP
SIPVHPIGYY
301 DAQKLLEKMG GSAPPDSSWR GSLKVPYNVG PGFTGNFSTQ KVKMHIHSTN
EVTRIYNVIG
361 TLRGAVEPDR YVILGGHRDS WVFGGIDPQS GAAVVHEIVR SFGTLKKEGW
RPRRTILFAS
421 WDAEEFGLLG STEWAEENSR LLQERGVAYI NADSSIEGNY TLRVDCTPLM
YSLVHNLTKE
481 LKSPDEGFEG KSLYESWTKK SPSPEFSGMP RISKLGSGND FEVFFQRLGI
ASGRARYTKN
541 WETNKFSGYP LYHSVYETYE LVEKFYDPMF KYHLTVAQVR GGMVFELANS
IVLPFDCRDY

601 AVVLRKYADK IYSISMKHPQ EMKTYSVSFD SLFSAVKNFT EIASKFSERL
QDFDKSNPIV
661 LRMMNDQLMF LERAFIDPLG LPDRPFYRHV IYAPSSHNKY AGESFPGIYD
ALFDIESKVD
721 PSKAWGEVKR QIYVAAFTVQ AAAETLSEVA

Homo sapiens tyrosinase (oculocutaneous albinism IA) (TYR), mRNA., ACCESSION NM_000372
VERSION NM_000372.1 GI:4507752

### SEQ ID NO 2

/translation="
















"

### SEQ ID NO 5 ORIGIN

1 atcactgtag tagtagctgg aaagagaaat ctgtgactcc aattagccag
ttcctgcaga
61 ccttgtgagg actagaggaa gaatgctcct ggctgttttg tactgcctgc
tgtggagttt
121 ccagacctcc gctggccatt tccctagagc ctgtgtctcc tctaagaacc
tgatggagaa
181 ggaatgctgt ccaccgtgga gcggggacag gagtccctgt ggccagcttt
caggcagagg
241 ttcctgtcag aatatccttc tgtccaatgc accacttggg cctcaatttc
ccttcacagg
301 ggtggatgac cgggagtcgt ggccttccgt cttttataat aggacctgcc
agtgctctgg
361 caacttcatg ggattcaact gtggaaactg caagtttggc ttttggggac
caaactgcac
421 agagagacga ctcttggtga gaagaaacat cttcgatttg agtgccccag
agaaggacaa
481 attttttgcc tacctcactt tagcaaagca taccatcagc tcagactatg
tcatccccat

541 agggacctat ggccaaatga aaaatggatc aacacccatg tttaacgaca
tcaatattta
601 tgacctcttt gtctggatgc attattatgt gtcaatggat gcactgcttg
ggggatctga
661 aatctggaga gacattgatt ttgcccatga agcaccagct tttctgcctt
ggcatagact
721 cttcttgttg cggtgggaac aagaaatcca gaagctgaca ggagatgaaa
acttcactat
781 tccatattgg gactggcggg atgcagaaaa gtgtgacatt tgcacagatg
agtacatggg
841 aggtcagcac cccacaaatc ctaacttact cagcccagca tcattcttct
cctcttggca
901 gattgtctgt agccgattgg aggagtacaa cagccatcag tctttatgca
atggaacgcc
961 cgagggacct ttacggcgta atcctggaaa ccatgacaaa tccagaaccc
caaggctccc
1021 ctcttcagct gatgtagaat tttgcctgag tttgacccaa tatgaatctg
gttccatgga
1081 taaagctgcc aatttcagct ttagaaatac actggaagga tttgctagtc
cacttactgg
1141 gatagcggat gcctctcaaa gcagcatgca caatgccttg cacatctata
tgaatggaac
1201 aatgtcccag gtacagggat ctgccaacga tcctatcttc cttcttcacc
atgcatttgt
1261 tgacagtatt tttgagcagt ggctccgaag gcaccgtcct cttcaagaag
tttatccaga
1321 agccaatgca cccattggac ataaccggga atcctacatg gttcctttta
taccactgta
1381 cagaaatggt gatttcttta tttcatccaa agatctgggc tatgactata
gctatctaca
1441 agattcagac ccagactctt ttcaagacta cattaagtcc tatttggaac
aagcgagtcg
1501 gatctggtca tggctccttg gggcggcgat ggtaggggcc gtcctcactg
ccctgctggc
1561 agggcttgtg agcttgctgt gtcgtcacaa gagaaagcag cttcctgaag
aaaagcagcc
1621 actcctcatg gagaaagagg attaccacag cttgtatcag agccatttat
aaaaggctta
1681 ggcaatagag tagggccaaa aagcctgacc tcactctaac tcaaagtaat
gtccaggttc
1741 ccagagaata tctgctggta tttttctgta aagaccattt gcaaaattgt
aacctaatac
1801 aaagtgtagc cttcttccaa ctcaggtaga acacacctgt ctttgtcttg
ctgttttcac
1861 tcagcccttt taacattttc ccctaagccc atatgtctaa ggaaaggatg
ctatttggta
1921 atgaggaact gttatttgta tgtgaattaa agtgctctta tttt

Homo sapiens synovial sarcoma, X breakpoint 2 (SSX2), mRNA. ACCESSION NM_003147
VERSION NM_003147.1 GI:10337582

### SEQ ID NO 3

/translation="MNGDDAFARRPTVGAQIPEKIQKAFDDIAKYFSKEEWEKMKASE
KIFYVYMKRKYEAMTKLGFKATLPPFMCNKRAEDFQGNDLDNDPNRGNQVERPQMTFG
RLQGISPKIMPKKPAEEGNDSEEVPEASGPQNDGKELCPPGKPTTSEKIHERSGPKRG
EHAWTHRLRERKQLVIYEEISDPEEDDE"

### SEQ ID NO 6

### ORIGIN

1 ctctctttcg attcttccat actcagagta cgcacggtct gattttctct
ttggattctt
61 ccaaaatcag agtcagactg ctcccggtgc catgaacgga gacgacgcct
ttgcaaggag
121 acccacggtt ggtgctcaaa taccagagaa gatccaaaag gccttcgatg
atattgccaa
181 atacttctct aaggaagagt gggaaaagat gaaagcctcg gagaaaatct
tctatgtgta
241 tatgaagaga aagtatgagg ctatgactaa actaggtttc aaggccaccc
tcccaccttt
301 catgtgtaat aaacgggccg aagacttcca ggggaatgat ttggataatg
accctaaccg
361 tgggaatcag gttgaacgtc ctcagatgac tttcggcagg ctccagggaa
tctccccgaa
421 gatcatgccc aagaagccag cagaggaagg aaatgattcg gaggaagtgc
cagaagcatc
481 tggcccacaa aatgatggga aagagctgtg cccccgggga aaaccaacta
cctctgagaa
541 gattcacgag agatctggac ccaaaagggg ggaacatgcc tggacccaca
gactgcgtga
601 gagaaaacag ctggtgattt atgaagagat cagcgaccct gaggaagatg
acgagtaact
661 cccctcaggg atacgacaca tgcccatgat gagaagcaga acgtggtgac
ctttcacgaa
721 catgggcatg gctgcggacc cctcgtcatc aggtgcatag caagtg

Homo sapiens folate hydrolase (prostate-specific membrane antigen) 1 (FOLH1), mRNA.
ACCESSION NM_004476
VERSION NM_004476.1 GI:4758397

/translation="MWNLLHETDSAVATARRPRWLCAGALVLAGGFFLLGFLFGWFIK

SSNEATNITPKHNMKAFLDELKAENIKKFLYNFTQIPHLAGTEQNFQLAKQIQSQWKE

FGLDSVELAHYDVLLSYPNKTHPNYISIINEDGNEIFNTSLFEPPPPGYENVSDIVPP

FSAFSPQGMPEGDLVYVNYARTEDFFKLERDMKINCSGKIVIARYGKVFRGNKVKNAQ

LAGAKGVILYSDPADYFAPGVKSYPDGWNLPGGGVQRGNILNLNGAGDPLTPGYPANE
YAYRRGIAEAVGLPSIPVHPIGYYDAQKLLEKMGGSAPPDSSWRGSLKVPYNVGPGFT
GNFSTQKVKMHIHSTNEVTRIYNVIGTLRGAVEPDRYVILGGHRDSWVFGGIDPQSGA
AVVHEIVRSFGTLKKEGWRPRRTILFASWDAEEFGLLGSTEWAEENSRLLQERGVAYI
NADSSIEGNYTLRVDCTPLMYSLVHNLTKELKSPDEGFEGKSLYESWTKKSPSPEFSG
MPRISKLGSGNDFEVFFQRLGIASGRARYTKNWETNKFSGYPLYHSVYETYELVEKFY
DPMFKYHLTVAQVRGGMVFELANSIVLPFDCRDYAVVLRKYADKIYSISMKHPQEMKT
YSVSFDSLFSAVKNFTEIASKFSERLQDFDKSNPIVLRMMNDQLMFLERAFIDPLGLP
DRPFYRHVIYAPSSHNKYAGESFPGIYDALFDIESKVDPSKAWGEVKRQIYVAAFTVQ
AAAETLSEVA"

### SEQ ID NO 7 ORIGIN

1 ctcaaaaggg gccggatttc cttctcctgg aggcagatgt tgcctctctc
tctcgctcgg
61 attggttcag tgcactctag aaacactgct gtggtggaga aactggaccc
caggtctgga
121 gcgaattcca gcctgcaggg ctgataagcg aggcattagt gagattgaga
gagactttac
181 cccgccgtgg tggttggagg gcgcgcagta gagcagcagc acaggcgcgg
gtcccgggag
241 gccggctctg ctcgcgccga gatgtggaat ctccttcacg aaaccgactc
ggctgtggcc
301 accgcgcgcc gcccgcgctg gctgtgcgct ggggcgctgg tgctggcggg
tggcttcttt
361 ctcctcggct tcctcttcgg gtggtttata aaatcctcca atgaagctac
taacattact
421 ccaaagcata atatgaaagc atttttggat gaattgaaag ctgagaacat
caagaagttc
481 ttatataatt ttacacagat accacattta gcaggaacag aacaaaactt
tcagcttgca
541 aagcaaattc aatcccagtg gaaagaattt ggcctggatt ctgttgagct
agcacattat
601 gatgtcctgt tgtcctaccc aaataagact catcccaact acatctcaat
aattaatgaa
661 gatggaaatg agattttcaa cacatcatta tttgaaccac ctcctccagg
atatgaaaat
721 gtttcggata ttgtaccacc tttcagtgct ttctctcctc aaggaatgcc
agagggcgat
781 ctagtgtatg ttaactatgc acgaactgaa gacttcttta aattggaacg
ggacatgaaa
841 atcaattgct ctgggaaaat tgtaattgcc agatatggga aagttttcag
aggaaataag

901 gttaaaaatg cccagctggc aggggccaaa ggagtcattc tctactccga
ccctgctgac
961 tactttgctc ctggggtgaa gtcctatcca gatggttgga atcttcctgg
aggtggtgtc
1021 cagcgtggaa atatcctaaa tctgaatggt gcaggagacc ctctcacacc
aggttaccca
1081 gcaaatgaat atgcttatag gcgtggaatt gcagaggctg ttggtcttcc
aagtattcct
1141 gttcatccaa ttggatacta tgatgcacag aagctcctag aaaaaatggg
tggctcagca
1201 ccaccagata gcagctggag aggaagtctc aaagtgccct acaatgttgg
acctggcttt
1261 actggaaact tttctacaca aaaagtcaag atgcacatcc actctaccaa
tgaagtgaca
1321 agaatttaca atgtgatagg tactctcaga ggagcagtgg aaccagacag
atatgtcatt
1381 ctgggaggtc accgggactc atgggtgttt ggtggtattg accctcagag
tggagcagct
1441 gttgttcatg aaattgtgag gagctttgga acactgaaaa aggaagggtg
gagacctaga
1501 agaacaattt tgtttgcaag ctgggatgca gaagaatttg gtcttcttgg
ttctactgag
1561 tgggcagagg agaattcaag actccttcaa gagcgtggcg tggcttatat
taatgctgac
1621 tcatctatag aaggaaacta cactctgaga gttgattgta caccgctgat
gtacagcttg
1681 gtacacaacc taacaaaaga gctgaaaagc cctgatgaag gctttgaagg
caaatctctt
1741 tatgaaagtt ggactaaaaa aagtccttcc ccagagttca gtggcatgcc
caggataagc
1801 aaattgggat ctggaaatga ttttgaggtg ttcttccaac gacttggaat
tgcttcaggc
1861 agagcacggt atactaaaaa ttgggaaaca aacaaattca gcggctatcc
actgtatcac
1921 agtgtctatg aaacatatga gttggtggaa aagttttatg atccaatgtt
taaatatcac
1981 ctcactgtgg cccaggttcg aggagggatg gtgtttgagc tagccaattc
catagtgctc
2041 ccttttgatt gtcgagatta tgctgtagtt ttaagaaagt atgctgacaa
aatctacagt
2101 atttctatga aacatccaca ggaaatgaag acatacagtg tatcatttga
ttcacttttt
2161 tctgcagtaa agaattttac agaaattgct tccaagttca gtgagagact
ccaggacttt
2221 gacaaaagca acccaatagt attaagaatg atgaatgatc aactcatgtt
tctggaaaga
2281 gcatttattg atccattagg gttaccagac aggccttttt ataggcatgt
catctatgct
2341 ccaagcagcc acaacaagta tgcaggggag tcattcccag gaatttatga
tgctctgttt
2401 gatattgaaa gcaaagtgga cccttccaag gcctggggag aagtgaagag
acagatttat

2461 gttgcagcct tcacagtgca ggcagctgca gagactttga gtgaagtagc
ctaagaggat
2521 tctttagaga atccgtattg aatttgtgtg gtatgtcact cagaaagaat
cgtaatgggt
2581 atattgataa attttaaaat tggtatattt gaaataaagt tgaatattat
atataaaaaa
2641 aaaaaaaaaa aaa

Human melanocyte-specific (pmel 17) gene, exons 2-5, and complete cds.
ACCESSION U20093
VERSION U20093.1 GI:1142634

### SEQ ID NO 70

/translation="









"

### SEQ ID NO 80

### ORIGIN

1 gtgctaaaaa gatgccttct tcatttggct gtgataggtg ctttgtggct
gtgggggcta
61 caaaagtacc cagaaaccag gactggcttg gtgtctcaag gcaactcaga
accaaagcct
121 ggaacaggca gctgtatcca gagtggacag aagcccagag acttgactgc
tggagaggtg
181 gtcaagtgtc cctcaaggtc agtaatgatg ggcctacact gattggtgca
aatgcctcct
241 tctctattgc cttgaacttc cctggaagcc aaaaggtatt gccagatggg
caggttatct
301 gggtcaacaa taccatcatc aatgggagcc aggtgtgggg aggacagcca
gtgtatcccc
361 aggaaactga cgatgcctgc atcttccctg atggtggacc ttgcccatct
ggctcttggt
421 ctcagaagag aagctttgtt tatgtctgga agacctgggg tgagggactc
ccttctcagc
481 ctatcatcca cacttgtgtt tacttctttc tacctgatca cctttctttt
ggccgcccct
541 tccaccttaa cttctgtgat tttctctaat cttcattttc ctcttagatc
ttttctcttt
601 cttagcacct agcccccttc aagctctatc ataattcttt ctggcaactc
ttggcctcaa

661 ttgtagtcct accccatgga atgcctcatt aggacccctt ccctgtcccc
ccatatcaca
721 gccttccaaa caccctcaga agtaatcata cttcctgacc tcccatctcc
agtgccgttt
781 cgaagcctgt ccctcagtcc cctttgacca gtaatctctt cttccttgct
tttcattcca
841 aaaatgcttc aggccaatac tggcaagttc taggggggcc agtgtctggg
ctgagcattg
901 ggacaggcag ggcaatgctg ggcacacaca ccatggaagt gactgtctac
catcgccggg
961 gatcccggag ctatgtgcct cttgctcatt ccagctcagc cttcaccatt
actggtaagg
1021 gttcaggaag ggcaaggcca gttgtagggc aaagagaagg cagggaggct
tggatggact
1081 gcaaaggaga aaggtgaaat gctgtgcaaa cttaaagtag aagggccagg
aagacctagg
1141 cagagaaatg tgaggcttag tgccagtgaa gggccagcca gtcagcttgg
agttggaggg
1201 tgtggctgtg aaaggagaag ctgtggctca ggcctggttc tcaccttttc
tggctccaat
1261 cccagaccag gtgcctttct ccgtgagcgt gtcccagttg cgggccttgg
atggagggaa
1321 caagcacttc ctgagaaatc agcctctgac ctttgccctc cagctccatg
accccagtgg
1381 ctatctggct gaagctgacc tctcctacac ctgggacttt ggagacagta
gtggaaccct
1441 gatctctcgg gcacctgtgg tcactcatac ttacctggag cctggcccag
tcactgccca
1501 ggtggtcctg caggctgcca ttcctctcac ctcctgtggc tcctccccag
ttccaggcac
1561 cacagatggg cacaggccaa ctgcagaggc ccctaacacc acagctggcc
aagtgcctac
1621 tacagaagtt gtgggtacta cacctggtca ggcgccaact gcagagccct
ctggaaccac
1681 atctgtgcag gtgccaacca ctgaagtcat aagcactgca cctgtgcaga
tgccaactgc
1741 agagagcaca ggtatgacac ctgagaaggt gccagtttca gaggtcatgg
gtaccacact
1801 ggcagagatg tcaactccag aggctacagg tatgacacct gcagaggtat
caattgtggt
1861 gctttctgga accacagctg cacaggtaac aactacagag tgggtggaga
ccacagctag
1921 agagctacct atccctgagc ctgaaggtcc agatgccagc tcaatcatgt
ctacggaaag
1981 tattacaggt tccctgggcc ccctgctgga tggtacagcc accttaaggc
tggtgaagag
2041 acaagtcccc ctggattgtg ttctgtatcg atatggttcc ttttccgtca
ccctggacat
2101 tgtccagggt attgaaagtg ccgagatcct gcaggctgtg ccgtccggtg
aggggggatgc
2161 atttgagctg actgtgtcct gccaaggcgg gctgcccaag gaagcctgca
tggagatctc

2221 atcgccaggg tgccagcccc ctgcccagcg gctgtgccag cctgtgctac
ccagcccagc
2281 ctgccagctg gttctgcacc agatactgaa gggtggctcg gggacatact
gcctcaatgt
2341 gtctctggct gataccaaca gcctggcagt ggtcagcacc cagcttatca
tgcctggtag
2401 gtccttggac agagactaag tgaggaggga agtggataga ggggacagct
ggcaagcagc
2461 agacatgagt gaagcagtgc ctgggattct tctcacaggt caagaagcag
gccttgggca
2521 ggttccgctg atcgtgggca tcttgctggt gttgatggct gtggtccttg
catctctgat
2581 atataggcgc agacttatga agcaagactt ctccgtaccc cagttgccac
atagcagcag
2641 tcactggctg cgtctacccc gcatcttctg ctcttgtccc attggtgaga
atagcccccct
2701 cctcagtggg cagcaggtct gagtactctc atatgatgct gtgattttcc
tggagttgac
2761 agaaacacct atatttcccc cagtcttccc tgggagacta ctattaactg
aaataaa
//

Homo sapiens kallikrein 3, (prostate specific antigen) (KLK3), mRNA.
ACCESSION NM_001648
VERSION NM_001648.1 GI:4502172

### SEQ ID NO 78

/translation="MWVPVVFLTLSVTWIGAAPLILSRIVGGWECEKHSQPWQVLVAS
RGRAVCGGVLVHPQWVLTAAHCIRNKSVILLGRHSLFHPEDTGQVFQVSHSFPHPLYDMSLLKNRF
LRPGDDSSHDLMLLRLSEPAELTDAVKVMDLPTQEPALGTTCYASGWGSIEPEEFLTPKKLQCVDL
HVISNDVCAQVHPQKVTKFMLCAGRWTGGKSTCSGDSGGPLVCNGVLQGITSWGSEPCALPERPSL
YTKVVHYRKWIKDTIVANP"

### SEQ ID NO 86

### ORIGIN

1 agccccaagc ttaccacctg caccoggaga gctgtgtgtc accatgtggg
tccoggttgt
61 cttcctcacc ctgtccgtga cgtggattgg tgctgcaccc ctcatcctgt
ctcggattgt
121 gggaggctgg gagtgcgaga agcattccca accctggcag gtgcttgtgg
cctctcgtgg
181 cagggcagtc tgcggcggtg ttctggtgca cccccagtgg gtcctcacag
ctgcccactg
241 catcaggaac aaaagcgtga tcttgctggg tcggcacagc ctgtttcatc
ctgaagacac
301 aggccaggta tttcaggtca gccacagctt cccacacccg ctctacgata
tgagcctcct
361 gaagaatcga ttcctcaggc caggtgatga ctccagccac gacctcatgc
tgctccgcct

421 gtcagagcct gccgagctca cggatgctgt gaaggtcatg gacctgccca
cccaggagcc
481 agcactgggg accacctgct acgcctcagg ctggggcagc attgaaccag
aggagttctt
541 gaccccaaag aaacttcagt gtgtggacct ccatgttatt tccaatgacg
tgtgtgcgca
601 agttcaccct cagaaggtga ccaagttcat gctgtgtgct ggacgctgga
caggggcaa
661 aagcacctgc tcgggtgatt ctgggggccc acttgtctgt aatggtgtgc
ttcaaggtat
721 cacgtcatgg ggcagtgaac catgtgccct gcccgaaagg ccttccctgt
acaccaaggt
781 ggtgcattac cggaagtgga tcaaggacac catcgtggcc aacccctgag
cacccctatc
841 aaccccctat tgtagtaaac ttggaacctt ggaaatgacc aggccaagac
tcaagcctcc
901 ccagttctac tgacctttgt ccttaggtgt gaggtccagg gttgctagga
aaagaaatca
961 gcagacacag gtgtagacca gagtgtttct taaatggtgt aattttgtcc
tctctgtgtc
1021 ctggggaata ctggccatgc ctggagacat atcactcaat ttctctgagg
acacagatag
1081 gatggggtgt ctgtgttatt tgtggggtac agagatgaaa gaggggtggg
atccacactg
1141 agagagtgga gagtgacatg tgctggacac tgtccatgaa gcactgagca
gaagctggag
1201 gcacaacgca ccagacactc acagcaagga tggagctgaa aacataaccc
actctgtcct
1261 ggaggcactg ggaagcctag agaaggctgt gagccaagga gggagggtct
tcctttggca
1321 tgggatgggg atgaagtaag gagagggact ggaccccctg gaagctgatt
cactatgggg
1381 ggaggtgtat tgaagtcctc cagacaaccc tcagatttga tgatttccta
gtagaactca
1441 cagaaataaa gagctgttat actgtg
//

Human autoimmunogenic cancer/testis antigen NY-ESO-1 mRNA, complete cds.
ACCESSION U87459
VERSION U87459.1 GI:1890098

### SEQ ID NO 74

/translation="MQAEGRGTGGSTGDADGPGGPGIPDGPGGNAGGPGEAG
ATGGRGPRGAGAARASGPGGGAPRGPHGGAASGLNGCCRCGARGPESRLLEF
YLAMPFATPMEAELARRSLAQDAPPLPVPGVLLKEFTVSGNILTIRLTAADH
RQLQLSISSCLQQLSLLMWITQCFLPVFLAQPPSGQRR"

### SEQ ID NO 84

### ORIGIN

1 atcctcgtgg gccctgacct tctctctgag agccgggcag aggctcggga
gccatgcagg
61 ccgaaggccg gggcacaggg ggttcgacgg gcgatgctga tggcccagga
ggccctggca
121 ttcctgatgg cccagggggc aatgctggcg gcccaggaga ggcgggtgcc
acgggcggca
181 gaggtccccg gggcgcaggg gcagcaaggg cctcggggcc gggaggaggc
gccccgcggg
241 gtccgcatgg cggcgcggct tcagggctga atggatgctg cagatgcggg
gccagggggc
301 cggagagccg cctgcttgag ttctacctcg ccatgccttt cgcgacaccc
atggaagcag
361 agctggcccg caggagcctg gcccaggatg ccccaccgct tcccgtgcca
ggggtgcttc
421 tgaaggagtt cactgtgtcc ggcaacatac tgactatccg actgactgct
gcagaccacc
481 gccaactgca gctctccatc agctcctgtc tccagcagct ttccctgttg
atgtggatca
541 cgcagtgctt tctgcccgtg tttttggctc agcctccctc agggcagagg
cgctaagccc
601 agcctggcgc cccttcctag gtcatgcctc ctcccctagg gaatggtccc
agcacgagtg
661 gccagttcat tgtgggggcc tgattgtttg tcgctggagg aggacggctt
acatgtttgt
721 ttctgtagaa aataaaactg agctacgaaa aa
//

LAGE-1a protein [Home sapiens].
ACCESSION CAA11116
PID g3255959
VERSION CAA11116.1 GI:3255959

### SEQ ID NO 75

### ORIGIN

1 mqaegrgtgg stgdadgpgg pgipdgpggn aggpgeagat ggrgprgaga
arasgprgga
61 prgphggaas aqdgrcpcga rrpdsrllel hitmpfsspm eaelvrrils
rdaaplprpg
121 avlkdftvsg nllfirltaa dhrqlqlsis sclqqlsllm witqcflpvf
laqapsgqrr
181
//

LAGE-1b protein [Homo sapiens].
ACCESSION CAA11117
PID g3255960
VERSION CAA11117.1 GI:3255960

### SEQ ID NO 76

### ORIGIN

1 mqaegrgtgg stgdadgpgg pgipdgpggn aggpgeagat ggrgprgaga
arasgprgga
61 prgphggaas aqdgrcpcga rrpdsrllel hitmpfsspm eaelvrrils
rdaaplprpg
121 avlkdftvsg nllfmsvwdq dregagrmrv vgwglgsasp eggkardlrt
pkhkvseqrp
181 gtpgppppeg aqgdgcrgva fnvmfsaphi
//

Human antigen (MAGE-1) gene, complete cds.
ACCESSION M77481
VERSION M77481.1 GI:416114

### SEQ ID NO 71

/translation="MSLEQRSLHCKPEEALEAQQEALGLVCVQAATSSSSPL
VLGTLEEVPTAGSTDPPQSPQGASAFPTTINFTRQRQPSEGSSSREEEGPST
SCILESLFRAVITKKVADLVGFLLLKYRAREPVTKAEMLESVIKNYKHCFPE
IFGKASESLQLVFGIDVKEADPTGHSYVLVTCLGLSYDGLLGDNQIMPKTGF
LIIVLVMIAMEGGHAPEEEIWEELSVMEVYDGREHSAYGEPRKLLTQDLVQE
KYLEYRQVPDSDPARYEFLWGPRALAETSYVKVLEYVIKVSARVRFFFPSLR
EAALREEEEGV"

### SEQ ID NO 81

### ORIGIN

1 ggatccaggc cctgccagga aaaatataag ggccctgcgt gagaacagag
ggggtcatcc
61 actgcatgag agtggggatg tcacagagtc cagcccaccc tcctggtagc
actgagaagc
121 cagggctgtg cttgcggtct gcaccctgag ggcccgtgga ttcctcttcc
tggagctcca
181 ggaaccaggc agtgaggcct tggtctgaga cagtatcctc aggtcacaga
gcagaggatg
241 cacagggtgt gccagcagtg aatgtttgcc ctgaatgcac accaagggcc
ccacctgcca
301 caggacacat aggactccac agagtctggc ctcacctccc tactgtcagt
cctgtagaat
361 cgacctctgc tggccggctg taccctgagt accctctcac ttcctccttc
aggttttcag
421 gggacaggcc aacccagagg acaggattcc ctggaggcca cagaggagca
ccaaggagaa
481 gatctgtaag taggcctttg ttagagtctc caaggttcag ttctcagctg
aggcctctca
541 cacactccct ctctccccag gcctgtgggt cttcattgcc cagctcctgc
ccacactcct
601 gcctgctgcc ctgacgagag tcatcatgtc tcttgagcag aggagtctgc
actgcaagcc
661 tgaggaagcc cttgaggccc aacaagaggc cctgggcctg gtgtgtgtgc
aggctgccac
721 ctcctcctcc tctcctctgg tcctgggcac cctggaggag gtgcccactg
ctgggtcaac

781 agatcctccc cagagtcctc agggagcctc cgcctttccc actaccatca
acttcactcg
841 acagaggcaa cccagtgagg gttccagcag ccgtgaagag gaggggccaa
gcacctcttg
901 tatcctggag tccttgttcc gagcagtaat cactaagaag gtggctgatt
tggttggttt
961 tctgctcctc aaatatcgag ccagggagcc agtcacaaag gcagaaatgc
tggagagtgt
1021 catcaaaaat tacaagcact gttttcctga gatcttcggc aaagcctctg
agtccttgca
1081 gctggtcttt ggcattgacg tgaaggaagc agaccccacc ggccactcct
atgtccttgt
1141 cacctgccta ggtctctcct atgatggcct gctgggtgat aatcagatca
tgcccaagac
1201 aggcttcctg ataattgtcc tggtcatgat tgcaatggag ggcggccatg
ctcctgagga
1261 ggaaatctgg gaggagctga gtgtgatgga ggtgtatgat gggagggagc
acagtgccta
1321 tggggagccc aggaagctgc tcacccaaga tttggtgcag gaaaagtacc
tggagtaccg
1381 gcaggtgccg gacagtgatc ccgcacgcta tgagttcctg tggggtccaa
gggccctcgc
1441 tgaaaccagc tatgtgaaag tccttgagta tgtgatcaag gtcagtgcaa
gagttcgctt
1501 tttcttccca tccctgcgtg aagcagcttt gagagaggag gaagagggag
tctgagcatg
1561 agttgcagcc aaggccagtg ggagggggac tgggccagtg caccttccag
ggccgcgtcc
1621 agcagcttcc cctgcctcgt gtgacatgag gcccattctt cactctgaag
agagcggtca
1681 gtgttctcag tagtaggttt ctgttctatt gggtgacttg gagatttatc
tttgttctct
1741 tttggaattg ttcaaatgtt tttttttaag ggatggttga atgaacttca
gcatccaagt
1801 ttatgaatga cagcagtcac acagttctgt gtatatagtt taagggtaag
agtcttgtgt
1861 tttattcaga ttgggaaatc cattctattt tgtgaattgg gataataaca
gcagtggaat
1921 aagtacttag aaatgtgaaa aatgagcagt aaaatagatg agataaagaa
ctaaagaaat
1981 taagagatag tcaattcttg ccttatacct cagtctattc tgtaaaattt
ttaaagatat
2041 atgcatacct ggatttcctt ggcttctttg agaatgtaag agaaattaaa
tctgaataaa
2101 gaattcttcc tgttcactgg ctcttttctt ctccatgcac tgagcatctg
ctttttggaa
2161 ggccctgggt tagtagtgga gatgctaagg taagccagac tcatacccac
ccatagggtc
2221 gtagagtcta ggagctgcag tcacgtaatc gaggtggcaa gatgtcctct
aaagatgtag
2281 ggaaaagtga gagaggggtg agggtgtggg gctccgggtg agagtggtgg
agtgtcaatg

2341 ccctgagctg gggcattttg ggctttggga aactgcagtt ccttctgggg
gagctgattg
2401 taatgatctt gggtggatcc
//

Human MAGE-2 gene exons 1-4, complete cds.
ACCESSION L18920
VERSION L18320.1 GI:436180

### SEQ ID NO 72

/translation="MPLEQRSQHCKPEEGLEARGEALGLVGAQAPATEEQQTASSSSTLVEVT
LGEVPAADSPSPPHSPQGASSFSTTINYTLWRQSDEGSSNQEEEGPRMFPDLE
SEFQAAISRKMVELVHFLLLKYRAREPVTKAEMLESVLRNCQDFFPVIFSKASEYLQLVFGIE
VVEVVPISHLYILVTCLGLSYDGLLGDNQVMPKTGLLIIVLAIIAIEGDCAPEEKIWEELSML
EVFEGREDSVFAHPRKLLMQDLVQENYLEYRQVPGSDPACYEFLWGPRALIETSYVKVLHHTL
KIGGEPHISYPPLHERALREGEE"

### SEQ ID NO 82

### ORIGIN

1 attccttcat caaacagcca ggagtgagga agaggaccct cctgagtgag
gactgaggat
61 ccaccctcac cacatagtgg gaccacagaa tccagctcag cccctcttgt
cagccctggt
121 acacactggc aatgatctca ccccgagcac acccctcccc ccaatgccac
ttcgggccga
181 ctcagagtca gagacttggt ctgaggggag cagacacaat cggcagagga
tggcggtcca
241 ggctcagtct ggcatccaag tcaggacctt gagggatgac caaaggcccc
tcccaccccc
301 aactcccccg accccaccag gatctacagc ctcaggatcc ccgtcccaat
ccctacccct
361 acaccaacac catcttcatg cttaccccca cccccccatc cagatcccca
tccgggcaga
421 atccggttcc acccttgccg tgaacccagg gaagtcacgg gcccggatgt
gacgccactg
481 acttgcacat tggaggtcag aggacagcga gattctcgcc ctgagcaacg
gcctgacgtc
541 ggcggaggga agcaggcgca ggctccgtga ggaggcaagg taagacgccg
agggaggact
601 gaggcgggcc tcaccccaga cagagggccc ccaataatcc agcgctgcct
ctgctgccgg
661 gcctggacca ccctgcaggg gaagacttct caggctcagt cgccaccacc
tcaccccgcc
721 accccccgcc gctttaaccg cagggaactc tggcgtaaga gctttgtgtg
accagggcag
781 ggctggttag aagtgctcag ggcccagact cagccaggaa tcaaggtcag
gaccccaaga
841 ggggactgag ggcaacccac cccctaccct cactaccaat cccatccccc
aacaccaacc
901 ccacccccat ccctcaaaca ccaaccccac ccccaaaccc cattcccatc
tcctccccca

961 ccaccatcct ggcagaatcc ggctttgccc ctgcaatcaa cccacggaag
ctccgggaat
1021 ggcggccaag cacgcggatc ctgacgttca catgtacggc taagggaggg
aaggggttgg
1081 gtctcgtgag tatggccttt gggatgcaga ggaagggccc aggcctcctg
gaagacagtg
1141 gagtccttag gggacccagc atgccaggac agggggccca ctgtacccct
gtctcaaact
1201 gagccacctt ttcattcagc cgagggaatc ctagggatgc agacccactt
cagcaggggg
1261 ttggggccca gcctgcgagg agtcaagggg aggaagaaga gggaggactg
aggggacctt
1321 ggagtccaga tcagtggcaa ccttgggctg ggggatcctg ggcacagtgg
ccgaatgtgc
1381 cccgtgctca ttgcaccttc agggtgacag agagttgagg gctgtggtct
gagggctggg
1441 acttcaggtc agcagaggga ggaatcccag gatctgccgg acccaaggtg
tgcccccttc
1501 atgaggactg gggatacccc cggcccagaa agaagggatg ccacagagtc
tggaagtccc
1561 ttgttcttag ctctggggga acctgatcag ggatggccct aagtgacaat
ctcatttgta
1621 ccacaggcag gaggttgggg aaccctcagg gagataaggt gttggtgtaa
agaggagctg
1681 tctgctcatt tcagggggtt gggggttgag aaagggcagt ccctggcagg
agtaaagatg
1741 agtaacccac aggaggccat cataacgttc accctagaac caaaggggtc
agccctggac
1801 aacgcacgtg ggggtaacag gatgtggccc ctcctcactt gtctttccag
atctcaggga
1861 gttgatgacc ttgttttcag aaggtgactc aggtcaacac aggggcccca
tctggtcgac
1921 agatgcagtg gttctaggat ctgccaagca tccaggtgga gagcctgagg
taggattgag
1981 ggtacccctg ggccagaatg cagcaagggg gccccataga aatctgccct
gcccctgcgg
2041 ttacttcaga gaccctgggc agggctgtca gctgaagtcc ctccattatc
ctgggatctt
2101 tgatgtcagg gaaggggagg ccttggtctg aaggggctgg agtcaggtca
gtagagggag
2161 ggtctcaggc cctgccagga gtggacgtga ggaccaagcg gactcgtcac
ccaggacacc
2221 tggactccaa tgaatttgga catctctcgt tgtccttcgc gggaggacct
ggtcacgtat
2281 ggccagatgt gggtcccctc atatccttct gtaccatatc agggatgtga
gttcttgaca
2341 tgagagattc tcaagccagc aaaagggtgg gattaggccc tacaaggaga
aaggtgaggg
2401 ccctgagtga gcacagaggg gaccctccac ccaagtagag tggggacctc
acggagtctg
2461 gccaaccctg ctgagacttc tgggaatccg tggctgtgct tgcagtctgc
acactgaagg

2521 cccgtgcatt cctctcccag gaatcaggag ctccaggaac caggcagtga
ggccttggtc
2581 tgagtcagtg tcctcaggtc acagagcaga ggggacgcag acagtgccaa
cactgaaggt
2641 ttgcctggaa tgcacaccaa gggccccacc cgcccagaac aaatgggact
ccagagggcc
2701 tggcctcacc ctccctattc tcagtcctgc agcctgagca tgtgctggcc
ggctgtaccc
2761 tgaggtgccc tcccacttcc tccttcaggt tctgaggggg acaggctgac
aagtaggacc
2821 cgaggcactg gaggagcatt gaaggagaag atctgtaagt aagcctttgt
cagagcctcc
2881 aaggttcagt tcagttctca cctaaggcct cacacacgct ccttctctcc
ccaggcctgt
2941 gggtcttcat tgcccagctc ctgcccgcac tcctgcctgc tgccctgacc
agagtcatca
3001 tgcctcttga gcagaggagt cagcactgca agcctgaaga aggccttgag
gcccgaggag
3061 aggccctggg cctggtgggt gcgcaggctc ctgctactga ggagcagcag
accgcttctt
3121 cctcttctac tctagtggaa gttaccctgg gggaggtgcc tgctgccgac
tcaccgagtc
3181 ctccccacag tcctcaggga gcctccagct tctcgactac catcaactac
actctttgga
3241 gacaatccga tgagggctcc agcaaccaag aagaggaggg gccaagaatg
tttcccgacc
3301 tggagtccga gttccaagca gcaatcagta ggaagatggt tgagttggtt
cattttctgc
3361 tcctcaagta tcgagccagg gagccggtca caaaggcaga aatgctggag
agtgtcctca
3421 gaaattgcca ggacttcttt cccgtgatct tcagcaaagc ctccgagtac
ttgcagctgg
3481 tctttggcat cgaggtggtg gaagtggtcc ccatcagcca cttgtacatc
cttgtcacct
3541 gcctgggcct ctcctacgat ggcctgctgg gcgacaatca ggtcatgccc
aagacaggcc
3601 tcctgataat cgtcctggcc ataatcgcaa tagagggcga ctgtgcccct
gaggagaaaa
3661 tctgggagga gctgagtatg ttggaggtgt ttgaggggag ggaggacagt
gtcttcgcac
3721 atcccaggaa gctgctcatg caagatctgg tgcaggaaaa ctacctggag
taccggcagg
3781 tgcccggcag tgatcctgca tgctacgagt tcctgtgggg tccaagggcc
ctcattgaaa
3841 ccagctatgt gaaagtcctg caccatacac taaagatcgg tggagaacct
cacatttcct
3901 acccacccct gcatgaacgg gctttgagag agggagaaga gtgagtctca
gcacatgttg
3961 cagccagggc cagtgggagg gggtctgggc cagtgcacct tccagggccc
catccattag
4021 cttccactgc ctcgtgtgat atgaggccca ttcctgcctc tttgaagaga
gcagtcagca

4081 ttcttagcag tgagtttctg ttctgttgga tgactttgag atttatcttt
ctttcctgtt
4141 ggaattgttc aaatgttcct tttaacaaat ggttggatga acttcagcat
ccaagtttat
4201 gaatgacagt agtcacacat agtgctgttt atatagttta ggggtaagag
tcctgttttt
4261 tattcagatt gggaaatcca ttccattttg tgagttgtca cataataaca
gcagtggaat
4321 atgtatttgc ctatattgtg aacgaattag cagtaaaata catgatacaa
ggaactcaaa
4381 agatagttaa ttcttgcctt atacctcagt ctattatgta aaattaaaaa
tatgtgtatg
4441 tttttgcttc tttgagaatg caaaagaaat taaatctgaa taaattcttc
ctgttcactg
4501 gctcatttct ttaccattca ctcagcatct gctctgtgga aggccctggt
agtagtggg
//

Human MAGE-3 antigen (MAGE-3) gene, complete cds.
ACCESSION U03735
VERSION U03735.1 GI:468825

### SEQ ID NO 73

/translation="MPLEQRSQHCKPEEGLEARGEALGLVGAQAPATEEQEAASSSSSTLVEVTLGE
VPAAESPDPPQSPQGASSLPTTMNYPLWSQSYEDSSNQEEEGPSTFPDLESEFQAALSRKVAELVH
FLLLKYRAREPVTKAEMLGSVVGNWQYFFPVIFSKASSSLQLVFGIELMEVDPIGHLYIFATCLGL
SYDGLLGDNQIMPKAGLLIIVLAIIAREGDCAPEEKIWEELSVLEVFEGREDSILGDPKKLLTQHF
VQENYLEYRQVPGSDPACYEFLWGPRALVETSYVKVLHHMVKISGGPHISYPPLHEWVLREGEE"

### SEQ ID NO 83

### ORIGIN

1 acgcaggcag tgatgtcacc cagaccacac cccttccccc aatgccactt
cagggggtac
61 tcagagtcag agacttggtc tgaggggagc agaagcaatc tgcagaggat
ggcggtccag
121 gctcagccag gcatcaactt caggaccctg agggatgacc gaaggccccg
cccacccacc
181 cccaactccc ccgaccccac caggatctac agcctcagga cccccgtccc
aatccttacc
241 ccttgcccca tcaccatctt catgcttacc tccaccccca tccgatcccc
atccaggcag
301 aatccagttc cacccctgcc cggaacccag ggtagtaccg ttgccaggat
gtgacgccac
361 tgacttgcgc attggaggtc agaagaccgc gagattctcg ccctgagcaa
cgagcgacgg
421 cctgacgtcg gcggagggaa gccggcccag gctcggtgag gaggcaaggt
aagacgctga
481 gggaggactg aggcgggcct cacctcagac agagggcctc aaataatcca
gtgctgcctc
541 tgctgccggg cctgggccac cccgcagggg aagacttcca ggctgggtcg
ccactacctc

601 accccgccga cccccgccgc tttagccacg gggaactctg gggacagagc
ttaatgtggc
661 cagggcaggg ctggttagaa gaggtcaggg cccacgctgt ggcaggaatc
aaggtcagga
721 ccccgagagg gaactgaggg cagcctaacc accaccctca ccaccattcc
cgtcccccaa
781 caccccaacc caccccccatc ccccattccc atccccaccc ccacccctat
cctggcagaa
841 tccgggcttt gcccctggta tcaagtcacg gaagctccgg gaatggcggc
caggcacgtg
901 agtcctgagg ttcacatcta cggctaaggg agggaagggg ttcggtatcg
cgagtatggc
961 cgttgggagg cagcgaaagg gcccaggcct cctggaagac agtggagtcc
tgaggggacc
1021 cagcatgcca ggacaggggg cccactgtac ccctgtctca aaccgaggca
cctttttcatt
1081 cggctacggg aatcctaggg atgcagaccc acttcagcag gggttgggg
cccagccctg
1141 cgaggagtca tggggaggaa gaagagggag gactgagggg accttggagt
ccagatcagt
1201 ggcaaccttg ggctggggga tgctgggcac agtggccaaa tgtgctctgt
gctcattgcg
1261 ccttcagggt gaccagagag ttgagggctg tggtctgaag agtgggactt
caggtcagca
1321 gagggaggaa tcccaggatc tgcagggccc aaggtgtacc cccaaggggc
ccctatgtgg
1381 tggacagatg cagtggtcct aggatctgcc aagcatccag gtgaagagac
tgagggagga
1441 ttgagggtac ccctgggaca gaatgcggac tgggggcccc ataaaaatct
gccctgctcc
1501 tgctgttacc tcagagagcc tgggcagggc tgtcagctga ggtccctcca
ttatcctagg
1561 atcactgatg tcagggaagg ggaagccttg gtctgagggg gctgcactca
gggcagtaga
1621 gggaggctct cagaccctac taggagtgga ggtgaggacc aagcagtctc
ctcacccagg
1681 gtacatggac ttcaataaat ttggacatct ctcgttgtcc tttccgggag
gacctgggaa
1741 tgtatggcca gatgtgggtc ccctcatgtt tttctgtacc atatcaggta
tgtgagttct
1801 tgacatgaga gattctcagg ccagcagaag ggagggatta ggccctataa
ggagaaaggt
1861 gagggccctg agtgagcaca gaggggatcc tccaccccag tagagtgggg
acctcacaga
1921 gtctggccaa ccctcctgac agttctggga atccgtggct gcgtttgctg
tctgcacatt
1981 gggggcccgt ggattcctct cccaggaatc aggagctcca ggaacaaggc
agtgaggact
2041 tggtctgagg cagtgtcctc aggtcacaga gtagaggggg ctcagatagt
gccaacggtg
2101 aaggtttgcc ttggattcaa accaagggcc ccacctgccc cagaacacat
ggactccaga

2161 gcgcctggcc tcaccctcaa tactttcagt cctgcagcct cagcatgcgc
tggccggatg
2221 taccctgagg tgccctctca cttcctcctt caggttctga ggggacaggc
tgacctggag
2281 gaccagaggc ccccggagga gcactgaagg agaagatctg taagtaagcc
tttgttagag
2341 cctccaaggt tccattcagt actcagctga ggtctctcac atgctccctc
tctccccagg
2401 ccagtgggtc tccattgccc agctcctgcc cacactcccg cctgttgccc
tgaccagagt
2461 catcatgcct cttgagcaga ggagtcagca ctgcaagcct gaagaaggcc
ttgaggcccg
2521 aggagaggcc ctgggcctgg tgggtgcgca ggctcctgct actgaggagc
aggaggctgc
2581 ctcctcctct tctactctag ttgaagtcac cctgggggag gtgcctgctg
ccgagtcacc
2641 agatcctccc cagagtcctc agggagcctc cagcctcccc actaccatga
actaccctct
2701 ctggagccaa tcctatgagg actccagcaa ccaagaagag gaggggccaa
gcaccttccc
2761 tgacctggag tccgagttcc aagcagcact cagtaggaag gtggccgagt
tggttcattt
2821 tctgctcctc aagtatcgag ccagggagcc ggtcacaaag gcagaaatgc
tggggagtgt
2881 cgtcggaaat tggcagtatt tctttcctgt gatcttcagc aaagcttcca
gttccttgca
2941 gctggtcttt ggcatcgagc tgatggaagt ggaccccatc ggccacttgt
acatctttgc
3001 cacctgcctg ggcctctcct acgatggcct gctgggtgac aatcagatca
tgcccaaggc
3061 aggcctcctg ataatcgtcc tggccataat cgcaagagag ggcgactgtg
cccctgagga
3121 gaaaatctgg gaggagctga gtgtgttaga ggtgtttgag gggagggaag
acagtatctt
3181 gggggatccc aagaagctgc tcacccaaca tttcgtgcag gaaaactacc
tggagtaccg
3241 gcaggtcccc ggcagtgatc ctgcatgtta tgaattcctg tggggtccaa
gggccctcgt
3301 tgaaaccagc tatgtgaaag tcctgcacca tatggtaaag atcagtggag
gacctcacat
3361 ttcctaccca cccctgcatg agtgggtttt gagagagggg gaagagtgag
tctgagcacg
3421 agttgcagcc agggccagtg ggagggggtc tgggccagtg caccttccgg
ggccgcatcc
3481 cttagtttcc actgcctcct gtgacgtgag gcccattctt cactctttga
agcgagcagt
3541 cagcattctt agtagtgggt ttctgttctg ttggatgact ttgagattat
tctttgtttc
3601 ctgttggagt tgttcaaatg ttccttttaa cggatggttg aatgagcgtc
agcatccagg
3661 tttatgaatg acagtagtca cacatagtgc tgtttatata gtttaggagt
aagagtcttg

3721 ttttttactc aaattgggaa atccattcca ttttgtgaat tgtgacataa
taatagcagt
3781 ggtaaaagta tttgcttaaa attgtgagcg aattagcaat aacatacatg
agataactca
3841 agaaatcaaa agatagttga ttcttgcctt gtacctcaat ctattctgta
aaattaaaca
3901 aatatgcaaa ccaggatttc cttgacttct ttgagaatgc aagcgaaatt
aaatctgaat
3961 aaataattct tcctcttcac tggctcgttt cttttccgtt cactcagcat
ctgctctgtg
4021 ggaggccctg ggttagtagt ggggatgcta aggtaagcca gactcacgcc
tacccatagg
4081 gctgtagagc ctaggacctg cagtcatata attaaggtgg tgagaagtcc
tgtaagatgt
4141 agaggaaatg taagagaggg gtgagggtgt ggcgctccgg gtgagagtag
tggagtgtca
4201 gtgc
//

Homo sapiens prostate stem cell antigen (PSCA) mRNA, complete cds.
ACCESSION AF043498
VERSION AF043498.1 GI:2909843

### SEQ ID NO 79

/translation="MKAVLLALLMAGLALQPGTALLCYSCKAQVSNEDCLQVENCTQLGEQCW
TARIRAVGLLTVISKGCSLNCVDDSQDYYVGKKNITCCDTDLCNASGAHALQPAAAILALLPA
LGLLLWGPGQL"

### SEQ ID NO 87

### ORIGIN

1 agggagaggc agtgaccatg aaggctgtgc tgcttgccct gttgatggca
ggcttggccc
61 tgcagccagg cactgccctg ctgtgctact cctgcaaagc ccaggtgagc
aacgaggact
121 gcctgcaggt ggagaactgc acccagctgg gggagcagtg ctggaccgcg
cgcatccgcg
181 cagttggcct cctgaccgtc atcagcaaag gctgcagctt gaactgcgtg
gatgactcac
241 aggactacta cgtgggcaag aagaacatca cgtgctgtga caccgacttg
tgcaacgcca
301 gcggggccca tgccctgcag ccggctgccg ccatccttgc gctgctccct
gcactcggcc
361 tgctgctctg gggacccggc cagctatagg ctctgggggg ccccgctgca
gcccacactg
421 ggtgtggtgc cccaggcctt tgtgccactc ctcacagaac ctggcccagt
gggagcctgt
481 cctggttcct gaggcacatc ctaacgcaag tttgaccatg tatgtttgca
cccctttcc
541 ccnaaccctg accttcccat gggccttttc caggattccn accnggcaga
tcagttttag

601 tganacanat ccgcntgcag atggcccctc caaccntttn tgttgntgtt
tccatggccc
661 agcattttcc acccttaacc ctgtgttcag gcacttnttc ccccaggaag
ccttccctgc
721 ccaccccatt tatgaattga gccaggtttg gtccgtggtg tcccccgcac
ccagcagggg
781 acaggcaatc aggagggccc agtaaaggct gagatgaagt ggactgagta
gaactggagg
841 acaagagttg acgtgagttc ctgggagttt ccagagatgg ggcctggagg
cctggaggaa
901 ggggccaggc ctcacatttg tggggntccc gaatggcagc ctgagcacag
cgtaggccct
961 taataaacac ctgttggata agccaaaaaa
//

GLANDULAR KALLIKREIN 1 PRECURSOR (TISSUE KALLIKREIN)
(KIDNEY/PANCREAS/SALIVARY GLAND KALLIKREIN).
ACCESSION P06870
PID g125170
VERSION P06870 GI:125170

### SEQ ID NO 600

### ORIGIN

1 

61 

121 

181 

241 
//

ELASTASE 2A PRECURSOR.
ACCESSION P08217
PID g119255
VERSION P08217 GI:119255

### SEQ ID NO 601

### ORIGIN

1 

61 

121 

181 

241 

pancreatic elastase IIB [Homo sapiens].
ACCESSION NP_056933
PID g7705648
VERSION NP_056933.1 GI:7705648

### SEQ ID NO 602

### ORIGIN

1 mirtlllstl vagalscgvs tyapdmsrml ggeearpnsw pwqvslqyss
ngqwyhtcgg
61 slianswvlt aahcisssri yrvmlgqhnl yvaesgslav svskivvhkd
wnsnqvskgn
121 diallklanp vsltdkiqla clppagtilp nnypcyvtgw grlqtngalp
ddlkqgrllv
181 vdyatcsssg wwgstvktnm icaggdgvic tcngdsggpl ncqasdgrwe
vhgigsltsv
241 lgcnyyykps iftrvsnynd winsviann
//

PRAME Homo sapiens preferentially expressed antigen in melanoma
(PRAME), mRNA,
ACCESSION NM_006115
VERSION NM_06115.1 GI:5174640

### SEQ ID NO 77

/translation="MERRRLWGSIQSRYISMSVWTSPRRLVELAGQSLLKDEALAIAALELLPREL
FPPLFMAAFDGRHSQTLKAMVQAWPFTCLPLGVLMKGQHLHLETFKAVLDGLDVLLAQEVRPRRWK
LQVLDLRKNSHQDFWTVWSGNRASLYSFPEPEAAQPMTKKRKVDGLSTEAEQPFIPVEVLVDLFLK
EGACDELFSYLIEKVKRKKNVLRLCCKKLKIFAMPMQDIKMILKMVQLDSIEDLEVTCTWKLPTLA
KFSPYLGQMINLRRLLLSHIHASSYISPEKEEQYIAQFTSQFLSLQCLQALYVDSLFFLRGRLDQL
LRHVMNPLETLSITNCRLSEGDVMHLSQSPSVSQLSVLSLSGVMLTDVSPEPLQALLERASATLQD
LVFDECGITDDQLLALLPSLSHCSQLTTLSFYGNSISISALQSLLQHLIGLSNLTHVLYPVPLESY
EDIHGTLHLERLAYLHARLRELLCELGRPSMVWLSANPCPHCGDRTFYDPEPILCPCFMPN"

### SEQ ID NO 85

### ORIGIN

1 gcttcagggt acagctcccc cgcagccaga agccgggcct gcagcccctc
agcaccgctc
61 cgggacaccc caccegcttc ccaggcgtga cctgtcaaca gcaacttcgc
ggtgtggtga
121 actctctgag gaaaaaccat tttgattatt actctcagac gtgcgtggca
acaagtgact
181 gagacctaga aatccaagcg ttggaggtcc tgaggccagc ctaagtcgct
tcaaaatgga
241 acgaaggcgt ttgtggggtt ccattcagag ccgatacatc agcatgagtg
tgtggacaag
301 cccacggaga cttgtggagc tggcagggca gagcctgctg aaggatgagg
ccctggccat
361 tgccgccctg gagttgctgc ccagggagct cttcccgcca ctcttcatgg
cagcctttga

421 cgggagacac agccagaccc tgaaggcaat ggtgcaggcc tggcccttca
cctgcctccc
481 tctgggagtg ctgatgaagg gacaacatct tcacctggag accttcaaag
ctgtgcttga
541 tggacttgat gtgctccttg cccaggaggt tcgccccagg aggtggaaac
ttcaagtgct
601 ggatttacgg aagaactctc atcaggactt ctggactgta tggtctggaa
acagggccag
661 tctgtactca tttccagagc cagaagcagc tcagcccatg acaaagaagc
gaaaagtaga
721 tggtttgagc acagaggcag agcagccctt cattccagta gaggtgctcg
tagacctgtt
781 cctcaaggaa ggtgcctgtg atgaattgtt ctcctacctc attgagaaag
tgaagcgaaa
841 gaaaaatgta ctacgcctgt gctgtaagaa gctgaagatt tttgcaatgc
ccatgcagga
901 tatcaagatg atcctgaaaa tggtgcagct ggactctatt gaagatttgg
aagtgacttg
961 tacctggaag ctacccacct tggcgaaatt ttctccttac ctgggccaga
tgattaatct
1021 gcgtagactc ctcctctccc acatccatgc atcttcctac atttccccgg
agaaggaaga
1081 gcagtatatc gcccagttca cctctcagtt cctcagtctg cagtgcctgc
aggctctcta
1141 tgtggactct ttatttttcc ttagaggccg cctggatcag ttgctcaggc
acgtgatgaa
1201 ccccttggaa accctctcaa taactaactg ccggctttcg gaaggggatg
tgatgcatct
1261 gtcccagagt cccagcgtca gtcagctaag tgtcctgagt ctaagtgggg
tcatgctgac
1321 cgatgtaagt cccgagcccc tccaagctct gctggagaga gcctctgcca
ccctccagga
1381 cctggtcttt gatgagtgtg ggatcacgga tgatcagctc cttgccctcc
tgccttccct
1441 gagccactgc tcccagctta caaccttaag cttctacggg aattccatct
ccatatctgc
1501 cttgcagagt ctcctgcagc acctcatcgg gctgagcaat ctgacccacg
tgctgtatcc
1561 tgtccccctg gagagttatg aggacatcca tggtaccctc cacctggaga
ggcttgccta
1621 tctgcatgcc aggctcaggg agttgctgtg tgagttgggg cggcccagca
tggtctggct
1681 tagtgccaac ccctgtcctc actgtgggga cagaaccttc tatgacccgg
agcccatcct
1741 gtgcccctgt ttcatgccta actagctggg tgcacatatc aaatgcttca
ttctgcatac
1801 ttggacacta aagccaggat gtgcatgcat cttgaagcaa caaagcagcc
acagtttcag
1861 acaaatgttc agtgtgagtg aggaaaacat gttcagtgag gaaaaaacat
tcagacaaat
1921 gttcagtgag gaaaaaaagg ggaagttggg gataggcaga tgttgacttg
aggagttaat

1981 gtgatctttg gggagataca tcttatagag ttagaaatag aatctgaatt
tctaaaggga
2041 gattctggct tgggaagtac atgtaggagt taatccctgt gtagactgtt
gtaaagaaac
2101 tgttgaaaat aaagagaagc aatgtgaagc aaaaaaaaaa aaaaaaa

ED-B domain of Fibronectin Human fibronectin gene ED-B region.
ACCESSION X07717
VERSION X07717.1 GI:31406

### SEQ ID NO 590

/translation="CTFDNLSPGLEYNVSVYTVKDDKESVPISDTIIPEVPQLTDLSF

VDITDSSIGLRWTPLNSSTIIGYRITVVAAGEGIPIFEDFVDSSVGYYTVTGLEPGID

YDISVITLINGGESAPTTLTQQTAVPPPTDLRFTNIGPDTMRVTW"

### SEQ ID NO 591

### ORIGIN

1 ctgcactttt gataacctga gtcccggcct ggagtacaat gtcagtgttc
acactgtcaa
61 ggatgacaag gaaagtgtcc ctatctctga taccatcatc ccaggtaata
gaaaataagc
121 tgctatcctg agagtgacat tccaataaga gtggggatta gcatcttaat
ccccagatgc
181 ttaagggtgt caactatatt tgggatttaa ttccgatctc ccagctgcac
tttccaaaac
241 caagaagtca aagcagcgat ttggacaaaa tgcttgctgt taacactgct
ttactgtctg
301 tgcttcactg ggatgctgtg tgttgcagcg agtatgtaat ggagtggcag
ccatggcttt
361 aactctgtat tgtctgctca catggaagta tgactaaaac actgtcacgt
gtctgtactc
421 agtactgata ggctcaaagt aatatggtaa atgcatccca tcagtacatt
tctgcccgat

481 tttacaatcc atatcaattt ccaacagctg cctatttcat cttgcagttt
caaatccttc
541 tttttgaaaa ttggatttta aaaaaaagtt aagtaaaagt cacaccttca
gggttgttct
601 ttcttgtggc cttgaaagac aacattgcaa aggcctgtcc taaggatagg
cttgtttgtc
661 cattgggtta taacataatg aaagcattgg acagatcgtg tccccctttg
gactcttcag
721 tagaatgctt ttactaacgc taattacatg ttttgattat gaatgaacct
aaaatagtgg
781 caatggcctt aacctaggcc tgtctttcct cagcctgaat gtgcttttga
atggcacatt
841 tcacaccata cattcataat gcattagcgt tatggccatg atgttgtcat
gagttttgta
901 tgggagaaaa aaaatcaatt tatcacccat ttattatttt ttccggttgt
tcatgcaagc
961 ttattttcta ctaaaacagt tttggaatta ttaaaagcat tgctgatact
tacttcagat
1021 attatgtcta ggctctaaga atggtttcga catcctaaac agccatatga
tttttaggaa
1081 tctgaacagt tcaaattgta ccctttaagg atgttttcaa aatgtaaaaa
atatatatat
1141 atatatatat tccctaaaag aatattcctg tttattcttc tagggaagca
aactgttcat
1201 gatgcttagg aagtcttttc agagaattta aaacagattg catattacca
tcattgcttt
1261 aacattccac caattttact actagtaacc tgatatacac tgctttattt
tttcctcttt
1321 ttttccctct attttccttt tgcctccccc tccctttgct ttgtaactca
atagaggtgc
1381 cccaactcac tgacctaagc tttgttgata taaccgattc aagcatcggc
ctgaggtgga
1441 ccccgctaaa ctcttccacc attattgggt accgcatcac agtagttgcg
gcaggagaag

1501 gtatccctat ttttgaagat tttgtggact cctcagtagg atactacaca
gtcacagggc
1561 tggagccggg cattgactat gatatcagcg ttatcactct cattaatggc
ggcgagagtg
1621 cccctactac actgacacaa caaacgggtg aattttgaaa acttctgcgt
ttgagacata
1681 gatggtgttg catgctgcca ccagttactc cggttaaata tggatgtttc
atgggggaag
1741 tcagcaattg gccaaagatt cagataggtg gaattggggg gataaggaat
caaatgcatc
1801 tgctaaactg attggagaaa aacacatgca atatcttcag tacactctca
tttaaaccac
1861 aagtagatat aaagcctaga gaaatacaga tgtctgctct gttaaatata
aaatagcaaa
1921 tgttcattca atttgaagac ctagaatttt tcttcttaaa taccaaacac
gaataccaaa
1981 ttgcgtaagt accaattgat aagaatatat caccaaaatg taccatcatg
ctcttccttc
2041 taccctttga taaactctac catgctcctt ctttgtagct aaaaacccat
caaaatttag
2101 ggtagagtgg atgggcattg ttttgaggta ggagaaaagt aaacttggga
ccattctagg
2161 ttttgttgct gtcactaggt aaagaaacac ctctttaacc acagtctggg
gacaagcatg
2221 caacatttta aaggttctct gctgtgcatg ggaaaagaaa catgctgaga
accaatttgc
2281 atgaacatgt tcacttgtaa gtagaattca ctgaatggaa ctgtagctct
agatatctca
2341 catgggggga agtttaggac cctcttgtct ttttgtctgt gtgcatgtat
ttctttgtaa
2401 agtactgcta tgtttctctt tgctgtgtgg caacttaagc ctcttcggcc
tgggataaaa
2461 taatctgcag tggtattaat aatgtacata aagtcaacat atttgaaagt
agattaaaat

2521 cttttttaaa tatatcaatg atggcaaaaa ggttaaaggg ggcctaacag
tactgtgtgt
2581 agtgttttat ttttaacagt agtacactat aacttaaaat agacttagat
tagactgttt
2641 gcatgattat gattctgttt cctttatgca tgaaatattg attttacctt
tccagctact
2701 tcgttagctt taattttaaa atacattaac tgagtcttcc ttcttgttcg
aaaccagctg
2761 ttcctcctcc cactgacctg cgattcacca acattggtcc agacaccatg
cgtgtcacct
2821 ggg
//

CEA Homo sapiens carcinoembryonic antigen-related cell adhesion molecule 5 (CEACAM5), mRNA.
ACCESSION NM_004363
VERSION NM_004363.1 GI:11386170

### SEQ ID NO 592

/translation="MESPSAPPHRWCIPWQRLLLTASLLTFWNPPTTAKLTIESTPFN
VAEGKEVLLLVHNLPQHLFGYSWYKGERVDGNRQIIGYVIGTQQATPGPAYSGREIIY
PNASLLIQNIIQNDTGFYTLHVIKSDLVNEEATGQFRVYPELPKPSISSNNSKPVEDK
DAVAFTCEPETQDATYLWWVNNQSLPVSPRLQLSNGNRTLTLFNVTRNDTASYKCETQ
NPVSARRSDSVILNVLYGPDAPTISPLNTSYRSGENLNLSCHAASNPPAQYSWFVNGT
FQQSTQELFIPNITVNNSGSYTCQAHNSDTGLNRTTVTTITVYAEPPKPFITSNNSNP
VEDEDAVALTCEPEIQNTTYLWWVNNQSLPVSPRLQLSNDNRTLTLLSVTRNDVGPYE
CGIQNELSVDHSDPVILNVLYGPDDPTISPSYTYYRPGVNLSLSCHAASNPPAQYSWL
IDGNIQQHTQELFISNITEKNSGLYTCQANNSASGHSRTTVKTITVSAELPKPSISSN
NSKPVEDKDAVAFTCEPEAQNTTYLWWVNGQSLPVSPRLQLSNGNRTLTLFNVTRNDA
RAYVCGIQNSVSANRSDPVTLDVLYGPDTPIISPPDSSYLSGANLNLSCHSASNPSPQ
YSWRINGIPQQHTQVLFIAKITPNNNGTYACFVSNLATGRNNSIVKSITVSASGTSPG
LSAGATVGIMIGVLVGVALI"

### SEQ ID NO 593

### ORIGIN

1 ctcagggcag agggaggaag gacagcagac cagacagtca cagcagcctt
gacaaaacgt
61 tcctggaact caagctcttc tccacagagg aggacagagc agacagcaga
gaccatggag
121 tctccctcgg cccctcccca cagatggtgc atcccctggc agaggctcct
gctcacagcc
181 tcacttctaa ccttctggaa cccgcccacc actgccaagc tcactattga
atccacgccg
241 ttcaatgtcg cagaggggaa ggaggtgctt ctacttgtcc acaatctgcc
ccagcatctt
301 tttggctaca gctggtacaa aggtgaaaga gtggatggca accgtcaaat
tataggatat
361 gtaataggaa ctcaacaagc taccccaggg cccgcataca gtggtcgaga
gataatatac
421 cccaatgcat ccctgctgat ccagaacatc atccagaatg acacaggatt
ctacacccta
481 cacgtcataa agtcagatct tgtgaatgaa gaagcaactg gccagttccg
ggtataccog
541 gagctgccca agccctccat ctccagcaac aactccaaac ccgtggagga
caaggatgct
601 gtggccttca cctgtgaacc tgagactcag gacgcaacct acctgtggtg
ggtaaacaat
661 cagagcctcc cggtcagtcc caggctgcag ctgtccaatg gcaacaggac
cctcactcta
721 ttcaatgtca caagaaatga cacagcaagc tacaaatgtg aaacccagaa
cccagtgagt
781 gccaggcgca gtgattcagt catcctgaat gtcctctatg gcccggatgc
ccccaccatt
841 tcccctctaa acacatctta cagatcaggg gaaaatctga acctctcctg
ccacgcagcc
901 tctaacccac ctgcacagta ctcttggttt gtcaatggga ctttccagca
atccacccaa
961 gagctcttta tccccaacat cactgtgaat aatagtggat cctatacgtg
ccaagcccat

1021 aactcagaca ctggcctcaa taggaccaca gtcacgacga tcacagtcta
tgcagagcca
1081 cccaaaccct tcatcaccag caacaactcc aaccccgtgg aggatgagga
tgctgtagcc
1141 ttaacctgtg aacctgagat tcagaacaca acctacctgt ggtgggtaaa
taatcagagc
1201 ctcccggtca gtcccaggct gcagctgtcc aatgacaaca ggaccctcac
tctactcagt
1261 gtcacaagga atgatgtagg accctatgag tgtggaatcc agaacgaatt
aagtgttgac
1321 cacagcgacc cagtcatcct gaatgtcctc tatggcccag acgaccccac
catttccccc
1381 tcatacacct attaccgtcc aggggtgaac ctcagcctct cctgccatgc
agcctctaac
1441 ccacctgcac agtattcttg gctgattgat gggaacatcc agcaacacac
acaagagctc
1501 tttatctcca acatcactga gaagaacagc ggactctata cctgccaggc
caataactca
1561 gccagtggcc acagcaggac tacagtcaag acaatcacag tctctgcgga
gctgcccaag
1621 ccctccatct ccagcaacaa ctccaaaccc gtggaggaca aggatgctgt
ggccttcacc
1681 tgtgaacctg aggctcagaa cacaacctac ctgtggtggg taaatggtca
gagcctccca
1741 gtcagtccca ggctgcagct gtccaatggc aacaggaccc tcactctatt
caatgtcaca
1801 agaaatgacg caagagccta tgtatgtgga atccagaact cagtgagtgc
aaaccgcagt
1861 gacccagtca ccctggatgt cctctatggg ccggacaccc ccatcatttc
ccccccagac
1921 tcgtcttacc tttcgggagc gaacctcaac ctctcctgcc actcggcctc
taacccatcc
1981 ccgcagtatt cttggcgtat caatgggata ccgcagcaac acacacaagt
tctctttatc

2041 gccaaaatca cgccaaataa taacgggacc tatgcctgtt ttgtctctaa
cttggctact
2101 ggccgcaata attccatagt caagagcatc acagtctctg catctggaac
ttctcctggt
2161 ctctcagctg gggccactgt cggcatcatg attggagtgc tggttggggt
tgctctgata
2221 tagcagccct ggtgtagttt cttcatttca ggaagactga cagttgtttt
gcttcttcct
2281 taaagcattt gcaacagcta cagtctaaaa ttgcttcttt accaaggata
tttacagaaa
2341 agactctgac cagagatcga gaccatccta gccaacatcg tgaaacccca
tctctactaa
2401 aaatacaaaa atgagctggg cttggtggcg cgcacctgta gtcccagtta
ctcgggaggc
2461 tgaggcagga gaatcgcttg aacccgggag gtggagattg cagtgagccc
agatcgcacc
2521 actgcactcc agtctggcaa cagagcaaga ctccatctca aaaagaaaag
aaaagaagac
2581 tctgacctgt actcttgaat acaagtttct gataccactg cactgtctga
gaatttccaa
2641 aactttaatg aactaactga cagcttcatg aaactgtcca ccaagatcaa
gcagagaaaa
2701 taattaattt catgggacta aatgaactaa tgaggattgc tgattcttta
aatgtcttgt
2761 ttcccagatt tcaggaaact ttttttcttt taagctatcc actcttacag
caatttgata
2821 aaatatactt ttgtgaacaa aaattgagac atttacattt tctccctatg
tggtcgctcc
2881 agacttggga aactattcat gaatatttat attgtatggt aatatagtta
ttgcacaagt
2941 tcaataaaaa tctgctcttt gtataacaga aaaa
//

Her2/Neu Human tyrosine kinase-type receptor (HER2) mRNA, complete cds.
ACCESSION M11730
VERSION M11730.1 GI:183986

### SEQ ID NO 594

/translation="MELAALCRWGLLLALLPPGAASTQVCTGTDMKLRLPASPETHLD
MLRHLYQGCQVVQGNLELTYLPTNASLSFLQDIQEVQGYVLIAHNQVRQVPLQRLRIV
RGTQLFEDNYALAVLDNGDPLNNTTPVTGASPGGLRELQLRSLTEILKGGVLIQRNPQ
LCYQDTILWKDIFHKNNQLALTLIDTNRSRACHPCSPMCKGSRCWGESSEDCQSLTRT
VCAGGCARCKGPLPTDCCHEQCAAGCTGPKHSDCLACLHFNHSGICELHCPALVTYNT
DTFESMPNPEGRYTFGASCVTACPYNYLSTDVGSCTLVCPLHNQEVTAEDGTQRCEKC
SKPCARVCYGLGMEHLREVRAVTSANIQEFAGCKKIFGSLAFLPESFDGDPASNTAPL
QPEQLQVFETLEEITGYLYISAWPDSLPDLSVFQNLQVIRGRILHNGAYSLTLQGLGI
SWLGLRSLRELGSGLALIHHNTHLCFVHTVPWDQLFRNPHQALLHTANRPEDECVGEG
LACHQLCARGHCWGPGPTQCVNCSQFLRGQECVEECRVLQGLPREYVNARHCLPCHPE
CQPQNGSVTCFGPEADQCVACAHYKDPPFCVARCPSGVKPDLSYMPIWKFPDEEGACQ
PCPINCTHSCVDLDDKGCPAEQRASPLTSIVSAVVGILLVVVLGVVFGILIKRRQQKI
RKYTMRRLLQETELVEPLTPSGAMPNQAQMRILKETELRKVKVLGSGAFGTVYKGIWI
PDGENVKIPVAIKVLRENTSPKANKEILDEAYVMAGVGSPYVSRLLGICLTSTVQLVT
QLMPYGCLLDHVRENRGRLGSQDLLNWCMQIAKGMSYLEDVRLVHRDLAARNVLVKSP
NHVKITDFGLARLLDIDETEYHADGGKVPIKWMALESILRRRFTHQSDVWSYGVTVWE
LMTFGAKPYDGIPAREIPDLLEKGERLPQPPICTIDVYMIMVKCWMIDSECRPRFREL
VSEFSRMARDPQRFVVIQNEDLGPASPLDSTFYRSLLEDDDMGDLVDAEEYLVPQQGF
FCPDPAPGAGGMVHHRHRSSSTRSGGGDLTLGLEPSEEEAPRSPLAPSEGAGSDVFDG
DLGMGAAKGLQSLPTHDPSPLQRYSEDPTVPLPSETDGYVAPLTCSPQPEYVNQPDVR
PQPPSPREGPLPAARPAGATLERAKTLSPGKNGVVKDVFAFGGAVENPEYLTPQGGAA
PQPHPPPAFSPAFDNLYYWDQDPPERGAPPSTFKGTPTAENPEYLGLDVPV"

### SEQ ID NO 595

ORIGIN Chromosome 17q21-q22.

1 aattctcgag ctcgtcgacc ggtcgacgag ctcgagggtc gacgagctcg
agggcgcgcg
61 cccggccccc acccctcgca gcaccccgcg ccccgcgccc tcccagccgg
gtccagccgg
121 agccatgggg ccggagccgc agtgagcacc atggagctgg cggccttgtg
ccgctggggg

181 ctcctcctcg ccctcttgcc ccccggagcc gcgagcaccc aagtgtgcac
cggcacagac
241 atgaagctgc ggctccctgc cagtcccgag acccacctgg acatgctccg
ccacctctac
301 cagggctgcc aggtggtgca gggaaacctg gaactcacct acctgcccac
caatgccagc
361 ctgtccttcc tgcaggatat ccaggaggtg cagggctacg tgctcatcgc
tcacaaccaa
421 gtgaggcagg tcccactgca gaggctgcgg attgtgcgag gcacccagct
ctttgaggac
481 aactatgccc tggccgtgct agacaatgga gacccgctga acaataccac
ccctgtcaca
541 ggggcctccc caggaggcct gcgggagctg cagcttcgaa gcctcacaga
gatcttgaaa
601 ggaggggtct tgatccagcg gaacccccag ctctgctacc aggacacgat
tttgtggaag
661 gacatcttcc acaagaacaa ccagctggct ctcacactga tagacaccaa
ccgctctcgg
721 gcctgccacc cctgttctcc gatgtgtaag ggctcccgct gctggggaga
gagttctgag
781 gattgtcaga gcctgacgcg cactgtctgt gccggtggct gtgcccgctg
caaggggcca
841 ctgcccactg actgctgcca tgagcagtgt gctgccggct gcacgggccc
caagcactct
901 gactgcctgg cctgcctcca cttcaaccac agtggcatct gtgagctgca
ctgcccagcc
961 ctggtcacct acaacacaga cacgtttgag tccatgccca atcccgaggg
ccggtataca
1021 ttcggcgcca gctgtgtgac tgcctgtccc tacaactacc tttctacgga
cgtgggatcc
1081 tgcaccctcg tctgccccct gcacaaccaa gaggtgacag cagaggatgg
aacacagcgg
1141 tgtgagaagt gcagcaagcc ctgtgcccga gtgtgctatg gtctgggcat
ggagcacttg

1201 cgagaggtga gggcagttac cagtgccaat atccaggagt ttgctggctg
caagaagatc
1261 tttgggagcc tggcatttct gccggagagc tttgatgggg acccagcctc
caacactgcc
1321 ccgctccagc cagagcagct ccaagtgttt gagactctgg aagagatcac
aggttaccta
1381 tacatctcag catggccgga cagcctgcct gacctcagcg tcttccagaa
cctgcaagta
1441 atccggggac gaattctgca caatggcgcc tactcgctga ccctgcaagg
gctgggcatc
1501 agctggctgg ggctgcgctc actgagggaa ctgggcagtg gactggccct
catccaccat
1561 aacacccacc tctgcttcgt gcacacggtg ccctgggacc agctctttcg
gaacccgcac
1621 caagctctgc tccacactgc caaccggcca gaggacgagt gtgtgggcga
gggcctggcc
1681 tgccaccagc tgtgcgcccg agggcactgc tggggtccag ggcccaccca
gtgtgtcaac
1741 tgcagccagt tccttcgggg ccaggagtgc gtggaggaat gccgagtact
gcaggggctc
1801 cccagggagt atgtgaatgc caggcactgt ttgccgtgcc accctgagtg
tcagccccag
1861 aatggctcag tgacctgttt tggaccggag gctgaccagt gtgtggcctg
tgcccactat
1921 aaggaccctc ccttctgcgt ggcccgctgc cccagcggtg tgaaacctga
cctctcctac
1981 atgcccatct ggaagtttcc agatgaggag ggcgcatgcc agccttgccc
catcaactgc
2041 acccactcct gtgtggacct ggatgacaag ggctgccccg ccgagcagag
agccagccct
2101 ctgacgtcca tcgtctctgc ggtggttggc attctgctgg tcgtggtctt
gggggtggtc
2161 tttgggatcc tcatcaagcg acggcagcag aagatccgga agtacacgat
gcggagactg

2221 ctgcaggaaa cggagctggt ggagccgctg acacctagcg gagcgatgcc
caaccaggcg
2281 cagatgcgga tcctgaaaga gacggagctg aggaaggtga aggtgcttgg
atctggcgct
2341 tttggcacag tctacaaggg catctggatc cctgatgggg agaatgtgaa
aattccagtg
2401 gccatcaaag tgttgaggga aaacacatcc cccaaagcca acaaagaaat
cttagacgaa
2461 gcatacgtga tggctggtgt gggctcccca tatgtctccc gccttctggg
catctgcctg
2521 acatccacgg tgcagctggt gacacagctt atgccctatg gctgcctctt
agaccatgtc
2581 cgggaaaacc gcggacgcct gggctcccag gacctgctga actggtgtat
gcagattgcc
2641 aaggggatga gctacctgga ggatgtgcgg ctcgtacaca gggacttggc
cgctcggaac
2701 gtgctggtca agagtcccaa ccatgtcaaa attacagact tcgggctggc
tcggctgctg
2761 gacattgacg agacagagta ccatgcagat gggggcaagg tgcccatcaa
gtggatggcg
2821 ctggagtcca ttctccgccg gcggttcacc caccagagtg atgtgtggag
ttatggtgtg
2881 actgtgtggg agctgatgac ttttggggcc aaaccttacg atgggatccc
agcccgggag
2941 atccctgacc tgctggaaaa gggggagcgg ctgccccagc cccccatctg
caccattgat
3001 gtctacatga tcatggtcaa atgttggatg attgactctg aatgtcggcc
aagattccgg
3061 gagttggtgt ctgaattctc ccgcatggcc agggaccccc agcgctttgt
ggtcatccag
3121 aatgaggact tgggcccagc cagtcccttg gacagcacct tctaccgctc
actgctggag
3181 gacgatgaca tgggggacct ggtggatgct gaggagtatc tggtacccca
gcagggcttc

3241 ttctgtccag accctgcccc gggcgctggg ggcatggtcc accacaggca
ccgcagctca
3301 tctaccagga gtggcggtgg ggacctgaca ctagggctgg agccctctga
agaggaggcc
3361 cccaggtctc cactggcacc ctccgaaggg gctggctccg atgtatttga
tggtgacctg
3421 ggaatggggg cagccaaggg gctgcaaagc ctccccacac atgaccccag
ccctctacag
3481 cggtacagtg aggaccccac agtacccctg ccctctgaga ctgatggcta
cgttgccccc
3541 ctgacctgca gcccccagcc tgaatatgtg aaccagccag atgttcggcc
ccagcccccct
3601 tcgccccgag agggccctct gcctgctgcc cgacctgctg gtgccactct
ggaaagggcc
3661 aagactctct ccccagggaa gaatggggtc gtcaaagacg tttttgcctt
tgggggtgcc
3721 gtggagaacc ccgagtactt gacaccccag ggaggagctg cccctcagcc
ccaccctcct
3781 cctgccttca gcccagcctt cgacaacctc tattactggg accaggaccc
accagagcgg
3841 ggggctccac ccagcacctt caaagggaca cctacggcag agaacccaga
gtacctgggt
3901 ctggacgtgc cagtgtgaac cagaaggcca agtccgcaga agccctgatg
tgtcctcagg
3961 gagcagggaa ggcctgactt ctgctggcat caagaggtgg gagggccctc
cgaccacttc
4021 caggggaacc tgccatgcca ggaacctgtc ctaaggaacc ttccttcctg
cttgagttcc
4081 cagatggctg gaaggggtcc agcctcgttg gaagaggaac agcactgggg
agtctttgtg
4141 gattctgagg ccctgcccaa tgagactcta gggtccagtg gatgccacag
cccagcttgg
4201 ccctttcctt ccagatcctg ggtactgaaa gccttaggga agctggcctg
agaggggaag

4261 cggccctaag ggagtgtcta agaacaaaag cgacccattc agagactgtc
cctgaaacct
4321 agtactgccc cccatgagga aggaacagca atggtgtcag tatccaggct
ttgtacagag
4381 tgcttttctg tttagttttt actttttttg ttttgttttt ttaaagacga
aataaagacc
4441 caggggagaa tgggtgttgt atggggaggc aagtgtgggg ggtccttctc
cacacccact
4501 ttgtccattt gcaaatatat tttggaaaac
//

H. sapiens mRNA for SCP1 protein.
ACCESSION X95654
VERSION X95654.1 GI:1212982

### SEQ ID NO 596

/translation="
















"

### SEQ ID NO 597

### ORIGIN

1 gccctcatag accgtttgtt gtagttcgcg tgggaacagc aacccacggt
ttcccgatag
61 ttcttcaaag atatttacaa ccgtaacaga gaaaatggaa aagcaaaagc
cctttgcatt
121 gttcgtacca ccgagatcaa gcagcagtca ggtgtctgcg gtgaaacctc
agaccctggg
181 aggcgattcc actttcttca agagtttcaa caaatgtact gaagatgatt
tggagtttcc
241 atttgcaaag actaatctct ccaaaaatgg ggaaaacatt gattcagatc
ctgctttaca
301 aaaagttaat ttcttgcccg tgcttgagca ggttggtaat tctgactgtc
actatcagga
361 aggactaaaa gactctgatt tggagaattc agagggattg agcagagtgt
tttcaaaact
421 gtataaggag gctgaaaaga taaaaaaatg gaaagtaagt acagaagctg
aactgagaca
481 gaaagaaagt aagttgcaag aaaacagaaa gataattgaa gcacagcgaa
aagccattca
541 ggaactgcaa tttggaaatg aaaaagtaag tttgaaatta gaagaaggaa
tacaagaaaa
601 taaagattta ataaaagaga ataatgccac aaggcattta tgtaatctac
tdaaagaaac
661 ctgtgctaga tctgcagaaa agacaaagaa atatgaatat gaacgggaag
aaaccaggca
721 agtttatatg gatctaaata ataacattga gaaaatgata acagctcatg
gggaacttcg
781 tgtgcaagct gagaattcca gactggaaat gcattttaag ttaaaggaag
attatgaaaa
841 aatccaacac cttgaacaag aatacaagaa ggaaataaat gacaaggaaa
agcaggtatc
901 actactattg atccaaatca ctgagaaaga aaataaaatg aaagatttaa
catttctgct

961 agaggaatcc agagataaag ttaatcaatt agaggaaaag acaaaattac
agagtgaaaa
1021 cttaaaacaa tcaattgaga aacagcatca tttgactaaa gaactagaag
atattaaagt
1081 gtcattacaa agaagtgtga gtactcaaaa ggctttagag gaagatttac
agatagcaac
1141 aaaaacaatt tgtcagctaa ctgaagaaaa agaaactcaa atggaagaat
ctaataaagc
1201 tagagctgct cattcgtttg tggttactga atttgaaact actgtctgca
gcttggaaga
1261 attattgaga acagaacagc aaagattgga aaaaaatgaa gatcaattga
aaatacttac
1321 catggagctt caaaagaaat caagtgagct ggaagagatg actaagctta
caaataacaa
1381 agaagtagaa cttgaagaat tgaaaaaagt cttgggagaa aaggaaacac
ttttatatga
1441 aaataaacaa tttgagaaga ttgctgaaga attaaaagga acagaacaag
aactaattgg
1501 tcttctccaa gccagagaga aagaagtaca tgatttggaa atacagttaa
ctgccattac
1561 cacaagtgaa cagtattatt caaaagaggt taaagatcta aaaactgagc
ttgaaaacga
1621 gaagcttaag aatactgaat taacttcaca ctgcaacaag ctttcactag
aaaacaaaga
1681 gctcacacag gaaacaagtg atatgaccct agaactcaag aatcagcaag
aagatattaa
1741 taataacaaa aagcaagaag aaaggatgtt gaaacaaata gaaaatcttc
aagaaacaga
1801 aacccaatta agaaatgaac tagaatatgt gagagaagag ctaaaacaga
aaagagatga
1861 agttaaatgt aaattggaca agagtgaaga aaattgtaac aatttaagga
aacaagttga
1921 aaataaaaac aagtatattg aagaacttca gcaggagaat aaggccttga
aaaaaaaagg

1981 tacagcagaa agcaagcaac tgaatgttta tgagataaag gtcaataaat
tagagttaga
2041 actagaaagt gccaaacaga aatttggaga aatcacagac acctatcaga
aagaaattga
2101 ggacaaaaag atatcagaag aaaatctttt ggaagaggtt gagaaagcaa
aagtaatagc
2161 tgatgaagca gtaaaattac agaaagaaat tgataagcga tgtcaacata
aaatagctga
2221 aatggtagca cttatggaaa aacataagca ccaatatgat aagatcattg
aagaaagaga
2281 ctcagaatta ggactttata agagcaaaga acaagaacag tcatcactga
gagcatcttt
2341 ggagattgaa ctatccaatc tcaaagctga acttttgtct gttaagaagc
aacttgaaat
2401 agaaagagaa gagaaggaaa aactcaaaag agaggcaaaa gaaaacacag
ctactcttaa
2461 agaaaaaaaa gacaagaaaa cacaaacatt tttattggaa acacctgaaa
tttattggaa
2521 attggattct aaagcagttc cttcacaaac tgtatctcga aatttcacat
cagttgatca
2581 tggcatatcc aaagataaaa gagactatct gtggacatct gccaaaaata
ctttatctac
2641 accattgcca aaggcatata cagtgaagac accaacaaaa ccaaaactac
agcaaagaga
2701 aaacttgaat atacccattg aagaaagtaa aaaaaagaga aaaatggcct
ttgaatttga
2761 tattaattca gatagttcag aaactactga tcttttgagc atggtttcag
aagaagagac
2821 attgaaaaca ctgtatagga acaataatcc accagcttct catctttgtg
tcaaaacacc
2881 aaaaaaggcc ccttcatctc taacaacccc tggacctaca ctgaagtttg
gagctataag
2941 aaaaatgcgg gaggaccgtt gggctgtaat tgctaaaatg gatagaaaaa
aaaaactaaa

3001 agaagctgaa aagttatttg tttaatttca gagaatcagt gtagttaagg
agcctaataa
3061 cgtgaaactt atagttaata ttttgttctt atttgccaga gccacatttt
atctggaagt
3121 tgagacttaa aaaatacttg catgaatgat ttgtgtttct ttatattttt
agcctaaatg
3181 ttaactacat attgtctgga aacctgtcat tgtattcaga taattagatg
attatatatt
3241 gttgttactt tttcttgtat tcatgaaaac tgtttttact aagttttcaa
atttgtaaag
3301 ttagcctttg aatgctagga atgcattatt gagggtcatt ctttattctt
tactattaaa
3361 atattttgga tgcaaaaaaa aaaaaaaaaa aaa
//

Homo sapiens synovial sarcoma, X breakpoint 4 (SSX4), mRNA.
ACCESSION NM_005636
VERSION NM_005636.1 GI:5032122

### SEQ ID NO 598

/translation="MNGDDAFARRPRDDAQISEKLRKAFDDIAKYFSKKEWEKMKSSEKIVY

VYMKLNYEVMTKLGFKVTLPPFMRSKRAADFHGNDFGNDRNHRNQVERPQMTFG

SLQRIFPKIMPKKPAEEENGLKEVPEASGPQNDGKQLCPPGNPSTLEKINKTSGPKRG

KHAWTHRLRERKQLVVYEEISDPEEDDE"

### SEQ ID NO 599

### ORIGIN

1 atgaacggag acgacgcctt tgcaaggaga cccagggatg atgctcaaat
atcagagaag
61 ttacgaaagg ccttcgatga tattgccaaa tacttctcta agaaagagtg
ggaaaagatg
121 aaatcctcgg agaaaatcgt ctatgtgtat atgaagctaa actatgaggt
catgactaaa

181 ctaggtttca aggtcaccct cccacctttc atgcgtagta aacgggctgc
agacttccac
241 gggaatgatt ttggtaacga tcgaaaccac aggaatcagg ttgaacgtcc
tcagatgact
301 ttcggcagcc tccagagaat cttcccgaag atcatgccca agaagccagc
agaggaagaa
361 aatggtttga aggaagtgcc agaggcatct ggcccacaaa atgatgggaa
acagctgtgc
421 cccccgggaa atccaagtac cttggagaag attaacaaga catctggacc
caaaaggggg
481 aaacatgcct ggacccacag actgcgtgag agaaagcagc tggtggttta
tgaagagatc
541 agcgaccctg aggaagatga cgagtaactc ccctcg

All patents and publications mentioned in the specification are indicative of the levels of those skilled in the art to which the invention pertains.

The invention illustratively described herein suitably may be practiced in the absence of any element or elements, limitation or limitations which is not specifically disclosed herein. The terms and expressions which have been employed are used as terms of description and not of limitation, and there is no intention that in the use of such terms and expressions indicates the exclusion of equivalents of the features shown and described or portions thereof. It is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.
1. An isolated epitope, comprising a component selected from the group consisting of:
   (i) a polypeptide having the sequence as disclosed in TABLE 1;
   (ii) an epitope cluster comprising the polypeptide of (i);
   (iii) a polypeptide having substantial similarity to (i) or (ii);
   (iv) a polypeptide having functional similarity to any of (i) through (iii); and
   (v) a nucleic acid encoding the polypeptide of any of (i) through (iv).
2. The epitope of claim 1, wherein the epitope is immunologically active,
3. The epitope of claim 1, wherein the polypeptide is less than about 30 amino acids in length.
4. The epitope of claim 1, wherein the polypeptide is 8 to 10 amino acids in length.
5. The epitope of claim 1, wherein the substantial or functional similarity comprises addition of at least one amino acid.
6. The epitope of claim 5, wherein the at least one additional amino acid is at an N-terminus of the polypeptide.
7. The epitope of claim 1, wherein the substantial or functional similarity comprises a substitution of at least one amino acid.
8. The epitope of claim 1, the polypeptide having affinity to an HLA-A2 molecule.
9. The epitope of claim 8, wherein the affinity is determined by an assay of binding.
10. The epitope of claim 8, wherein the affinity is determined by an assay of restriction of epitope recognition.
11. The epitope of claim 8, wherein the affinity is determined by a prediction algorithm.
12. The epitope of claim 1, the polypeptide having affinity to an HLA-B7 or HLA-B51 molecule.
13. The epitope of claim 1, wherein the polypeptide is a housekeeping epitope.
14. The epitope of claim 1, wherein the polypeptide corresponds to an epitope displayed on a tumor cell.
15. The epitope of claim 1, wherein the polypeptide corresponds to an epitope displayed on a neovasculature cell.
16. The epitope of claim 1, wherein the polypeptide is an immune epitope.
17. The epitope of claim 1 wherein the epitope is a nucleic acid.
18. A pharmaceutical composition comprising the polypeptide of claim 1 and a pharmaceutically acceptable adjuvant, carrier, diluent, or excipient.
19. The composition of claim 18, where the adjuvant is a polynucleotide.
20. The composition of claim 19 wherein the polynucleotide comprises a dinucleotide.
21. The composition of claim 20 wherein the dinucleotide is CpG.
22. The composition of claim 18, wherein the adjuvant is encoded by a polynucleotide.
23. The composition of claim 18 wherein the adjuvant is a cytokine.
24. The composition of claim 23 wherein the cytokine is GM-CSF.
25. The composition of claim 18 further comprising a professional antigen-presenting cell (pAPC).
26. The composition of claim 25, wherein the pAPC is a dendritic cell.
27. The composition of claim 18, further comprising a second epitope.
28. The composition of claim 27, wherein the second epitope is a polypeptide.
29. The composition of claim 27, wherein the second epitope is a nucleic acid.
30. The composition of claim 27, wherein the second epitope is a housekeeping epitope.
31. The composition of claim 27, wherein the second epitope is an immune epitope.
32. A pharmaceutical composition comprising the nucleic acid of claim 1 and a pharmaceutically acceptable adjuvant, carrier, diluent, or excipient.
33. A recombinant construct comprising the nucleic acid of Claim 1.
34. The construct of claim 33, further comprising a plasmid, a viral vector, or an artificial chromosome.
35. The construct of claim 33, further comprising a sequence encoding at least one feature selected from the group consisting of a second epitope, an IRES, an ISS, an NIS, and ubiquitin.
36. A purified antibody that specifically binds to the epitope of claim 1.
37. A purified antibody that specifically binds to a peptide-MHC protein complex comprising the epitope of claim 1.
38. The antibody of claim 36 or claim 37, wherein the antibody is a monoclonal antibody.
39. A multimeric MHC-peptide complex comprising the epitope of claim 1.
40. An isolated T cell expressing a T cell receptor specific for an MHC-peptide complex, the complex comprising the epitope of claim 1.
41. The T cell of claim 40, produced by an in vitro immunization.
42. The T cell of claim 40, isolated from an immunized animal.
43. A T cell done comprising the T cell of claim 40.
44. A polyclonal population of T cells comprising the T cell of claim 40.
45. A pharmaceutical composition comprising the T cell of claim 40 and a pharmaceutically acceptable adjuvant, carrier, diluent, or excipient.
46. An isolated protein molecule comprising the binding domain of a T cell receptor specific for an MHC-peptide complex, the complex comprising the epitope of claim 1.
47. The protein of claim 46, wherein the protein is multivalent.
48. An isolated nucleic acid encoding the protein of claim 46.
49. A. recombinant construct comprising the nucleic acid of claim 48.
50. A host cell expressing the recombinant construct, the construct comprising the nucleic acid of claim 1, or the construct encoding a protein molecule comprising the binding domain of a T cell receptor specific for an MHC-peptide complex.
51. The host cell of claim 50, wherein the host cell is a dendritic cell, macrophage, tumor cell, or tumor-derived cell,
52. The host cell of claim 50, wherein the host cell is a bacterium, fungus, or protozoan.
53. A pharmaceutical composition comprising the host cell of claim 50 and a pharmaceutically acceptable adjuvant, carrier, diluent, or excipient
54. A vaccine or immunotherapeutic composition comprising at least one component selected from the group consisting of the epitope of claim 1; the composition of claim 18, 32, or 45, the construct of claim 33; the T cell of claim 40, a host cell expressing a recombinant construct comprising a nucleic acid encoding a T cell receptor binding domain specific for an MHC-peptide complex and a composition comprising the same, and a host cell expressing a recombinant construct comprising the nucleic acid of claim 1 and a composition comprising the same.
55. A method of treating an animal, comprising:
   administering to an animal the vaccine or immunotherapeutic composition of claim 54.
56. The method of claim 55, wherein the administering step comprises a mode of delivery selected from the group consisting of transdermal, intranodal, perinodal, oral, intravenous, intradermal, intramuscular, intraperitoneal, mucosal, aerosol inhalation, and instillation.
57. The method of claim 55, further comprising a step of assaying to determine a characteristic indicative of a state of a target cell or target cells.
58. The method of claim 57, comprising a first assaying step and a second assaying step, wherein the first assaying step precedes the administering step, and wherein the second assaying step follows the administering step.
59. The method of claim 58, further comprising a step of comparing the characteristic determined in the first assaying step with the characteristic determined in the second assaying step to obtain a result.
60. The method of claim 59, wherein the result is selected from the group consisting of: evidence of an immune response, a diminution in number of target cells, a loss of mass or size of a tumor comprising target cells, a decrease in number or concentration of an intracellular parasite infecting target cells,
61. A method of evaluating immunogenicity of a vaccine or immunotherapeutic composition, comprising:
   administering to an animal the vaccine or immunotherapeutic composition of claim 54; and
   evaluating immunogenicity based on a characteristic of the animal.
62. The method of claim 61, wherein the animal is HLA-transgenic.
63. A method of evaluating immunogenicity, comprising:
   *in vitro* stimulation of a T cell with the vaccine or immunotherapeutic composition of claim 54; and
   evaluating immunogenicity based on a characteristic of the T cell,
64. The method of claim 63, wherein the stimulation is a primary stimulation.
65. A method of making a passive/adoptive immunotherapeutic, comprising:
   combining the T cell of claim 40, or a host cell expressing a recombinant construct comprising a nucleic acid encoding a T cell receptor binding domain specific for an MHC-peptide complex, or a host cell expressing a recombinant construct comprising the nucleic acid of claim 1 with a phamaceutically acceptable adjuvant, carrier, diluent, or excipient.
66. A method of determining specific T cell frequency comprising the step of contacting T cells with a MHC-peptide complex comprising the epitope of claim 1,
67. The method of claim 66, wherein the contacting step comprises at least one feature selected from the group consisting of immunization, restimulation, detection, and enumeration,
68. The method of Claim 66, further comprising ELISPOT analysis, limiting dilution analysis, flow cytometry, in situ hybridization, the polymerase chain reaction or any combination thereof.
69. A method of evaluating immunologic response, comprising the method of claim 66 carried out prior to and subsequent to an immunization step.
70. A. method of evaluating immunologic response, comprising:
   determining frequency, cytokine production, or cytolytic activity of T cells, prior to and subsequent to a step of stimulation with MHC-peptide complexes comprising the epitope of claim 1.
71. A method of diagnosing a disease comprising:
   contacting a subject tissue with at least one component selected from the group consisting of the T cell of claim 40, the host cell of claim 50, the antibody of claim 36, and the protein of claim 46; and
   diagnosing the disease based on a characteristic of the tissue or of the component.
72. The method of claim 71, wherein the contacting step takes place *in vivo*.
73. The method of claim 71, wherein the contacting step takes place *in vitro*.
74. A method of making a vaccine, comprising:
   combining at least one component selected from the group consisting of the epitope of claim 1; the composition of claim 18; 32, 45, or 53; the construct of claim 33; the T cell of claim 40, and the host cell of claim 50, with a pharmaceutically acceptable adjuvant, carrier, diluent, or excipient,
75. A computer readable medium having recorded thereon the sequence of any one of SEQ ID NOS: 1-602, in a machine having a hardware or software that calculates the physical, biochemical, immunologic, or molecular genetic properties of a molecule embodying said sequence.
76. A method of treating an animal comprising combining the method of claim 55 combined with at least one mode of treatment selected from the group of radiation therapy, chemotherapy, biochemotherapy, and surgery.
77. An isolated polypeptide comprising an epitope cluster from a target-associated antigen having the sequence as disclosed in Tables 25-44, wherein the acid sequence consists of not more than about 80% of the amino acid sequence of the antigen.
78. A vaccine or immunotherapeutic product comprising the polypeptide of claim 78.
79. An isolated polynucleotide encoding the polypeptide of claim 78.
80. A vaccine, or immunotherapeutic product comprising the polynucleotide of claim 80.
81. The polynucleotide of claim 79 or 80, wherein the polynucleotide is DNA.
82. The polynucleotide of claim 79 or 80, wherein the polynucleotide is RNA.

## Claims

1. An isolated polypeptide comprising an epitope cluster from a target-associated antigen, wherein said target associated antigen is PSMA (SEQ ID NO. 4), and wherein said epitope cluster comprises 9-mer epitopes predicted for HLA-A2 binding, and wherein said cluster has a sequence as disclosed in Table 42 selected from the group consisting of amino acids 13-45, amino acids 278-297, amino acids 247-268, amino acids 217-227, amino acids 3-12, amino acids 20-43, amino acids 57-69, amino acids 100-138, amino acids 193-208, amino acids 354-381, amino acids 385-405, amino acids 415-435, amino acids 440-450, amino acids 454-481, amino acids 507-523, amino acids 547-562, amino acids 568-591, amino acids 603-614, amino acids 631-650, amino acids 660-681, amino acids 663-676, amino acids 700-715, amino acids 700-719, amino acids 726-749, and amino acids 731-749 of PSMA (SEQ ID NO. 4), and wherein the amino acid sequence consists of not more than 80% of the amino acid sequence of the antigen.

2. Isolated polypeptide of claim 1, wherein the epitope cluster has a sequence as disclosed in Table 42 selected from the group consisting of amino acids 13-45, amino acids 278-297, amino acids 247-268, amino acids 217-227, and amino acids 3-12 of PSMA (SEQ ID NO. 4).

3. Isolated polypeptide of claim 1, wherein the epitope cluster has a sequence as disclosed in Table 42 of amino acids 13-45 of PSMA (SEQ ID NO. 4).

4. Isolated polypeptide of claim 1, wherein the cluster has a sequence as disclosed in Table 42 of amino acids 278-297 of PSMA (SEQ ID NO. 4).

5. Isolated polypeptide of claim 1, wherein the cluster has a sequence as disclosed in Table 42 of amino acids 247-268 of PSMA (SEQ ID NO. 4).

6. Isolated polypeptide of claim 1, wherein the cluster has a sequence as disclosed in Table 42 of amino acids 217-227 of PSMA (SEQ ID NO. 4).

7. Isolated polypeptide of claim 1, wherein the cluster has a sequence as disclosed in Table 42 of amino acids 3-12 of PSMA (SEQ ID NO. 4).

8. A vaccine or immunotherapeutic product comprising the polypeptide of any one of the preceding claims.

9. An isolated polynucleotide encoding the polypeptide of any one of claims 1-7.

10. A vaccine or immunotherapeutic product comprising the polynucleotide of claim 9.
